(19) Europäisches Patentamt
European Patent Office
Office européen des brevets

(11) EP 1 331 266 B1

(12) EUROPEAN PATENT SPECIFICATION

(45) Date of publication and mention of the grant of the patent:
**04.01.2017 Bulletin 2017/01**

(21) Application number: **01974718.7**

(22) Date of filing: **05.10.2001**

(51) Int Cl.:
*C12N 15/10* (2006.01) *C12P 21/08* (2006.01)
*C07K 16/00* (2006.01) *A01K 67/00* (2006.01)
*A61K 39/395* (2006.01) *C12N 9/00* (2006.01)
*C12N 15/52* (2006.01) *G01N 33/53* (2006.01)

(86) International application number:
**PCT/JP2001/008804**

(87) International publication number:
**WO 2002/031140 (18.04.2002 Gazette 2002/16)**

(54) **CELLS PRODUCING ANTIBODY COMPOSITIONS**

ANTIKÖRPERZUSAMMENSETZUNGEN PRODUZIERENDE ZELLEN

CELLULES PRODUISANT DES COMPOSITIONS D'ANTICORPS

(84) Designated Contracting States:
**AT BE CH CY DE DK ES FI FR GB GR IE IT LI LU MC NL PT SE TR**
Designated Extension States:
**AL LT LV MK RO SI**

(30) Priority: **06.10.2000 JP 2000308526**

(43) Date of publication of application:
**30.07.2003 Bulletin 2003/31**

(60) Divisional application:
**10180013.4 / 2 314 685**
**10180018.3 / 2 314 686**

(73) Proprietor: **Kyowa Hakko Kirin Co., Ltd.**
**Tokyo 100-8185 (JP)**

(72) Inventors:
- **KANDA, Yutaka**
**Machida-shi, Tokyo 194-0023 (JP)**
- **SATOH, Mitsuo**
**Machida-shi, Tokyo 194-0023 (JP)**
- **NAKAMURA, Kazuyasu**
**North Bethesda, MD 20852 (US)**
- **UCHIDA, Kazuhisa**
**Machida-shi, Tokyo 194-0023 (JP)**
- **SHINKAWA, Toyohide**
**Machida-shi, Tokyo 194-0023 (JP)**
- **YAMANE, Naoko**
**Machida-shi, Tokyo 194-0023 (JP)**
- **HOSAKA, Emi**
**Machida-shi, Tokyo 194-0023 (JP)**
- **YAMANO, Kazuya**
**Machida-shi,**
**Tokyo 194-8533 (JP)**
- **YAMASAKI, Motoo**
**Tokyo 100-8185 (JP)**
- **HANAI, Nobuo**
**Tokyo 100-8185 (JP)**

(74) Representative: **Hoffmann Eitle**
**Patent- und Rechtsanwälte PartmbB**
**Arabellastraße 30**
**81925 München (DE)**

(56) References cited:
**EP-A- 1 176 195**
**WO-A1-00/61739**
**WO-A1-97/27303**
**WO-A1-97/37683**
**WO-A1-99/54342**
**WO-A1-99/64618**
**US-A- 5 728 568**

- **RIPKA J ET AL: "Two Chinese hamster ovary glycosylation mutants affected in the conversion of GDP-mannose to GDP-fucose" ARCHIVES OF BIOCHEMISTRY AND BIOPHYSICS, NEW YORK, US, US, vol. 249, no. 2, September 1986 (1986-09), pages 533-545, XP002971006 ISSN: 0003-9861**
- **OHYAMA C ET AL: "Molecular cloning and expression of GDP-D-mannose-4,6-dehydratase, a key enzyme for fucose metabolism defective in Lec13 cells." THE JOURNAL OF BIOLOGICAL CHEMISTRY. 5 JUN 1998, vol. 273, no. 23, 5 June 1998 (1998-06-05), pages 14582-14587, XP002328593 ISSN: 0021-9258**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- WANG Y ET AL: "Purification and characterization of a GDP-fucose:polypeptide fucosyltransferase from Chinese hamster ovary cells." THE JOURNAL OF BIOLOGICAL CHEMISTRY. 3 APR 1998, vol. 273, no. 14, 3 April 1998 (1998-04-03), pages 8112-8118, XP002328594 ISSN: 0021-9258
- UMANA P ET AL: "ENGINEERED GLYCOFORMS OF AN ANTINEUROBLASTOMA IGG1 WITH OPTIMIZED ANTIBODY-DEPENDENT CELLULAR CYTOTOXIC ACTIVITY" NATURE BIOTECHNOLOGY, NATURE PUBLISHING, US, vol. 17, February 1999 (1999-02), pages 176-180, XP002921620 ISSN: 1087-0156
- WRIGHT A ET AL: "Effect of glycosylation on antibody function: implications for genetic engineering" TRENDS IN BIOTECHNOLOGY, ELSEVIER PUBLICATIONS, CAMBRIDGE, GB, vol. 15, no. 1, 1997, pages 26-32, XP004016809 ISSN: 0167-7799
- SHITARA K. ET AL.: 'A new veator for the high level expression of chimeric antibodies in myeloma cells' JOURNAL OF IMMUNOLOGICAL METHODS vol. 167, 1994, pages 271 - 278, XP002911931
- FURUKAWA KIYOSHI: 'Seitai ni okeru totanpakushitsu tousa no kinou kaiseki' TANPAKUSHITAU KAKUSAN KOUSO vol. 43, no. 16, 1998, pages 2309 - 2317, XP002907925

SEQUENCE LISTING

<110> KYOWA HAKKO KOGYO CO., LTD.

<120> ANTIBODY COMPOSITION-PRODUCING CELL

<130> P-38524

<150> JP 2000-308526
<151> 2000-10-06

<160> 73

<170> PatentIn Ver. 2. 1

<210> 1
<211> 2008
<212> DNA
<213> Cricetulus griseus

<400> 1

aacagaaact tattttcctg tgtggctaac tagaaccaga gtacaatgtt tccaattctt 60

tgagctccga gaagacagaa gggagttgaa actctgaaaa tgcgggcatg gactggttcc 120

tggcgttgga ttatgctcat tctttttgcc tgggggacct tattgtttta tataggtggt 180

catttggttc gagataatga ccaccctgac cattctagca gagaactctc caagattctt 240

gcaaagctgg agcgcttaaa acaacaaaat gaagacttga ggagaatggc tgagtctctc 300

cgaataccag aaggccctat tgatcagggg acagctacag gaagagtccg tgttttagaa 360

gaacagcttg ttaaggccaa agaacagatt gaaaattaca agaaacaagc taggaatgat 420

ctgggaaagg atcatgaaat cttaaggagg aggattgaaa atggagctaa agagctctgg 480

tttttctac aaagtgaatt gaagaaatta aagaaattag aaggaaacga actccaaaga 540

catgcagatg aaattctttt ggatttagga catcatgaaa ggtctatcat gacagatcta 600

tactacctca gtcaaacaga tggagcaggt gagtggcggg aaaaagaagc caaagatctg 660

acagagctgg tccagcggag aataacatat ctgcagaatc ccaaggactg cagcaaagcc 720

agaaagctgg tatgtaatat caacaaaggc tgtggctatg gatgtcaact ccatcatgtg 780

gtttactgct tcatgattgc ttatggcacc cagcgaacac tcatcttgga atctcagaat 840

tggcgctatg ctactggagg atgggagact gtgtttagac ctgtaagtga gacatgcaca 900

gacaggtctg gcctctccac tggacactgg tcaggtgaag tgaaggacaa aaatgttcaa 960

gtggtcgagc tccccattgt agacagcctc catcctcgtc ctccttactt acccttggct 1020

gtaccagaag accttgcaga tcgactcctg agagtccatg gtgatcctgc agtgtggtgg 1080

gtatcccagt ttgtcaaata cttgatccgt ccacaacctt ggctggaaag ggaaatagaa 1140

gaaaccacca agaagcttgg cttcaaacat ccagttattg gagtccatgt cagacgcact 1200

gacaaagtgg gaacagaagc agccttccat cccattgagg aatacatggt acacgttgaa 1260

gaacattttc agcttctcga acgcagaatg aaagtggata aaaaaagagt gtatctggcc 1320

actgatgacc cttctttgtt aaaggaggca aagacaaagt actccaatta tgaatttatt 1380

agtgataact ctatttcttg gtcagctgga ctacacaacc gatacacaga aaattcactt 1440

cggggcgtga tcctggatat acactttctc tcccaggctg acttccttgt gtgtactttt 1500

tcatcccagg tctgtagggt tgcttatgaa atcatgcaaa cactgcatcc tgatgcctct 1560

gcaaacttcc attctttaga tgacatctac tattttggag gccaaaatgc ccacaaccag 1620

attgcagttt atcctcacca acctcgaact aaagaggaaa tccccatgga acctggagat 1680

atcattggtg tggctggaaa ccattggaat ggttactcta aaggtgtcaa cagaaaacta 1740

ggaaaaacag gcctgtaccc ttcctacaaa gtccgagaga agatagaaac agtcaaatac 1800

cctacatatc ctgaagctga aaaatagaga tggagtgtaa gagattaaca acagaattta 1860

gttcagacca tctcagccaa gcagaagacc cagactaaca tatggttcat tgacagacat 1920

gctccgcacc aagagcaagt gggaaccctc agatgctgca ctggtggaac gcctctttgt 1980

gaagggctgc tgtgccctca agcccatg 2008

<210> 2
<211> 1728
<212> DNA
<213> Mus musculus

<400> 2

atgcgggcat ggactggttc ctggcgttgg attatgctca ttctttttgc ctgggggacc 60
ttgttatttt atataggtgg tcatttggtt cgagataatg accaccctga tcactccagc 120
agagaactct ccaagattct tgcaaagctt gaacgcttaa aacagcaaaa tgaagacttg 180
aggcgaatgg ctgagtctct ccgaatacca gaaggcccca ttgaccaggg gacagctaca 240
ggaagagtcc gtgttttaga agaacagctt gttaaggcca aagaacagat tgaaaattac 300
aagaaacaag ctagaaatgg tctggggaag gatcatgaaa tcttaagaag gaggattgaa 360
aatggagcta aagagctctg gtttttcta caaagcgaac tgaagaaatt aaagcattta 420
gaaggaaatg aactccaaag acatgcagat gaaattcttt tggatttagg acaccatgaa 480
aggtctatca tgacagatct atactacctc agtcaaacag atggagcagg ggattggcgt 540
gaaaaagagg ccaaagatct gacagagctg gtccagcgga gaataacata tctccagaat 600
cctaaggact gcagcaaagc caggaagctg gtgtgtaaca tcaataaagg ctgtggctat 660
ggttgtcaac tccatcacgt ggtctactgt ttcatgattg cttatggcac ccagcgaaca 720
ctcatcttgg aatctcagaa ttggcgctat gctactggtg gatgggagac tgtgtttaga 780
cctgtaagtg agacatgtac agacagatct ggcctctcca ctggacactg gtcaggtgaa 840
gtaaatgaca aaaacattca agtggtcgag ctccccattg tagacagcct ccatcctcgg 900
cctccttact taccactggc tgttccagaa gaccttgcag accgactcct aagagtccat 960
ggtgaccctg cagtgtggtg ggtgtcccag tttgtcaaat acttgattcg tccacaacct 1020

tggctggaaa aggaaataga agaagccacc aagaagcttg gcttcaaaca tccagttatt 1080

ggagtccatg tcagacgcac agacaaagtg ggaacagaag cagccttcca ccccatcgag 1140

gagtacatgg tacacgttga agaacatttt cagcttctcg cacgcagaat gcaagtggat 1200

aaaaaaagag tatatctggc tactgatgat cctactttgt taaaggaggc aaagacaaag 1260

tactccaatt atgaatttat tagtgataac tctatttctt ggtcagctgg actacacaat 1320

cggtacacag aaaattcact tcggggtgtg atcctggata tacactttct ctcacaggct 1380

gactttctag tgtgtacttt ttcatcccag gtctgtcggg ttgcttatga aatcatgcaa 1440

accctgcatc ctgatgcctc tgcgaacttc cattctttgg atgacatcta ctattttgga 1500

ggccaaaatg cccacaatca gattgctgtt tatcctcaca aacctcgaac tgaagaggaa 1560

attccaatgg aacctggaga tatcattggt gtggctggaa accattggga tggttattct 1620

aaaggtatca acagaaaact tggaaaaaca ggcttatatc cctcctacaa agtccgagag 1680

aagatagaaa cagtcaagta tcccacatat cctgaagctg aaaaatag 1728

<210> 3
<211> 9196
<212> DNA
<213> Cricetulus griseus

<400> 3

tctagaccag gctggtctcg aactcacaga gaaccacctg cctctgccac ctgagtgctg 60

ggattaaagg tgtgcaccac caccgcccgg cgtaaaatca tatttttgaa tattgtgata 120

atttacatta taattgtaag taaaaatttt cagcctattt tgttatacat ttttgcgtaa 180

attattcttt tttgaaagtt ttgttgtcca taatagtcta gggaaacata aagttataat 240

ttttgtctat gtatttgcat atatatctat ttaatctcct aatgtccagg aaataaatag 300

ggtatgtaat agcttcaaca tgtggtatga tagaattttt cagtgctata taagttgtta 360

cagcaaagtg ttattaattc atatgtccat atttcaattt tttatgaatt attaaattga 420

atccttaagc tgccagaact agaattttat tttaatcagg aagccccaaa tctgttcatt 480

ctttctatat atgtggaaag gtaggcctca ctaactgatt cttcacctgt tttagaacat 540

ggtccaagaa tggagttatg taaggggaat tacaagtgtg agaaaactcc tagaaaacaa 600

gatgagtctt gtgaccttag tttctttaaa aacacaaaat tcttggaatg tgttttcatg 660

ttcctcccag gtggatagga gtgagtttat ttcagattat ttattacaac tggctgttgt 720

tacttgtttc tatgtcttta tagaaaaaca tatttttttt gccacatgca gcttgtcctt 780

atgattttat acttgtgtga ctcttaactc tcagagtata aattgtctga tgctatgaat 840

aaagttggct attgtatgag acttcagccc acttcaatta ttggcttcat tctctcagat 900

cccaccacct ccagagtggt aaacaacttg aaccattaaa cagactttag tctttatttg 960

aatgatagat ggggatatca gatttatagg cacagggttt tgagaaaggg agaaggtaaa 1020

cagtagagtt taacaacaac aaaaagtata ctttgtaaac gtaaaactat ttattaaagt 1080

agtagacaag acattaaata ttccttggga ttagtgcttt ttgaattttg ctttcaaata 1140

atagtcagtg agtatacccc tcccccattc tatattttag cagaaatcag aataaatggt 1200

gtttctggta cattcttttg tagagaattt attttctttg ggtttttgtg catttaaagt 1260

caataaaaat taaggttcag taatagaaaa aaaactctga tttttggaat cccctttctt 1320

cagcttttct atttaatctc ttaatgataa tttaatttgt ggccatgtgg tcaaagtata 1380

tagccttgta tatgtaaatg ttttaaccaa cctgccttta cagtaactat ataattttat 1440

tctataatat atgacttttc ttccatagct ttagagttgc ccagtcactt taagttacat 1500

tttcatatat gttctttgtg ggaggagata attttatttc taagagaatc ctaagcatac 1560

tgattgagaa atggcaaaca aaacacataa ttaaagctga taaagaacga acatttggag 1620

tttaaaatac atagccaccc taagggttta actgttgtta gccttctttt ggaattttta 1680

ttagttcata tagaaaaatg gattttatcg tgacatttcc atatatgtat ataatatatt 1740

tacatcatat ccacctgtaa ttattagtgt ttttaaatat atttgaaaaa ataatggtct 1800

ggtttgatcc atttgaacct tttgatgttt ggtgtggttg ccaattggtt gatggttatg 1860

ataacctttg cttctctaag gttcaagtca gtttgagaat atgtcctcta aaaatgacag 1920

gttgcaagtt aagtagtgag atgacagcga gatggagtga tgagaatttg tagaaatgaa 1980

ttcacttata ctgagaactt gttttgcttt tagataatga acatattagc ctgaagtaca 2040

tagccgaatt gattaattat tcaaagatat aatcttttaa tccctataaa agaggtatta 2100

cacaacaatt caagaaagat agaattagac ttccagtatt ggagtgaacc atttgttatc 2160

aggtagaacc ctaacgtgtg tggttgactt aaagtgttta ctttttacct gatactgggt 2220

agctaattgt ctttcagcct cctggccaaa gataccatga aagtcaactt acgttgtatt 2280

ctatatctca aacaactcag ggtgtttctt actctttcca cagcatgtag agcccaggaa 2340

gcacaggaca agaaagctgc ctccttgtat caccaggaag atctttttgt aagagtcatc 2400

acagtatacc agagagacta attttgtctg aagcatcatg tgttgaaaca acagaaactt 2460

attttcctgt gtggctaact agaaccagag tacaatgttt ccaattcttt gagctccgag 2520

aagacagaag ggagttgaaa ctctgaaaat gcgggcatgg actggttcct ggcgttggat 2580

tatgctcatt ctttttgcct gggggacctt attgttttat ataggtggtc atttggttcg 2640

agataatgac caccctgacc attctagcag agaactctcc aagattcttg caaagctgga 2700

gcgcttaaaa caacaaaatg aagacttgag gagaatggct gagtctctcc ggtaggtttg 2760

aaatactcaa ggatttgatg aaatactgtg cttgaccttt aggtataggg tctcagtctg 2820

ctgttgaaaa atataatttc tacaaaccgt ctttgtaaaa ttttaagtat tgtagcagac 2880

tttttaaaag tcagtgatac atctatatag tcaatatagg tttacatagt tgcaatctta 2940

ttttgcatat gaatcagtat atagaagcag tggcatttat atgcttatgt tgcatttaca 3000

attatgttta gacgaacaca aactttatgt gatttggatt agtgctcatt aaattttttt 3060

attctatgga ctacaacaga gacataaatt ttgaaaggct tagttactct taaattctta 3120

tgatgaaaag caaaaattca ttgttaaata gaacagtgca tccggaatgt gggtaattat 3180

tgccatattt ctagtctact aaaaattgtg gcataactgt tcaaagtcat cagttgtttg 3240

gaaagccaaa gtctgattta aatggaaaac ataaacaatg atatctattt ctagatacct 3300

ttaacttgca gttactgagt ttacaagttg tctgacaact ttggattctc ttacttcata 3360

tctaagaatg atcatgtgta cagtgcttac tgtcacttta aaaaactgca gggctagaca 3420

tgcagatatg aagactttga cattagatgt ggtaattggc actaccagca agtggtatta 3480

agatacagct gaatatatta ctttttgagg aacataattc atgaatggaa agtggagcat 3540

tagagaggat gccttctggc tctcccacac cactgtttgc atccattgca tttcacactg 3600

cttttagaac tcagatgttt catatggtat attgtgtaac tcaccatcag ttttatcttt 3660

aaatgtctat ggatgataat gttgtatgtt aacactttta caaaaacaaa tgaagccata 3720

tcctcggtgt gagttgtgat ggtggtaatt gtcacaatag gattattcag caaggaacta 3780

agtcagggac aagaagtggg cgatactttg ttggattaaa tcattttact ggaagttcat 3840

cagggagggt tatgaaagtt gtggtctttg aactgaaatt atatgtgatt cattattctt 3900

gatttaggcc ttgctaatag taactatcat ttattgggaa tttgtcatat gtgccaattt 3960

gtcatgggcc agacagcgtg ttttactgaa tttctagata tctttatgag attctagtac 4020

tgttttcagc cattttacag atgaagaatc ttaaaaaatg ttaaataatt tagtttgccc 4080

aagattatac gttaacaaat ggtagaacct tctttgaatt ctggcagtat ggctacacag 4140

tccgaactct tatcttccta agctgaaaac agaaaaagca atgacccaga aaattttatt 4200

taaaagtctc aggagagact tcccatcctg agaagatctc ttttcccttt tataatttag 4260

gctcctgaat aatcactgaa ttttctccat gttccatcta tagtactgtt atttctgttt 4320

tccttttttc ttaccacaaa gtatcttgtt tttgctgtat gaaagaaaat gtgttattgt 4380

aatgtgaaat tctctgtccc tgcagggtcc cacatccgcc tcaatcccaa ataaacacac 4440

agaggctgta ttaattatga aactgttggt cagttggcta gggcttctta ttggctagct 4500

ctgtcttaat tattaaacca taactactat tgtaagtatt tccatgtggt cttatcttac 4560

caaggaaagg gtccagggac ctcttactcc tctggcgtgt tggcagtgaa gaggagagag 4620

cgatttccta tttgtctctg cttattttct gattctgctc agctatgtca cttcctgcct 4680

ggccaatcag ccaatcagtg ttttattcat tagccaataa aagaaacatt tacacagaag 4740

gacttccccc atcatgttat ttgtatgagt tcttcagaaa atcatagtat cttttaatac 4800

taatttttat aaaaaattaa ttgtattgaa aattatgtgt atatgtgtct gtgtgtcgat 4860

ttgtgctcat aagtagcatg gagtgcagaa gagggaatca gatctttttt taagggacaa 4920

agagtttatt cagattacat tttaaggtga taatgtatga ttgcaaggtt atcaacatgg 4980

cagaaatgtg aagaagctgg tcacattaca tccagagtca agagtagaga gcaatgaatt 5040

gatgcatgca ttcctgtgct cagctcactt ttcctggagc tgagctgatt gtaagccatc 5100

tgatgtcttt gctgggaact aactcaaagg caagttcaaa acctgttctt aagtataagc 5160

catctctcca gtccctcata tggtctctta agacactttc tttatattct tgtacataga 5220

aattgaattc ctaacaactg cattcaaatt acaaaatagt ttttaaaagc tgatataata 5280

aatgtaaata caatctagaa catttttata aataagcata ttaactcagt aaaaataaat 5340

gcatggttat tttccttcat tagggaagta tgtctcccca ggctgttctc tagattctac 5400

tagtaatgct gtttgtacac catccacagg ggttttattt taaagctaag acatgaatga 5460

tggacatgct tgttagcatt tagacttttt tccttactat aattgagcta gtatttttgt 5520

gctcagtttg atatctgtta attcagataa atgtaatagt aggtaatttc tttgtgataa 5580

aggcatataa attgaagttg gaaaacaaaa gcctgaaatg acagttttta agattcagaa 5640

caataatttt caaaagcagt tacccaactt tccaaataca atctgcagtt ttcttgatat 5700

gtgataaatt tagacaaaga aatagcacat tttaaaatag ctatttactc ttgatttttt 5760

tttcaaattt aggctagttc actagttgtg tgtaaggtta tggctgcaaa catctttgac 5820

tcttggttag ggaatccagg atgatttacg tgtttggcca aaatcttgtt ccattctggg 5880

tttcttctct atctaggtag ctagcacaag ttaaaggtgt ggtagtattg gaaggctctc 5940

aggtatatat ttctatattc tgtatttttt tcctctgtca tatatttgct ttctgtttta 6000

ttgatttcta ctgttagttt gatacttact ttcttacact ttctttggga tttattttgc 6060

tgttctaaga tttcttagca agttcatatc actgatttta acagttgctt cttttgtaat 6120

atagactgaa tgccccttat ttgaaatgct tgggatcaga aactcagatt tgaacttttc 6180

tttttttaata tttccatcaa gtttaccagc tgaatgtcct gatccaagaa tatgaaatct 6240

gaaatgcttt gaaatctgaa acttttagag tgataaagct tccctttaaa ttaatttgtg 6300

ttctatattt tttgacaatg tcaacctttc attgttatcc aatgagtgaa catattttca 6360

atttttttgt ttgatctgtt atattttgat ctgaccatat ttataaaatt ttatttaatt 6420

tgaatgttgt gctgttactt atctttatta ttatttttgc ttattttcta gccaaatgaa 6480

attatattct gtattatttt agtttgaatt ttactttgtg gcttagtaac tgccttttgt 6540

tggtgaatgc ttaagaaaaa cgtgtggtct actgatattg gttctaatct tatatagcat 6600

gttgtttgtt aggtagttga ttatgctggt cagattgtct tgagtttatg caaatgtaaa 6660

atatttagat gcttgttttg ttgtctaaga acaaagtatg cttgctgtct cctatcggtt 6720

ctggtttttc cattcatctc ttcaagctgt tttgtgtgtt gaatactaac tccgtactat 6780

cttgttttct gtgaattaac ccctttttcaa aggtttcttt tcttttttttt tttaagggac 6840

aacaagttta ttcagattac attttaagct gataatgtat gattgcaagg ttatcaacat 6900

ggcagaaatg tgaagaagct aggcacatta catccacatg gagtcaagag cagagagcag 6960

tgaattaatg catgcattcc tgtggtcagc tcacttttcc tattcttaga tagtctagga 7020

tcataaacct ggggaatagt gctaccacaa tgggcatatc cacttacttc agttcatgca 7080

atcaaccaag gcacatccac aggaaaaact gatttagaca acctctcatt gagactcttc 7140

ccagatgatt agactgtgtc aagttgacaa ttaaaactat cacacctgaa gccatcacta 7200

gtaaatataa tgaaaatgtt gattatcacc ataattcatc tgtatccctt tgttattgta 7260

gattttgtga agttcctatt caagtccctg ttccttcctt aaaaacctgt tttttagtta 7320

aataggtttt ttagtgttcc tgtctgtaaa tactttttta aagttagata ttattttcaa 7380

gtatgttctc ccagtctttg gcttgtattt tcatcccttc aatacatata tttttgtaat 7440

ttattttttt tatttaaatt agaaacaaag ctgcttttac atgtcagtct cagttccctc 7500

tccctcccct cctcccctgc tccccaccta agccccaatt ccaactcctt tcttctcccc 7560

aggaagggtg aggccctcca tgggggaaat cttcaatgtc tgtcatatca tttggagcag 7620

ggcctagacc ctccccagtg tgtctaggct gagagagtat ccctctatgt ggagagggct 7680

cccaaagttc atttgtgtac taggggtaaa tactgatcca ctatcagtgg ccccatagat 7740

tgtccggacc tccaaactga cttcctcctt cagggagtct ggaacagttc tatgctggtt 7800

tcccagatat cagtctgggg tccatgagca accccttgtt caggtcagtt gtttctgtag 7860

gtttccccag cccggtcttg accccctttgc tcatcacttc tccctctctg caactggatt 7920

ccagagttca gctcagtgtt tagctgtggg tgtctgcatc tgcttccatc agctactgga 7980

tgagggctct aggatggcat ataaggtagt catcagtctc attatcagag aagggctttt 8040

aaggtagcct cttgattatt gcttagattg ttagttgggg tcaaccttgt aggtctctgg 8100

acagtgacag aattctcttt aaacctataa tggctccctc tgtggtggta tccctttttct 8160

tgctctcatc cgttcctccc ctgactagat cttcctgctc cctcatgtcc tcctctcccc 8220

tcccccttctc cccttctctt tcttctaact ccctctcccc tccacccacg atccccatta 8280

gcttatgaga tcttgtcctt attttagcaa aacctttttg gctataaaat taattaattt 8340

aatatgctta tatcaggttt attttggcta gtatttgtat gtgtttggtt agtgtttttta 8400

accttaattg acatgtatcc ttatatttag acacagattt aaatatttga agtttttttt 8460

tttttttttt ttaaagattt atttattttt tatgtcttct gcctgcatgc cagaagaggg 8520

caccagatct cattcaaggt ggttgtgagc caccatgtgg ttgctgggaa ttgaactcag 8580

gacctctgga agaacagtca gtgctcttaa ccgctgagcc atctctccag cccctgaagt 8640

gtttctttta aagaggatag cagtgcatca tttttccctt tgaccaatga ctcctacctt 8700

actgaattgt tttagccatt tatatgtaat gctgttacca ggtttacatt ttcttttatc 8760

ttgctaaatt tcttccctgt ttgtctcatc tcttattttt gtctgttgga ttatataggc 8820

ttttattttt ctgtttttac agtaagttat atcaaattaa aattatttta tggaatgggt 8880

gtgttgacta catgtatgtc tgtgcaccat gtgctgacct ggtcttggcc agaagaaggt 8940

gtcatattct ctgaaactgg tattgtggat gttacgaact gccatagggt gctaggaatc 9000

aaaccccagc tcctctggaa aagcagccac tgctctgagc cactgagtcc tctcttcaag 9060

caggtgatgc caacttttaa tggttaccag tggataagag tgcttgtatc tctagcaccc 9120

atgaaaattt atgcattgct atatgggctt gtcacttcag cattgtgtga cagagacagg 9180

aggatcccaa gagctc 9196

<210> 4
<211> 25
<212> DNA
<213> Artificial Sequence

<220>
<223> Description of Artificial Sequense: Synthetic DNA

<400> 4
actcatcttg gaatctcaga attgg 25

<210> 5
<211> 24
<212> DNA
<213> Artificial Sequence

<220>
<223> Description of Artificial Sequense: Synthetic DNA

<400> 5
cttgaccgtt tctatcttct ctcg 24

<210> 6
<211> 979
<212> DNA
<213> Cricetulus griseus

<400> 6

actcatcttg gaatctcaga attggcgcta tgctactgga ggatgggaga ctgtgtttag 60

acctgtaagt gagacatgca cagacaggtc tggcctctcc actggacact ggtcaggtga 120

agtgaaggac aaaaatgttc aagtggtcga gctccccatt gtagacagcc tccatcctcg 180

tcctccttac ttacccttgg ctgtaccaga agaccttgca gatcgactcc tgagagtcca 240

tggtgatcct gcagtgtggt gggtatccca gtttgtcaaa tacttgatcc gtccacaacc 300

ttggctggaa agggaaatag aagaaaccac caagaagctt ggcttcaaac atccagttat 360

tggagtccat gtcagacgca ctgacaaagt gggaacagaa gcagccttcc atcccattga 420

ggaatacatg gtacacgttg aagaacattt tcagcttctc gaacgcagaa tgaaagtgga 480

taaaaaaaga gtgtatctgg ccactgatga cccttctttg ttaaaggagg caaagacaaa 540

gtactccaat tatgaattta ttagtgataa ctctatttct tggtcagctg gactacacaa 600

ccgatacaca gaaaattcac ttcggggcgt gatcctggat atacactttc tctcccaggc 660

tgacttcctt gtgtgtactt tttcatccca ggtctgtagg gttgcttatg aaatcatgca 720

aacactgcat cctgatgcct ctgcaaactt ccattcttta gatgacatct actattttgg 780

aggccaaaat gcccacaacc agattgcagt ttatcctcac caacctcgaa ctaaagagga 840

aatccccatg gaacctggag atatcattgg tgtggctgga aaccattgga atggttactc 900

taaaggtgtc aacagaaaac taggaaaaac aggcctgtac ccttcctaca aagtccgaga 960

gaagatagaa acggtcaag 979

<210> 7
<211> 979
<212> DNA
<213> Rattus norvegicus

<400> 7

actcatcttg gaatctcaga attggcgcta tgctactggt ggatgggaga ctgtgtttag 60

acctgtaagt gagacatgca cagacagatc tggcctctcc actggacact ggtcaggtga 120

agtgaatgac aaaaatattc aagtggtgga gctccccatt gtagacagcc ttcatcctcg 180

gcctccttac ttaccactgg ctgttccaga agaccttgca gatcgactcg taagagtcca 240

tggtgatcct gcagtgtggt gggtgtccca gttcgtcaaa tatttgattc gtccacaacc 300

ttggctagaa aaggaaatag aagaagccac caagaagctt ggcttcaaac atccagtcat 360

tggagtccat gtcagacgca cagacaaagt gggaacagag gcagccttcc atcccatcga 420

agagtacatg gtacatgttg aagaacattt tcagcttctc gcacgcagaa tgcaagtgga 480

taaaaaaaga gtatatctgg ctaccgatga ccctgctttg ttaaaggagg caaagacaaa 540

gtactccaat tatgaattta ttagtgataa ctctatttct tggtcagctg gactacacaa 600

tcggtacaca gaaaattcac ttcggggcgt gatcctggat atacactttc tctctcaggc 660

tgacttccta gtgtgtactt tttcatccca ggtctgtcgg gttgcttatg aaatcatgca 720

aaccctgcat cctgatgcct ctgcaaactt ccactcttta gatgacatct actattttgg 780

aggccaaaat gcccacaacc agattgccgt ttatcctcac aaacctcgaa ctgatgagga 840

aattccaatg gaacctggag atatcattgg tgtggctgga aaccattggg atggttattc 900

taaaggtgtc aacagaaaac ttggaaaaac aggcttatat ccctcctaca aagtccgaga 960

gaagatagaa acggtcaag 979

<210> 8
<211> 40
<212> DNA
<213> Artificial Sequence

<220>
<223> Description of Artificial Sequense: Synthetic DNA

<400> 8
aagtataagc ttacatggat gacgatatcg ctgcgctcgt 40

<210> 9
<211> 40
<212> DNA
<213> Artificial Sequence

<220>
<223> Description of Artificial Sequense: Synthetic DNA

<400> 9
atttaactgc aggaagcatt tgcggtggac gatggagggg 40

<210> 10
<211> 40
<212> DNA
<213> Artificial Sequence

<220>
<223> Description of Artificial Sequense: Synthetic DNA

<400> 10
atttaaggta ccgaagcatt tgcggtgcac gatggagggg 40

<210> 11
<211> 23
<212> DNA
<213> Artificial Sequence

<220>
<223> Description of Artificial Sequense: Synthetic DNA

<400> 11
ctccaattat gaatttatta gtg 23

<210> 12
<211> 25
<212> DNA
<213> Artificial Sequence

<220>
<223> Description of Artificial Sequense: Synthetic DNA

<400> 12
ggatgtttga agccaagctt cttgg 25

<210> 13
<211> 24
<212> DNA
<213> Artificial Sequence

<220>
<223> Description of Artificial Sequense: Synthetic DNA

<400> 13
gtccatggtg atcctgcagt gtgg 24

<210> 14
<211> 23
<212> DNA
<213> Artificial Sequence

<220>
<223> Description of Artificial Sequense: Synthetic DNA

<400> 14
caccaatgat atctccaggt tcc 23

<210> 15
<211> 24
<212> DNA
<213> Artificial Sequence

<220>
<223> Description of Artificial Sequense: Synthetic DNA

<400> 15
gatatcgctg cgctcgttgt cgac 24

<210> 16
<211> 24
<212> DNA
<213> Artificial Sequence

<220>
<223> Description of Artificial Sequense: Synthetic DNA

<400> 16
caggaaggaa ggctggaaaa gagc 24

<210> 17
<211> 24
<212> DNA
<213> Artificial Sequence

<220>
<223> Description of Artificial Sequense: Synthetic DNA

<400> 17
gatatcgctg cgctcgtcgt cgac 24

<210> 18
<211> 24
<212> DNA
<213> Artificial Sequence

<220>
<223> Description of Artificial Sequense: Synthetic DNA

<400> 18
caggaaggaa ggctggaaga gagc 24

<210> 19
<211> 24
<212> DNA
<213> Artificial Sequence

<220>
<223> Description of Artificial Sequense: Synthetic DNA

<400> 19
atgcgggcat ggactggttc ctgg 24

<210> 20
<211> 27
<212> DNA
<213> Artificial Sequence

<220>
<223> Description of Artificial Sequense: Synthetic DNA

<400> 20
ctatttttca gcttcaggat atgtggg 27

<210> 21
<211> 24
<212> DNA
<213> Artificial Sequence

<220>
<223> Description of Artificial Sequense: Synthetic DNA

<400> 21
gtctgaagca ttatgtgttg aagc 24

<210> 22
<211> 23
<212> DNA
<213> Artificial Sequence

<220>
<223> Description of Artificial Sequense: Synthetic DNA

<400> 22
gtgagtacat tcattgtact gtg 23

<210> 23
<211> 575
<212> PRT
<213> Cricetulus griseus

<400> 23

```
Met Arg Ala Trp Thr Gly Ser Trp Arg Trp Ile Met Leu Ile Leu Phe
1               5                   10                  15

Ala Trp Gly Thr Leu Leu Phe Tyr Ile Gly Gly His Leu Val Arg Asp
            20                  25                  30

Asn Asp His Pro Asp His Ser Ser Arg Glu Leu Ser Lys Ile Leu Ala
        35                  40                  45

Lys Leu Glu Arg Leu Lys Gln Gln Asn Glu Asp Leu Arg Arg Met Ala
    50                  55                  60

Glu Ser Leu Arg Ile Pro Glu Gly Pro Ile Asp Gln Gly Thr Ala Thr
```

65 70 75 80

Gly Arg Val Arg Val Leu Glu Glu Gln Leu Val Lys Ala Lys Glu Gln
85 90 95

Ile Glu Asn Tyr Lys Lys Gln Ala Arg Asn Asp Leu Gly Lys Asp His
100 105 110

Glu Ile Leu Arg Arg Arg Ile Glu Asn Gly Ala Lys Glu Leu Trp Phe
115 120 125

Phe Leu Gln Ser Glu Leu Lys Lys Leu Lys Lys Leu Glu Gly Asn Glu
130 135 140

Leu Gln Arg His Ala Asp Glu Ile Leu Leu Asp Leu Gly His His Glu
145 150 155 160

Arg Ser Ile Met Thr Asp Leu Tyr Tyr Leu Ser Gln Thr Asp Gly Ala
165 170 175

Gly Glu Trp Arg Glu Lys Glu Ala Lys Asp Leu Thr Glu Leu Val Gln
180 185 190

Arg Arg Ile Thr Tyr Leu Gln Asn Pro Lys Asp Cys Ser Lys Ala Arg
195 200 205

Lys Leu Val Cys Asn Ile Asn Lys Gly Cys Gly Tyr Gly Cys Gln Leu
210 215 220

His His Val Val Tyr Cys Phe Met Ile Ala Tyr Gly Thr Gln Arg Thr
225 230 235 240

Leu Ile Leu Glu Ser Gln Asn Trp Arg Tyr Ala Thr Gly Gly Trp Glu
245 250 255

Thr Val Phe Arg Pro Val Ser Glu Thr Cys Thr Asp Arg Ser Gly Leu
260 265 270

Ser Thr Gly His Trp Ser Gly Glu Val Lys Asp Lys Asn Val Gln Val
275 280 285

Val Glu Leu Pro Ile Val Asp Ser Leu His Pro Arg Pro Pro Tyr Leu
290 295 300

Pro Leu Ala Val Pro Glu Asp Leu Ala Asp Arg Leu Leu Arg Val His
305 310 315 320

Gly Asp Pro Ala Val Trp Trp Val Ser Gln Phe Val Lys Tyr Leu Ile
325 330 335

Arg Pro Gln Pro Trp Leu Glu Arg Glu Ile Glu Glu Thr Thr Lys Lys
340 345 350

Leu Gly Phe Lys His Pro Val Ile Gly Val His Val Arg Arg Thr Asp
355 360 365

Lys Val Gly Thr Glu Ala Ala Phe His Pro Ile Glu Glu Tyr Met Val
370 375 380

His Val Glu Glu His Phe Gln Leu Leu Glu Arg Arg Met Lys Val Asp
385 390 395 400

Lys Lys Arg Val Tyr Leu Ala Thr Asp Asp Pro Ser Leu Leu Lys Glu
405 410 415

Ala Lys Thr Lys Tyr Ser Asn Tyr Glu Phe Ile Ser Asp Asn Ser Ile
420 425 430

Ser Trp Ser Ala Gly Leu His Asn Arg Tyr Thr Glu Asn Ser Leu Arg
435 440 445

Gly Val Ile Leu Asp Ile His Phe Leu Ser Gln Ala Asp Phe Leu Val
450 455 460

Cys Thr Phe Ser Ser Gln Val Cys Arg Val Ala Tyr Glu Ile Met Gln
465 470 475 480

Thr Leu His Pro Asp Ala Ser Ala Asn Phe His Ser Leu Asp Asp Ile
485 490 495

Tyr Tyr Phe Gly Gly Gln Asn Ala His Asn Gln Ile Ala Val Tyr Pro
500 505 510

His Gln Pro Arg Thr Lys Glu Glu Ile Pro Met Glu Pro Gly Asp Ile
515 520 525

Ile Gly Val Ala Gly Asn His Trp Asn Gly Tyr Ser Lys Gly Val Asn
530 535 540

Arg Lys Leu Gly Lys Thr Gly Leu Tyr Pro Ser Tyr Lys Val Arg Glu
545 550 555 560

Lys Ile Glu Thr Val Lys Tyr Pro Thr Tyr Pro Glu Ala Glu Lys
565 570 575

<210> 24
<211> 575
<212> PRT
<213> Mus musculus

<400> 24

Met Arg Ala Trp Thr Gly Ser Trp Arg Trp Ile Met Leu Ile Leu Phe
1 5 10 15

Ala Trp Gly Thr Leu Leu Phe Tyr Ile Gly Gly His Leu Val Arg Asp
20 25 30

Asn Asp His Pro Asp His Ser Ser Arg Glu Leu Ser Lys Ile Leu Ala
35 40 45

Lys Leu Glu Arg Leu Lys Gln Gln Asn Glu Asp Leu Arg Arg Met Ala
50 55 60

Glu Ser Leu Arg Ile Pro Glu Gly Pro Ile Asp Gln Gly Thr Ala Thr
65 70 75 80

Gly Arg Val Arg Val Leu Glu Glu Gln Leu Val Lys Ala Lys Glu Gln
85 90 95

Ile Glu Asn Tyr Lys Lys Gln Ala Arg Asn Gly Leu Gly Lys Asp His
100 105 110

Glu Ile Leu Arg Arg Arg Ile Glu Asn Gly Ala Lys Glu Leu Trp Phe
115 120 125

Phe Leu Gln Ser Glu Leu Lys Lys Leu Lys His Leu Glu Gly Asn Glu
130 135 140

Leu Gln Arg His Ala Asp Glu Ile Leu Leu Asp Leu Gly His His Glu
145 150 155 160

Arg Ser Ile Met Thr Asp Leu Tyr Tyr Leu Ser Gln Thr Asp Gly Ala
165 170 175

Gly Asp Trp Arg Glu Lys Glu Ala Lys Asp Leu Thr Glu Leu Val Gln
180 185 190

Arg Arg Ile Thr Tyr Leu Gln Asn Pro Lys Asp Cys Ser Lys Ala Arg
195 200 205

Lys Leu Val Cys Asn Ile Asn Lys Gly Cys Gly Tyr Gly Cys Gln Leu
210 215 220

His His Val Val Tyr Cys Phe Met Ile Ala Tyr Gly Thr Gln Arg Thr
225 230 235 240

Leu Ile Leu Glu Ser Gln Asn Trp Arg Tyr Ala Thr Gly Gly Trp Glu
245 250 255

Thr Val Phe Arg Pro Val Ser Glu Thr Cys Thr Asp Arg Ser Gly Leu
260 265 270

Ser Thr Gly His Trp Ser Gly Glu Val Asn Asp Lys Asn Ile Gln Val
275 280 285

Val Glu Leu Pro Ile Val Asp Ser Leu His Pro Arg Pro Pro Tyr Leu
290 295 300

Pro Leu Ala Val Pro Glu Asp Leu Ala Asp Arg Leu Leu Arg Val His
305 310 315 320

Gly Asp Pro Ala Val Trp Trp Val Ser Gln Phe Val Lys Tyr Leu Ile
325 330 335

Arg Pro Gln Pro Trp Leu Glu Lys Glu Ile Glu Glu Ala Thr Lys Lys
340 345 350

Leu Gly Phe Lys His Pro Val Ile Gly Val His Val Arg Arg Thr Asp
355 360 365

Lys Val Gly Thr Glu Ala Ala Phe His Pro Ile Glu Glu Tyr Met Val
370 375 380

His Val Glu Glu His Phe Gln Leu Leu Ala Arg Arg Met Gln Val Asp
385 390 395 400

Lys Lys Arg Val Tyr Leu Ala Thr Asp Asp Pro Thr Leu Leu Lys Glu
405 410 415

Ala Lys Thr Lys Tyr Ser Asn Tyr Glu Phe Ile Ser Asp Asn Ser Ile
420 425 430

Ser Trp Ser Ala Gly Leu His Asn Arg Tyr Thr Glu Asn Ser Leu Arg
435 440 445

Gly Val Ile Leu Asp Ile His Phe Leu Ser Gln Ala Asp Phe Leu Val
450 455 460

Cys Thr Phe Ser Ser Gln Val Cys Arg Val Ala Tyr Glu Ile Met Gln
465 470 475 480

Thr Leu His Pro Asp Ala Ser Ala Asn Phe His Ser Leu Asp Asp Ile
485 490 495

Tyr Tyr Phe Gly Gly Gln Asn Ala His Asn Gln Ile Ala Val Tyr Pro
500 505 510

His Lys Pro Arg Thr Glu Glu Glu Ile Pro Met Glu Pro Gly Asp Ile
515 520 525

Ile Gly Val Ala Gly Asn His Trp Asp Gly Tyr Ser Lys Gly Ile Asn
530 535 540

Arg Lys Leu Gly Lys Thr Gly Leu Tyr Pro Ser Tyr Lys Val Arg Glu
545 550 555 560

Lys Ile Glu Thr Val Lys Tyr Pro Thr Tyr Pro Glu Ala Glu Lys
565 570 575

<210> 25
<211> 18
<212> PRT
<213> Homo sapiens

<400> 25

Asp Glu Ser Ile Tyr Ser Asn Tyr Tyr Leu Tyr Glu Ser Ile Pro Lys
1 5 10 15

Pro Cys

<210> 26
<211> 25
<212> DNA
<213> Artificial Sequence

<220>
<223> Description of Artificial Sequense: Synthetic DNA

<400> 26
cttgtgtgac tcttaactct cagag 25

<210> 27
<211> 23
<212> DNA
<213> Artificial Sequence

<220>
<223> Description of Artificial Sequense: Synthetic DNA

<400> 27
ccctcgagat aacttcgtat agc 23

<210> 28
<211> 18
<212> DNA
<213> Artificial Sequence

<220>
<223> Description of Artificial Sequense: Synthetic DNA

<400> 28
ggtaggcctc actaactg 18

<210> 29
<211> 25
<212> DNA
<213> Artificial Sequence

<220>
<223> Description of Artificial Sequense: Synthetic DNA

<400> 29
catagaaaca agtaacaaca gccag 25

<210> 30
<211> 28
<212> DNA
<213> Artificial Sequence

<220>
<223> Description of Artificial Sequense: Synthetic DNA

<400> 30
gagacttcag cccacttcaa ttattggc 28

<210> 31
<211> 25
<212> DNA
<213> Artificial Sequence

<220>
<223> Description of Artificial Sequense: Synthetic DNA

<400> 31
gaggccactt gtgtagcgcc aagtg 25

<210> 32
<211> 24
<212> DNA
<213> Artificial Sequence

<220>
<223> Description of Artificial Sequence: Synthetic DNA

<400> 32
aggaaggtgg cgctcatcac gggc 24

<210> 33
<211> 26
<212> DNA
<213> Artificial Sequence

<220>
<223> Description of Artificial Sequence: Synthetic DNA

<400> 33
taaggccaca agtcttaatt gcatcc 26

<210> 34
<211> 27
<212> DNA
<213> Artificial Sequence

<220>
<223> Description of Artificial Sequence: Synthetic DNA

<400> 34
caggggtgtt cccttgagga ggtggaa 27

<210> 35
<211> 23
<212> DNA
<213> Artificial Sequence

<220>
<223> Description of Artificial Sequence: Synthetic DNA

<400> 35
cccctcacgc atgaagcctg gag 23

<210> 36
<211> 28
<212> DNA
<213> Artificial Sequence

<220>
<223> Description of Artificial Sequence: Synthetic DNA

<400> 36
ggcaggagac caccttgcga gtgcccac 28

<210> 37
<211> 28
<212> DNA
<213> Artificial Sequence

<220>
<223> Description of Artificial Sequence: Synthetic DNA

<400> 37
ggcgctggct tacccggaga ggaatggg 28

<210> 38
<211> 28
<212> DNA
<213> Artificial Sequence

<220>
<223> Description of Artificial Sequence: Synthetic DNA

<400> 38
aaaaggcctc agttagtgaa ctgtatgg 28

<210> 39
<211> 29
<212> DNA
<213> Artificial Sequence

<220>
<223> Description of Artificial Sequence: Synthetic DNA

<400> 39
cgcggatcct caagcgttgg ggttggtcc 29

<210> 40
<211> 45
<212> DNA
<213> Artificial Sequence

<220>
<223> Description of Artificial Sequence: Synthetic DNA

<400> 40
cccaagcttg ccaccatggc tcacgctccc gctagctgcc cgagc 45

<210> 41
<211> 31
<212> DNA

<213> Artificial Sequence

<220>
<223> Description of Artificial Sequence: Synthetic DNA

<400> 41
ccggaattct gccaagtatg agccatcctg g 31

<210> 42
<211> 17
<212> DNA
<213> Artificial Sequence

<220>
<223> Description of Artificial Sequence: Synthetic DNA

<400> 42
gccatccaga aggtggt 17

<210> 43
<211> 17
<212> DNA
<213> Artificial Sequence

<220>
<223> Description of Artificial Sequence: Synthetic DNA

<400> 43
gtcttgtcag ggaagat 17

<210> 44
<211> 28
<212> DNA
<213> Artificial Sequence

<220>
<223> Description of Artificial Sequence: Synthetic DNA

<400> 44
ggcaggagac caccttgcga gtgcccac 28

<210> 45
<211> 28
<212> DNA
<213> Artificial Sequence

<220>
<223> Description of Artificial Sequence: Synthetic DNA

<400> 45
gggtgggctg taccttctgg aacagggc 28

<210> 46
<211> 28
<212> DNA
<213> Artificial Sequence

<220>

<223> Description of Artificial Sequence: Synthetic DNA

<400> 46
ggcgctggct tacccggaga ggaatggg 28

<210> 47
<211> 28
<212> DNA
<213> Artificial Sequence

<220>
<223> Description of Artificial Sequence: Synthetic DNA

<400> 47
ggaatgggtg tttgtctcctc caaagatgc 28

<210> 48
<211> 1316
<212> DNA
<213> Cricetulus griseus

<400> 48

gccccgcccc ctccacctgg accgagagta gctggagaat tgtgcaccgg aagtagctct 60

tggactggtg gaaccctgcg caggtgcagc aacaatgggt gagccccagg gatccaggag 120

gatcctagtg acagggggct ctggactggt gggcagagct atccagaagg tggtcgcaga 180

tggcgctggc ttacccggag aggaatgggt gtttgtctcc tccaaagatg cagatctgac 240

ggatgcagca caaacccaag ccctgttcca gaaggtacag cccacccatg tcatccatct 300

tgctgcaatg gtaggaggcc ttttccggaa tatcaaatac aacttggatt tctggaggaa 360

gaatgtgcac atcaatgaca acgtcctgca ctcagctttc gaggtgggca ctcgcaaggt 420

ggtctcctgc ctgtccacct gtatcttccc tgacaagacc acctatccta ttgatgaaac 480

aatgatccac aatggtccac cccacagcag caattttggg tactcgtatg ccaagaggat 540

gattgacgtg cagaacaggg cctacttcca gcagcatggc tgcaccttca ctgctgtcat 600

ccctaccaat gtctttggac ctcatgacaa cttcaacatt gaagatggcc atgtgctgcc 660

tggcctcatc cataaggtgc atctggccaa gagtaatggt tcagccttga ctgtttgggg 720

tacagggaaa ccacggaggc agttcatcta ctcactggac ctagcccggc tcttcatctg 780

ggtcctgcgg gagtacaatg aagttgagcc catcatcctc tcagtgggcg aggaagatga 840

agtctccatt aaggaggcag ctgaggctgt agtggaggcc atggacttct gtggggaagt 900

cacttttgat tcaacaaagt cagatgggca gtataagaag acagccagca atggcaagct 960

tcgggcctac ttgcctgatt tccgtttcac acccttcaag caggctgtga aggagacctg 1020

tgcctggttc accgacaact atgagcaggc ccggaagtga agcatgggac aagcgggtgc 1080

tcagctggca atgcccagtc agtaggctgc agtctcatca tttgcttgtc aagaactgag 1140

gacagtatcc agcaacctga gccacatgct ggtctctctg ccagggggct tcatgcagcc 1200

atccagtagg gcccatgttt gtccatcctc gggggaaggc cagaccaaca ccttgtttgt 1260

ctgcttctgc cccaacctca gtgcatccat gctggtcctg ctgtcccttg tctaga 1316

<210> 49
<211> 23
<212> DNA
<213> Artificial Sequence

<220>
<223> Description of Artificial Sequence: Synthetic DNA

<400> 49
gatcctgctg ggaccaaaat tgg 23

<210> 50
<211> 22
<212> DNA
<213> Artificial Sequence

<220>
<223> Description of Artificial Sequence: Synthetic DNA

<400> 50
cttaacatcc caagggatgc tg 22

<210> 51
<211> 1965
<212> DNA
<213> Cricetulus griseus

<400> 51

acggggggct cccggaagcg gggaccatgg cgtctctgcg cgaagcgagc ctgcggaagc 60

tgcggcgctt ttccgagatg agaggcaaac ctgtggcaac tgggaaattc tgggatgtag 120

ttgtaataac agcagctgac gaaaagcagg agcttgctta caagcaacag ttgtcggaga 180

agctgaagag aaaggaattg ccccttggag ttaactacca tgttttcact gatcctcctg 240

gaaccaaaat tggaaatgga ggatcaacac tttgttctct tcagtgcctg gaaagcctct 300

atggagacaa gtggaattcc ttcacagtcc tgttaattca ctctggtggc tacagtcaac 360

gacttcccaa tgcaagcgct ttaggaaaaa tcttcacggc tttaccactt ggtgagccca 420

tttatcagat gttggactta aaactagcca tgtacatgga tttcccctca cgcatgaagc 480

ctggagtttt ggtcacctgt gcagatgata ttgaactata cagcattggg gactctgagt 540

ccattgcatt tgagcagcct ggctttactg ccctagccca tccatctagt ctggctgtag 600

gcaccacaca tggagtattt gtattggact ctgccggttc tttgcaacat ggtgacctag 660

agtacaggca atgccaccgt ttcctccata agcccagcat tgaaaacatg caccacttta 720

atgccgtgca tagactagga agctttggtc aacaggactt gagtgggggt gacaccacct 780

gtcatccatt gcactctgag tatgtctaca cagatagcct attttacatg gatcataaat 840

cagccaaaaa gctacttgat ttctatgaaa gtgtaggccc actgaactgt gaaatagatg 900

cctatggtga ctttctgcag gcactgggac ctggagcaac tgcagagtac accaagaaca 960

cctcacacgt cactaaagag gaatcacact tgttggacat gaggcagaaa atattccacc 1020

tcctcaaggg aacacccctg aatgttgttg tccttaataa ctccaggttt tatcacattg 1080

gaacaacgga ggagtatctg ctacatttca cttccaatgg ttcgttacag gcagagctgg 1140

gcttgcaatc catagctttc agtgtctttc caaatgtgcc tgaagactcc catgagaaac 1200

cctgtgtcat tcacagcatc ctgaattcag gatgctgtgt ggcccctggc tcagtggtag 1260

aatattccag attaggacct gaggtgtcca tctcggaaaa ctgcattatc agcggttctg 1320

tcatagaaaa agctgttctg cccccatgtt ctttcgtgtg ctctttaagt gtggagataa 1380

atggacactt agaatattca actatggtgt ttggcatgga agacaacttg aagaacagtg 1440

ttaaaaccat atcagatata aagatgcttc agttctttgg agtctgtttc ctgacttgtt 1500

tagatatttg gaaccttaaa gctatggaag aactattttc aggaagtaag acgcagctga 1560

gcctgtggac tgctcgaatt ttccctgtct gttcttctct gagtgagtcg gttgcagcat 1620

cccttgggat gttaaatgcc attcgaaacc attcgccatt cagcctgagc aacttcaagc 1680

tgctgtccat ccaggaaatg cttctctgca aagatgtagg agacatgctt gcttacaggg 1740

agcaactctt tctagaaatc agttcaaaga gaaaacagtc tgattcggag aaatcttaaa 1800

tacaatggat tttgcctgga aacaggattg caaatgcagg catattctat agatctctgg 1860

gttcttcttt ctttctcccc tctctccttt cctttccctt tgatgtaatg acaaaggtaa 1920

aaatggccac ttctgatgga aaaaaaaaaa aaaaaaaaaa aaaaa 1965

<210> 52
<211> 27
<212> DNA
<213> Artificial Sequence

<220>
<223> Description of Artificial Sequence: Synthetic DNA

<400> 52
caggggtgtt cccttgagga ggtggaa 27

<210> 53
<211> 27

<212> DNA
<213> Artificial Sequence

<220>
<223> Description of Artificial Sequence: Synthetic DNA

<400> 53
cactgagcca ggggccacac agcatcc 27

<210> 54
<211> 23
<212> DNA
<213> Artificial Sequence

<220>
<223> Description of Artificial Sequence: Synthetic DNA

<400> 54
cccctcacgc atgaagcctg gag 23

<210> 55
<211> 27
<212> DNA
<213> Artificial Sequence

<220>
<223> Description of Artificial Sequence: Synthetic DNA

<400> 55
tgccaccgtt tcctccataa gcccagc 27

<210> 56
<211> 28
<212> DNA
<213> Artificial Sequence

<220>
<223> Description of Artificial Sequence: Synthetic DNA

<400> 56
atggctcaag ctcccgctaa gtgcccga 28

<210> 57
<211> 27
<212> DNA
<213> Artificial Sequence

<220>
<223> Description of Artificial Sequence: Synthetic DNA

<400> 57
tcaagcgttt gggttggtcc tcatgag 27

<210> 58
<211> 25
<212> DNA
<213> Artificial Sequence

<220>
<223> Description of Artificial Sequence: Synthetic DNA

<400> 58
tccggggatg gcgagatggg caagc 25

<210> 59
<211> 24
<212> DNA
<213> Artificial Sequence

<220>
<223> Description of Artificial Sequence: Synthetic DNA

<400> 59
cttgacatgg ctctgggctc caag 24

<210> 60
<211> 25
<212> DNA
<213> Artificial Sequence

<220>
<223> Description of Artificial Sequence: Synthetic DNA

<400> 60
ccacttcagt cggtcggtag tattt 25

<210> 61
<211> 24
<212> DNA
<213> Artificial Sequence

<220>
<223> Description of Artificial Sequence: Synthetic DNA

<400> 61
cgctcacccg cctgaggcga catg 24

<210> 62
<211> 32
<212> DNA
<213> Artificial Sequence

<220>
<223> Description of Artificial Sequence: Synthetic DNA

<400> 62
ggcaggtgct gtcggtgagg tcaccatagt gc 32

<210> 63
<211> 24
<212> DNA
<213> Artificial Sequence

<220>
<223> Description of Artificial Sequence: Synthetic DNA

<400> 63
ggggccatgc caaggactat gtcg 24

<210> 64
<211> 25
<212> DNA
<213> Artificial Sequence

<220>
<223> Description of Artificial Sequence: Synthetic DNA

<400> 64
atgtggctga tgttacaaaa tgatg 25

<210> 65
<211> 1504
<212> DNA
<213> Cricetulus griseus

<220>
<221> CDS
<222> (1) .. (1119)

<400> 65

```
atg gct cac gct ccc gct agc tgc ccg agc tcc agg aac tct ggg gac      48
Met Ala His Ala Pro Ala Ser Cys Pro Ser Ser Arg Asn Ser Gly Asp
  1               5                  10                  15

ggc gat aag ggc aag ccc agg aag gtg gcg ctc atc acg ggc atc acc      96
Gly Asp Lys Gly Lys Pro Arg Lys Val Ala Leu Ile Thr Gly Ile Thr
             20                  25                  30

ggc cag gat ggc tca tac ttg gca gaa ttc ctg ctg gag aaa gga tac     144
Gly Gln Asp Gly Ser Tyr Leu Ala Glu Phe Leu Leu Glu Lys Gly Tyr
         35                  40                  45

gag gtt cat gga att gta cgg cga tcc agt tca ttt aat aca ggt cga     192
Glu Val His Gly Ile Val Arg Arg Ser Ser Ser Phe Asn Thr Gly Arg
```

```
        50                  55                  60

att gaa cat tta tat aag aat cca cag gct cat att gaa gga aac atg     240
Ile Glu His Leu Tyr Lys Asn Pro Gln Ala His Ile Glu Gly Asn Met
 65                  70                  75                  80

aag ttg cac tat ggt gac ctc acc gac agc acc tgc cta gta aaa atc     288
Lys Leu His Tyr Gly Asp Leu Thr Asp Ser Thr Cys Leu Val Lys Ile
 85                  90                  95                 100

atc aat gaa gtc aaa cct aca gag atc tac aat ctt ggt gcc cag agc     336
Ile Asn Glu Val Lys Pro Thr Glu Ile Tyr Asn Leu Gly Ala Gln Ser
                105                 110                 115

cat gtc aag att tcc ttt gac tta gca gag tac act gca gat gtt gat     384
His Val Lys Ile Ser Phe Asp Leu Ala Glu Tyr Thr Ala Asp Val Asp
            120                 125                 130

gga gtt ggc acc ttg cgg ctt ctg gat gca att aag act tgt ggc ctt     432
Gly Val Gly Thr Leu Arg Leu Leu Asp Ala Ile Lys Thr Cys Gly Leu
        135                 140                 145

ata aat tct gtg aag ttc tac cag gcc tca act agt gaa ctg tat gga     480
Ile Asn Ser Val Lys Phe Tyr Gln Ala Ser Thr Ser Glu Leu Tyr Gly
    150                 155                 160

aaa gtg caa gaa ata ccc cag aaa gag acc acc cct ttc tat cca agg     528
Lys Val Gln Glu Ile Pro Gln Lys Glu Thr Thr Pro Phe Tyr Pro Arg
165                 170                 175                 180

tcg ccc tat gga gca gcc aaa ctt tat gcc tat tgg att gta gtg aac     576
Ser Pro Tyr Gly Ala Ala Lys Leu Tyr Ala Tyr Trp Ile Val Val Asn
                185                 190                 195

ttt cga gag gct tat aat ctc ttt gcg gtg aac ggc att ctc ttc aat     624
Phe Arg Glu Ala Tyr Asn Leu Phe Ala Val Asn Gly Ile Leu Phe Asn
            200                 205                 210

cat gag agt cct aga aga gga gct aat ttt gtt act cga aaa att agc     672
His Glu Ser Pro Arg Arg Gly Ala Asn Phe Val Thr Arg Lys Ile Ser
        215                 220                 225

cgg tca gta gct aag att tac ctt gga caa ctg gaa tgt ttc agt ttg     720
```

```
Arg Ser Val Ala Lys Ile Tyr Leu Gly Gln Leu Glu Cys Phe Ser Leu
    230                 235                 240

gga aat ctg gac gcc aaa cga gac tgg ggc cat gcc aag gac tat gtc     768
Gly Asn Leu Asp Ala Lys Arg Asp Trp Gly His Ala Lys Asp Tyr Val
245                 250                 255                 260

gag gct atg tgg ctg atg tta caa aat gat gaa cca gag gac ttt gtc     816
Glu Ala Met Trp Leu Met Leu Gln Asn Asp Glu Pro Glu Asp Phe Val
                265                 270                 275

ata gct act ggg gaa gtt cat agt gtc cgt gaa ttt gtt gag aaa tca     864
Ile Ala Thr Gly Glu Val His Ser Val Arg Glu Phe Val Glu Lys Ser
            280                 285                 290

ttc atg cac att gga aag acc att gtg tgg gaa gga aag aat gaa aat     912
Phe Met His Ile Gly Lys Thr Ile Val Trp Glu Gly Lys Asn Glu Asn
        295                 300                 305

gaa gtg ggc aga tgt aaa gag acc ggc aaa att cat gtg act gtg gat     960
Glu Val Gly Arg Cys Lys Glu Thr Gly Lys Ile His Val Thr Val Asp
    310                 315                 320

ctg aaa tac tac cga cca act gaa gtg gac ttc ctg cag gga gac tgc     1008
Leu Lys Tyr Tyr Arg Pro Thr Glu Val Asp Phe Leu Gln Gly Asp Cys
325                 330                 335                 340

tcc aag gcg cag cag aaa ctg aac tgg aag ccc cgc gtt gcc ttt gac     1056
Ser Lys Ala Gln Gln Lys Leu Asn Trp Lys Pro Arg Val Ala Phe Asp
                345                 350                 355

gag ctg gtg agg gag atg gtg caa gcc gat gtg gag ctc atg aga acc     1104
Glu Leu Val Arg Glu Met Val Gln Ala Asp Val Glu Leu Met Arg Thr
            360                 365                 370

aac ccc aac gcc tga gcacctctac aaaaaaattc gcgagacatg gactatggtg     1159
Asn Pro Asn Ala
        375

cagagccagc caaccagagt ccagccactc ctgagaccat cgaccataaa ccctcgactg 1219
cctgtgtcgt ccccacagct aagagctggg ccacaggttt gtgggcacca ggacggggac 1279
actccagagc taaggccact tcgcttttgt caaaggctcc tctcaatgat tttgggaaat 1339
caagaagttt aaaatcacat actcatttta cttgaaatta tgtcactaga caacttaaat 1399
```

ttttgagtct tgagattgtt tttctctttt cttattaaat gatctttcta tgacccagca 1459
aaaaaaaaaa aaaaaaggga tataaaaaaa aaaaaaaaaa aaaaa 1504

<210> 66
<211> 25
<212> DNA
<213> Artificial Sequence

<220>
<223> Description of Artificial Sequence: Synthetic DNA

<400> 66
atgaagttgc actatggtga cctca 25

<210> 67
<211> 59
<212> DNA
<213> Cricetulus griseus

<400> 67
ccgacagcac ctgcctagta aaaatcatca atgaagtcaa acctacagag atctacaat 59

<210> 68
<211> 25
<212> DNA
<213> Artificial Sequence

<220>
<223> Description of Artificial Sequence: Synthetic DNA

<400> 68
gacttagcag agtacactgc agatg 25

<210> 69
<211> 25
<212> DNA
<213> Artificial Sequence

<220>
<223> Description of Artificial Sequence: Synthetic DNA

<400> 69
accttggata gaaaggggtg gtctc 25

<210> 70
<211> 125
<212> DNA
<213> Cricetulus griseus

<400> 70

ttgatggagt tggcaccttg cggcttctgg atgcaattaa gacttgtggc cttataaatt 60
ctgtgaagtt ctaccaggcc tcaactagtg aactgtatgg aaaagtgcaa gaaatacccc 120
agaaa 125

<210> 71

<211> 376
<212> PRT
<213> Cricetulus griseus

<400> 71

```
Met Ala His Ala Pro Ala Ser Cys Pro Ser Ser Arg Asn Ser Gly Asp
1               5                   10                  15

Gly Asp Lys Gly Lys Pro Arg Lys Val Ala Leu Ile Thr Gly Ile Thr
            20                  25                  30

Gly Gln Asp Gly Ser Tyr Leu Ala Glu Phe Leu Leu Glu Lys Gly Tyr
        35                  40                  45

Glu Val His Gly Ile Val Arg Arg Ser Ser Ser Phe Asn Thr Gly Arg
    50                  55                  60

Ile Glu His Leu Tyr Lys Asn Pro Gln Ala His Ile Glu Gly Asn Met
65                  70                  75                  80

Lys Leu His Tyr Gly Asp Leu Thr Asp Ser Thr Cys Leu Val Lys Ile
85                  90                  95                  100

Ile Asn Glu Val Lys Pro Thr Glu Ile Tyr Asn Leu Gly Ala Gln Ser
                105                 110                 115
```

```
His Val Lys Ile Ser Phe Asp Leu Ala Glu Tyr Thr Ala Asp Val Asp
            120                 125                 130

Gly Val Gly Thr Leu Arg Leu Leu Asp Ala Ile Lys Thr Cys Gly Leu
        135                 140                 145

Ile Asn Ser Val Lys Phe Tyr Gln Ala Ser Thr Ser Glu Leu Tyr Gly
    150                 155                 160

Lys Val Gln Glu Ile Pro Gln Lys Glu Thr Thr Pro Phe Tyr Pro Arg
165                 170                 175                 180

Ser Pro Tyr Gly Ala Ala Lys Leu Tyr Ala Tyr Trp Ile Val Val Asn
                185                 190                 195

Phe Arg Glu Ala Tyr Asn Leu Phe Ala Val Asn Gly Ile Leu Phe Asn
            200                 205                 210

His Glu Ser Pro Arg Arg Gly Ala Asn Phe Val Thr Arg Lys Ile Ser
        215                 220                 225

Arg Ser Val Ala Lys Ile Tyr Leu Gly Gln Leu Glu Cys Phe Ser Leu
    230                 235                 240

Gly Asn Leu Asp Ala Lys Arg Asp Trp Gly His Ala Lys Asp Tyr Val
245                 250                 255                 260

Glu Ala Met Trp Leu Met Leu Gln Asn Asp Glu Pro Glu Asp Phe Val
                265                 270                 275

Ile Ala Thr Gly Glu Val His Ser Val Arg Glu Phe Val Glu Lys Ser
            280                 285                 290

Phe Met His Ile Gly Lys Thr Ile Val Trp Glu Gly Lys Asn Glu Asn
        295                 300                 305

Glu Val Gly Arg Cys Lys Glu Thr Gly Lys Ile His Val Thr Val Asp
    310                 315                 320

Leu Lys Tyr Tyr Arg Pro Thr Glu Val Asp Phe Leu Gln Gly Asp Cys
325                 330                 335                 340
```

Ser Lys Ala Gln Gln Lys Leu Asn Trp Lys Pro Arg Val Ala Phe Asp
345 350 355

Glu Leu Val Arg Glu Met Val Gln Ala Asp Val Glu Leu Met Arg Thr
360 365 370

Asn Pro Asn Ala
375

<210> 72
<211> 321
<212> PRT
<213> Cricetulus griseus

<400> 72

Met Gly Glu Pro Gln Gly Ser Arg Arg Ile Leu Val Thr Gly Gly Ser
1 5 10 15

Gly Leu Val Gly Arg Ala Ile Gln Lys Val Val Ala Asp Gly Ala Gly
20 25 30

Leu Pro Gly Glu Glu Trp Val Phe Val Ser Ser Lys Asp Ala Asp Leu
35 40 45

Thr Asp Ala Ala Gln Thr Gln Ala Leu Phe Gln Lys Val Gln Pro Thr
50 55 60

His Val Ile His Leu Ala Ala Met Val Gly Gly Leu Phe Arg Asn Ile
65 70 75 80

Lys Tyr Asn Leu Asp Phe Trp Arg Lys Asn Val His Ile Asn Asp Asn
85 90 95

Val Leu His Ser Ala Phe Glu Val Gly Thr Arg Lys Val Val Ser Cys
100 105 110

Leu Ser Thr Cys Ile Phe Pro Asp Lys Thr Thr Tyr Pro Ile Asp Glu
115 120 125

Thr Met Ile His Asn Gly Pro Pro His Ser Ser Asn Phe Gly Tyr Ser
130 135 140

Tyr Ala Lys Arg Met Ile Asp Val Gln Asn Arg Ala Tyr Phe Gln Gln
145 150 155 160

His Gly Cys Thr Phe Thr Ala Val Ile Pro Thr Asn Val Phe Gly Pro
165 170 175

His Asp Asn Phe Asn Ile Glu Asp Gly His Val Leu Pro Gly Leu Ile
180 185 190

His Lys Val His Leu Ala Lys Ser Asn Gly Ser Ala Leu Thr Val Trp
195 200 205

Gly Thr Gly Lys Pro Arg Arg Gln Phe Ile Tyr Ser Leu Asp Leu Ala
210 215 220

Arg Leu Phe Ile Trp Val Leu Arg Glu Tyr Asn Glu Val Glu Pro Ile
225 230 235 240

Ile Leu Ser Val Gly Glu Glu Asp Glu Val Ser Ile Lys Glu Ala Ala
245 250 255

Glu Ala Val Val Glu Ala Met Asp Phe Cys Gly Glu Val Thr Phe Asp
260 265 270

Ser Thr Lys Ser Asp Gly Gln Tyr Lys Lys Thr Ala Ser Asn Gly Lys
275 280 285

Leu Arg Ala Tyr Leu Pro Asp Phe Arg Phe Thr Pro Phe Lys Gln Ala
290 295 300

Val Lys Glu Thr Cys Ala Trp Phe Thr Asp Asn Tyr Glu Gln Ala Arg
305 310 315 320

Lys

<210> 73
<211> 590
<212> PRT
<213> Cricetulus griseus

<400> 73

Met Ala Ser Leu Arg Glu Ala Ser Leu Arg Lys Leu Arg Arg Phe Ser

1 5 10 15

Glu Met Arg Gly Lys Pro Val Ala Thr Gly Lys Phe Trp Asp Val Val
20 25 30

Val Ile Thr Ala Ala Asp Glu Lys Gln Glu Leu Ala Tyr Lys Gln Gln
35 40 45

Leu Ser Glu Lys Leu Lys Arg Lys Glu Leu Pro Leu Gly Val Asn Tyr
50 55 60

His Val Phe Thr Asp Pro Pro Gly Thr Lys Ile Gly Asn Gly Gly Ser
65 70 75 80

Thr Leu Cys Ser Leu Gln Cys Leu Glu Ser Leu Tyr Gly Asp Lys Trp
85 90 95

Asn Ser Phe Thr Val Leu Leu Ile His Ser Gly Gly Tyr Ser Gln Arg
100 105 110

Leu Pro Asn Ala Ser Ala Leu Gly Lys Ile Phe Thr Ala Leu Pro Leu
115 120 125

Gly Glu Pro Ile Tyr Gln Met Leu Asp Leu Lys Leu Ala Met Tyr Met
130 135 140

Asp Phe Pro Ser Arg Met Lys Pro Gly Val Leu Val Thr Cys Ala Asp
145 150 155 160

Asp Ile Glu Leu Tyr Ser Ile Gly Asp Ser Glu Ser Ile Ala Phe Glu
165 170 175

Gln Pro Gly Phe Thr Ala Leu Ala His Pro Ser Ser Leu Ala Val Gly
180 185 190

Thr Thr His Gly Val Phe Val Leu Asp Ser Ala Gly Ser Leu Gln His
195 200 205

Gly Asp Leu Glu Tyr Arg Gln Cys His Arg Phe Leu His Lys Pro Ser
210 215 220

Ile Glu Asn Met His His Phe Asn Ala Val His Arg Leu Gly Ser Phe
225 230 235 240

Gly Gln Gln Asp Leu Ser Gly Gly Asp Thr Thr Cys His Pro Leu His
245 250 255

Ser Glu Tyr Val Tyr Thr Asp Ser Leu Phe Tyr Met Asp His Lys Ser
260 265 270

Ala Lys Lys Leu Leu Asp Phe Tyr Glu Ser Val Gly Pro Leu Asn Cys
275 280 285

Glu Ile Asp Ala Tyr Gly Asp Phe Leu Gln Ala Leu Gly Pro Gly Ala
290 295 300

Thr Ala Glu Tyr Thr Lys Asn Thr Ser His Val Thr Lys Glu Glu Ser
305 310 315 320

His Leu Leu Asp Met Arg Gln Lys Ile Phe His Leu Leu Lys Gly Thr
325 330 335

Pro Leu Asn Val Val Val Leu Asn Asn Ser Arg Phe Tyr His Ile Gly
340 345 350

Thr Thr Glu Glu Tyr Leu Leu His Phe Thr Ser Asn Gly Ser Leu Gln
355 360 365

Ala Glu Leu Gly Leu Gln Ser Ile Ala Phe Ser Val Phe Pro Asn Val
370 375 380

Pro Glu Asp Ser His Glu Lys Pro Cys Val Ile His Ser Ile Leu Asn
385 390 395 400

Ser Gly Cys Cys Val Ala Pro Gly Ser Val Val Glu Tyr Ser Arg Leu
405 410 415

Gly Pro Glu Val Ser Ile Ser Glu Asn Cys Ile Ile Ser Gly Ser Val
420 425 430

Ile Glu Lys Ala Val Leu Pro Pro Cys Ser Phe Val Cys Ser Leu Ser
435 440 445

Val Glu Ile Asn Gly His Leu Glu Tyr Ser Thr Met Val Phe Gly Met
450 455 460

```
Glu Asp Asn Leu Lys Asn Ser Val Lys Thr Ile Ser Asp Ile Lys Met
465                 470                 475                 480

Leu Gln Phe Phe Gly Val Cys Phe Leu Thr Cys Leu Asp Ile Trp Asn
                485                 490                 495

Leu Lys Ala Met Glu Glu Leu Phe Ser Gly Ser Lys Thr Gln Leu Ser
            500                 505                 510

Leu Trp Thr Ala Arg Ile Phe Pro Val Cys Ser Ser Leu Ser Glu Ser
        515                 520                 525

Val Ala Ala Ser Leu Gly Met Leu Asn Ala Ile Arg Asn His Ser Pro
    530                 535                 540

Phe Ser Leu Ser Asn Phe Lys Leu Leu Ser Ile Gln Glu Met Leu Leu
545                 550                 555                 560

Cys Lys Asp Val Gly Asp Met Leu Ala Tyr Arg Glu Gln Leu Phe Leu
                565                 570                 575

Glu Ile Ser Ser Lys Arg Lys Gln Ser Asp Ser Glu Lys Ser
            580                 585                 590
```

## Description

### TECHNICAL FIELD

Described herein is a cell for the production of an antibody molecule such as an antibody useful for various diseases, a fragment of the antibody and a fusion protein having the Fc region of the antibody or the like, a process for producing an antibody composition using the cell, the antibody composition and use thereof.

### BACKGROUND ART

Since antibodies have high binding activity, binding specificity and high stability in blood, their applications to diagnosis, prevention and treatment of various human diseases have been attempted [Monoclonal Antibodies: Principles and Applications, Wiley-Liss, Inc., Chapter 2.1 (1995)]. Also, production of a humanized antibody such as a human chimeric antibody or a human complementarity determining region (hereinafter referred to as "CDR")-grafted antibody from an antibody derived from an animal other than human have been attempted by using genetic recombination techniques. The human chimeric antibody is an antibody in which its antibody variable region (hereinafter referred to as "V region") is an antibody derived from an animal other than human and its constant region (hereinafter referred to as "C region") is derived from a human antibody. The human CDR-grafted antibody is an antibody in which the CDR of a human antibody is replaced by CDR of an antibody derived from an animal other than human.

It has been revealed that five classes, namely IgM, IgD, IgG, IgA and IgE, are present in antibodies derived from mammals. Antibodies of human IgG class are mainly used for the diagnosis, prevention and treatment of various human diseases because they have functional characteristics such as long half-life in blood, various effector functions and the like [Monoclonal Antibodies: Principles and Applications, Wiley-Liss, Inc., Chapter 1 (1995)]. The human IgG class antibody is further classified into the following 4 subclasses: IgG1, IgG2, IgG3 and IgG4. A large number of studies have so far been conducted for antibody-dependent cell-mediated cytotoxic activity (hereinafter referred to as "ADCC activity") and complement-dependent cytotoxic activity (hereinafter referred to as "CDC activity") as effector functions of the IgG class antibody, and it has been reported that among antibodies of the human IgG class, the IgG1 subclass has the highest ADCC activity and CDC activity [Chemical Immunology, 65, 88 (1997)]. In view of the above, most of the anti-tumor humanized antibodies, including commercially available Rituxan and Herceptin, which require high effector functions for the expression of their effects, are antibodies of the human IgG1 subclass.

Expression of ADCC activity and CDC activity of the human IgG1 subclass antibodies requires binding of the Fc region of the antibody to an antibody receptor existing on the surface of an effector cell, such as a killer cell, a natural killer cell, an activated macrophage or the like (hereinafter referred to as "Fc$\gamma$R") and various complement components are bound. Regarding the binding, it has been suggested that several amino acid residues in the hinge region and the second domain of C region (hereinafter referred to as "C$\gamma$2 domain") of the antibody are important [Eur. J. Immunol., 23, 1098 (1993), Immunology, 86, 319 (1995), Chemical Immunology, 65, 88 (1997)] and that a sugar chain binding to the C$\gamma$2 domain [Chemical Immunology, 65, 88 (1997)] is also important.

Regarding the sugar chain, Boyd *et al.* have examined effects of a sugar chain on the ADCC activity and CDC activity by treating a human CDR-grafted antibody CAMPATH-1H (human IgG1 subclass) produced by a Chinese hamster ovary cell (CHO cell) or a mouse myeloma NS0 cell (NS0 cell) with various sugar hydrolyzing enzymes, and reported that elimination of the non-reducing end sialic acid did not have influence upon both activities, but the CDC activity alone was affected by further removal of galactose residue and about 50% of the activity was decreased, and that complete removal of the sugar chain caused disappearance of both activities [Molecular Immunol., 32, 1311 (1995)]. Also, Lifely *et al.* have analyzed the sugar chain bound to a human CDR-grafted antibody CAMPATH-1H (human IgG1 subclass) which was produced by CHO cell, NS0 cell or rat myeloma YO cell, measured its ADCC activity, and reported that the CAMPATH-1H derived from YO cell showed the highest ADCC activity, suggesting that *N*-acetylglucosamine (hereinafter referred also to as "GlcNAc") at the bisecting position is important for the activity *[*Glycobiology, 5, 813 (1995); WO 99/54342]. These reports indicate that the structure of the sugar chain plays an important role in the effector functions of human antibodies of IgG1 subclass and that it is possible to prepare an antibody having more higher effector function by changing the structure of the sugar chain. However, actually, structures of sugar chains are various and complex, and it cannot be said that an actual important structure for the effector function was identified.

Sugar chains of glycoproteins are roughly divided into two types, namely a sugar chain which binds to asparagine (*N*-glycoside-linked sugar chain) and a sugar chain which binds to other amino acid such as serine, threonine (*O*-glycoside-linked sugar chain), based on the binding form to the protein moiety. The *N*-glycoside-linked sugar chains have various structures [Biochemical Experimentation Method 23 - Method for Studying Glycoprotein Sugar Chain (Gakujutsu Shuppan Center), edited by Reiko Takahashi (1989)], but it is known that they have a basic common core structure shown by the following structural formula (I).

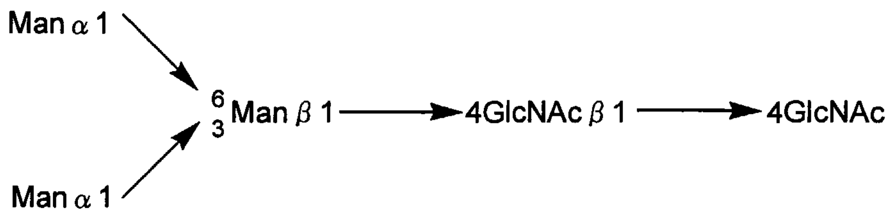

Structural formula (I)

The sugar chain terminus which binds to asparagine is called a reducing end, and the opposite side is called a non-reducing end. It is known that the *N*-glycoside-linked sugar chain includes a high mannose type in which mannose alone binds to the non-reducing end of the core structure; a complex type in which the non-reducing end side of the core structure has at least one parallel branches of galactose-*N*-acetylglucosamine (hereinafter referred to as "Gal-GlcNAc") and the non-reducing end side of Gal-GlcNAc has a structure of sialic acid, bisecting *N*-acetylglucosamine or the like; a hybrid type in which the non-reducing end side of the core structure has branches of both of the high mannose type and complex type; and the like.

In the Fc region of an antibody of an IgG type, two *N*-glycoside-linked sugar chain binding sites are present. In serum IgG, to the sugar chain binding site, generally, binds a complex type sugar chain having plural branches and in which addition of sialic acid or bisecting *N*-acetylglucosamine is low. It is known that there is variety regarding the addition of galactose to the non-reducing end of the complex type sugar chain and the addition of fucose to the *N*-acetylglucosamine in the reducing end *[*Biochemistry, 36, 130 (1997)].

It has been considered that such a structure of a sugar chain is determined by sugar chain genes, namely a gene for a glycosyltransferase which synthesizes a sugar chain and a gene for a glycolytic enzyme which hydrolyzes the sugar chain.

Synthesis of an *N*-glycoside-linked sugar chain is described below.

Glycoproteins are modified with a sugar chain in the endoplasmic reticulum (hereinafter referred to as "ER") lumen. During the biosynthesis step of the *N*-glycoside-linked sugar chain, a relatively large sugar chain is transferred to the polypeptide chain which is elongating in the ER lumen. In the transformation, the sugar chain is firstly added in succession to phosphate groups of a long chain lipid carrier comprising about 20 α-isoprene units, which is called dolichol phosphate (hereinafter referred also to as "P-Dol"). That is, *N*-acetylglucosamine is transferred to dolichol phosphate to thereby form GlcNAc-P-P-Dol and then one more GleNAc is transferred to form GlcNAc-GlcNAc-P-P-Dol. Next, five mannoses (hereinafter mannose is also referred to as "Man") are transferred to thereby form $(Man)_5$-$(GlcNAc)_2$-P-P-Dol and then four Man's and three glucoses (hereinafter glucose is also referred to as "Glc") are transferred. Thus, a sugar chain precursor, $(Glc)_3$-$(Man)_9$-$(GlcNAc)_2$-P-P-Dol, called core oligosaccharide is formed. The sugar chain precursor comprising 14 sugars is transferred as a mass to a polypeptide having an asparagine-X-serine or asparagine-X-threonine sequence in the ER lumen. In the reaction, dolichol pyrophosphate (P-P-Dol) bound to the core oligosaccharide is released but again becomes dolichol phosphate by hydrolysis with pyrophosphatase and is recycled. Trimming of the sugar chain immediately starts after the sugar chain binds to the polypeptide. That is, 3 Glc's and 1 or 2 Man's are eliminated on the ER, and it is known that α-1,2-glucosidase I, α-1,3-glucosidase II and α-1,2-mannosidase relates to the elimination.

The glycoprotein which was subjected to trimming on the ER is transferred to the Golgi body and are variously modified. In the *cis* part of the Golgi body, *N*-acetylglucosamine phosphotransferase which relates to addition of mannose phosphate, *N*-acetylglucosamine 1-phosphodiester α-*N*-acetylglucosaminidase and α-mannosidase I are present and reduce the Man residues to 5. In the medium part of the Golgi body, *N*-acetylglucosamine transferase I (GnTI) which relates to addition of the first outside GlcNAc of the complex type *N*-glycoside-linked sugar chain, α-mannosidase II which relates to elimination of 2 Man's, *N*-acetylglucosamine transferase II (GnTII) which relates to addition of the second GlcNAc from the outside and α-1,6-fucosyltransferase which relates to addition of fucose to the reducing end *N*-acetylglucosamine are present. In the *trans* part of the Golgi body, galactose transferase which relates to addition of galactose and sialyltransferase which relates to addition of sialic acid such as *N*-acetylneuraminic acid or the like are present. It is known that *N*-glycoside-linked sugar chain is formed by activities of these various enzymes.

In general, most of the humanized antibodies of which application to medicaments is in consideration are prepared using genetic recombination techniques and produced using Chinese hamster ovary tissue-derived CHO cell as the host cell. But as described above, since the sugar chain structure plays a remarkably important role in the effector function of antibodies and differences are observed in the sugar chain structure of glycoproteins expressed by host cells, development of a host cell which can be used for the production of an antibody having higher effector function is desired.

In order to modify the sugar chain structure of the produced glycoprotein, various methods have been attempted, such as 1) application of an inhibitor against an enzyme relating to the modification of a sugar chain, 2) selection of a

mutant, 3) introduction of a gene encoding an enzyme relating to the modification of a sugar chain, and the like. Specific examples are described below.

Examples of an inhibitor against an enzyme relating to the modification of a sugar chain include tunicamycin which selectively inhibits formation of GlcNAc-P-P-Dol which is the first step of the formation of a core oligosaccharide which is a precursor of an *N*-glycoside-linked sugar chain, castanospermin and *N*-methyl-1-deoxynojirimycin which are inhibitors of glycosidase I, bromocondulitol which is an inhibitor of glycosidase II, 1-deoxynojirimycin and 1,4-dioxy-1,4-imino-D-mannitol which are inhibitors of mannosidase I, swainsonine which is an inhibitor of mannosidase II and the like. Examples of an inhibitor specific for a glycosyltransferase include deoxy derivatives of substrates against *N*-acetylglucosamine transferase V (GnTV) and the like [Glycobiology Series 2 - Destiny of Sugar Chain in Cell (Kodan-sha Scientific), edited by Katsutaka Nagai, Senichiro Hakomori and Akira Kobata (1993)]. Also, it is known that 1-deoxynojirimycin inhibits synthesis of a complex type sugar chain and increases the ratio of high mannose type and hybrid type sugar chains. Actually, it has been reported that sugar chain structure of IgG was changed and properties such as antigen binding activity and the like was changed when the inhibitors were added to a medium [Molecular Immunol., 26, 1113 (1989)].

Mutants regarding the activity of an enzyme relating to the modification of a sugar chain are mainly selected and obtained as a lectin-resistant cell line. For example, CHO cell mutants having various sugar chain structures have been obtained as a lectin-resistant cell line using a lectin such as WGA (wheat-germ agglutinin derived from T. *vulgaris*), ConA (cocanavalin A derived from *C. ensiformis*), RIC (a toxin derived from R. *communis*), L-PHA (leucoagglutinin derived from *P. vulgaris*), LCA (lentil agglutinin derived from *L. culinaris*), PSA (pea lectin derived from *P. sativum*) or the like [Somatic Cell Mol. Genet., 12, 51 (1986)].

As an example of the modification of the sugar chain structure of a product obtained by introducing the gene of an enzyme relating to the modification of a sugar chain into a host cell, it has been reported that a protein in which a number of sialic acid is added to the non-reducing end of the sugar chain can be produced by introducing rat β-galactoside-α-2,6-sialyltransferase into CHO cell [J. Biol. Chem., 261, 13848 (1989)].

Also, it was confirmed that an H antigen (Fucα1-2Ga1β1-) in which fucose (hereinafter also referred to as "Fuc") was added to the non-reducing end of the sugar chain was expressed by introducing human β-galactoside-2-α-fucosyltransferase into mouse L cell [Science, 252, 1668 (1991)]. In addition, based on knowledge that addition of the bisecting-positioned *N*-acetylglucosamine of *N*-glycoside-linked sugar chain is important for the ADCC activity of antibody, *Umana et al.* have prepared CHO cell which expresses β-1,4-*N*-acetylglucosamine transferase III (GnTIII) and compared it with the parent cell line on the expression of GnTIII. It was confirmed that express of GnTIII was not observed in the parent cell line of CHO cell [J. Biol. Chem., 261, 13370 (1984)], and that the antibody expressed using the produced GnTIII expressing CHO cell had ADCC activity 16 times higher than the antibody expressed using the parent cell line [Glycobiology, 5, 813 (1995): WO 99/54342]. At this time, *Umana et al.* have also produced CHO cell into which β-1,4-*N*-acetylglucosamine transferase V (GnTV) was introduced and reported that excess expression of GnTIII or GnTV shows toxicity for CHO cell.

## DISCLOSURE OF THE INVENTION

Thus, in order to modify the sugar chain structure of the glycoprotein to be produced, control of the activity of the enzyme relating to the modification of a sugar chain in the host cell has been attempted, but actually, the structures of sugar chains are various and complex, and solution of the physiological roles of sugar chains would be insufficient, so that trial and error are repeated. Particularly, although it has been revealed little by little that the effector function of antibodies is greatly influenced by the sugar chain structure, a truly important sugar chain structure has not been specified yet. Accordingly, identification of a sugar chain which has influence upon the effector function of antibodies and development of a host cell to which such a sugar chain structure can be added are expected for developing medicaments.

An object of the present invention is to provide a host cell which produces an antibody composition and can control a sugar chain structure bound to an antibody molecule, a cell which can produce an antibody composition having high ADCC activity, a production method of an antibody composition using the cell and an antibody composition produced by the production method.

The present invention relates to the following (1) to (61).

(1) A Chinese hamster ovary tissue-derived CHO cell into which a gene encoding an antibody molecule is introduced, which produces an antibody composition comprising an antibody molecule having complex *N*-glycoside-linked sugar chains bound to the Fc region, wherein among the total complex *N*-glycoside-linked sugar chains bound to the Fc was expressed by introducing human β-galactoside-2-α-fucosyltransferase into mouse L cell [Science, 252, 1668 (1991)]. In addition, based on knowledge that addition of the bisecting-positioned *N*-acetylglucosamine of *N*-glycoside-linked sugar chain is important for the ADCC activity of antibody, *Umana et al.* have prepared CHO cell which expresses β-1,4-*N*-acetylglucosamine transferase III (GnTIII) and compared it with the parent cell line on the expression of GnTIII. It was confirmed that express of GnTIII was not observed in the parent cell line of CHO cell [J. Biol Chem., 261. 13370 (1984)], and that the antibody expressed using the produced GnTIII expressing CHO cell had ADCC activity 16 times higher than the antibody expressed using the parent cell line [Glycobiology, 5, 813 (1995): WO 99/54342]. At this time, *Umana et al.* have also produced CHO cell into which β-1,4-*N*-acetylglucosamine transferase V (GnTV) was introduced and reported that excess expression of GnTIII or GnTV shows toxicity for CHO cell.

DISCLOSURE OF THE INVENTION

Thus, in order to modify the sugar chain structure of the glycoprotein to be produced, control of the activity of the enzyme relating to the modification of a sugar chain in the host cell has been attempted, but actually, the structures of sugar chains are various and complex, and solution of the physiological roles of sugar chains would be insufficient, so that trial and error are repeated. Particularly, although it has been revealed little by little that the effector function of antibodies is greatly influenced by the sugar chain structure, a truly important sugar chain structure has not been specified yet. Accordingly, identification of a sugar chain which has influence upon the effector function of antibodies and development of a host cell to which such a sugar chain structure can be added are expected for developing medicaments.

An object of the present invention is to provide a host cell which produces an antibody composition and can control a sugar chain structure bound to an antibody molecule, a cell which can produce an antibody composition having high ADCC activity, a production method of an antibody composition using the cell and an antibody composition produced by the production method.

The present invention relates to the following (1) to (22).

[1]. A cell in which

(i) the activity of an enzyme relating to the synthesis of GDP-fucose is deleted, or
(ii) the activity of an enzyme relating to the synthesis of GDP-fucose and the activity of α1,6-fucosyltransferase are deleted,

by a genetic engineering technique, wherein said enzyme relating to the synthesis of GDP-fucose is an enzyme selected from the group consisting of the following (a), (b) and (c):

(a) GMD (GDP-mannose 4,6-dehydratase);
(b) Fx (GDP-keto-6-deoxymannose 3,5-epimerase, 4-reductase);.
(c) GFPP (GDP-beta-L-fucose pyrophosphorylase),

and wherein the genetic engineering technique is a technique selected from the group consisting of the following (A), (B), (C) and (D):

(A) a gene disruption technique targeting a gene encoding the enzyme;
(B) a technique for introducing a dominant negative mutant of a gene encoding the enzyme;
(C) a technique for introducing mutation into the enzyme;
(D) a technique for inhibiting transcription and/or translation of a gene encoding the enzyme.

[2]. A transgenic non-human animal or plant or the progenies thereof, comprising a genome which is modified such that the activity of an enzyme relating to the synthesis of GDP-fucose and the activity of α1,6-fucosyltransferase are decreased, by a genetic engineering technique, wherein said enzyme relating to the synthesis of GDP-fucose is an enzyme selected from the group consisting of the following (a), (b) and (c):

(a) GMD (GDP-mannose 4,6-dehydratase);
(b) Fx (GDP-keto-6-deoxymannose 3,5-epimerase, 4-reductase);
(c) GFPP (GDP-beta-L-fucose pyrophosphorylase),

and wherein the genetic engineering technique is a technique selected from the group consisting of the following (A), (B), (C) and (D):

(A) a gene disruption technique targeting a gene encoding the enzyme;
(B) a technique for introducing a dominant negative mutant of a gene encoding the enzyme;
(C) a technique for introducing mutation into the enzyme;

(D) a technique for inhibiting transcription and/or translation of a gene encoding the enzyme

wherein in said transgenic non-human animal or plant or progenies thereof a gene encoding an antibody has been introduced and the transgenic non-human animal or plant or progenies thereof is capable of producing an antibody composition in which fucose is not bound to N-acetylglucosamine at the reducing end of the sugar chain in 20% or more among the total complex N-glycoside-linked sugar chains binding to the Fc region contained in the antibody composition.

[3]. The transgenic non-human animal or plant or the progenies thereof according to [2], comprising a genome which is modified such that a gene encoding an enzyme relating to the synthesis of GDP-fucose or $\alpha$1,6-fucosyltransferase is knocked out.

[4]. The cell according to [1] or the transgenic non-human animal or plant or the progenies thereof according to [2] or [3], wherein the GMD is a protein encoded by a DNA of the following (a) or (b):

(a) a DNA comprising the nucleotide sequence represented by SEQ ID NO:65;
(b) a DNA which hybridizes with the DNA comprising the nucleotide sequence represented by SEQ ID NO:65 under stringent conditions and encodes a protein having GMD activity.

[5]. The cell according to [1] or the transgenic non-human animal or plant or the progenies thereof according to [2] or [3], wherein the GMD is a protein selected from the group consisting of the following (a), (b) and (c):

(a) a protein comprising the amino acid sequence represented by SEQ ID NO:71;
(b) a protein which comprises an amino acid sequence in which 1 to 20 amino acids are deleted, substituted, inserted and/or added in the amino acid sequence represented by SEQ ID NO:71 and has GMD activity;
(c) a protein which comprises an amino acid sequence having a homology of at least 80 % with the amino acid sequence represented by SEQ ID NO:71 and has GMD activity.

[6]. The cell according to [1] or the transgenic non-human animal or plant or the progenies thereof according to [2] or [3], wherein the Fx is a protein encoded by a DNA of the following (a) or (b):

(a) a DNA comprising the nucleotide sequence represented by SEQ ID NO:48;
(b) a DNA which hybridizes with the DNA comprising the nucleotide sequence represented by SEQ ID NO:48 under stringent conditions and encodes a protein having Fx activity.

[7]. The cell according to [1] or the transgenic non-human animal or plant or the progenies thereof according to [2] or [3], wherein the Fx is a protein selected from the group consisting of the following (a), (b) and (c):

(a) a protein comprising the amino acid sequence represented by SEQ ID NO:72;
(b) a protein which comprises an amino acid sequence in which 1 to 20 amino acids are deleted, substituted, inserted and/or added in the amino acid sequence represented by SEQ ID NO:72 and has Fx activity.
(c) a protein which comprises an amino acid sequence having a homology of at least 80 % with the amino acid sequence represented by SEQ ID NO:72 and has Fx activity.

[8]. The cell according to [1] or the transgenic non-human animal or plant or the progenies thereof according to [2] or [3], wherein the GFPP is a protein encoded by a DNA of the following (a) or (b):

(a) a DNA comprising the nucleotide sequence represented by SEQ ID NO:51;
(b) a DNA which hybridizes with the DNA comprising the nucleotide sequence represented by SEQ ID NO:51 under stringent conditions and encodes a protein having GFPP activity.

[9]. The cell according to [1] or the transgenic non-human animal or plant or the progenies thereof according to [2] or [3], wherein the GFPP is a protein selected from the group consisting of the following (a), (b) and (c):

(a) a protein comprising the amino acid sequence represented by SEQ ID NO:73;
(b) a protein which comprises an amino acid sequence in which 1 to 20 amino acids are deleted, substituted, inserted and/or added in the amino acid sequence represented by SEQ ID NO:73 and has GFPP activity;
(c) a protein which comprises an amino acid sequence having a homology of at least 80 % with the amino acid

sequence represented by SEQ ID NO:73 and has GFPP activity.

[10]. The cell, transgenic non-human animal or plant or the progenies thereof according to any one of [1] to [3], wherein the α1,6-fucosyltransferase is a protein encoded by a DNA selected from the group consisting of the following (a), (b), (c) and (d):

(a) a DNA comprising the nucleotide sequence represented by SEQ ID NO:1;
(b) a DNA comprising the nucelotide sequence represented by SEQ ID NO:2;
(c) a DNA which hybridizes with the DNA comprising the nucleotide sequence represented by SEQ ID NO:1 under stringent conditions and encodes a protein having α1,6-fucosyltransferase activity;
(d) a DNA which hybridizes with the DNA comprising the nucleotide sequence represented by SEQ ID NO:2 under stringent conditions and encodes a protein having α1,6-fucosyltransferase activity.

[11]. The cell, transgenic non-human animal or plant or the progenies thereof according to any one of [1] to [3], wherein the α1,6-fucosyltransferase is a protein selected from the group consisting of the following (a), (b), (c), (d), (e) and (f):

(a) a protein comprising the amino acid sequence represented by SEQ ID:23;
(b) a protein comprising the amino acid sequence represented by SEQ ID:24;
(c) a protein which comprises an amino acid sequence in which 1 to 20 amino acids are deleted, substituted, inserted and/or added in the amino acid sequence represented by SEQ ID NO:23 and has a α1,6-fucosyltransferase activity;
(d) a protein which comprises an amino acid sequence in which 1 to 20 amino acids are deleted, substituted, inserted and/or added in the amino acid sequence represented by SEQ ID NO:24 and has a α1,6-fucosyltransferase activity;
(e) a protein which comprises an amino acid sequence having a homology of at least 80 % with the amino acid sequence represented by SEQ ID NO:23 and has a α1,6-fucosyltransferase activity;
(f) a protein which comprises an amino acid sequence having a homology of at least 80 % with the amino acid sequence represented by SEQ ID NO:24 and has a α1,6-fucosyltransferase activity.

[12]. The cell according to any one of [1] and [4] to [11], which is resistant to at least a lectin which recognizes a sugar chain in which 1-position of fucose is bound to 6-position of N-acetylglucosamine in the reducing end through α-bond in the complex N-glycoside-linked sugar chain.

[13]. The cell according to any one of [1] and [4] to [12], which is a cell selected from the group consisting of the following (a) to (i):

(a) a CHO cell derived from a Chinese hamster ovary tissue;
(b) a rat myeloma cell line, YB2/3HL.P2.G11.16.AG.20 cell;
(c) a mouse myeloma cell line, NS0 cell;
(d) a mouse myeloma cell line, SP2/0-Ag14 cell;
(e) a BHK cell derived from a syrian hamster kidney tissue;
(f) an antibody-producing hybridoma cell;
(g) a human leukemia cell line Namalwa cell;
(h) a non-human embryonic stem cell;
(i) a non-human fertilized egg cell.

[14]. The cell according to any one of [1] or [4] to [13] into which a gene encoding an antibody molecule is introduced.

[15]. The cell according to [14], wherein the antibody molecule belongs to an IgG class.

[16]. A process for producing an antibody composition, which comprises culturing the cell according to [14] or [15] in a medium to produce and accumulate the antibody composition in the culture; and recovering the antibody composition from the culture.

[17]. The process according to [16], which produces an antibody composition having higher antibody-dependent cell-mediated cytotoxic activity than an antibody composition obtained from its parent cell line.

[18]. The transgenic non-human animal or plant or the progenies thereof according to any one of [2] to [11], wherein the transgenic non-human animal is an animal selected from the group consisting of cattle, sheep, goat, pig, horse, mouse, rat, fowl, monkey and rabbit.

[19]. A process for producing an antibody composition, which comprises
recovering an antibody composition from tissue or body fluid comprising the antibody, which tissue or body fluid has been isolated from a transgenic non-human animal or plant or the progenies thereof according to any one of [2] to [11] and [18], into which a gene encoding an antibody molecule has been introduced.

[20]. The process according to [19], wherein the antibody molecule belongs to an IgG class.

[21]. The process according to [19] or [20], which produces an antibody composition having higher antibody-dependent cell-mediated cytotoxic activity than an antibody composition obtained from a non-human animal or plant or the progenies thereof whose genome is not modified.

[22]. The cell according to any one of [1] or [4] to [15] or the transgenic non-human animal or plant or the progenies thereof according to any one of [2] to [11] or [18], wherein said technique according to (D) is targeting a gene of an enzyme relating to the synthesis of GDP-fucose or a gene of α1,6-fucosyltransferase with an RNAi gene construct, wherein said enzyme relating to the synthesis of GDP-fucose is an enzyme selected from the group consisting of the following (a), (b) and (c):

(a) GMD (GDP-mannose 4,6-dehydratase);
(b) Fx (GDP-keto-6-deoxymannose 3,5-epimerase, 4-reductase);
(c) GFPP (GDP-beta-L-fucose pyrophosphorylase).

Also disclosed herein are the following (1) to (61):

(1) A Chinese hamster ovary tissue-derived CHO cell into which a gene encoding an antibody molecule is introduced, which produces an antibody composition comprising an antibody molecule having complex *N*-glycoside-linked sugar chains bound to the Fc region, wherein among the total complex *N*-glycoside-linked sugar chains bound to the Fc region in the composition, the ratio of a sugar chain in which fucose is not bound to *N*-acetylglucosamine in the reducing end in the sugar chain is 20% or more.
(2) The CHO cell according to (1), wherein the sugar chain to which fucose is not bound is a complex *N*-glycoside-linked sugar chain in which 1-position of fucose is not bound to 6-position of *N*-acetylglucosamine in the reducing end through α-bond.
(3) The CHO cell according to (1) or (2), wherein the antibody molecule belongs to an IgG class.
(4) The CHO cell according to any one of (1) to (3), wherein the activity of an enzyme relating to the synthesis of an intracellular sugar nucleotide, GDP-fucose and/or the activity of an enzyme relating to the modification of a sugar chain in which 1-position of fucose is bound to 6-position of *N*-acetylglucosamine in the reducing end through α-bond in the complex *N*-glycoside-linked sugar chain is decreased or deleted.
(5) The CHO cell according to (4), wherein the enzyme relating to the synthesis of an intracellular sugar nucleotide, GDP-fucose is an enzyme selected from the group consisting of the following (a), (b) and (c):

(a) GMD (GDP-mannose 4,6-dehydratase);
(b) Fx (GDP-keto-6-deoxymannose 3,5-epimerase, 4-reductase);
(c) GFPP (GDP-beta-L-fucose pyrophosphorylase).

(6) The CHO cell according to (5), wherein the GMD is a protein encoded by a DNA of the following (a) or (b):

(a) a DNA comprising the nucleotide sequence represented by SEQ ID NO:65;
(b) a DNA which hybridizes with the DNA comprising the nucleotide sequence represented by SEQ ID NO:65 under stringent conditions and encodes a protein having GMD activity.

(7) The CHO cell according to (5), wherein the GMD is a protein selected from the group consisting of the following (a), (b) and (c):

(a) a protein comprising the amino acid sequence represented by SEQ ID NO:71;
(b) a protein which comprises an amino acid sequence in which at least one amino acid is deleted, substituted,

inserted and/or added in the amino acid sequence represented by SEQ ID NO:71 and has GMD activity;
(c) a protein which comprises an amino acid sequence having a homology of at least 80% with the amino acid sequence represented by SEQ ID NO:71 and has GMD activity.

(8) The CHO cell according to (5), wherein the Fx is a protein encoded by a DNA of the following (a) or (b):

(a) a DNA comprising the nucleotide sequence represented by SEQ ID NO:48;
(b) a DNA which hybridizes with the DNA comprising the nucleotide sequence represented by SEQ ID NO:48 under stringent conditions and encodes a protein having Fx activity.

(9) The CHO cell according to (5), wherein the Fx is a protein selected from the group consisting of the following (a), (b) and (c):

(a) a protein comprising the amino acid sequence represented by SEQ ID NO:72;
(b) a protein which comprises an amino acid sequence in which at least one amino acid is deleted, substituted, inserted and/or added in the amino acid sequence represented by SEQ ID NO:72 and has Fx activity;
(c) a protein which comprises an amino acid sequence having a homology of at least 80% with the amino acid sequence represented by SEQ ID NO:72 and has Fx activity.

(10) The CHO cell according to (5), wherein the GFPP is a protein encoded by a DNA of the following (a) or (b):

(a) a DNA comprising the nucleotide sequence represented by SEQ ID NO:51;
(b) a DNA which hybridizes with the DNA comprising the nucleotide sequence represented by SEQ ID NO:51 under stringent conditions and encodes a protein having GFPP activity.

(11) The CHO cell according to (5), wherein the GFPP is a protein selected from the group consisting of the following (a), (b) and (c):

(a) a protein comprising the amino acid sequence represented by SEQ ID NO:73;
(b) a protein which comprises an amino acid sequence in which at least one amino acid is deleted, substituted, inserted and/or added in the amino acid sequence represented by SEQ ID NO:73 and has GFPP activity;
(c) a protein which comprises an amino acid sequence having a homology of at least 80% with the amino acid sequence represented by SEQ ID NO:73 and has GFPP activity.

(12) The CHO cell according to (4), wherein the enzyme relating to the modification of a sugar chain in which 1-position of fucose is bound to 6-position of the *N*-acetylglucosamine in the reducing end through $\alpha$-bond in the complex *N*-glycoside-linked sugar chain is $\alpha$-1,6-fucosyltransferase.
(13) The CHO cell according to (12), wherein the $\alpha$-1,6-fucosyltransferase is a protein encoded by a DNA of the following (a) or (b):

(a) a DNA comprising the nucleotide sequence represented by SEQ ID NO:1;
(b) a DNA which hybridizes with the DNA comprising the nucleotide sequence represented by SEQ ID NO:1 under stringent conditions and encodes a protein having $\alpha$-1,6-fucosyltransferase activity.

(14) The CHO cell according to (12), wherein the $\alpha$-1,6-fucosyltransferase is a protein selected from the group consisting of the following (a), (b) and (c):

(a) a protein comprising the amino acid sequence represented by SEQ ID NO:23;
(b) a protein which comprises an amino acid sequence in which at least one amino acid is deleted, substituted, inserted and/or added in the amino acid sequence represented by SEQ ID NO:23 and has $\alpha$-1,6-fucosyltransferase activity;
(c) a protein which comprises an amino acid sequence having a homology of at least 80% with the amino acid sequence represented by SEQ ID NO:23 and has $\alpha$-1,6-fucosyltransferase activity.

(15) The CHO cell according to any one of (4) to (14), wherein the enzyme activity is decreased or deleted by a technique selected from the group consisting of the following (a), (b), (c), (d) and (e):

(a) a gene disruption technique targeting a gene encoding the enzyme;

(b) a technique for introducing a dominant negative mutant of a gene encoding the enzyme;
(c) a technique for introducing mutation into the enzyme;
(d) a technique for inhibiting transcription or translation of a gene encoding the enzyme;
(e) a technique for selecting a cell line resistant to a lectin which recognizes a sugar chain in which 1-position of fucose is bound to 6-position of *N*-acetylglucosamine in the reducing end through α-bond in the complex *N*-glycoside-linked sugar chain.

(16) The CHO cell according to any one of (4) to (15), which is resistant to at least a lectin which recognizes a sugar chain in which 1-position of fucose is bound to 6-position of *N*-acetylglucosamine in the reducing end through α-bond in the complex *N*-glycoside-linked sugar chain.
(17) The CHO cell according to any one of (4) to (16), which produces an antibody composition having higher antibody-dependent cell-mediated cytotoxic activity than an antibody composition produced by its parent CHO cell.
(18) The CHO cell according to (17), which produces an antibody composition having higher antibody-dependent cell-mediated cytotoxic activity than an antibody composition in which among the total complex *N*-glycoside-linked sugar chains bound to the Fc region contained in the antibody composition, the ratio of a sugar chain in which fucose is not bound to *N*-acetylglucosamine in the reducing end in the sugar chain is less than 20%.
(19) The CHO cell according to (18), wherein the sugar chain to which fucose is not bound is a complex *N*-glycoside-linked sugar chain in which 1-position of fucose is not bound to 6-position of *N*-acetylglucosamine in the reducing end through α-bond.
(20) A process for producing an antibody composition, which comprises culturing the CHO cell according to any one of (1) to (19) in a medium to produce and accumulate an antibody composition in the culture; and recovering the antibody composition from the culture.
(21) An antibody composition which is produced using the method according to (20).
(22) An antibody composition which comprises an antibody molecule having complex *N*-glycoside-linked sugar chains bound to the Fc region which is produced by a CHO cell, wherein among the total complex *N*-glycoside-linked sugar chains bound to the Fc region in the composition, the ratio of a sugar chain in which fucose is not bound to *N*-acetylglucosamine in the reducing end in the sugar chain is 20% or more.
(23) A cell in which the activity of an enzyme relating to the synthesis of an intracellular sugar nucleotide, GDP-fucose and/or the activity of an enzyme relating to the modification of a sugar chain wherein 1-position of fucose is bound to 6-position of *N*-acetylglucosamine in the reducing end through α-bond in the complex N-glycoside-linked sugar chain is decreased or deleted by a genetic engineering technique.
(24) The cell according to (23), wherein the enzyme relating to the synthesis of an intracellular sugar nucleotide, GDP-fucose is an enzyme selected from the group consisting of the following (a), (b) and (c):

(a) GMD (GDP-mannose 4,6-dehydratase);
(b) Fx (GDP-keto-6-deoxymannose 3,5-epimerase, 4-reductase);
(c) GFPP (GDP-beta-L-fucose pyrophosphorylase).

(25) The cell according to (24), wherein the GMD is a protein encoded by a DNA of the following (a) or (b):

(a) a DNA comprising the nucleotide sequence represented by SEQ ID NO:65;
(b) a DNA which hybridizes with the DNA comprising the nucleotide sequence represented by SEQ ID NO:65 under stringent conditions and encodes a protein having GMD activity.

(26) The cell according to (24), wherein the GMD is a protein selected from the group consisting of the following (a), (b) and (c):

(a) a protein comprising the amino acid sequence represented by SEQ ID NO:71;
(b) a protein which comprises an amino acid sequence in which at least one amino acid is deleted, substituted, inserted and/or added in the amino acid sequence represented by SEQ ID NO:71 and has GMD activity;
(c) a protein which comprises an amino acid sequence having a homology of at least 80% with the amino acid sequence represented by SEQ ID NO:71 and has GMD activity.

(27) The cell according to (24), wherein the Fx is a protein encoded by a DNA of the following (a) or (b):

(a) a DNA comprising the nucleotide sequence represented by SEQ ID NO:48;
(b) a DNA which hybridizes with the DNA comprising the nucleotide sequence represented by SEQ ID NO:48 under stringent conditions and encodes a protein having Fx activity.

(28) The cell according to (24), wherein the Fx is a protein selected from the group consisting of the following (a), (b) and (c):

(a) a protein comprising the amino acid sequence represented by SEQ ID NO:72;
(b) a protein which comprises an amino acid sequence in which at least one amino acid is deleted, substituted, inserted and/or added in the amino acid sequence represented by SEQ ID NO:72 and has Fx activity;
(c) a protein which comprises an amino acid sequence having a homology of at least 80% with the amino acid sequence represented by SEQ ID NO:72 and has Fx activity.

(29) The cell according to (24), wherein the GFPP is a protein encoded by a DNA of the following (a) or (b):

(a) a DNA comprising the nucleotide sequence represented by SEQ ID NO:51;
(b) a DNA which hybridizes with the DNA comprising the nucleotide sequence represented by SEQ ID NO:51 under stringent conditions and encodes a protein having GFPP activity.

(30) The cell according to (24), wherein the GFPP is a protein selected from the group consisting of the following (a), (b) and (c):

(a) a protein comprising the amino acid sequence represented by SEQ ID NO:73;
(b) a protein which comprises an amino acid sequence in which at least one amino acid is deleted, substituted, inserted and/or added in the amino acid sequence represented by SEQ ID NO:73 and has GFPP activity;
(c) a protein which comprises an amino acid sequence having a homology of at least 80% with the amino acid sequence represented by SEQ ID NO:73 and has GFPP activity.

(31) The cell according to (23), wherein the enzyme relating to the modification of a sugar chain in which 1-position of fucose is bound to 6-position of *N*-acetylglucosamine in the reducing end through α-bond in the *N*-glycoside-linked sugar chain is α-1,6-fucosyltransferase.
(32) The cell according to (31), wherein the α-1,6-fucosyltransferase is a protein encoded by a DNA selected from the group consisting of the following (a), (b), (c) and (d):

(a) a DNA comprising the nucleotide sequence represented by SEQ ID NO:1;
(b) a DNA comprising the nucleotide sequence represented by SEQ ID NO:2;
(c) a DNA which hybridizes with the DNA comprising the nucleotide sequence represented by SEQ ID NO:1 under stringent conditions and encodes a protein having α-1,6-fucosyltransferase activity,;
(d) a DNA which hybridizes with the DNA comprising the nucleotide sequence represented by SEQ ID NO:2 under stringent conditions and encodes a protein having α-1,6-fucosyltransferase activity.

(33) The cell according to (31), wherein the α-1,6-fucosyltransferase is a protein selected from the group consisting of the following (a), (b), (c), (d), (e) and (f):

(a) a protein comprising the amino acid sequence represented by SEQ ID NO:23;
(b) a protein comprising the amino acid sequence represented by SEQ ID NO:24;
(c) a protein which comprises an amino acid sequence in which at least one amino acid is deleted, substituted, inserted and/or added in the amino acid sequence represented by SEQ ID NO:23 and has α-1,6-fucosyltransferase activity;
(d) a protein which comprises an amino acid sequence in which at least one amino acid is deleted, substituted, inserted and/or added in the amino acid sequence represented by SEQ ID NO:24 and has α-1,6-fucosyltransferase activity;
(e) a protein which comprises an amino acid sequence having a homology of at least 80% with the amino acid sequence represented by SEQ ID NO:23 and has α-1,6-fucosyltransferase activity;
(f) a protein which comprises an amino acid sequence having a homology of at least 80% with the amino acid sequence represented by SEQ ID NO:24 and has α-1,6-fucosyltransferase activity.

(34) The cell according to any one of (23) to (33), wherein the genetic engineering technique is a technique selected from the group consisting of the following (a), (b), (c) and (d):

(a) a gene disruption technique targeting a gene encoding the enzyme;
(b) a technique for introducing a dominant negative mutant of a gene encoding the enzyme;

(c) a technique for introducing mutation into the enzyme;
(d) a technique for inhibiting transcription and/or translation of a gene encoding the enzyme.

(35) The cell according to any one of (23) to (34), which is resistant to at least a lectin which recognizes a sugar chain in which 1-position of fucose is bound to 6-position of *N*-acetylglucosamine in the reducing end through α-bond in the *N*-glycoside-linked sugar chain.
(36) The cell according to any one of (23) to (35), which is a cell selected from the group consisting of the following (a) to (i):

(a) a CHO cell derived from a Chinese hamster ovary tissue;
(b) a rat myeloma cell line, YB2/3HL.P2.G11.16Ag.20 cell;
(c) a mouse myeloma cell line, NS0 cell;
(d) a mouse myeloma cell line, SP2/0-Ag14 cell;
(e) a BHK cell derived from a syrian hamster kidney tissue;
(f) an antibody-producing hybridoma cell;
(g) a human leukemia cell line, Namalwa cell;
(h) an embryonic stem cell;
(i) a fertilized egg cell.

(37) The cell according to any one of (23) to (36) into which a gene encoding an antibody molecule is introduced.
(38) The cell according to (37), wherein the antibody molecule belongs to an IgG class.
(39) A process for producing an antibody composition, which comprises culturing the cell according to (37) or (38) in a medium to produce and accumulate the antibody composition in the culture; and recovering the antibody composition from the culture.
(40) The process according to (39), which produces an antibody composition having higher antibody-dependent cell-mediated cytotoxic activity than an antibody composition obtained from its parent cell line.
(41) An antibody composition which is produced using the process according to (39) or (40).
(42) A transgenic non-human animal or plant or the progenies thereof, comprising a genome which is modified such that the activity of an enzyme relating to the synthesis of an intracellular sugar nucleotide, GDP-fucose and/or the activity of an enzyme relating to the modification of a sugar chain in which 1-position of fucose is bound to 6-position of *N*-acetylglucosamine in the reducing end through α-bond in the *N*-glycoside-linked sugar chain is decreased.
(43) The transgenic non-human animal or plant or the progenies thereof according to (42), wherein a gene encoding the enzyme relating to the synthesis of an intracellular sugar nucleotide, GDP-fucose or a gene encoding the enzyme relating to the modification of a sugar chain in which 1-position of fucose is bound to 6-position of *N*-acetylglucosamine in the reducing end through α-bond in the *N*-glycoside-linked sugar chain is knocked out.
(44) The transgenic non-human animal or plant or the progenies thereof according to (42) or (43), wherein the enzyme relating to the synthesis of an intracellular sugar nucleotide, GDP-fucose is an enzyme selected from the group consisting of the following (a), (b) and (c):

(a) GMD (GDP-mannose 4,6-dehydratase);
(b) Fx (GDP-keto-6-deoxymannose 3,5-epimerase, 4-reductase);
(c) GFPP (GDP-beta-L-fucose pyrophosphorylase).

(45) The transgenic non-human animal or plant or the progenies thereof according to (44), wherein the GMD is a protein encoded by a DNA of the following (a) or (b):

(a) a DNA comprising the nucleotide sequence represented by SEQ ID NO:65;
(b) a DNA which hybridizes with the DNA comprising the nucleotide sequence represented by SEQ ID NO:65 under stringent conditions and encodes a protein having GMD activity.

(46) The transgenic non-human animal or plant or the progenies thereof according to (44), wherein the Fx is a protein encoded by a DNA of the following (a) or (b):

(a) a DNA comprising the nucleotide sequence represented by SEQ ID NO:48;
(b) a DNA which hybridizes with the DNA comprising the nucleotide sequence represented by SEQ ID NO:48 under stringent conditions and encodes a protein having Fx activity.

(47) The transgenic non-human animal or plant or the progenies thereof according to (44), wherein the GFPP is a

protein encoded by a DNA of the following (a) or (b):

(a) a DNA comprising the nucleotide sequence represented by SEQ ID NO:51;
(b) a DNA which hybridizes with the DNA comprising the nucleotide sequence represented by SEQ ID NO:51 under stringent conditions and encodes a protein having GFPP activity.

(48) The transgenic non-human animal or plant or the progenies thereof according to (42) or (43), wherein the enzyme relating to the modification of a sugar chain in which 1-position of fucose is bound to 6-position of *N*-acetylglucosamine in the reducing end through α-bond in the *N*-glycoside-linked sugar chain is α-1-6-fucosyltransferase.
(49) The transgenic non-human animal or plant or the progenies thereof according to (48), wherein the α-1,6-fucosyltransferase is a protein encoded by a DNA selected from the group consisting of the following (a), (b), (c) and (d):

(a) a DNA comprising the nucleotide sequence represented by SEQ ID NO:1;
(b) a DNA comprising the nucleotide sequence represented by SEQ ID NO:2;
(c) a DNA which hybridizes with the DNA comprising the nucleotide sequence represented by SEQ ID NO:1 under stringent conditions and encodes a protein having α-1,6-fucosyltransferase activity;
(d) a DNA which hybridizes with the DNA comprising the nucleotide sequence represented by SEQ ID NO:2 under stringent conditions and encodes a protein having α-1,6-fucosyltransferase activity.

(50) The transgenic non-human animal or plant or the progenies thereof according to any one of (42) to (49), wherein the transgenic non-human animal is an animal selected from the group consisting of cattle, sheep, goat, pig, horse, mouse, rat, fowl, monkey and rabbit.
(51) A process for producing an antibody composition, which comprises introducing a gene encoding an antibody molecule into the transgenic non-human animal or plant or the progenies thereof according to any one of (42) to (50); rearing the animal or plant; isolating tissue or body fluid comprising the introduced antibody from the reared animal or plant; and recovering the antibody composition from the isolated tissue or body fluid.
(52) The process according to (51), wherein the antibody molecule belongs to an IgG class.
(53) The process according to (51) or (52), which produces an antibody composition having higher antibody-dependent cell-mediated cytotoxic activity than an antibody composition obtained from a non-human animal or plant or the progenies thereof whose genome is not modified.
(54) An antibody composition which is produced using the process according to any one of (51) to (53).
(55) A medicament comprising the antibody composition according to any one of (21), (22), (41) and (54) as an active ingredient.
(56) The medicament according to (55), wherein the medicament is a diagnostic drug, a preventive drug or a therapeutic drug for diseases accompanied by tumors, diseases accompanied by allergies, diseases accompanied by inflammations, autoimmune diseases, circulatory organ diseases, diseases accompanied by viral infections or diseases accompanied by bacterial infections.
(57) A protein selected from the group consisting of the following (a), (b), (c), (d), (e), (f), (g), (h), (i) and (j):

(a) a protein comprising the amino acid sequence represented by SEQ ID NO:71;
(b) a protein which comprises an amino acid sequence in which at least one amino acid is deleted, substituted, inserted and/or added in the amino acid sequence represented by SEQ ID NO:71 and has GMD activity;
(c) a protein comprising the amino acid sequence represented by SEQ ID NO:72;
(d) a protein which comprises an amino acid sequence in which at least one amino acid is deleted, substituted, inserted and/or added in the amino acid sequence represented by SEQ ID NO:72 and has Fx activity;
(e) a protein comprising the amino acid sequence represented by SEQ ID NO:73;
(f) a protein which comprises an amino acid sequence in which at least one amino acid is deleted, substituted, inserted and/or added in the amino acid sequence represented by SEQ ID NO:73 and has GFPP activity;
(g) a protein comprising the amino acid sequence represented by SEQ ID NO:23;
(h) a protein which comprises an amino acid sequence in which at least one amino acid is deleted, substituted, inserted and/or added in the amino acid sequence represented by SEQ ID NO:23 and has α-1,6-fucosyltransferase activity;
(i) a protein comprising the amino acid sequence represented by SEQ ID NO:24;
(j) a protein which comprises an amino acid sequence in which at least one amino acid is deleted, substituted, inserted and/or added in the amino acid sequence represented by SEQ ID NO:24 and the α-1,6-fucosyltransferase activity.

(58) A DNA which encodes the protein according to (57).

(59) A DNA selected from the group consisting of the following (a), (b), (c), (d) and (e):

(a) a DNA comprising the nucleotide sequence represented by SEQ ID NO:1;
(b) a DNA comprising the nucleotide sequence represented by SEQ ID NO:2;
(c) a DNA comprising the nucleotide sequence represented by SEQ ID NO:65;
(d) a DNA comprising the nucleotide sequence represented by SEQ ID NO:48;
(e) a DNA comprising the nucleotide sequence represented by SEQ ID NO:51.

(60) A genome DNA selected from the group consisting of the following (a), (b) and (c):

(a) a genome DNA comprising the nucleotide sequence represented by SEQ ID NO:3;
(b) a genome DNA comprising the nucleotide sequence represented by SEQ ID NO:67;
(c) a genome DNA comprising the nucleotide sequence represented by SEQ ID NO:70.

(61) A target vector for homologous recombination, comprising a full length of the DNA according to any one of (58) to (60), or a part thereof.

The Chinese hamster ovary tissue-derived CHO cell into which a gene encoding an antibody molecule is introduced according to the present invention may be any CHO cell, so long as it is a Chinese hamster ovary tissue-derived CHO cell into which a gene encoding an antibody molecule is introduced, which produces an antibody composition comprising complex *N*-glycoside-linked sugar chains bound to the Fc region of an antibody molecule, wherein among the total complex *N*-glycoside-linked sugar chains bound to the Fc region in the composition, the ratio of a sugar chain in which fucose is not bound to *N*-acetylglucosamine in the reducing end in the sugar chain is 20% or more.

In the present invention, the antibody molecule includes any molecule, so long as it comprises the Fc region of an antibody. Examples include an antibody, an antibody fragment, a fusion protein comprising an Fc region, and the like.

The antibody is a protein which is produced in the living body by immune reaction as a result of exogenous antigen stimulation and has an activity to specifically bind to the antigen. Examples of the antibody include an antibody secreted by a hybridoma cell prepared from a spleen cell of an animal immunized with an antigen; an antibody prepared by a genetic recombination technique, namely an antibody obtained by introducing an antibody gene-inserted antibody expression vector into a host cell; and the like. Specific examples include an antibody produced by a hybridoma, a humanized antibody, a human antibody and the like.

A hybridoma is a cell which is obtained by cell fusion between a B cell obtained by immunizing a mammal other than human with an antigen and a myeloma cell derived from mouse or the like and can produce a monoclonal antibody having the desired antigen specificity.

Examples of the humanized antibody include a human chimeric antibody, a human CDR-grafted antibody and the like.

A human chimeric antibody is an antibody which comprises an antibody heavy chain variable region (hereinafter referred to as "HV" or "VH", the heavy chain being "H chain") and an antibody light chain variable region (hereinafter referred to as "LV" or "VL", the light chain being "L chain"), both of an animal other than human, a human antibody heavy chain constant region (hereinafter also referred to as "CH") and a human antibody light chain constant region (hereinafter also referred to as "CL"). As the animal other than human, any animal such as mouse, rat, hamster, rabbit or the like can be used, so long as a hybridoma can be prepared therefrom.

The human chimeric antibody can be produced by obtaining cDNA's encoding VH and VL from a monoclonal antibody-producing hybridoma, inserting them into an expression vector for host cell having genes encoding human antibody CH and human antibody CL to thereby construct a human chimeric antibody expression vector, and then introducing the vector into a host cell to express the antibody.

As the CH of human chimeric antibody, any CH can be used, so long as it belongs to human immunoglobulin (hereinafter referred to as "hIg") can be used. But those belonging to the hIgG class are preferable and any one of the subclasses belonging to the hIgG class, such as hIgG1, hIgG2, hIgG3 and hIgG4, can be used. Also, as the CL of human chimeric antibody, any CL can be used, so long as it belongs to the hIg class, and those belonging to the $\kappa$ class or $\lambda$ class can also be used.

A human CDR-grafted antibody is an antibody in which amino acid sequences of CDR's of VH and VL of an antibody derived from an animal other than human are grafted into appropriate positions of VH and VL of a human antibody.

The human CDR-grafted antibody can be produced by constructing cDNA's encoding V regions in which CDR's of VH and VL of an antibody derived from an animal other than human are grafted into CDR's of VH and VL of a human antibody, inserting them into an expression vector for host cell having genes encoding human antibody CH and human antibody CL to thereby construct a human CDR-grafted antibody expression vector, and then introducing the expression

vector into a host cell to express the human CDR-grafted antibody.

As the CH of human CDR-grafted antibody, any CH can be used, so long as it belongs to the hIg, but those of the hIgG class are preferable and any one of the subclasses belonging to the hIgG class, such as hIgG1, hIgG2, hIgG3 and hIgG4, can be used. Also, as the CL of human CDR-grafted antibody, any CL can be used, so long as it belongs to the hIg class, and those belonging to the κ class or λ, class can also be used.

A human antibody is originally an antibody naturally existing in the human body, but it also includes antibodies obtained from a human antibody phage library, a human antibody-producing transgenic animal and a human antibody-producing transgenic plant, which are prepared based on the recent advance in genetic engineering, cell engineering and developmental engineering techniques.

Regarding the antibody existing in the human body, a lymphocyte capable of producing the antibody can be cultured by isolating a human peripheral blood lymphocyte, immortalizing it by its infection with EB virus or the like and then cloning it, and the antibody can be purified from the culture.

The human antibody phage library is a library in which antibody fragments such as Fab, single chain antibody and the like are expressed on the phage surface by inserting a gene encoding an antibody prepared from a human B cell into a phage gene. A phage expressing an antibody fragment having the desired antigen binding activity can be recovered from the library, using its activity to bind to an antigen-immobilized substrate as the marker. The antibody fragment can be converted further into a human antibody molecule comprising two full H chains and two full L chains by genetic engineering techniques.

A human antibody-producing transgenic non-human animal is an animal in which a human antibody gene is introduced into cells. Specifically, a human antibody-producing transgenic animal can be prepared by introducing a human antibody gene into ES cell of a mouse, transplanting the ES cell into an early stage embryo of other mouse and then developing it. By introducing a human chimeric antibody gene into a fertilized egg and developing it, the transgenic animal can be also prepared. Regarding the preparation method of a human antibody from the human antibody-producing transgenic animal, the human antibody can be produced and accumulated in a culture by obtaining a human antibody-producing hybridoma by a hybridoma preparation method usually carried out in mammals other than human and then culturing it.

Examples of the transgenic non-human animal include cattle, sheep, goat, pig, horse, mouse, rat, fowl, monkey, rabbit and the like.

Also, in the present invention, it is preferable that the antibody is an antibody which recognizes a tumor-related antigen, an antibody which recognizes an allergy- or inflammation-related antigen, an antibody which recognizes circulatory organ disease-related antigen, an antibody which recognizes an autoimmune disease-related antigen or an antibody which recognizes a viral or bacterial infection-related antigen, and a human antibody which belongs to the IgG class is preferable.

An antibody fragment is a fragment which comprises the Fc region of an antibody. Examples of the antibody fragment include an H chain monomer, an H chain dimer and the like.

A fusion protein comprising an Fc region is a composition in which an antibody comprising the Fc region of an antibody or the antibody fragment is fused with a protein such as an enzyme, a cytokine or the like.

In the present invention, examples of the sugar chain which binds to the Fc region of an antibody molecule includes an *N*-glycoside-linked sugar chain. Examples of the *N*-glycoside-linked sugar chain include a complex type in which the non-reducing end side of the core structure has one or plural parallel branches of galactose-*N*-acetylglucosamine (hereinafter referred to as "Gal-GlcNAc") and the non-reducing end side of Gal-GlcNAc has a structure such as sialic acid, bisecting *N*-acetylglucosamine or the like.

In one antibody, the Fc region has positions to which an *N*-glycoside-linked sugar chain is bound which will be described later. Accordingly, two sugar chains are bound per one antibody molecule. Since the *N*-glycoside-linked sugar chain which binds to an antibody includes any sugar chain having the core structure represented by the structural formula (I), a number of combinations of sugar chains may possible for the two *N*-glycoside-linked sugar chains which bind to the antibody. Accordingly, identity of substances can be judged from the viewpoint of the sugar structure bound to the Fc region.

In the present invention, the composition which comprises an antibody molecule having complex *N*-glycoside-linked sugar chains in the Fc region may comprise an antibody having the same sugar chain structure or an antibody having different sugar chain structures, so long as the effect of the present invention is obtained from the composition.

In the present invention, the ratio of a sugar chain in which fucose is not bound to *N*-acetylglucosamine in the reducing end in the sugar chain among the total complex *N*-glycoside-linked sugar chains bound to the Fc region contained in the antibody composition is a ratio of the number of a sugar chain in which fucose is not bound to *N*-acetylglucosamine in the reducing end in the sugar chain to the total number of the complex *N*-glycoside-linked sugar chains bound to the Fc region contained in the composition.

In the present invention, the sugar chain in which fucose is not bound to *N*-acetylglucosamine in the reducing end in the complex *N*-glycoside-linked sugar chain is a sugar chain in which the fucose is not bound to *N*-acetylglucosamine in the reducing end through α-bond in the complex *N*-glycoside-linked sugar chain. Examples include a complex *N*-glycoside-linked sugar chain in which 1-position of fucose is not bound to 6-position of *N*-acetylglucosamine through α-bond.

The antibody composition shows high ADCC activity when the ratio of a sugar chain in which fucose is not bound to *N*-acetylglucosamine in the reducing end in the sugar chain among the total complex *N*-glycoside-linked sugar chains binding to the Fc region contained in the antibody composition produced in accordance with the present invention is preferably 20% or more, more preferably 25% or more, still more preferably 30% or more, far preferably 40% or more, and most preferably 50% or more. As the antibody concentration is decreased, the ADCC activity is decreased, but high ADCC activity can be obtained even when the antibody concentration is low, so long as the ratio of a sugar chain in which fucose is not bound to *N*-acetylglucosamine in the reducing end in the sugar chain is 20% or more.

The ratio of a sugar chain in which fucose is not bound to *N*-acetylglucosamine in the reducing end in the sugar chain contained in the composition which comprises an antibody molecule having complex *N*-glycoside-linked sugar chains in the Fc region can be determined by releasing the sugar chain from the antibody molecule using a known method such as hydrazinolysis, enzyme digestion or the like [Biochemical Experimentation Methods 23 - Method for Studying Glycoprotein Sugar Chain (Japan Scientific Societies Press), edited by Reiko Takahashi (1989)], carrying out fluorescence labeling or radioisotope labeling of the released sugar chain and then separating the labeled sugar chain by chromatography. Also, the released sugar chain can also be determined by analyzing it with the HPAED-PAD method [J. Liq. Chromatogr., 6,1557 (1983)].

In the present invention, the Chinese hamster ovary tissue-derived CHO cell includes any cell which is a cell line established from an ovary tissue of Chinese hamster *(Cricetulus griseus).* Examples include CHO cells described in documents such as Journal of Experimental Medicine, 108, 945 (1958); Proc. Natl. Acad Sci. USA, 60, 1275 (1968); Genetics, 55, 513 (1968); Chromosoma, 41, 129 (1973); Methods in Cell Science, 18, 115 (1996); Radiation Research, 148, 260 (1997); Proc. Natl. Acad Sci. USA, 77, 4216 (1980); Proc. Natl. Acad Sci., 60, 1275 (1968); Cell, 6, 121 (1975); Molecular Cell Genetics, Appendix I, II (pp. 883-900); and the like. In addition, CHO-K1 (ATCC CCL-61), DUXB11 (ATCC CCL-9096) and Pro-5 (ATCC CCL-1781) registered in ATCC (The American Type Culture Collection) and a commercially available CHO-S (Life Technologies, Cat # 11619) or sub-cell lines obtained by adapting the cell lines using various media can also be exemplified.

In the present invention, the enzyme relating to the synthesis of an intracellular sugar nucleotide, GDP-fucose may be any enzyme, so long as it is an enzyme relating to the synthesis of the intracellular sugar nucleotide, GDP-fucose as a supply source of fucose to a sugar chain. The enzyme relating to the synthesis of an intracellular sugar nucleotide, GDP-fucose is an enzyme which has influence on the synthesis of the intracellular sugar nucleotide, GDP-fucose.

The intracellular sugar nucleotide, GDP-fucose is supplied by a *de novo* synthesis pathway or a salvage synthesis pathway. Thus, all enzymes relating to the synthesis pathways are included in the enzyme relating to the synthesis of an intracellular sugar nucleotide, GDP-fucose.

Examples of the enzyme relating to the *de novo* synthesis pathway of the intracellular sugar nucleotide, GDP-fucose include GDP-mannose 4,6-dehydratase (hereinafter referred to as "GMD"), GDP-keto-6-deoxymannose 3,5-epimerase 4,6-reductase (hereinafter referred to as "Fx") and the like.

Examples of the enzyme relating to the salvage synthesis pathway of the intracellular sugar nucleotide, GDP-fucose include GDP-beta-L-fucose pyrophosphorylase (hereinafter referred to as "GFPP"), fucokinase and the like.

As the enzyme which has influence on the synthesis of an intracellular sugar nucleotide, GDP-fucose, an enzyme which has influence on the activity of the enzyme relating to the synthesis of the intracellular sugar nucleotide, GDP-fucose and an enzyme which has influence on the structure of substances as the substrate of the enzyme are also included.

In the present invention, examples of the GMD include:

a protein encoded by a DNA of the following (a) or (b):

(a) a DNA comprising the nucleotide sequence represented by SEQ ID NO:65;
(b) a DNA which hybridizes with the DNA comprising the nucleotide sequence represented by SEQ ID NO:65 under stringent conditions and encodes a protein having GMD activity,
(c) a protein comprising the amino acid sequence represented by SEQ ID NO:71,
(d) a protein which comprises an amino acid sequence in which at least one amino acid is deleted, substituted, inserted and/or added in the amino acid sequence represented by SEQ ID NO:71 and has GMD activity,
(e) a protein which comprises an amino acid sequence having a homology of at least 80% with the amino acid sequence represented by SEQ ID NO:71 and has GMD activity, and the like.

Also, examples of the DNA encoding the amino acid sequence of GMD include a DNA comprising the nucleotide

sequence represented by SEQ ID NO:65 and a DNA which hybridizes with the DNA comprising the nucleotide sequence represented by SEQ ID NO:65 under stringent conditions and encodes an amino acid sequence having GMD activity.

In the present invention, examples of the Fx include:

a protein encoded by a DNA of the following (a) or (b):

(a) a DNA comprising the nucleotide sequence represented by SEQ ID NO:48;
(b) a DNA which hybridizes with the DNA comprising the nucleotide sequence represented by SEQ ID NO:48 under stringent conditions and encodes a protein having Fx activity,
(c) a protein comprising the amino acid sequence represented by SEQ ID NO:72,
(d) a protein which comprises an amino acid sequence in which at least one amino acid is deleted, substituted, inserted and/or added in the amino acid sequence represented by SEQ ID NO:72 and has Fx activity,
(e) a protein which comprises an amino acid sequence having a homology of at least 80% with the amino acid sequence represented by SEQ ID NO:72 and has Fx activity, and the like.

Also, examples of the DNA encoding the amino acid sequence of Fx include a DNA comprising the nucleotide sequence represented by SEQ ID NO:48 and a DNA which hybridizes with the DNA comprising the nucleotide sequence represented by SEQ ID NO:48 under stringent conditions and encodes an amino acid sequence having Fx activity.

In the present invention, examples of the GFPP include:

a protein encoded by a DNA of the following (a) or (b):

(a) a DNA comprising the nucleotide sequence represented by SEQ ID NO:51;
(b) a DNA which hybridizes with the DNA comprising the nucleotide sequence represented by SEQ ID NO:51 under stringent conditions and encodes a protein having GFPP activity,
(c) a protein comprising the amino acid sequence represented by SEQ ID NO:73,
(d) a protein which comprises an amino acid sequence in which at least one amino acid is deleted, substituted, inserted and/or added in the amino acid sequence represented by SEQ ID NO:73 and has GFPP activity,
(e) a protein which comprises an amino acid sequence having a homology of at least 80% with the amino acid sequence represented by SEQ ID NO:73 and has GFPP activity, and the like.

Also, examples of the DNA encoding the amino acid sequence of GFPP include a DNA comprising the nucleotide sequence represented by SEQ ID NO:51 and a DNA which hybridizes with the DNA comprising the nucleotide sequence represented by SEQ ID NO:51 under stringent conditions and encodes an amino acid sequence having Fx activity.

In the present invention, the enzyme relating to the modification of a sugar chain in which 1-position of fucose is bound to 6-position of *N*-acetylglucosamine in the reducing end through α-bond in the complex *N*-glycoside-linked sugar chain includes any enzyme, so long as it is an enzyme relating to the reaction of binding of 1-position of fucose to 6-position of *N*-acetylglucosamine in the reducing end through α-bond in the complex *N*-glycoside-linked sugar chain. The enzyme relating to the reaction of binding of 1-position of fucose to 6-position of *N*-acetylglucosamine in the reducing end through α-bond in the complex *N*-glycoside-linked sugar chain means an enzyme which has influence on the reaction of binding of 1-position of fucose to 6-position of *N*-acetylglucosamine in the reducing end through α-bond in the complex *N*-glycoside-linked sugar chain.

Examples of the enzyme relating to the reaction of binding of 1-position of fucose to 6-position of *N*-acetylglucosamine in the reducing end through α-bond in the complex *N*-glycoside-linked sugar chain include α-1,6-fucosyltransferase, α-L-fucosidase and the like.

Also, examples include an enzyme which has influence on the activity of the enzyme relating to the reaction of binding of 1-position of fucose to 6-position of *N*-acetylglucosamine in the reducing end through α-bond in the complex *N*-glycoside-linked sugar chain and an enzyme which has influence on the structure of substances as the substrate of the enzyme.

In the present invention, examples of the a-1,6-fucosyltransferase include:

a protein encoded by a DNA of the following (a), (b), (c) or (d):

(a) a DNA comprising the nucleotide sequence represented by SEQ ID NO:1;
(b) a DNA comprising the nucleotide sequence represented by SEQ ID NO:2;
(c) a DNA which hybridizes with the DNA comprising the nucleotide sequence represented by SEQ ID NO:1 under stringent conditions and encodes a protein having α-1,6-fucosyltransferase activity;
(d) a DNA which hybridizes with the DNA comprising the nucleotide sequence represented by SEQ ID NO:2

under stringent conditions and encodes a protein having α-1,6-fucosyltransferase activity;
(e) a protein comprising the amino acid sequence represented by SEQ ID NO:23,
(f) a protein comprising the amino acid sequence represented by SEQ ID NO:24,
(g) a protein which comprises an amino acid sequence in which at least one amino acid is deleted, substituted, inserted and/or added in the amino acid sequence represented by SEQ ID NO:23 and has α-1,6-fucosyltransferase activity,
(h) a protein which comprises an amino acid sequence in which at least one amino acid is deleted, substituted, inserted and/or added in the amino acid sequence represented by SEQ ID NO:24 and has α-1,6-fucosyltransferase activity,
(i) a protein which comprises an amino acid sequence having a homology of at least 80% with the amino acid sequence represented by SEQ ID NO:23 and has α-1,6-fucosyltransferase activity,
(j) a protein which comprises an amino acid sequence having a homology of at least 80% with the amino acid sequence represented by SEQ ID NO:24 and has α-1,6-fucosyltransferase activity, and the like.

Also, examples of the DNA encoding the amino acid sequence of α-1,6-fucosyltransferase include a DNA having the nucleotide sequence represented by SEQ ID NO:1 or 2 and a DNA which hybridizes with the DNA having the nucleotide sequence represented by SEQ ID NO:1 or 2 under stringent conditions and encodes an amino acid sequence having α-1,6-fucosyltransferase activity.

In the present invention, a DNA which hybridizes under stringent conditions is a DNA obtained, e.g., by a method such as colony hybridization, plaque hybridization or Southern blot hybridization using a DNA such as the DNA having the nucleotide sequence represented by SEQ ID NO:1, 2, 48, 51 or 65 or a partial fragment thereof as the probe, and specifically includes a DNA which can be identified by carrying out hybridization at 65°C in the presence of 0.7 to 1.0 M sodium chloride using a filter to which colony- or plaque-derived DNA fragments are immobilized, and then washing the filter at 65°C using 0.1 to 2 x SSC solution (composition of the 1 x SSC solution comprising 150 mM sodium chloride and 15 mM sodium citrate). The hybridization can be carried out in accordance with the methods described, e.g., in Molecular Cloning, A Laboratory Manual, 2nd Ed., Cold Spring Harbor Laboratory Press (1989) (hereinafter referred to as "Molecular Cloning, Second Edition"), Current Protocols in Molecular Biology, John Wiley & Sons, 1987-1997 (hereinafter referred to as "Current Protocols in Molecular Biology"); DNA Cloning 1: Core Techniques, A Practical Approach, Second Edition, Oxford University (1995); and the like. Examples of the hybridizable DNA include a DNA having at least 60% or more, preferably 70% or more, more preferably 80% or more, still more preferably 90% or more, far more preferably 95% or more, and most preferably 98% or more, of homology with the nucleotide sequence represented by SEQ ID NO:1, 2, 48, 51 or 65.

In the present invention, the protein which comprises an amino acid sequence in which at least one amino acid is deleted, substituted, inserted and/or added in the amino acid sequence represented by SEQ ID NO:23, 24, 71, 72 or 73 and has α-1,6-fucosyltransferase activity, GMD activity, Fx activity or GFPP activity can be obtained, e.g., by introducing a site-directed mutation into a DNA encoding a protein having the amino acid sequence represented by SEQ ID NO:1, 2, 65, 48 or 51, respectively, using the site-directed mutagenesis described, e.g., in Molecular Cloning, Second Edition; Current Protocols in Molecular Biology; Nucleic Acids Research, 10, 6487 (1982); Proc. Natl. Acad. Sci. USA, 79, 6409 (1982); Gene, 34, 315 (1985); Nucleic Acids Research, 13, 4431 (1985); Proc. Natl. Acad. Sci. USA, 82, 488 (1985); and the like. The number of amino acids to be deleted, substituted, inserted and/or added is one or more, and the number is not particularly limited, but is a number which can be deleted, substituted or added by a known technique such as the site-directed mutagenesis, e.g., it is 1 to several tens, preferably 1 to 20, more preferably 1 to 10, and most preferably 1 to 5.

Also, in order to maintain the α-1,6-fucosyltransferase activity, GMD activity, Fx activity or GFPP activity of the protein to be used in the present invention, it has at least 80% or more, preferably 85% or more, more preferably 90% or more, still more preferably 95% or more, far more preferably 97% or more, and most preferably 99% or more, of homology with the amino acid sequence represented by SEQ ID NO:23, 24, 71, 72 or 73, when calculated using an analyzing soft such as BLAST [J. Mol. Biol., 215, 403 (1990)], FASTA [Methods in Enzymology, 183, 63 (1990)] or the like.

Examples of the CHO cell of the present disclosure include a cell in which the enzyme activity is decreased or deleted.

The cell in which the enzyme activity is decreased or deleted include cells in which the activity of an enzyme relating to the synthesis of an intracellular sugar nucleotide, GDP-fucose or the activity of an enzyme relating to the modification of a sugar chain wherein 1-position of fucose is bound to 6-position of *N*-acetylglucosamine in the reducing end through α-bond in the complex *N*-glycoside-linked sugar chain is decreased or deleted. As the method for obtaining such cells, any technique can be used, so long as it can reduce or delete the enzyme activity of interest. Examples of the technique for reducing or deleting the enzyme activity include:

(a) a gene disruption technique targeting a gene encoding the enzyme,

(b) a technique for introducing a dominant negative mutant of a gene encoding the enzyme,
(c) a technique for introducing mutation into the enzyme,
(d) a technique for inhibiting transcription and/or translation of a gene encoding the enzyme,
(e) a technique for selecting a cell line resistant to a lectin which recognizes a sugar chain in which 1-position of fucose is bound to 6-position of *N*-acetylglucosamine in the reducing end through α-bond in the complex *N*-glycoside-linked sugar chain, and the like.

Herein, the lectin-resistant cell line can be obtained by culturing a cell line in a medium comprising a predetermined concentration of lectin and then by selecting a cell line which acquires such a property that its survival rate is increased at least 2 times, preferably 3 times, and more preferably 5 times or more, than the parent cell line with statistical significance. Also, it can also be obtained by culturing a cell line in a medium comprising lectin and then by selecting a cell line which can be cultured at a certain survival rate, e.g., 80% survival rate, at a lectin concentration of at least 2 times, preferably 5 times, more preferably 10 times, and most preferably 20 times or more, than the parent cell line.
As the lectin which recognizes a sugar chain structure in which 1-position of fucose is bound to 6-position of *N*-acetylglucosamine in the reducing end through α-bond in the *N*-glycoside-linked sugar chain, any lectin which can recognize the sugar chain structure can be used. Examples include a *Lens culinaris* lectin LCA (lentil agglutinin derived from *Lens culinaris),* a pea lectin PSA (pea lectin derived from *Pisum sativum*), a broad bean lectin VFA (agglutinin derived from *Vicia faba*)*,* an *Aleuria aurantia* lectin AAL (lectin derived from *Aleuria aurantia*) and the like.
The CHO cell of the present invention can produce an antibody composition having higher ADCC activity than that of an antibody composition produced by the parent CHO cell before applying the technique for decreasing or deleting the enzyme activity of interest.
Also, the CHO cell of the present invention can produce an antibody composition having higher ADCC activity than that of an antibody composition in which, among the total complex *N*-glycoside-linked sugar chains bound to the Fc region contained in the antibody composition, the ratio of a sugar chain in which fucose is not bound to *N*-acetylglucosamine in the reducing end in the sugar chain is less than 20%.
An example of the parent cell line to be used in the present invention is a cell in which the activity of an enzyme relating to the synthesis of an intracellular sugar nucleotide, GDP-fucose or the activity of an enzyme relating to the modification of a sugar chain wherein 1-position of fucose is bound to 6-position of *N*-acetylglucosamine in the reducing end through α-bond in the complex *N*-glycoside-linked sugar chain is not decreased. Specifically, a cell which is not treated to decrease or delete the activity of an enzyme relating to the synthesis of an intracellular sugar nucleotide, GDP-fucose or the activity of an enzyme relating to the modification of a sugar chain wherein 1-position of fucose is bound to 6-position of *N*-acetylglucosamine in the reducing end through α-bond in the complex *N*-glycoside-linked sugar chain is used.
In the present invention, the ADCC activity is a cytotoxic activity in which an antibody bound to a cell surface antigen on a tumor cell in the living body activate an effector cell through an Fc receptor existing on the antibody Fc region and effector cell surface and thereby obstruct the tumor cell and the like *[*Monoclonal Antibodies: Principles and Applications, Wiley-Liss, Inc., Chapter 2.1 (1955)]. Examples of the effector cell include a killer cell, a natural killer cell, an activated macrophage and the like.
The present disclosure also relates to a cell in which the activity of an enzyme relating to the synthesis of an intracellular sugar nucleotide, GDP-fucose or the activity of an enzyme relating to the modification of a sugar chain wherein 1-position of fucose is bound to 6-position of *N*-acetylglucosamine in the reducing end through α-bond in the complex *N*-glycoside-linked sugar chain is decreased by a genetic engineering technique. The host cell of the present invention is useful as a host cell for producing an antibody composition having high ADCC activity.
The host cell of the present invention may be any host, so long as it can express an antibody molecule in accordance with the present invention. Examples include a yeast cell, an animal cell, an insect cell, a plant cell and the like. Examples of the cells include those which will be later in the item 3. Among animal cells, preferred examples include a CHO cell derived from a Chinese hamster ovary tissue, a rat myeloma cell line YB2/3HL.P2.G11.16Ag.20 cell, a mouse myeloma cell line NS0 cell, a mouse myeloma SP2/0-Ag14 cell, a BHK cell derived from a syrian hamster kidney tissue, an antibody producing-hybridoma cell, a human leukemia cell line Namalwa cell, an embryonic stem cell, a fertilized egg cell and the like.
The present invention is described below in detail.

1. Preparation of the host cell of the present invention

The host cell of the present invention can be prepared by the following techniques.

(1) Gene disruption technique targeting a gene encoding an enzyme

The host cell of the present invention can be prepared using a gene disruption technique by targeting an enzyme relating to the synthesis of an intracellular sugar nucleotide, GDP-fucose or an enzyme relating to the modification of a sugar chain wherein 1-position of fucose is bound to 6-position of *N*-acetylglucosamine in the reducing end through α-bond in the complex *N*-glycoside-linked sugar chain. Examples of the enzyme relating to the synthesis of an intracellular sugar nucleotide, GDP-fucose include GMD, Fx, GFPP, fucokinase and the like. Examples of the enzyme relating to the modification of a sugar chain wherein 1-position of fucose is bound to 6-position of *N*-acetylglucosamine in the reducing end through α-bond in the complex *N*-glycoside-linked sugar chain include α-1,6-fucosyltransferase, α-L-fucosidase and the like.

The gene as used herein includes DNA and RNA.

The gene disruption method may be any method, so long as it can disrupt the gene of the target enzyme is included. Examples include an antisense method, a ribozyme method, a homologous recombination method, an RDO method, an RNAi method, a retrovirus-employed method, a transposon-employed method and the like. The methods are specifically described below.

(a) Preparation of the host cell of the present invention by the antisense method or the ribozyme method

The host cell of the present invention can be prepared by the ribozyme method described in Cell Technology, 12, 239 (1993); BIO/TECHNOLOGY, 17, 1097 (1999); Hum. Mol. Genet., 5, 1083 (1995); Cell Technology, 13, 255 (1994); Proc. Natl. Acad Sci. USA, 96, 1886 (1999); or the like, e.g., in the following manner by targeting an enzyme relating to the synthesis of an intracellular sugar nucleotide, GDP-fucose or an enzyme relating to the modification of a sugar chain wherein 1-position of fucose is bound to 6-position of *N*-acetylglucosamine in the reducing end through α-bond in the complex *N*-glycoside-linked sugar chain.

A cDNA or a genome DNA encoding an enzyme relating to the synthesis of an intracellular sugar nucleotide, GDP-fucose or an enzyme relating to the modification of a sugar chain wherein 1-position of fucose is bound to 6-position of *N*-acetylglucosamine in the reducing end through α-bond in the complex *N*-glycoside-linked sugar chain is prepared.

The nucleotide sequence of the prepared cDNA or genome DNA is determined.

Based on the determined DNA sequence, an appropriate length of an antisense gene or ribozyme construct comprising a DNA moiety which encodes the enzyme relating to the synthesis of an intracellular sugar nucleotide, GDP-fucose or the enzyme relating to the modification of a sugar chain wherein 1-position of fucose is bound to 6-position of *N*-acetylglucosamine in the reducing end through α-bond in the complex *N*-glycoside-linked sugar chain a part of its non-translation region or an intron, is designed.

In order to express the antisense gene or ribozyme in a cell, a recombinant vector is prepared by inserting a fragment or total length of the prepared DNA into downstream of the promoter of an appropriate expression vector.

A transformant is obtained by introducing the recombinant vector into a host cell suitable for the expression vector.

The host cell of the present invention can be obtained by selecting a transformant on the basis of the activity of the enzyme relating to the synthesis of an intracellular sugar nucleotide, GDP-fucose or the enzyme relating to the modification of a sugar chain wherein 1-position of fucose is bound to 6-position of *N*-acetylglucosamine in the reducing end through α-bond in the complex *N*-glycoside-linked sugar chain. The host cell of the present invention can also be obtained by selecting a transformant on the basis of the sugar chain structure of a glycoprotein on the cell membrane or the sugar chain structure of the produced antibody molecule.

As the host cell used for the production of the host cell of the present invention, any cell such as yeast, animal cell, insect cell or plant cell can be used, so long as it has a gene encoding the target enzyme relating to the synthesis of an intracellular sugar nucleotide, GDP-fucose or the target enzyme relating to the modification of a sugar chain wherein 1-position of fucose is bound to 6-position of *N*-acetylglucosamine in the reducing end through α-bond in the complex *N*-glycoside-linked sugar chain. Examples include host cells which will be described later in the item 3.

As the expression vector, a vector which is autonomously replicable in the host cell or can be integrated into the chromosome and comprises a promoter at such a position that the designed antisense gene or ribozyme can be transferred is used. Examples include expression vectors which will be described later in the item 3.

Regarding the method for introducing a gene into various host cells, the methods for introducing recombinant vectors suitable for various host cells, which will be described later in the item 3, can be used.

The following method can be exemplified as the method for selecting a transformant on the basis of the activity of an enzyme relating to the synthesis of an intracellular sugar nucleotide, GDP-fucose or the activity of an enzyme relating to the modification of a sugar chain wherein 1-position of fucose is bound to 6-position of *N*-acetylglucosamine in the reducing end through α-bond in the complex *N*-glycoside-linked sugar chain.

Method for selecting transformant:

Examples of the method for selecting a cell in which the activity of an enzyme relating to the synthesis of an intracellular sugar nucleotide, GDP-fucose or the activity of an enzyme relating to the modification of a sugar chain wherein 1-position of fucose is bound to 6-position of *N*-acetylglucosamine in the reducing end through α-bond in the complex *N*-glycoside-linked sugar chain is decreased include biochemical methods or genetic engineering techniques described in New Biochemical Experimentation Series 3 - Saccharides I, Glycoprotein (Tokyo Kagaku Dojin), edited by Japanese Biochemical society (1988); Cell Engineering, Supplement, Experimental Protocol Series, Glycobiology Experimental Protocol, Glycoprotein, Glycolipid and Proteoglycan (Shujun-sha), edited by Naoyuki Taniguchi, Akemi Suzuki, Kiyoshi Furukawa and Kazuyuki Sugawara (1996);, Molecular Cloning, Second Edition; Current Protocols in Molecular Biology; and the like. Examples of the biochemical method include a method in which the enzyme activity is evaluated using an enzyme-specific substrate and the like. Examples of the genetic engineering technique include the Northern analysis, RT-PCR and the like which measures the amount of mRNA of a gene encoding the enzyme.

Examples of the method for selecting a transformant on the basis of the sugar chain structure of a glycoprotein on the cell membrane include the methods which will be described later in the item 1(5). Examples of the method for selecting a transformant on the basis of the sugar chain structure of a produced antibody molecule include the methods which will be described later in the items 5 and 6.

As the method for preparing cDNA encoding an enzyme relating to the synthesis of an intracellular sugar nucleotide, GDP-fucose or an enzyme relating to the modification of a sugar chain wherein 1-position of fucose is bound to 6-position of *N*-acetylglucosamine in the reducing end through α-bond in the complex *N*-glycoside-linked sugar chain, the following method is exemplified.

Preparation of DNA:

A total RNA or mRNA is prepared from a human or non-human animal tissue or cell.

A cDNA library is prepared from the prepared total RNA or mRNA.

Degenerative primers are produced based on the amino acid sequence of an enzyme relating to the synthesis of an intracellular sugar nucleotide, GDP-fucose or an enzyme relating to the modification of a sugar chain wherein 1-position of fucose is bound to 6-position of *N*-acetylglucosamine in the reducing end through α-bond in the complex *N*-glycoside-linked sugar chain, and a gene fragment encoding the enzyme relating to the synthesis of an intracellular sugar nucleotide, GDP-fucose or the enzyme relating to the modification of a sugar chain wherein 1-position of fucose is bound to 6-position of *N*-acetylglucosamine in the reducing end through α-bond in the complex *N*-glycoside-linked sugar chain is obtained by PCR using the prepared cDNA library as the template.

A DNA encoding the enzyme relating to the synthesis of an intracellular sugar nucleotide, GDP-fucose or the enzyme relating to the modification of a sugar chain wherein 1-position of fucose is bound to 6-position of *N*-acetylglucosamine in the reducing end through α-bond in the complex *N*-glycoside-linked sugar chain can be obtained by screening the cDNA library using the obtained gene fragment as a probe.

Regarding the mRNA of a human or non-human tissue or cell, a commercially available product (e.g., manufactured by Clontech) may be used or it may be prepared from a human or non-human animal tissue or cell in the following manner. Examples of the method for preparing a total RNA from a human or non-human animal tissue or cell include the guanidine thiocyanate-cesium trifluoroacetate method [Methods in Enrymology, 154, 3 (1987)], the acidic guanidine thiocyanate phenol chloroform (AGPC) method [Analytical Biochemistry, 162, 156 (1987); Experimental Medicine, 9, 1937 (1991)] and the like.

Also, examples of the method for preparing mRNA from a total RNA as poly(A)$^+$ RNA include an oligo(dT)-immobilized cellulose column method (*Molecular Cloning,* Second Edition) and the like.

In addition, mRNA can be prepared using a kit such as Fast Track mRNA Isolation Kit (manufactured by Invitrogen), Quick Prep mRNA Purification Kit (manufactured by Pharmacia) or the like.

A cDNA library is prepared from the prepared mRNA of a human or non-human animal tissue or cell. Examples of the method for preparing cDNA libraries include the methods described in Molecular Cloning, Second Edition; Current Protocols in Molecular Biology*;* A Laboratory Manual, Second Edition (1989); and the like, or methods using commercially available kits such as SuperScript Plasmid System for cDNA Synthesis and Plasmid Cloning (manufactured by Life Technologies), ZAP-cDNA Synthesis Kit (manufactured by STRATAGENE) and the like.

As the cloning vector for use in the preparation of the cDNA library, any vector such as a phage vector, a plasmid vector or the like can be used, so long as it is autonomously replicable in *Escherichia coli* K12. Examples include ZAP Express [manufactured by STRATAGENE, Strategies, 5, 58 (1992)], pBluescript II SK(+) [Nucleic Acids Research, 17, 9494 (1989)], Lambda ZAP II (manufactured by STRATAGENE), λgt10 and λgt11 [DNA Cloning, A Practical Approach, 1, 49 (1985)], λTriplEx (manufactured by Clontech), XExCell (manufactured by Pharmacia), pcD2 [Mol. Cell. Biol., 3, 280 (1983)], pUC18 *[*Gene, 33, 103 (1985)] and the like.

Any microorganism can be used as the host microorganism, but *Escherichia coli* is preferably used. Examples include *Escherichia coli* XL1-Blue MRF' [manufactured by STRATAGENE, Strategies, 5, 81 (1992)], *Escherichia coli* C600 [Genetics, 39, 440 (1954)], *Escherichia coli* Y1088 [Science, 222, 778 (1983)], *Escherichia coli* Y1090 [Science, 222, 778 (1983)], *Escherichia coli* NM522 [J. Mol. Biol., 166, 1 (1983)], *Escherichia coli* K802 [J. Mol. Biol., 16, 118 (1966)], *Escherichia coli* JM105 [Gene, 38, 275 (1985)] and the like

The cDNA library may be used as such in the succeeding analysis, and in order to obtain a full length cDNA as efficient as possible by decreasing the ratio of an infull length cDNA, a cDNA library prepared using the oligo cap method developed by Sugano et al. [Gene, 138, 171 (1994); Gene, 200, 149 (1997); Protein, Nucleic Acid and Protein, 41, 603 (1996); Experimental Medicine, 11, 2491 (1993); cDNA Cloning (Yodo-sha) (1996); Methods for Preparing Gene Libraries (Yodo-sha) (1994)] may be used in the following analysis.

Degenerative primers specific for the 5'-terminal and 3'-terminal nucleotide sequences of a nucleotide sequence presumed to encode the amino acid sequence are prepared based on the amino acid sequence of the enzyme relating to the synthesis of an intracellular sugar nucleotide, GDP-fucose or the enzyme relating to the modification of a sugar chain wherein 1-position of fucose is bound to 6-position of *N*-acetylglucosamine in the reducing end through α-bond in the complex *N*-glycoside-linked sugar chain, and DNA is amplified by PCR [PCR Protocols, Academic Press (1990)] using the prepared cDNA library as the template to obtain a gene fragment encoding the enzyme relating to the synthesis of an intracellular sugar nucleotide, GDP-fucose or the enzyme relating to the modification of a sugar chain wherein 1-position of fucose is bound to 6-position of *N*-acetylglucosamine in the reducing end through α-bond in the complex *N*-glycoside-linked sugar chain.

It can be confirmed that the obtained gene fragment is a DNA encoding the enzyme relating to the synthesis of an intracellular sugar nucleotide, GDP-fucose or the enzyme relating to the modification of a sugar chain wherein 1-position of fucose is bound to 6-position of *N*-acetylglucosamine in the reducing end through α-bond in the complex *N*-glycoside-linked sugar chain, by a method usually used for analyzing a nucleotide, such as the dideoxy method of Sanger et al. [Proc. Natl. Acad. Sci. USA, 74, 5463 (1977)], a nucleotide sequence analyzer such as ABIPRISM 377 DNA Sequencer (manufactured by PE Biosystems) or the like.

A DNA encoding the enzyme relating to the synthesis of an intracellular sugar nucleotide, GDP-fucose or the enzyme relating to the modification of a sugar chain wherein 1-position of fucose is bound to 6-position of *N*-acetylglucosamine in the reducing end through α-bond in the complex *N*-glycoside-linked sugar chain can be obtained by carrying out colony hybridization or plaque hybridization (Molecular Cloning, Second Edition) for the cDNA or cDNA library synthesized from the mRNA contained in the human or non-human animal tissue or cell, using the gene fragment as a DNA probe.

Also, a DNA encoding the enzyme relating to the synthesis of an intracellular sugar nucleotide, GDP-fucose or the enzyme relating to the modification of a sugar chain wherein 1-position of fucose is bound to 6-position of *N*-acetylglucosamine in the reducing end through α-bond in the complex *N*-glycoside-linked sugar chain can also be obtained by carrying out screening by PCR using the cDNA or cDNA library synthesized from the mRNA contained in a human or non-human animal tissue or cell as the template and using the primers used for obtaining the gene fragment encoding the enzyme relating to the synthesis of an intracellular sugar nucleotide, GDP-fucose or the enzyme relating to the modification of a sugar chain wherein 1-position of fucose is bound to 6-position of *N*-acetylglucosamine in the reducing end through α-bond in the complex *N*-glycoside-linked sugar chain.

The nucleotide sequence of the obtained DNA encoding the enzyme relating to the synthesis of an intracellular sugar nucleotide, GDP-fucose or the enzyme relating to the modification of a sugar chain wherein 1-position of fucose is bound to 6-position of *N*-acetylglucosamine in the reducing end through α-bond in the complex *N*-glycoside-linked sugar chain is analyzed from its terminus and determined by a method usually used for analyzing a nucleotide, such as the dideoxy method of Sanger et al. [Proc. Natl. Acad. Sci. USA, 74, 5463 (1977)], a nucleotide sequence analyzer such as ABIPRISM 377 DNA Sequencer (manufactured by PE Biosystems) or the like.

A gene encoding the enzyme relating to the synthesis of an intracellular sugar nucleotide, GDP-fucose or the enzyme relating to the modification of a sugar chain wherein 1-position of fucose is bound to 6-position of *N*-acetylglucosamine in the reducing end through α-bond in the complex *N*-glycoside-linked sugar chain can also be determined from genes in data bases by searching nucleotide sequence data bases such as GenBank, EMBL, DDBJ and the like using a homology retrieving program such as BLAST based on the determined cDNA nucleotide sequence.

Examples of the nucleotide sequence of the gene obtained by the method encoding the enzyme relating to the synthesis of an intracellular sugar nucleotide, GDP-fucose include the nucleotide sequence represented by SEQ ID NO:48, 51 or 65. Examples of the nucleotide sequence of the gene encoding the enzyme relating to the modification of a sugar chain wherein 1-position of fucose is bound to 6-position of *N*-acetylglucosamine in the reducing end through α-bond in the complex *N*-glycoside-linked sugar chain include the nucleotide sequence represented by SEQ ID NO:1 or 2.

The cDNA encoding the enzyme relating to the synthesis of an intracellular sugar nucleotide, GDP-fucose or the enzyme relating to the modification of a sugar chain wherein 1-position of fucose is bound to 6-position of *N*-acetylglucosamine in the reducing end through α-bond in the complex *N*-glycoside-linked sugar chain can also be obtained by

chemically synthesizing it with a DNA synthesizer such as DNA Synthesizer model 392 manufactured by Perkin Elmer or the like using the phosphoamidite method, based on the determined DNA nucleotide sequence.

As an example of the method for preparing a genome DNA encoding the enzyme relating to the synthesis of an intracellular sugar nucleotide, GDP-fucose or the enzyme relating to the modification of a sugar chain wherein 1-position of fucose is bound to 6-position of *N*-acetylglucosamine in the reducing end through α-bond in the complex *N*-glycoside-linked sugar chain, the method described below is exemplified.

Preparation of genome DNA:

Examples of the method for preparing genome DNA include known methods described in Molecular Cloning, Second Edition; Current Protocols in Molecular Biology; and the like. In addition, a genome DNA encoding the enzyme relating to the synthesis of an intracellular sugar nucleotide, GDP-fucose or the enzyme relating to the modification of a sugar chain wherein 1-position of fucose is bound to 6-position of *N*-acetylglucosamine in the reducing end through α-bond in the complex *N*-glycoside-linked sugar chain can also be isolated using a kit such as Genome DNA Library Screening System (manufactured by Genome Systems), Universal GenomeWalker™ Kits (manufactured by CLONTECH) or the like.

Examples of the nucleotide sequence of the genome DNA obtained by the method encoding the enzyme relating to the synthesis of an intracellular sugar nucleotide, GDP-fucose include the nucleotide sequence represented by SEQ ID NO:67 or 70. Examples of the nucleotide sequence of the genome DNA encoding the enzyme relating to the modification of a sugar chain wherein 1-position of fucose is bound to 6-position of *N*-acetylglucosamine in the reducing end through α-bond in the complex N-glycoside-linked sugar chain include the nucleotide sequence represented by SEQ ID NO:3.

In addition, the host cell of the present invention can also be obtained without using an expression vector, by directly introducing an antisense oligonucleotide or ribozyme into a host cell, which is designed based on the nucleotide sequence encoding the enzyme relating to the synthesis of an intracellular sugar nucleotide, GDP-fucose or the enzyme relating to the modification of a sugar chain wherein 1-position of fucose is bound to 6-position of *N*-acetylglucosamine in the reducing end through α-bond in the complex *N*-glycoside-linked sugar chain.

The antisense oligonucleotide or ribozyme can be prepared in the usual method or using a DNA synthesizer. Specifically, it can be prepared based on the sequence information of an oligonucleotide having a corresponding sequence of continued 5 to 150 bases, preferably 5 to 60 bases, and more preferably 10 to 40 bases, among nucleotide sequences of a cDNA and a genome DNA encoding the enzyme relating to the synthesis of an intracellular sugar nucleotide, GDP-fucose or the enzyme relating to the modification of a sugar chain wherein 1-position of fucose is bound to 6-position of *N*-acetylglucosamine in the reducing end through α-bond in the complex *N*-glycoside-linked sugar chain, by synthesizing an oligonucleotide which corresponds to a sequence complementary to the oligonucleotide (antisense oligonucleotide) or a ribozyme comprising the oligonucleotide sequence.

Examples of the oligonucleotide include oligo RNA and derivatives of the oligonucleotide (hereinafter referred to as "oligonucleotide derivatives").

Examples of the oligonucleotide derivatives include oligonucleotide derivatives in which a phosphodiester bond in the oligonucleotide is converted into a phosphorothioate bond, an oligonucleotide derivative in which a phosphodiester bond in the oligonucleotide is converted into an N3'-P5' phosphoamidate bond, an oligonucleotide derivative in which ribose and a phosphodiester bond in the oligonucleotide are converted into a peptide-nucleic acid bond, an oligonucleotide derivative in which uracil in the oligonucleotide is substituted with C-5 propynyluracil, an oligonucleotide derivative in which uracil in the oligonucleotide is substituted with C-5 thiazoleuracil, an oligonucleotide derivative in which cytosine in the oligonucleotide is substituted with C-5 propynylcytosine, an oligonucleotide derivative in which cytosine in the oligonucleotide is substituted with phenoxazine-modified cytosine, an oligonucleotide derivative in which ribose in the oligonucleotide is substituted with 2'-O-propylribose and an oligonucleotide derivative in which ribose in the oligonucleotide is substituted with 2'-methoxyethoxyribose [Cell Technology, 16, 1463 (1997)].

(b) Preparation of the host cell of the present invention by homologous recombination

The host cell of the present invention can be produced by modifying a target gene on chromosome through a homologous recombination technique, using a gene encoding an enzyme relating to the synthesis of an intracellular sugar nucleotide, GDP-fucose or an enzyme relating to the modification of a sugar chain wherein 1-position of fucose is bound to 6-position of *N*-acetylglucosamine in the reducing end through α-bond in the complex *N*-glycoside-linked sugar chain as the target gene.

The target gene on the chromosome can be modified by using a method described in Manipulating the Mouse Embryo, A Laboratory Manual, Second Edition, Cold Spring Harbor Laboratory Press (1994) (hereinafter referred to as "*Manipulating the Mouse Embryo, A Laboratory Manual*"); Gene Targeting, A Practical Approach, IRL Press at Oxford

University Press (1993); Biomanual Series 8, Gene Targeting, Preparation of Mutant Mice using ES Cells, Yodo-sha (1995) (hereinafter referred to as "*Preparation of Mutant Mice using ES Cells*"); or the like, for example, as follows.

A genome DNA encoding an enzyme relating to the synthesis of an intracellular sugar nucleotide, GDP-fucose or an enzyme relating to the modification of a sugar chain wherein 1-position of fucose is bound to 6-position of *N*-acetylglucosamine in the reducing end through α-bond in the complex *N*-glycoside-linked sugar chain is prepared.

Based on the nucleotide sequence of the genome DNA, a target vector is prepared for homologous recombination of a target gene to be modified (e.g., structural gene of the enzyme relating to the synthesis of an intracellular sugar nucleotide, GDP-fucose or the enzyme relating to the modification of a sugar chain wherein 1-position of fucose is bound to 6-position of *N*-acetylglucosamine in the reducing end through α-bond in the complex *N*-glycoside-linked sugar chain, or a promoter gene).

The host cell of the present invention can be produced by introducing the prepared target vector into a host cell and selecting a cell in which homologous recombination occurred between the target gene and target vector.

As the host cell, any cell such as yeast, animal cell, insect cell or plant cell can be used, so long as it has a gene encoding the enzyme relating to the synthesis of an intracellular sugar nucleotide, GDP-fucose or the enzyme relating to the modification of a sugar chain wherein 1-position of fucose is bound to 6-position of *N*-acetylglucosamine in the reducing end through α-bond in the complex *N*-glycoside-linked sugar chain. Examples include the host cells which will be described later in the item 3.

Examples of the method for preparing a genome DNA encoding the enzyme relating to the synthesis of an intracellular sugar nucleotide, GDP-fucose or the enzyme relating to the modification of a sugar chain wherein 1-position of fucose is bound to 6-position of *N*-acetylglucosamine in the reducing end through α-bond in the complex *N*-glycoside-linked sugar chain include the methods described in the preparation of genome DNA in the item 1(1)(a) and the like.

Examples of the nucleotide sequence of genome DNA encoding the enzyme relating to the synthesis of an intracellular sugar nucleotide, GDP-fucose include the nucleotide sequence represented by SEQ ID NO:67 or 70. Examples of the nucleotide sequence of genome DNA encoding the enzyme relating to the modification of a sugar chain wherein 1-position of fucose is bound to 6-position of *N*-acetylglucosamine in the reducing end through α-bond in the complex *N*-glycoside-linked sugar chain include the nucleotide sequence represented by SEQ ID NO:3.

The target vector for use in the homologous recombination of the target gene can be prepared in accordance with a method described in Gene Targeting, A Practical Approach, IRL Press at Oxford University Press (1993); Biomanual Series 8, Gene Targeting, Preparation ofMutant Mice using ES Cells, Yodo-sha (1995); or the like. The target vector can be used as either a replacement type or an insertion type.

For introducing the target vector into various host cells, the methods for introducing recombinant vectors suited for various host cells, which will be described later in the item 3, can be used.

Examples of the method for efficiently selecting a homologous recombinant include a method such as the positive selection, promoter selection, negative selection or polyA selection described in Gene Targeting, A Practical Approach, IRL Press at Oxford University Press (1993); Biomanual Series 8, Gene Targeting, Preparation of Mutant Mice using ES Cells, Yodo-sha (1995); or the like. Examples of the method for selecting the homologous recombinant of interest from the selected cell lines include the Southern hybridization method for genome DNA (Molecular Cloning, Second Edition), PCR [PCR Protocols, Academic Press (1990)], and the like.

(c) Preparation of the host cell of the present invention by RDO method

The host cell of the present invention can be prepared by an RDO (RNA-DNA oligonucleotide) method by targeting a gene encoding an enzyme relating to the synthesis of an intracellular sugar nucleotide, GDP-fucose or an enzyme relating to the modification of a sugar chain wherein 1-position of fucose is bound to 6-position of *N*-acetylglucosamine in the reducing end through α-bond in the complex *N*-glycoside-linked sugar chain, for example, as follows

A cDNA or a genome DNA encoding an enzyme relating to the synthesis of an intracellular sugar nucleotide, GDP-fucose or an enzyme relating to the modification of a sugar chain wherein 1-position of fucose is bound to 6-position of *N*-acetylglucosamine in the reducing end through α-bond in the complex *N*-glycoside-linked sugar chain is prepared.

The nucleotide sequence of the prepared cDNA or genome DNA is determined.

Based on the determined DNA sequence, an appropriate length of an RDO construct comprising a DNA moiety which encodes the enzyme relating to the synthesis of an intracellular sugar nucleotide, GDP-fucose or the enzyme relating to the modification of a sugar chain wherein 1-position of fucose is bound to 6-position of *N*-acetylglucosamine in the reducing end through α-bond in the complex *N*-glycoside-linked sugar chain or a part of its non-translation region or an intron, is designed and synthesized.

The host cell of the present invention can be obtained by introducing the synthesized RDO into a host cell and then selecting a transformant in which a mutation occurred in the target enzyme, namely the enzyme relating to the synthesis of an intracellular sugar nucleotide, GDP-fucose or the enzyme relating to the modification of a sugar chain wherein 1-position of fucose is bound to 6-position of *N*-acetylglucosamine in the reducing end through α-bond in the

complex *N*-glycoside-linked sugar chain.

As the host cell, any cell such as yeast, animal cell, insect cell or plant cell can be used, so long as it has a gene encoding the target enzyme relating to the synthesis of an intracellular sugar nucleotide, GDP-fucose or of the target enzyme relating to the modification of a sugar chain wherein 1-position of fucose is bound to 6-position of *N*-acetylglucosamine in the reducing end through α-bond in the complex *N*-glycoside-linked sugar chain. Examples include the host cells which will be described later in the item 3.

Examples of the method for introducing RDO into various host cells include the methods for introducing recombinant vectors suited for various host cells, which will be described later in the item 3.

Examples of the method for preparing cDNA encoding the enzyme relating to the synthesis of an intracellular sugar nucleotide, GDP-fucose or the enzyme relating to the modification of a sugar chain wherein 1-position of fucose is bound to 6-position of *N*-acetylglucosamine in the reducing end through α-bond in the complex *N*-glycoside-linked sugar chain include the methods described in the preparation of DNA in the item 1(1)(a) and the like.

Examples of the method for preparing a genome DNA encoding the enzyme relating to the synthesis of an intracellular sugar nucleotide, GDP-fucose or the enzyme relating to the modification of a sugar chain wherein 1-position of fucose is bound to 6-position of *N*-acetylglucosamine in the reducing end through α-bond in the complex *N*-glycoside-linked sugar chain include the methods in preparation of genome DNA described in the item 1(1)(a) and the like.

The nucleotide sequence of the DNA can be determined by digesting it with appropriate restriction enzymes, cloning the fragments into a plasmid such as pBluescript SK(-) (manufactured by Stratagene) or the like, subjecting the clones to the reaction generally used as a method for analyzing a nucleotide sequence such as the dideoxy method [Proc. Natl. Acad. Sci. USA, 74, 5463 (1977)] of Sanger *et al.* or the like, and then analyzing the clones using an automatic nucleotide sequence analyzer such as A.L.F. DNA Sequencer (manufactured by Pharmacia) or the like.

The RDO can be prepared by a usual method or using a DNA synthesizer.

Examples of the method for selecting a cell in which a mutation occurred, by introducing the ROD into the host cell, in the gene encoding the enzyme relating to the synthesis of an intracellular sugar nucleotide, GDP-fucose or the enzyme relating to the modification of a sugar chain wherein 1-position of fucose is bound to 6-position of *N*-acetylglucosamine in the reducing end through α-bond in the complex *N*-glycoside-linked sugar chain include the methods for directly detecting mutations in chromosomal genes described in Molecular Cloning, Second Edition, Current Protocols in Molecular Biology and the like;

the methods described in the item 1(1)(a) for selecting a transformant through the evaluation of the activity of the introduced enzyme relating to the synthesis of an intracellular sugar nucleotide, GDP-fucose or the enzyme relating to the modification of a sugar chain wherein 1-position of fucose is bound to 6-position of *N*-acetylglucosamine in the reducing end through α-bond in the complex *N*-glycoside-linked sugar chain; the method for selecting a transformant using the sugar structure of a glycoprotein on the cell membrane which will be described later in the item 1(5); and the method for selecting a transformant on the basis of the sugar structure of the produced antibody molecule which will be described later in the item 5 or 6, and the like.

The construct of the ROD can be designed in accordance with the methods described in Science, 273, 1386 (1996); Nature Medicine, 4, 285 (1998); Hepatology, 25, 1462 (1997); Gene Therapy, 5, 1960 (1999); J Mol. Med., 75, 829 (1997); Proc. Natl. Acad. Sci. USA, 96, 8774 (1999); Proc. Natl. Acad. Sci. USA, 96, 8768 (1999); Nuc. Acids. Res., 27, 1323 (1999); Invest. Dematol., 111, 1172 (1998); Nature Biotech., 16, 1343 (1998); Nature Biotech., 18, 43 (2000); Nature Biotech., 18, 555 (2000); and the like.

(d) Preparation of the host cell of the present invention by RNAi method

The host cell of the present invention can be prepared by the RNAi (RNA interference) method by targeting a gene of an enzyme relating to the synthesis of an intracellular sugar nucleotide, GDP-fucose or of an enzyme relating to the modification of a sugar chain wherein 1-position of fucose is bound to 6-position of *N*-acetylglucosamine in the reducing end through α-bond in the complex *N*-glycoside-linked sugar chain, for example, as follows.

A cDNA encoding an enzyme relating to the synthesis of an intracellular sugar nucleotide, GDP-fucose or an enzyme relating to the modification of a sugar chain wherein 1-position of fucose is bound to 6-position of *N*-acetylglucosamine in the reducing end through α-bond in the complex *N*-glycoside-linked sugar chain is prepared.

The nucleotide sequence of the prepared cDNA is determined.

Based on the determined DNA sequence, an appropriate length of an RNAi gene construct comprising the DNA coding moiety encoding the enzyme relating to the synthesis of an intracellular sugar nucleotide, GDP-fucose or the enzyme relating to the modification of a sugar chain wherein 1-position of fucose is bound to 6-position of *N*-acetylglucosamine in the reducing end through α-bond in the complex *N*-glycoside-linked sugar chain or a part of its non-translation region, is designed.

In order to express the RNAi gene in a cell, a recombinant vector is prepared by inserting a fragment or full length of the prepared DNA into downstream of the promoter of an appropriate expression vector.

A transformant is obtained by introducing the recombinant vector into a host cell suitable for the expression vector.

The host cell of the present invention can be obtained by selecting a transformant on the basis of the activity of the enzyme relating to the synthesis of an intracellular sugar nucleotide, GDP-fucose or the enzyme relating to the modification of a sugar chain wherein 1-position of fucose is bound to 6-position of *N*-acetylglucosamine in the reducing end through α-bond in the complex *N*-glycoside-linked sugar chain, or the sugar chain structure of a glycoprotein on the cell membrane or of the produced antibody molecule.

As the host cell, any cell such as yeast, animal cell, insect cell or plant cell can be used, so long as it has a gene encoding the target enzyme relating to the synthesis of an intracellular sugar nucleotide, GDP-fucose or the target enzyme relating to the modification of a sugar chain wherein 1-position of fucose is bound to 6-position of *N*-acetylglucosamine in the reducing end through α-bond in the complex *N*-glycoside-linked sugar chain. Examples include the host cells which will be described later in the item 3.

As the expression vector, a vector which is autonomously replicable in the host cell or can be integrated into the chromosome and comprises a promoter at such a position that the designed RNAi gene can be transferred is used. Examples include the expression vectors which will be described later in the item 3.

As the method for introducing a gene into various host cells, the methods for introducing recombinant vectors suitable for various host cells, which will be described later in the item 3, can be used.

Examples of the method for selecting a transformant on the basis of the activity of the enzyme relating to the synthesis of an intracellular sugar nucleotide, GDP-fucose or the enzyme relating to the modification of a sugar chain wherein 1-position of fucose is bound to 6-position of *N*-acetylglucosamine in the reducing end through α-bond in the complex *N*-glycoside-linked sugar chain include the methods described in the item 1(1)(a).

Examples of the method for selecting a transformant on the basis of the sugar chain structure of a glycoprotein on the cell membrane include the methods which will be described later in the item 1(5). Examples of the method for selecting a transformant on the basis of the sugar chain structure of a produced antibody molecule include the methods which will be described later in the item 5 or 6.

Examples of the method for preparing cDNA encoding the enzyme relating to the synthesis of an intracellular sugar nucleotide, GDP-fucose or the enzyme relating to the modification of a sugar chain wherein 1-position of fucose is bound to 6-position of *N*-acetylglucosamine in the reducing end through α-bond in the complex *N*-glycoside-linked sugar chain include the methods described in preparation of DNA in the item 1(1)(a) and the like.

In addition, the host cell of the present invention can also be obtained without using an expression vector, by directly introducing an RNAi gene designed based on the nucleotide sequence encoding the enzyme relating to the synthesis of an intracellular sugar nucleotide, GDP-fucose or the enzyme relating to the modification of a sugar chain wherein 1-position of fucose is bound to 6-position of *N*-acetylglucosamine in the reducing end through α-bond in the complex *N*-glycoside-linked sugar chain.

The RNAi gene can be prepared in the usual method or using a DNA synthesizer.

The RNAi gene construct can be designed in accordance with the methods described in Nature, 391, 806 (1998); Proc. Natl. Acad. Sci. USA, 95, 15502 (1998); Nature, 395, 854 (1998); Proc. Natl. Acad. Sci. USA, 96, 5049 (1999); Cell, 95, 1017 (1998); Proc. Natl. Acad. Sci. USA, 96, 1451 (1999); Proc. Natl. Acad. Sci. USA, 95, 13959 (1998); Nature Cell Biol., 2, 70 (2000); and the like.

(e) Preparation of the host cell of the present invention by a method using transposon

The host cell of the present invention can be prepared by inducing mutation using a transposon system described in Nature Genet., 25, 35 (2000) or the like, and then by selecting a mutant on the basis of the activity of the enzyme relating to the synthesis of an intracellular sugar nucleotide, GDP-fucose or the enzyme relating to the modification of a sugar chain wherein 1-position of fucose is bound to 6-position of *N*-acetylglucosamine in the reducing end through α-bond in the complex *N*-glycoside-linked sugar chain, or the sugar chain structure of a glycoprotein of a produced antibody molecule or on the cell membrane.

The transposon system is a system in which a mutation is induced by randomly inserting an exogenous gene into chromosome, wherein an exogenous gene interposed between transposons is generally used as a vector for inducing a mutation, and a transposase expression vector for randomly inserting the gene into chromosome is introduced into the cell at the same time.

Any transposase can be used, so long as it is suitable for the sequence of the transposon to be used.

As the exogenous gene, any gene can be used, so long as it can induce a mutation in the DNA of a host cell.

As the host cell, any cell such as yeast, animal cell, insect cell or plant cell can be used, so long as it has a gene encoding the target enzyme relating to the synthesis of an intracellular sugar nucleotide, GDP-fucose or of the target enzyme relating to the modification of a sugar chain wherein 1-position of fucose is bound to 6-position of *N*-acetylglucosamine in the reducing end through α-bond in the complex *N*-glycoside-linked sugar chain. Examples

include the host cells which will be described later in the item 3. For introducing the gene into various host cells, the method for introducing recombinant vectors suitable for various host cells, which will be described later in the item 3, can be used.

Examples of the method for selecting a mutant on the basis of the activity of the enzyme relating to the synthesis of an intracellular sugar nucleotide, GDP-fucose or the enzyme relating to the modification of a sugar chain wherein 1-position of fucose is bound to 6-position of *N*-acetylglucosamine in the reducing end through α-bond in the complex *N*-glycoside-linked sugar chain include the methods described in the item 1(1)(a).

Examples of the method for selecting a mutant on the basis of the sugar chain structure of a glycoprotein on the cell membrane include the methods which will be described later in the item 1(5). Examples of the method for selecting a mutant on the basis of the sugar chain structure of a produced antibody molecule include the methods which will be described later in the item 5 or 6.

(2) Method for introducing a dominant negative mutant of a gene encoding an enzyme

The host cell of the present invention can be prepared by targeting a gene encoding an enzyme relating to the synthesis of an intracellular sugar nucleotide, GDP-fucose or an enzyme relating to the modification of a sugar chain wherein 1-position of fucose is bound to 6-position of *N*-acetylglucosamine in the reducing end through α-bond in the complex *N*-glycoside-linked sugar chain, using a technique for introducing a dominant negative mutant of the enzyme. Examples of the enzyme relating to the synthesis of an intracellular sugar nucleotide, GDP-fucose include GMD, Fx, GFPP, fucokinase and the like. Examples of the enzyme relating to the modification of a sugar chain wherein 1-position of fucose is bound to 6-position of *N*-acetylglucosamine in the reducing end through α-bond in the complex *N*-glycoside-linked sugar chain include α-1,6-fucosyltransferase, α-L-fucosidase and the like.

The enzymes catalyze specific reactions having substrate specificity, and dominant negative mutants of the enzymes can be prepared by disrupting the active center of the enzymes which catalyze the catalytic activity having substrate specificity. The method for preparing a dominant negative mutant is specifically described as follows with reference to GMD among the target enzymes.

As a result of the analysis of the three-dimensional structure of *E. coli*-derived GMD, it has been revealed that 4 amino acids (threonine at position 133, glutamic acid at position 135, tyrosine at position 157 and lysine at position 161) have an important function on the enzyme activity (Structure, 8, 2, 2000). That is, when mutants were prepared by substituting the 4 amino acids with other different amino acids based on the three-dimensional structure information, the enzyme activity of all of the mutants was significantly decreased. On the other hand, changes in the ability of GMD to bind to GMD coenzyme NADP and its substrate GDP-mannose were hardly observed in the mutants. Accordingly, a dominant negative mutant can be prepared by substituting the 4 amino acids which control the enzyme activity of GMD. For example, in GMD (SEQ ID NO:65) derived from CHO cell, a dominant negative mutant can be prepared by substituting threonine position 155, glutamic acid at position 157, tyrosine at position 179 and lysine at position 183 with other amino acids, by comparing the homology and predicting the three-dimensional structure using the amino acid sequence information based on the results of the *E. coli*-derived GMD. Such a gene into which amino acid substitution is introduced can be prepared by the site-directed mutagenesis described in Molecular Cloning, Second Edition, Current Protocols in Molecular Biology or the like.

The host cell of the present invention can be prepared in accordance with the method described in Molecular Cloning, Second Edition, Current Protocols in Molecular Biology or the like, using the prepared dominant negative mutant gene of the target enzyme, for example, as follows.

A gene encoding a dominant negative mutant (hereinafter referred to as "dominant negative mutant gene") of the enzyme relating to the synthesis of an intracellular sugar nucleotide, GDP-fucose or the enzyme relating to the modification of a sugar chain wherein 1-position of fucose is bound to 6-position of *N*-acetylglucosamine in the reducing end through α-bond in the complex *N*-glycoside-linked sugar chain is prepared.

Based on the prepared full length DNA of dominant negative mutant gene, a DNA fragment of an appropriate length containing a moiety encoding the protein is prepared, if necessary.

A recombinant vector is produced by inserting the DNA fragment or full length DNA into downstream of the promoter of an appropriate expression vector.

A transformant is obtained by introducing the recombinant vector into a host cell suitable for the expression vector.

The host cell of the present invention can be prepared by selecting a transformant on the basis of the activity of the enzyme relating to the synthesis of an intracellular sugar nucleotide, GDP-fucose or the activity of the enzyme relating to the modification of a sugar chain wherein 1-position of fucose is bound to 6-position of *N*-acetylglucosamine in the reducing end through α-bond in the complex *N*-glycoside-linked sugar chain, or the sugar chain structure of a glycoprotein of a produced antibody molecule or on the cell membrane.

As the host cell, any cell such as yeast, animal cell, insect cell or plant cell can be used, so long as it has a

gene encoding the target enzyme relating to the synthesis of an intracellular sugar nucleotide, GDP-fucose or of the target enzyme relating to the modification of a sugar chain wherein 1-position of fucose is bound to 6-position of *N*-acetylglucosamine in the reducing end through α-bond in the complex *N*-glycoside-linked sugar chain. Examples include the host cells which will be described later in the item 3.

As the expression vector, a vector which is autonomously replicable in the host cell or can be integrated into the chromosome and comprises a promoter at a position where transcription of the DNA encoding the dominant negative mutant of interest can be effected is used. Examples include the expression vectors which will be described later in the item 3.

For introducing the gene into various host cells, the method for introducing recombinant vectors suitable for various host cells, which will be described later in the item 3, can be used.

Examples of the method for selecting a transformant on the basis of the activity of the enzyme relating to the synthesis of an intracellular sugar nucleotide, GDP-fucose or the enzyme relating to the modification of a sugar chain wherein 1-position of fucose is bound to 6-position of *N*-acetylglucosamine in the reducing end through α-bond in the complex *N*-glycoside-linked sugar chain include the methods described in the item 1(1)(a).

Examples of the method for selecting a transformant on the basis of the sugar chain structure of a glycoprotein on the cell membrane include the methods which will be described later in the item 1(5). Examples of the method for selecting a transformant on the basis of the sugar chain structure of a produced antibody molecule include the methods which will be described later in the item 5 or 6.

(3) Method for introducing a mutation into an enzyme

The host cell of the present invention can be prepared by introducing a mutation into a gene encoding an enzyme relating to the synthesis of an intracellular sugar nucleotide, GDP-fucose or an enzyme relating to the modification of a sugar chain wherein 1-position of fucose is bound to 6-position of *N*-acetylglucosamine in the reducing end through α-bond in the complex *N*-glycoside-linked sugar chain, and then by selecting a cell line of interest in which the mutation occurred in the enzyme.

Examples of the enzyme relating to the synthesis of an intracellular sugar nucleotide, GDP-fucose include GMD, Fx, GFPP, fucokinase and the like. Examples of the enzyme relating to the modification of a sugar chain wherein 1-position of fucose is bound to 6-position of *N*-acetylglucosamine in the reducing end through α-bond in the complex *N*-glycoside-linked sugar chain include α-1,6-fucosyltransferase, α-L-fucosidase and the like.

Examples of the method include 1) a method in which a desired cell line is selected from mutants obtained by a mutation-inducing treatment of a parent cell line with a mutagen or spontaneously generated mutants, on the basis of the activity of an enzyme relating to the synthesis of an intracellular sugar nucleotide, GDP-fucose or the activity of an enzyme relating to the modification of a sugar chain wherein 1-position of fucose is bound to 6-position of *N*-acetylglucosamine in the reducing end through α-bond in the complex *N*-glycoside-linked sugar chain, 2) a method in which a desired cell line is selected from mutants obtained by a mutation-inducing treatment of a parent cell line with a mutagen or spontaneously generated mutants, on the basis of the sugar chain structure of a produced antibody molecule and 3) a method in which a desired cell line is selected from mutants obtained by a mutation-inducing treatment of a parent cell line with a mutagen or spontaneously generated mutants, on the basis of the sugar chain structure of a glycoprotein on the cell membrane.

As the mutation-inducing treatment, any treatment can be used, so long as it can induce a point mutation or a deletion or frame shift mutation in the DNA of cells of the parent cell line.

Examples include treatment with ethyl nitrosourea, nitrosoguanidine, benzopyrene or an acridine pigment and treatment with radiation. Also, various alkylating agents and carcinogens can be used as mutagens. Examples of the method for allowing a mutagen to act upon cells include the methods described in Tissue Culture Techniques, 3rd edition (Asakura Shoten), edited by Japanese Tissue Culture Association (1996), Nature Genet., 24, 314 (2000) and the like.

Examples of the spontaneously generated mutant include mutants which are spontaneously formed by continuing subculture under general cell culture conditions without applying special mutation-inducing treatment.

Examples of the method for measuring the activity of the enzyme relating to the synthesis of an intracellular sugar nucleotide, GDP-fucose or the activity of the enzyme relating to the modification of a sugar chain wherein 1-position of fucose is bound to 6-position of *N*-acetylglucosamine in the reducing end through α-bond in the complex *N*-glycoside-linked sugar chain include the methods described in the item 1(1)(a). Examples of the method for discriminating the sugar chain structure of a prepared antibody molecule include the methods which will be described later in the item 5 or 6. Examples of the method for discriminating the sugar chain structure of a glycoprotein on the cell membrane include the methods which will be described later in the item 1(5).

(4) Method for inhibiting transcription and/or translation of a gene encoding an enzyme

The host cell of the present invention can be prepared by inhibiting transcription and/or translation of a target gene through a method such as the antisense RNA/DNA technique [Bioscience and Industry, 50, 322 (1992); Chemistry, 46, 681 (1991); Biotechnology, 9, 358 (1992); Trends in Biotechnology, 10, 87 (1992); Trends in Biotechnology, 10, 152 (1992); Cell Engineering, 16, 1463 (1997)], the triple helix technique [Trends in Biotechnology, 10, 132 (1992)] or the like, using a gene encoding an enzyme relating to the synthesis of an intracellular sugar nucleotide, GDP-fucose and/or an enzyme relating to the modification of a sugar chain wherein 1-position of fucose is bound to 6-position of *N*-acetylglucosamine in the reducing end through α-bond in the complex *N*-glycoside-linked sugar chain, as the target.
Examples of the enzyme relating to the synthesis of an intracellular sugar nucleotide, GDP-fucose include GMD, Fx, GFPP, fucokinase and the like. Examples of the enzyme relating to the modification of a sugar chain wherein 1-position of fucose is bound to 6-position of *N*-acetylglucosamine in the reducing end through α-bond in the complex *N*-glycoside-linked sugar chain include α-1,6-fucosyltransferase, α-L-fucosidase and the like.

(5) Method for selecting a cell line resistant to a lectin which recognizes a sugar chain structure in which 1-position of fucose is bound to 6-position of *N*-acetylglucosamine in the reducing end through α-bond in the *N*-glycoside-linked sugar chain

The host cell of the present invention can be prepared by using a method for selecting a cell line resistant to a lectin which recognizes a sugar chain structure in which 1-position of fucose is bound to 6-position of *N*-acetylglucosamine in the reducing end through α-bond in the *N*-glycoside-linked sugar chain.
Examples of the method for selecting a cell line resistant to a lectin which recognizes a sugar chain structure in which 1-position of fucose is bound to 6-position of *N*-acetylglucosamine in the reducing end through α-bond in the *N*-glycoside-linked sugar chain include the methods using lectin described in Somatic Cell Mol. Genet., 12, 51 (1986) and the like. As the lectin, any lectin can be used, so long as it is a lectin which recognizes a sugar chain structure in which 1-position of fucose is bound to 6-position of *N*-acetylglucosamine in the reducing end through α-bond in the *N*-glycoside-linked sugar chain. Examples include a *Lens culinaris* lectin LCA (lentil agglutinin derived from *Lens culinaris*), a pea lectin PSA (pea lectin derived from *Pisum sativum*), a broad bean lectin VFA (agglutinin derived from *Vicia faba*), an *Aleuria aurantia* lectin AAL (lectin derived from *Aleuria aurantia*) and the like.
Specifically, the cell line described herein resistant to a lectin which recognizes a sugar chain structure in which 1-position of fucose is bound to 6-position of *N*-acetylglucosamine in the reducing end through α-bond in the *N*-glycoside-linked sugar chain can be selected by culturing cells for 1 day to 2 weeks, preferably from 1 day to 1 week, using a medium comprising the lectin at a concentration of 1 μg/ml to 1 mg/ml, subculturing surviving cells or picking up a colony and transferring it into a culture vessel, and subsequently continuing the culturing using the lectin-containing medium. Examples of the cell line obtained by the method include CHO/CCR4-LCA Nega-13 (FERM BP-7756) obtained in Example 14(2) which will be described later.

2. Preparation of a transgenic non-human animal or plant or the progenies thereof of the present invention

The transgenic non-human animal or plant or the progenies thereof of the present invention is a transgenic non-human animal or plant or the progenies thereof in which a genome gene is modified in such a manner that the activity of an enzyme relating to the modification of a sugar chain of an antibody molecule can be controlled, and it can be prepared according to the method similar to that in the item 1, using a gene encoding an enzyme relating to the synthesis of an intracellular sugar nucleotide, GDP-fucose or an enzyme relating to the modification of a sugar chain wherein 1-position of fucose is bound to 6-position of *N*-acetylglucosamine in the reducing end through α-bond in the complex *N*-glycoside-linked sugar chain, as the target.
In a transgenic non-human animal, the embryonic stem cell of the present invention in which the activity of the enzyme relating to the synthesis of an intracellular sugar nucleotide, GDP-fucose or the activity of the enzyme relating to the modification of a sugar chain wherein 1-position of fucose is bound to 6-position of *N*-acetylglucosamine in the reducing end through α-bond in the complex *N*-glycoside-linked sugar chain is controlled can be prepared applying the method similar to that in the item 1 to an embryonic stem cell of the intended non-human animal such as cattle, sheep, goat, pig, horse, mouse, rat, fowl, monkey, rabbit or the like.
Specifically, a mutant clone is prepared in which a gene encoding the enzyme relating to the synthesis of an intracellular sugar nucleotide, GDP-fucose or the enzyme relating to the modification of a sugar chain wherein 1-position of fucose is bound to 6-position of *N*-acetylglucosamine in the reducing end through α-bond in the complex *N*-glycoside-linked sugar chain is inactivated or substituted with any sequence, by a known homologous recombination technique [e.g., Nature, 326, 6110, 295 (1987); Cell, 51, 3, 503 (1987); or the like]. Using the prepared mutant clone, a chimeric individual comprising an embryonic stem cell clone and a normal cell can be prepared by an injection chimera method

into blastocyst of fertilized egg of an animal or by an aggregation chimera method. The chimeric individual is crossed with a normal individual, so that a transgenic non-human animal in which the activity of the enzyme relating to the synthesis of an intracellular sugar nucleotide, GDP-fucose or the activity of the enzyme relating to the modification of a sugar chain wherein 1-position of fucose is bound to 6-position of *N*-acetylglucosamine in the reducing end through α-bond in the complex N-glycoside-linked sugar chain is decreased or deleted in the whole body cells can be obtained.

Also, a non-human fertilized egg cell of the present invention in which the activity of an enzyme relating to the synthesis of an intracellular sugar nucleotide, GDP-fucose or the activity of an enzyme relating to the modification of a sugar chain wherein 1-position of fucose is bound to 6-position of *N*-acetylglucosamine in the reducing end through α-bond in the complex *N*-glycoside-linked sugar chain is deleted can be prepared by applying the method similar to that in the item 1 to fertilized egg of a non-human animal of interest such as cattle, sheep, goat, pig, horse, mouse, rat, fowl, monkey, rabbit or the like.

A transgenic non-human animal in which the activity of an enzyme relating to the synthesis of an intracellular sugar nucleotide, GDP-fucose or the activity of an enzyme relating to the modification of a sugar chain wherein 1-position of fucose is bound to 6-position of *N*-acetylglucosamine in the reducing end through α-bond in the complex *N*-glycoside-linked sugar chain is decreased can be prepared by transplanting the prepared fertilized egg cell into the oviduct or uterus of a pseudopregnant female using the embryo transplantation method described in Manipulating Mouse Embryo, Second Edition or the like, followed by childbirth by the animal.

In a transgenic plant, the callus of the present invention in which the activity of an enzyme relating to the synthesis of an intracellular sugar nucleotide, GDP-fucose or the activity of an enzyme relating to the modification of a sugar chain wherein 1-position of fucose is bound to 6-position of *N*-acetylglucosamine in the reducing end through α-bond in the complex *N*-glycoside-linked sugar chain is deleted can be prepared by applying the method similar to that in the item 1 to a callus or cell of the plant of interest.

A transgenic plant in which the activity of an enzyme relating to the synthesis of an intracellular sugar nucleotide, GDP-fucose or the activity of an enzyme relating to the modification of a sugar chain wherein 1-position of fucose is bound to 6-position of *N*-acetylglucosamine in the reducing end through α-bond in the complex *N*-glycoside-linked sugar chain is decreased can be prepared by culturing the prepared callus using a medium comprising auxin and cytokinin to redifferentite it in accordance with a known method [Tissue Culture, 20 (1994); Tissue Culture, 21 (1995); Trends in Biotechnology, 15, 45 (1997)].

3. Process for producing an antibody composition

The antibody composition can be obtained by expressing it in a host cell using the methods described in Molecular Cloning, Second Edition; Current Protocols in Molecular Biology; Antibodies, A Laboratory Manual, Cold Spring Harbor Laboratory, 1988 (hereinafter referred also to as "*Antibodies*"); Monoclonal Antibodies: Principles and Practice, Third Edition, Acad. Press, 1993 (hereinafter referred also to as "*Monoclonal Antibodies*"); and Antibody Engineering, A Practical Approach, IRL Press at Oxford University Press (hereinafter referred also to as "*Antibody Engineering*"), for example, as follows.

A full length cDNA encoding an antibody molecule is prepared, and an appropriate length of a DNA fragment comprising a moiety encoding the antibody molecule is prepared.

A recombinant vector is prepared by inserting the DNA fragment or the full length cDNA into downstream of the promoter of an appropriate expression vector.

A transformant which produces the antibody molecule can be obtained by introducing the recombinant vector into a host cell suitable for the expression vector.

As the host cell, any of yeast, animal cell, insect cell, plant cell or the like can be used, so long as it can express the gene of interest.

A cell such as yeast, animal cell, insect cell, plant cell or the like into which an enzyme relating to the modification of an *N*-glycoside-linked sugar chain which binds to the Fc region of the antibody molecule is introduced by a genetic engineering technique can also be used as the host cell.

As the expression vector, a vector which is autonomously replicable in the host cell or can be integrated into the chromosome and comprises a promoter at such a position that the DNA encoding the antibody molecule of interest can be transferred is used.

The cDNA can be prepared from a human or non-human tissue or cell using, e.g., a probe primer specific for the antibody molecule of interest, in accordance with the methods described in the preparation of DNA in the item 1(1)(a).

When a yeast is used as the host cell, examples of the expression vector include YEP13 (ATCC 37115), YEp24 (ATCC 37051), YCp50 (ATCC 37419) and the like.

Any promoter can be used, so long as it can function in yeast. Examples include a promoter of a gene of the glycolytic pathway such as a hexose kinase gene, *etc.,* PH05 promoter, PGK promoter, GAP promoter, ADH promoter, gal 1 promoter, gal 10 promoter, heat shock protein promoter, MF α1 promoter, CUP 1 promoter and the like.

Examples of the host cell include microorganisms belonging to the genus *Saccharomyces,* the genus *Schizosaccharomyces,* the genus *Kluyveromyces,* the genus *Trichosporon,* the genus *Schwanniomyces* and the like, such as *Saccharomyces cerevisiae, Schizosaccharomyces pombe, Kluyveromyces lactis, Trichosporon pullulans* and *Schwanniomyces alluvius, etc.*

As the method for introducing the recombinant vector, any method can be used, so long as it can introduce DNA into yeast. Examples include electroporation [Methods in Enzymology, 194, 182 (1990)], spheroplast method [Proc. Natl. Acad. Sci. USA, 84, 1929 (1978)], lithium acetate method [J. Bacteriol., 153, 163 (1983)], a method described in Proc. Natl. Acad. Sci. USA, 75, 1929 (1978) and the like.

When an animal cell is used as the host, examples of the expression vector include pcDNAI, pcDM8 (available from Funakoshi), pAGE107 [Japanese Published Examined Patent Application No. 22979/91; Cytotechnology, 3, 133 (1990)], pAS3-3 (Japanese Published Examined Patent Application No. 227075/90), pCDM8 [Nature, 329, 840 (1987)], pcDNAI/Amp (manufactured by Invitrogen), pREP4 (manufactured by Invitrogen), pAGE103 [J. Biochemistry, 101, 1307 (1987)], pAGE210 and the like.

Any promoter can be used, so long as it can function in an animal cell. Examples include a promoter of IE (immediate early) gene of cytomegalovirus (CMV), an early promoter of SV40, a promoter of retrovirus, a promoter of metallothionein, a heat shock promoter, an SRα promoter and the like. Also, an enhancer of the IE gene of human CMV may be used together with the promoter.

Examples of the host cell include a human cell such as Namalwa cell, a monkey cell such as COS cell, a Chinese hamster cell such as CHO cell or HBT5637 (Japanese Published Examined Patent Application No. 299/88), a rat myeloma cell, a mouse myeloma cell, a cell derived from syrian hamster kidney, an embryonic stem cell, a fertilized egg cell and the like.

As the method for introducing the recombinant vector, any method can be used, so long as it can introduce DNA into an animal cell. Examples include electroporation [Cytotechnology, 3, 133 (1990)], the calcium phosphate method (Japanese Published Examined Patent Application No. 227075/90), the lipofection method [Proc. Natl. Acad. Sci. USA, 84, 7413 (1987)], the injection method [Manipulating the Mouse Embryo, A Laboratory Manual], a method using particle gun (gene gun) (Japanese Patent No. 2606856, Japanese Patent No. 2517813), the DEAE-dextran method [Biomanual Series 4-Gene Transfer and Expression Analysis (Yodo-sha), edited by Takashi Yokota and Kenichi Arai (1994)], the virus vector method (Manipulating Mouse Embryo, Second Edition) and the like.

When an insect cell is used as the host, the protein can be expressed by the method described in Current Protocols in Molecular Biology, Baculovirus Expression Vectors, A Laboratory Manual, W.H. Freeman and Company, New York (1992), Bio/Technology, 6, 47 (1988) or the like.

That is, the protein can be expressed by simultaneously introducing a recombinant gene-introducing vector and a baculovirus into an insect cell to obtain a recombinant virus in an insect cell culture supernatant and then infecting the insect cell with the recombinant virus.

Examples of the gene introducing vector used in the method include pVL1392, pVL1393, pBlueBacIII (all manufactured by Invitrogen) and the like.

Examples of the baculovirus include *Autographa californica* nuclear polyhedrosis virus which is infected with an insect of the family *Barathra.*

Examples of the insect cell include *Spodoptera frugiperda* oocytes Sf9 and Sf21 [Current Protocols in Molecular Biology, Baculovirus Expression Vectors, A Laboratory Manual, W.H. Freeman and Company, New York (1992)], a *Trichoplusia ni* oocyte High 5 (manufactured by Invitrogen) and the like.

Examples of the method for the simultaneously introducing the recombinant gene-introducing vector and the baculovirus for preparing the recombinant virus include the calcium phosphate method (Japanese Published Examined Patent Application No. 227075/90), the lipofection method [Proc. Natl. Acad. Sci. USA, 84, 7413 (1987)] and the like.

When a plant cell is used as the host, examples of the expression vector include Ti plasmid, tobacco mosaic virus and the like.

As the promoter, any promoter can be used, so long as it can function in a plant cell. Examples include cauliflower mosaic virus (CaMV) 35S promoter, rice actin 1 promoter and the like.

Examples of the host cell include plant cells of tobacco, potato, tomato, carrot, soybean, rape, alfalfa, rice, wheat, barley, *etc*., and the like.

As the method for introducing the recombinant vector, any method can be used, so long as it can introduce DNA into a plant cell. Examples include a method using *Agrobacterium* (Japanese Published Examined Patent Application No. 140885/84, Japanese Published Examined Patent Application No. 70080/85, WO 94/00977), electroporation (Japanese Published Examined Patent Application No. 251887/85), a method using a particle gun (gene gun) (Japanese Patent No. 2606856, Japanese Patent No. 2517813) and the like.

As the method for expressing a gene, secretion production, expression of a fusion protein of the Fc region with other protein and the like can be carried out in accordance with the method described in Molecular Cloning, Second Edition or the like, in addition to the direct expression.

When a gene is expressed by a bacterium, a yeast, an animal cell, an insect cell or a plant cell into which a gene relating to the synthesis of a sugar chain is introduced, an antibody molecule to which a sugar or a sugar chain is added by the introduced gene can be obtained.

An antibody composition can be obtained by culturing the obtained transformant in a medium to produce and accumulate the antibody molecule in the culture and then recovering it from the resulting culture. The method for culturing the transformant using a medium can be carried out in accordance with a general method which is used for the culturing of host cells.

As the medium for culturing a transformant obtained using a prokaryote such as *Escherichia coli etc.* or a eukaryote such as yeast *etc.* as the host cell, the medium may be either a natural medium or a synthetic medium, so long as it comprises materials such as a carbon source, a nitrogen source, an inorganic salt and the like which can be assimilated by the organism and culturing of the transformant can be efficiently carried out.

As the carbon source, those which can be assimilated by the organism can be used. Examples include carbohydrates such as glucose, fructose, sucrose, molasses containing them, starch, starch hydrolysate, *etc*.; organic acids such as acetic acid, propionic acid, *etc*.; alcohols such as ethanol, propanol, *etc*.; and the like.

Examples of the nitrogen source include ammonia; ammonium salts of inorganic acid or organic acid such as ammonium chloride, ammonium sulfate, ammonium acetate, ammonium phosphate, *etc*.; other nitrogen-containing compounds; peptone; meat extract; yeast extract; corn steep liquor; casein hydrolysate; soybean meal; soybean meal hydrolysate; various fermented cells and hydrolysates thereof; and the like.

Examples of the inorganic material include potassium dihydrogen phosphate, dipotassium hydrogen phosphate, magnesium phosphate, magnesium sulfate, sodium chloride, ferrous sulfate, manganese sulfate, copper sulfate, calcium carbonate, and the like

The culturing is carried out generally under aerobic conditions such as a shaking culture, submerged-aeration stirring culture or the like. The culturing temperature is preferably 15 to 40°C, and the culturing time is generally 16 hours to 7 days. During the culturing, the pH is maintained at 3.0 to 9.0. The pH is adjusted using an inorganic or organic acid, an alkali solution, urea, calcium carbonate, ammonia or the like.

If necessary, an antibiotic such as ampicillin, tetracycline or the like may be added to the medium during the culturing.

When a microorganism transformed with a recombinant vector obtained using an inducible promoter as the promoter is cultured, an inducer may be added to the medium, if necessary. For example, when a microorganism transformed with a recombinant vector obtained using *lac* promoter is cultured, isopropyl-β-D-thiogalactopyranoside may be added to the medium, and when a microorganism transformed with a recombinant vector obtained using *trp* promoter is cultured, indoleacrylic acid may be added to the medium.

When a transformant obtained using an animal cell as the host cell is cultured, examples of the medium include generally used RPMI 1640 medium [The Journal of the American Medical Association, 199, 519 (1967)], Eagle's MEM medium [Science, 122, 501 (1952)], Dulbecco's modified MEM medium [Virology, 8, 396 (1959)], 199 medium [Proceeding of the Society for the Biological Medicine, 73, 1 (1950)] and Whitten's medium [Developmental Engineering Experimentation Manual-Preparation of Transgenic Mice (Kodan-sha), edited by M. Katshuki (1987)], the media to which fetal calf serum, *etc.* is added, and the like.

The culturing is carried out generally at a pH of 6 to 8 and 30 to 40°C for 1 to 7 days in the presence of 5% $CO_2$. If necessary, an antibiotic such as kanamycin, penicillin or the like may be added to the medium during the culturing.

Examples of the medium for use in the culturing of a transformant obtained using an insect cell as the host include usually used TNM-FH medium (manufactured by Pharmingen), Sf-900 II SFM medium (manufactured by Life Technologies), ExCell 400 and ExCell 405 (both manufactured by JRH Biosciences), Grace's Insect Medium [Nature, 195, 788 (1962)] and the like.

The culturing is carried out generally at a medium pH of 6 to 7 and 25 to 30°C for 1 to 5 days.

In addition, antibiotics such as gentamicin may be added to the medium during the culturing as occasion demands.

A transformant obtained using a plant cell as the host can be cultured as a cell or by differentiating it into a plant cell or organ. Examples of the medium for culturing the transformant include generally used Murashige and Skoog (MS) medium and White medium, the media to which a plant hormone such as auxin, cytokinin, *etc.* is added, and the like.

The culturing is carried out generally at a pH of 5 to 9 and 20 to 40°C for 3 to 60 days.

If necessary, an antibiotic such as kanamycin, hygromycin or the like may be added to the medium during the culturing.

Accordingly, an antibody composition can be produced by culturing a transformant derived from a microorganism, an animal cell or a plant cell, which comprises a recombinant vector into which a DNA encoding an antibody molecule is inserted, in accordance with a general culturing method, to thereby produce and accumulate the antibody composition, and then recovering the antibody composition from the culture.

As the method for expressing the gene, secretion production, expression of a fusion protein and the like can

be carried out in accordance with the method described in Molecular Cloning, Second Edition, in addition to the direct expression.

Examples of the process for producing an antibody composition include a method of intracellular expression in a host cell, a method of extracellular secretion from a host cell, and a method of production on a host cell membrane outer envelope. The method can be selected by changing the host cell used or the structure of the antibody composition produced.

When the antibody composition of the present disclosure is produced in a host cell or on a host cell membrane outer envelope, it can be positively secreted extracellularly in accordance with the method of Paulson et al. [J. Biol. Chem., 264, 17619 (1989)], the method of Lowe et al. [Proc. Natl. Acad. Sci. USA, 86, 8227 (1989), Genes Develop., 4, 1288 (1990)], the methods described in Japanese Published Examined Patent Application No. 336963/93 and Japanese Published Examined Patent Application No. 823021/94 and the like.

That is, an antibody molecule of interest can be positively secreted extracellularly from a host cell by inserting a DNA encoding the antibody molecule and a DNA encoding a signal peptide suitable for the expression of the antibody molecule into an expression vector using a gene recombination technique, introducing the expression vector into the host cell and then expressing the antibody molecule.

Also, its production amount can be increased in accordance with the method described in Japanese Published Examined Patent Application No. 227075/90 using a gene amplification system using a dihydrofolate reductase gene.

In addition, the antibody composition can also be produced using a gene-introduced animal individual (transgenic non-human animal) or a plant individual (transgenic plant) which is constructed by the redifferentiation of an animal or plant cell into which the gene is introduced.

When the transformant is an animal individual or a plant individual, an antibody composition can be produced in accordance with a general method by rearing or cultivating it to thereby produce and accumulate the antibody composition and then recovering the antibody composition from the animal or plant individual.

Examples of the process for producing an antibody composition using an animal individual include a method in which the antibody composition of interest is produced in an animal constructed by introducing a gene in accordance with a known method [American Journal of Clinical Nutrition, 63, 627S (1996); Bio/Technology, 9, 830 (1991)].

In the case of an animal individual, an antibody composition can be produced by rearing a transgenic non-human animal into which a DNA encoding an antibody molecule is introduced to thereby produce and accumulate the antibody composition in the animal, and then recovering the antibody composition from the animal. Examples of the place of the animal where the composition is produced and accumulated include milk (Japanese Published Examined Patent Application No. 309192/88) and eggs of the animal. As the promoter used in this case, any promoter can be used, so long as it can function in an animal. Preferred examples include mammary gland cell-specific promoters such as $\alpha$ casein promoter, $\beta$ casein promoter, $\beta$ lactoglobulin promoter, whey acidic protein promoter and the like.

Example of the process for producing an antibody composition using a plant individual include a method in which an antibody composition is produced by cultivating a transgenic plant into which a DNA encoding an antibody molecule is introduced by a known method [Tissue Culture, 20 (1994); Tissue Culture, 21 (1995); Trends in Biotechnology, 15, 45 (1997)] to produce and accumulate the antibody composition in the plant, and then recovering the antibody composition from the plant.

Regarding purification of an antibody composition produced by a transformant into which a gene encoding an antibody molecule is introduced, for example, when the antibody composition is intracellularly expressed in a dissolved state, the cells after culturing are recovered by centrifugation, suspended in an aqueous buffer and then disrupted using ultrasonic oscillator, French press, Manton Gaulin homogenizer, dynomill or the like to obtain a cell-free extract. A purified product of the antibody composition can be obtained from a supernatant obtained by centrifuging the cell-free extract, by using a general enzyme isolation purification techniques such as solvent extraction; salting out; desalting with ammonium sulfate, *etc*.; precipitation with an organic solvent; anion exchange chromatography using a resin such as diethylaminoethyl (DEAE)-Sepharose, DIAION HPA-75 (manufactured by Mitsubishi Chemical), *etc*.; cation exchange chromatography using a resin such as S-Sepharose FF (manufactured by Pharmacia), *etc*.; hydrophobic chromatography using a resin such as butyl-Sepharose, phenyl-Sepharose, *etc*.; gel filtration using a molecular sieve; affinity chromatography; chromatofocusing; electrophoresis such as isoelectric focusing, *etc*.; and the like which may be used alone or in combination.

Also, when the antibody composition is expressed intracellularly by forming an insoluble body, the cells are recovered, disrupted and centrifuged in the same manner, and the insoluble body of the antibody composition is recovered as a precipitation fraction. The recovered insoluble body of the antibody composition is solubilized using a protein denaturing agent. The antibody composition is made into a normal three-dimensional structure by diluting or dialyzing the solubilized solution, and then a purified product of the antibody composition is obtained by the same isolation purification method.

When the antibody composition is secreted extracellularly, the antibody composition or derivatives thereof can be recovered from the culture supernatant. That is, the culture is treated by a technique such as centrifugation or the

like to obtain a soluble fraction, and a purified preparation of the antibody composition can be obtained from the soluble fraction by the same isolation purification method.

Examples of the thus obtained antibody composition include an antibody, the fragment of the antibody, a fusion protein comprising the Fc region of the antibody, and the like.

As an example for obtaining the antibody composition, a process for producing a composition of a humanized antibody is described below in detail, but other antibody compositions can also be obtained in a manner similar to the method.

(1) Construction of vector for humanized antibody expression

A vector for humanized antibody expression is an expression vector for animal cell into which genes encoding the heavy chain (H chain) and light chain (L chain) C regions of a human antibody are inserted, which can be constructed by cloning each of genes encoding the H chain and L chain C regions of a human antibody into an expression vector for animal cell.

The C regions of a human antibody may be the H chain and L chain of any human antibody. Examples include the C region belonging to IgG1 subclass in the H chain of a human antibody (hereinafter referred to as "hCγ1"), the C region belonging to κ class in the L chain of a human antibody (hereinafter referred to as "hCκ"), and the like.

As the genes encoding the H chain and L chain C regions of a human antibody, a chromosomal DNA comprising an exon and an intron can be used or a cDNA can also be used.

As the expression vector for animal cell, any vector can be used, so long as a gene encoding the C region of a human antibody can be inserted thereinto and expressed therein. Examples include pAGE107 [Cytotechnology, 3, 133 (1990)], pAGE103 [J. Biochem., 101, 1307 (1987)], pHSG274 [Gene, 27, 223 (1984)], pKCR [Proc. Natl. Acad. Sci. USA, 78, 1527 (1981), pSG1 β d2-4 [Cytotechnology, 4, 173 (1990)] and the like. Examples of the promoter and enhancer in the expression vector for animal cell include SV40 early promoter and enhancer [J. Biochem., 101, 1307 (1987)], Moloney mouse leukemia virus LTR promoter [Biochem. Biophys. Res. Commun., 149, 960 (1987)], immunoglobulin H chain promoter [Cell, 41, 479 (1985)] and enhancer [Cell, 33, 717 (1983)], and the like.

The humanized antibody expression vector may be either of a type in which genes encoding the H chain and L chain of an antibody exist on separate vectors or of a type in which both genes exist on the same vector (tandem type). In respect of easiness of construction of a humanized antibody expression vector, easiness of introduction into animal cells, and balance between the expression amounts of the H and L chains of an antibody in animal cells, a tandem type of the humanized antibody expression vector is more preferred [J. Immunol. Methods, 167, 271 (1994)].

The constructed humanized antibody expression vector can be used for expression of a human chimeric antibody and a human CDR-grafted antibody in animal cells.

(2) Preparation of cDNA encoding V region of antibody derived from animal other than human

cDNAs encoding the H chain and L chain V regions of an antibody derived from an animal other than human, such as a mouse antibody, can be obtained in the following manner.

A cDNA is synthesized by extracting mRNA from a hybridoma cell which produces the mouse antibody of interest. The synthesized cDNA is cloned into a vector such as a phage or a plasmid to obtain a cDNA library. Each of a recombinant phage or recombinant plasmid comprising a cDNA encoding the H chain V region and a recombinant phage or recombinant plasmid comprising a cDNA encoding the L chain V region is isolated from the library using a C region part or a V region part of an existing mouse antibody as the probe. Full nucleotide sequences of the H chain and L chain V regions of the mouse antibody of interest on the recombinant phage or recombinant plasmid are determined, and full amino acid sequences of the H chain and L chain V regions are deduced from the nucleotide sequences.

As the animal other than human, any animal such as mouse, rat, hamster, rabbit or the like can be used so long as a hybridoma cell can be produced therefrom.

Examples of the method for preparing total RNA from a hybridoma cell include the guanidine thiocyanate-cesium trifluoroacetate method [Methods in Enzymology, 154, 3 (1987)] and the like, and examples of the method for preparing mRNA from total RNA, an oligo(dT)-immobilized cellulose column method (Molecular Cloning, Second Edition) and the like. In addition, examples of a kit for preparing mRNA from a hybridoma cell include Fast Track mRNA Isolation Kit (manufactured by Invitrogen), Quick Prep mRNA Purification Kit (manufactured by Pharmacia) and the like.

Examples of the method for synthesizing cDNA and preparing a cDNA library include the usual methods (Molecular Cloning, Second Edition, Current Protocols in Molecular Biology, Supplement 1-34), methods using a commercially available kit such as SuperScript™, Plasmid System for cDNA Synthesis and Plasmid Cloning (manufactured by GIBCO BRL) or ZAP-cDNA Synthesis Kit (manufactured by Stratagene), and the like.

In preparing the cDNA library, the vector into which a cDNA synthesized using mRNA extracted from a hybridoma cell as the template is inserted may be any vector so long as the cDNA can be inserted. Examples include ZAP Express

[Strategies, 5, 58 (1992)], pBluescript II SK(+) [Nucleic Acids Research, 17, 9494 (1989)], λzapII (manufactured by Stratagene), λgt10 and λgt11 [DNA Cloning, A Practical Approach, I, 49 (1985)], Lambda BlueMid (manufactured by Clontech), λExCell, pT7T3 18U (manufactured by Pharmacia), pcD2 [Mol. Cell. Biol., 3, 280 (1983)], pUC18 [Gene, 33, 103 (1985)] and the like.

As *Escherichia coli* into which the cDNA library constructed from a phage or plasmid vector is introduced, any *Escherichia coli* can be used, so long as the cDNA library can be introduced, expressed and maintained. Examples include XL1-Blue MRF' [Strategies, 5, 81 (1992)], C600 [Genetics, 39, 440 (1954)], Y1088 and Y1090 [Science, 222, 778 (1983)], NM522 [J. Mol. Biol., 166, 1 (1983)], K802 [J. Mol. Biol., 16, 118 (1966)], JM105 [Gene, 38, 275 (1985)] and the like.

As the method for selecting a cDNA clone encoding the H chain and L chain V regions of an antibody derived from an animal other than human from the cDNA library, a colony hybridization or a plaque hybridization using an isotope- or fluorescence-labeled probe can be used (Molecular Cloning, Second Edition). The cDNA encoding the H chain and L chain V regions can also be prepared by preparing primers and carrying out polymerase chain reaction (hereinafter referred to as "PCR"; Molecular Cloning, Second Edition; Current Protocols in Molecular Biology, Supplement 1-34) using a cDNA synthesized from mRNA or a cDNA library as the template.

The nucleotide sequences of the cDNAs can be determined by digesting the selected cDNAs with appropriate restriction enzymes, cloning the fragments into a plasmid such as pBluescript SK(-) (manufactured by Stratagene) or the like, carrying out the reaction of a generally used nucleotide sequence analyzing method such as the dideoxy method [Proc. Natl. Acad. Sci. USA, 74, 5463 (1977)] of Sanger *et al.* or the like and then analyzing the clones using an automatic nucleotide sequence analyzer such as A.L.F. DNA Sequencer (manufactured by Pharmacia) or the like. Whether or not the obtained cDNAs are encoding the full amino acid sequences of the H chain and L chain V regions of the antibody containing a secretory signal sequence can be confirmed by deducing the full amino acid sequences of the H chain and L chain V regions from the determined nucleotide sequence and comparing them with the full amino acid sequences of the H chain and L chain V regions of known antibodies [Sequences of Proteins of Immunological Interest, US Dep. Health and Human Services (1991)].

(3) Analysis of amino acid sequence of V region of antibody derived from animal other than human

Regarding the full amino acid sequences of the H chain and L chain V regions of the antibody containing a secretory signal sequence, the length of the secretory signal sequence and the *N*-terminal amino acid sequences can be deduced and subgroups to which they belong can also be found, by comparing them with the full amino acid sequences of the H chain and L chain V regions of known antibodies [Sequences of Proteins of Immunological Interest, US Dep. Health and Human Services, (1991)]. In addition, the amino acid sequences of the H chain and L chain V regions of each CDR can also be found by comparing them with the amino acid sequences of the H chain and L chain V regions of known antibodies [Sequences of Proteins of Immunological Interest, US Dep. Health and Human Services, (1991)].

(4) Construction of human chimeric antibody expression vector

A human chimeric antibody expression vector can be constructed by cloning cDNAs encoding the H chain and L chain V regions of an antibody derived from an animal other than human into upstream of genes encoding the H chain and L chain C regions of a human antibody in the vector for humanized antibody expression constructed in the item 3(1). For example, a human chimeric antibody expression vector can be constructed by linking each of cDNAs encoding the H chain and L chain V regions of an antibody derived from an animal other than human to a synthetic DNA comprising nucleotide sequences at the 3'-terminals of the H chain and L chain V regions of an antibody derived from an animal other than human and nucleotide sequences at the 5'-terminals of the H chain and L chain C regions of a human antibody and also having a recognition sequence of an appropriate restriction enzyme at both terminals, and by cloning them into upstream of genes encoding the H chain and L chain C regions of a human antibody contained in the vector for humanized antibody expression constructed described in the item 3(1).

(5) Construction of cDNA encoding V region of human CDR-grafted antibody

cDNAs encoding the H chain and L chain V regions of a human CDR-grafted antibody can be obtained as follows. First, amino acid sequences of the frameworks (hereinafter referred to as "FR") of the H chain and L chain V regions of a human antibody for grafting CDR of the H chain and L chain V regions of an antibody derived from an animal other than human is selected. As the amino acid sequences of FRs of the H chain and L chain V regions of a human antibody, any amino acid sequences can be used so long as they are derived from a human antibody. Examples include amino acid sequences of FRs of the H chain and L chain V regions of human antibodies registered at databases such as Protein Data Bank, *etc.*, amino acid sequences common in each subgroup of FRs of the H chain and L chain V regions

of human antibodies [Sequences of Proteins of Immunological Interest, US Dep. Health and Human Services (1991)] and the like. But in order to produce a human CDR-grafted antibody having potent activity, it is preferable to select an amino acid sequence having a homology as high as possible (at least 60% or more) with amino acid sequences of the H chain and L chain V regions of an antibody of interest derived from an animal other than human.

Next, the amino acid sequences of CDRs of the H chain and L chain V regions of the antibody of interest derived from an animal other than human are grafted to the selected amino acid sequences of FRs of the H chain and L chain V regions of a human antibody to design amino acid sequences of the H chain and L chain V regions of the human CDR-grafted antibody. The designed amino acid sequences are converted into DNA sequences by considering the frequency of codon usage found in nucleotide sequences of antibody genes [Sequences of Proteins of Immunological Interest, US Dep. Health and Human Services (1991)], and the DNA sequences encoding the amino acid sequences of the H chain and L chain V regions of the human CDR-grafted antibody are designed. Based on the designed DNA sequences, several synthetic DNA fragments having a length of about 100 bases are synthesized, and PCR is carried out using them. In this case, it is preferable in each of the H chain and the L chain that 6 synthetic DNAs are designed in view of the reaction efficiency of PCR and the lengths of DNAs which can be synthesized.

Also, they can be easily cloned into the vector for humanized antibody expression constructed in the item 3(1) by introducing recognition sequences of an appropriate restriction enzyme into the 5'-terminals of the synthetic DNA present on both terminals. After the PCR, the amplified product is cloned into a plasmid such as pBluescript SK(-) (manufactured by Stratagene) or the like and the nucleotide sequences are determined by the method in the item 3(2) to thereby obtain a plasmid having DNA sequences encoding the amino acid sequences of the H chain and L chain V regions of the desired human CDR-grafted antibody.

(6) Construction of human CDR-grafted antibody expression vector

A human CDR-grafted antibody expression vector can be constructed by cloning the cDNAs encoding the H chain and L chain V regions of the human CDR-grafted antibody constructed in the item 3(5) into upstream of the gene encoding H chain and L chain C regions of a human antibody in the vector for humanized antibody expression described in the item 3(1). For example, the human CDR-grafted antibody expression vector can be constructed by introducing recognizing sequences of an appropriate restriction enzyme into the 5'-terminals of both terminals of a synthetic DNA fragment, among the synthetic DNA fragments which are used when PCR is carried out in the item 3(5) for constructing the H chain and L chain V regions of the human CDR-grafted antibody, so that they are cloned into upstream of the genes encoding the H chain and L chain C regions of a human antibody in the vector for humanized antibody expression described in the item 3(1) in such a manner that they can be expressed in a suitable form.

(7) Stable production of humanized antibody

A transformant capable of stably producing a human chimeric antibody and a human CDR-grafted antibody (both hereinafter referred to as "humanized antibody") can be obtained by introducing the humanized antibody expression vectors described in the items 3(4) and (6) into an appropriate animal cell.

Examples of the method for introducing a humanized antibody expression vector into an animal cell include electroporation [Japanese Published Examined Patent Application No. 257891/90, Cytotechnology, 3, 133 (1990)] and the like.

As the animal cell into which a humanized antibody expression vector is introduced, any cell can be used so long as it is an animal cell which can produce the humanized antibody.

Examples include mouse myeloma cells such as NS0 cell and SP2/0 cell, Chinese hamster ovary cells such as CHO/dhfr$^-$ cell and CHO/DG44 cell, rat myeloma such as YB2/0 cell and IR983F cell, BHK cell derived from a syrian hamster kidney, a human myeloma cell such as Namalwa cell, and the like, and a Chinese hamster ovary cell CHO/DG44 cell, a rat myeloma YB2/0 cell and the host cells of the present invention described in the item 5 are preferred.

After introduction of the humanized antibody expression vector, a transformant capable of stably producing the humanized antibody can be selected using a medium for animal cell culture comprising an agent such as G418 sulfate (hereinafter referred to as "G418"; manufactured by SIGMA) and the like in accordance with the method disclosed in Japanese Published Examined Patent Application No. 257891/90. Examples of the medium for animal cell culture include RPMI 1640 medium (manufactured by Nissui Pharmaceutical), GIT medium (manufactured by Nihon Pharmaceutical), EX-CELL 302 medium (manufactured by JRH), IMDM medium (manufactured by GIBCO BRL), Hybridoma-SFM medium (manufactured by GIBCO BRL) media obtained by adding various additives such as fetal bovine serum (hereinafter referred to as "FBS") to these media, and the like. The humanized antibody can be produced and accumulated in the culture supernatant by culturing the obtained transformant in a medium. The expression level and antigen binding activity of the humanized antibody in the culture supernatant can be measured by a method such as enzyme-linked immunosorbent assay [hereinafter referred to as "ELISA"; Antibodies: A Laboratory Manual, Cold Spring Harbor Laboratory,

Chapter 14 (1998), Monoclonal Antibodies: Principles and Practice, Academic Press Limited (1996)] or the like. Also, the expression level of the humanized antibody by the transformant can be increased using a DHFR gene amplification system in accordance with the method disclosed in Japanese Published Examined Patent Application No. 257891/90.

The humanized antibody can be purified from a culture supernatant of the transformant using a protein A column [Antibodies, A Laboratory Manual, Cold Spring Harbor Laboratory, Chapter 8 (1988), Monoclonal Antibodies: Principles and Practice, Academic Press Limited (1996)]. In addition, purification methods generally used for the purification of proteins can also be used. For example, the purification can be carried out through the combination of a gel filtration, an ion exchange chromatography and an ultrafiltration. The molecular weight of the H chain, L chain or antibody molecule as a whole of the purified humanized antibody can be measured, e.g., by polyacrylamide gel electrophoresis [hereinafter referred to as "SDS-PAGE"; Nature, 227, 680 (1970)], Western blotting [Antibodies, A Laboratory Manual, Cold Spring Harbor Laboratory, Chapter 12, (1988), Monoclonal Antibodies: Principles and Practice, Academic Press Limited (1996)] or the like.

Thus, methods for producing an antibody composition using an animal cell as the host have been described, but, as described above, the antibody composition can also be produced by a yeast, an insect cell, a plant cell, an animal individual or a plant individual by the same methods on the animal cell.

When a host cell has the ability to express an antibody molecule innately, the antibody composition of the present disclosure can be produced by preparing a cell expressing an antibody molecule using the method described in the item 1, culturing the cell and then purifying the antibody composition of interest from the resulting culture.

4. Activity evaluation of antibody composition

As the method for measuring the amount of the purified antibody composition, the activity to bind to an antibody and the effector function of the purified antibody composition, the known method described in *Monoclonal Antibodies, Antibody Engineering* and the like can be used.

As the examples, when the antibody composition is a humanized antibody, the binding activity with an antigen and the binding activity with an antigen-positive cultured cell line can be measured by methods such as ELISA, an immunofluorescent method [Cancer Immunol. Immunother., 36, 373 (1993)] and the like. The cytotoxic activity against an antigen-positive cultured cell line can be evaluated by measuring CDC activity, ADCC activity *[*Cancer Immunol. Immunother., 36, 373 (1993)] and the like.

Also, safety and therapeutic effect of the antibody composition in human can be evaluated using an appropriate model of animal species relatively close to human, such as *Macaca fascicularis* or the like.

5. Analysis of sugar chains binding to antibody molecules expressed in various cells

The sugar chain structure binding to an antibody molecule expressed in various cells can be analyzed in accordance with the general analysis of the sugar chain structure of a glycoprotein. For example, the sugar chain which is bound to IgG molecule comprises a neutral sugar such as galactose, mannose, fucose or the like, an amino sugar such as *N*-acetylglucosamine or the like and an acidic sugar such as sialic acid or the like, and can be analyzed by a method such as a sugar chain structure analysis or the like using sugar composition analysis, two dimensional sugar chain mapping or the like.

(1) Analysis of neutral sugar and amino sugar compositions

The sugar chain composition binding to an antibody molecule can be analyzed by carrying out acid hydrolysis of sugar chains with an acid such as trifluoroacetic acid or the like to release a neutral sugar or an amino sugar and measuring the composition ratio.

Example include a method using a sugar composition analyzer (BioLC) manufactured by Dionex. The BioLC is an apparatus which analyzes a sugar composition by HPAEC-PAD (high performance anion-exchange chromatography-pulsed amperometric detection) [J. Liq. Chromatogr., 6, 1577 (1983)].

The composition ratio can also be analyzed by a fluorescence labeling method using 2-aminopyridine. Specifically, the compositional ratio can be calculated in accordance with a known method *[*Agric. Biol. Chem., 55(1), 283-284 (1991)], by labeling an acid-hydrolyzed sample with a fluorescence with 2-aminopyridylation and then analyzing the composition by HPLC.

(2) Analysis of sugar chain structure

The sugar chain structure binding to an antibody molecule can be analyzed by the two dimensional sugar chain mapping method [Anal. Biochem., 171, 73 (1988), Biochemical Experimentation Methods 23 - Methods for Studying

Glycoprotein Sugar Chains (Japan Scientific Societies Press) edited by Reiko Takahashi (1989)]. The two dimensional sugar chain mapping method is a method for deducing a sugar chain structure by, e.g., plotting the retention time or elution position of a sugar chain by reverse phase chromatography as the X axis and the retention time or elution position of the sugar chain by normal phase chromatography as the Y axis, respectively, and comparing them with such results of known sugar chains.

Specifically, sugar chains are released from an antibody by subjecting the antibody to hydrazinolysis, and the released sugar chain is subjected to fluorescence labeling with 2-aminopyridine (hereinafter referred to as "PA") [J. Biochem., 95, 197 (1984)], and then the sugar chains are separated from an excess PA-treating reagent by gel filtration, and subjected to reverse phase chromatography. Thereafter, each peak of the separated sugar chains are subjected to normal phase chromatography. The sugar chain structure can be deduced by plotting the results on a two dimensional sugar chain map and comparing them with the spots of a sugar chain standard (manufactured by Takara Shuzo) or a literature [Anal. Biochem., 171, 73 (1988)].

The structure deduced by the two dimensional sugar chain mapping method can be confirmed by further carrying out mass spectrometry such as MALDI-TOF-MS of each sugar chain or the like.

6. Immunological determination method for discriminating sugar chain structure of antibody molecule

An antibody composition comprises an antibody molecule in which sugar chains binding to the Fc region of the antibody are different in structure. The antibody composition in which the ratio of a sugar chain in which fucose is not bound to *N*-acetylglucosamine in the reducing end in the sugar chain is 20% or more among the total complex *N*-glycoside-linked sugar chains binding to the Fc region in the antibody composition reducing end has potent ADCC activity. The antibody composition can be identified by using the method for analyzing the sugar chain structure of an antibody molecule described in the item 6. Also, it can also be identified by an Immunological determination method using a lectin.

The sugar chain structure of an antibody molecule can be identified by the Immunological determination method using a lectin in accordance with the known Immunological determination method such as Western staining, IRA (radioimmunoassay), VIA (viroimmunoassay), EIA (enzymoimmunoassay), FIA (fluoroimmunoassay), MIA (metalloimmunoassay) and the like described in Monoclonal Antibodies: Principles and Applications, Wiley-Liss, Inc. (1995); Immunoassay, 3rd Ed., Igakushoin (1987); Enzyme Antibody Method, Revised Edition, Gakusai Kikaku (1985); and the like.

A lectin which recognizes the sugar chain structure of an antibody molecule comprised in an antibody composition is labeled, and the labeled lectin is allowed to react with an antibody composition which is a sample. Then, the amount of the complex of the labeled lectin with the antibody molecule is measured.

Examples of the lectin used for identifying the sugar chain structure of an antibody molecule include WGA (wheat-germ agglutinin derived from *T. vulgaris*), ConA (cocanavalin A derived from *C. ensiformis*), RIC (a toxin derived from *R. communis*), L-PHA (leucoagglutinin derived from *P. vulgaris*), LCA (lentil agglutinin derived from *L. culinaris*), PSA (pea lectin derived from *P. sativum*), AAL (*Aleuria aurantia* lectin), ACL (*Amaranthus caudatus* lectin), BPL (*Bauhinia purpurea* lectin), DSL (*Datura stramonium* lectin), DBA (*Dolichos biflorus* agglutinin), EBL (elderberry balk lectin), ECL (*Erythrina cristagalli* lectin), EEL (*Euonymus eoropaeus* lectin), GNL (*Galanthus nivalis* lectin), GSL (*Griffonia simplicifolia* lectin), HPA (*Helix pomatia* agglutinin), HHL (*Hippeastrum* hybrid lectin), Jacalin, LTL (*Lotus tetragonolobus* lectin), LEL (*Lycopersicon esculentum* lectin), MAL (*Maackia amurensis* lectin), MPL (*Maclura pomifera* lectin), NPL (*Narcissus pseudonarcissus* lectin), PNA (peanut agglutinin), E-PHA (*Phaseolus vulgaris* erythroagglutinin), PTL (*Psophocarpus tetragonolobus* lectin), RCA (*Ricinus communis* agglutinin), STL (*Solanum tuberosum* lectin), SJA (*Sophora japonica* agglutinin), SBA (soybean agglutinin), UEA (*Ulex europaeus* agglutinin), VVL (*Vicia villosa* lectin) and WFA (*Wisteria floribunda* agglutinin).

It is preferable to use a lectin which specifically recognizes a sugar chain structure wherein fucose binds to the *N*-acetylglucosamine in the reducing end in the complex *N*-glycoside-linked sugar chain. Examples include *Lens culinaris* lectin LCA (lentil agglutinin derived from *Lens culinaris*), pea lectin PSA (pea lectin derived from *Pisum sativum*), broad bean lectin VFA (agglutinin derived from *Vicia faba*) and *Aleuria aurantia* lectin AAL (lectin derived from *Aleuria aurantia*).

7. Application of antibody molecule produced in accordance with the present invention

The antibody composition produced in accordance with the present invention has potent antibody-dependent cell-mediated cytotoxic activity. An antibody having potent antibody-dependent cell-mediated cytotoxic activity is useful for preventing and treating various diseases including cancers, inflammatory diseases, immune diseases such as autoimmune diseases, allergies and the like, circulatory organ diseases and viral or bacterial infections.

In the case of cancers, namely malignant tumors, cancer cells grow. General anti-tumor agents inhibit the growth of cancer cells. In contrast, an antibody having potent antibody-dependent cell-mediated cytotoxic activity can treat cancers by injuring cancer cells through its cell killing effect, and therefore, it is more effective as a therapeutic agent than the general anti-tumor agents. At present, in the therapeutic agent for cancers, an anti-tumor effect of an

antibody medicament alone is insufficient so that combination therapy with chemotherapy has been carried out [Science, 280, 1197 (1998)]. If more potent anti-tumor effect is found by the antibody composition produced in accordance with the present invention alone, the dependency on chemotherapy will be decreased and side effects will be reduced.

In immune diseases such as inflammatory diseases, autoimmune diseases, allergies and the like, *in vivo* reactions of the diseases are induced by the release of a mediator molecule by immunocytes, so that the allergy reaction can be inhibited by eliminating immunocytes using an antibody having potent antibody-dependent cell-mediated cytotoxic activity.

Examples of the circulatory organ diseases include arteriosclerosis and the like. The arteriosclerosis is treated using balloon catheter at present, but circulatory organ diseases can be prevented and treated by inhibiting growth of arterial cells in restricture after treatment using an antibody having potent antibody-dependent cell-mediated cytotoxic activity.

Various diseases including viral and bacterial infections can be prevented and treated by inhibiting proliferation of cells infected with a virus or bacterium using an antibody having potent antibody-dependent cell-mediated cytotoxic activity.

Examples of an antibody which recognizes a tumor-related antigen, an antibody which recognizes an allergy- or inflammation-related antigen, an antibody which recognizes circulatory organ disease-related antigen and an antibody which recognizes a viral or bacterial infection-related antigen are described below.

Examples of the antibody which recognizes a tumor-related antigen include anti-GD2 antibody *(*Ohta et al., Anticancer Res., 13, 331-336, 1993), anti-GD3 antibody *(*Ohta et al., Cancer Immunol. Immunother., 36, 260-266, 1993), anti-GM2 antibody *(*Nakamura et al., Cancer Res., 54, 1511-1516, 1994), anti-HER2 antibody (Carter et al., Proc. Natl. Acad. Sci. USA, 89, 4285-4289, 1992), anti-CD52 antibody (Carter et al., Proc. Natl. Acad. Sci. USA, 89, 4285-4289, 1992), anti-MAGE antibody (Jungbluth et al., British J. Cancer, 83, 493-497, 2000), anti-HM1.24 antibody (Ono et al., Molecular Immunol., 36, 387-395, 1999), anti-parathyroid hormone-related protein (PTHrP) antibody (Ogata et al., Cancer, 88, 2909-2911, 2000), anti-basic fibroblast growth factor antibody and anti-FGF8 antibody (Matsuzaki et al., Proc. Natl. Acad. Sci. USA, 86, 9911-9915, 1989), anti-basic fibroblast growth factor receptor antibody and anti-FGF8 receptor antibody (Kuo et al., J. Biol. Chem., 265, 16455-16463, 1990), anti-insulin-like growth factor antibody (Yao et al., J. Neurosci. Res., 40, 647-659, 1995), anti-insulin-like growth factor receptor antibody (Yao et al., J. Neurosci. Res., 40, 647-659, 1995), anti-PMSA antibody (Murphy et al., J. Urology, 160, 2396-2401, 1998), anti-vascular endothelial cell growth factor antibody (Presta et al., Cancer Res., 57, 4593-4599, 1997), anti-vascular endothelial cell growth factor receptor antibody (Kanno et al., Oncogene, 19, 2138-2146, 2000) and the like.

Examples of the antibody which recognizes an allergy- or inflammation-related antigen include anti-interleukin 6 antibody *(*Abrams et al., Immunol. Rev., 127, 5-24, 1992), anti-interleukin 6 receptor antibody *(*Sato et al., Molecular Immunol., 31, 371-381, 1994), anti-interleukin 5 antibody *(*Abrams et al., Immunol. Rev., 127, 5-24, 1992), anti-interleukin 5 receptor antibody and anti-interleukin 4 antibody *(*Biord et al., Cytokine, 3, 562-567, 1991), anti-tumor necrosis factor antibody *(*Tempest et al., Hybridoma, 13, 183-190, 1994), anti-tumor necrosis factor receptor antibody (Amrani et al., Molecular Pharmacol., 58, 237-245, 2000), anti-CCR4 antibody (Campbell et al., Nature, 400, 776-780, 1999), anti-chemokine antibody (Peri et al., J. Immuno. Meth., 174, 249-257, 1994), anti-chemokine receptor antibody (Wu et al., J. Exp. Med., 186, 1373-1381, 1997) and the like. Examples of the antibody which recognizes a circulatory organ disease-related antigen include anti-GpIIb/IIIa antibody (Co et al., J. Immunol., 152, 2968-2976, 1994), anti-platelet-derived growth factor antibody (Ferns et al., Science, 253, 1129-1132, 1991), anti-platelet-derived growth factor receptor antibody (Shulman et al., J. Biol. Chem., 272, 17400-17404, 1997) and anti-blood coagulation factor antibody (Peter et al., Circulation, 101, 1158-1164, 2000) and the like.

Examples of the antibody which recognizes a viral or bacterial infection-related antigen include anti-gp120 antibody (Tugarinov et al., Structure, 8, 385-395, 2000), anti-CD4 antibody (Schulze-Koops et al., J. Rheumatology, 25, 2065-2076, 1998), anti-CCR4 antibody and anti-Vero toxin antibody (Karnali et al., J. Clin. Microbiol., 37, 396-399, 1999) and the like.

These antibodies can be obtained from public organizations such as ATCC (The American Type Culture Collection), RIKEN Gene Bank at The Institute of Physical and Chemical Research, National Institute of Bioscience and Human Technology, Agency of Industrial Science and Technology (present name, International Patent Organism Depositary, National Institute of Advanced Industrial Science and Technology) and the like, or private reagent sales companies such as Dainippon Pharmaceutical, R & D SYSTEMS, PharMingen, Cosmo Bio, Funakoshi and the like.

The medicament comprising the antibody composition produced in accordance with the present invention can be administered as a therapeutic agent alone, but generally, it is preferable to provide it as a pharmaceutical formulation produced by an appropriate method well known in the technical field of manufacturing pharmacy, by mixing it with at least one pharmaceutically acceptable carrier.

It is desirable to select a route of administration which is most effective in treatment. Examples include oral administration and parenteral administration, such as buccal, tracheal, rectal, subcutaneous, intramuscular, intravenous or the like. In an antibody preparation, intravenous administration is preferable.

The dosage form includes sprays, capsules, tablets, granules, syrups, emulsions, suppositories, injections, ointments, tapes and the like.

Examples of the pharmaceutical preparation suitable for oral administration include emulsions, syrups, capsules, tablets, powders, granules and the like.

Liquid preparations, such as emulsions and syrups, can be produced using, as additives, water; saccharides, such as sucrose, sorbitol, fructose, *etc*.; glycols, such as polyethylene glycol, propylene glycol, *etc*.; oils, such as sesame oil, olive oil, soybean oil, *etc.;* antiseptics, such as p-hydroxybenzoic acid esters, *etc*.; flavors, such as strawberry flavor, peppermint, *etc*.; and the like.

Capsules, tablets, powders, granules and the like can be produced using, as additive, fillers, such as lactose, glucose, sucrose, mannitol, *etc*.; disintegrating agents, such as starch, sodium alginate, *etc*.; lubricants, such as magnesium stearate, talc, *etc*.; binders, such as polyvinyl alcohol, hydroxypropylcellulose, gelatin, *etc*.; surfactants, such as fatty acid ester, *etc*.; plasticizers, such as glycerine, *etc*.; and the like.

Examples of the pharmaceutical preparation suitable for parenteral administration include injections, suppositories, sprays and the like.

Injections may be prepared using a carrier, such as a salt solution, a glucose solution, a mixture of both thereof or the like. Also, powdered injections can be prepared by freeze-drying the antibody composition in the usual way and adding sodium chloride thereto.

Suppositories may be prepared using a carrier such as cacao butter, hydrogenated fat, carboxylic acid or the like.

Also, sprays may be prepared using the antibody composition as such or using a carrier which does not stimulate the buccal or airway mucous membrane of the patient and can facilitate absorption of the antibody composition by dispersing it as fine particles.

Examples of the carrier include lactose, glycerol and the like. Depending on the properties of the antibody composition and the carrier, it is possible to produce pharmaceutical preparations such as aerosols, dry powders and the like. In addition, the components exemplified as additives for oral preparations can also be added to the parenteral preparations.

Although the clinical dose or the frequency of administration varies depending on the objective therapeutic effect, administration method, treating period, age, body weight and the like, it is usually 10 μg/kg to 20 mg/kg per day and per adult.

Also, as the method for examining antitumor effect of the antibody composition against various tumor cells, *in vitro* tests include CDC activity measuring method, ADCC activity measuring method and the like, and *in vivo* tests include antitumor experiments using a tumor system in an experimental animal such as a mouse, *etc*. and the like.

CDC activity and ADCC activity measurements and antitumor experiments can be carried out in accordance with the methods described in Cancer Immunology Immunotherapy, 36, 373 (1993); Cancer Research, 54, 1511 (1994) and the like.

The present invention will be described below in detail based on Examples; however, Examples are only simple illustrations, and the scope of the present invention is not limited thereto.

BRIEF DESCRIPTION OF THE DRAWINGS

Fig. 1 shows electrophoresis patterns of SDS-PAGE of five purified anti-GD3 chimeric antibodies (using gradient gel from 4 to 15%). Fig. 1A and Fig. 1B show results of the electrophoresis under non-reducing conditions and that under reducing conditions, respectively. Lanes 1 to 7 show electrophoresis patterns of high molecular weight markers, YB2/0-GD3 chimeric antibody, CHO/DG44-GD3 chimeric antibody, SP2/0-GD3 chimeric antibody, NSO-GD3 chimeric antibody (302), NSO-GD3 chimeric antibody (GIT) and low molecular weight markers, respectively.

Fig. 2 shows activities of five purified anti-GD3 chimeric antibodies to bind to GD3, measured by changing the antibody concentration. The ordinate and the abscissa show the binding activity with GD3 and the antibody concentration, respectively. "○", "●", "□", "■" and "Δ" show the activities of YB2/0-GD3 chimeric antibody, CHO/DG44-GD3 chimeric antibody, SP2/0-GD3 chimeric antibody, NS0-GD3 chimeric antibody (302) and NS0-GD3 chimeric antibody (GIT), respectively.

Fig. 3 shows ADCC activities of five purified anti-GD3 chimeric antibodies for a human melanoma cell line G-361. The ordinate and the abscissa show the cytotoxic activity and the antibody concentration, respectively. "○", "●", "□", "■" and "Δ" show the activities of YB2/0-GD3 chimeric antibody, CHO/DG44-GD3 chimeric antibody, SP2/0-GD3 chimeric antibody, NS0-GD3 chimeric antibody (302) and NS0-GD3 chimeric antibody (GIT), respectively.

Fig. 4 shows electrophoresis patterns of SDS-PAGE of three purified anti-hIL-5Rα CDR-grafted antibodies (using gradient gel from 4 to 15%). Fig. 4A and Fig. 4B show results of the electrophoresis carried out under non-reducing conditions and those under reducing conditions, respectively. Lanes 1 to 5 show electrophoresis patterns of high

molecular weight markers, YB2/0-hIL-5R CDR antibody, CHO/d-hIL-5R CDR antibody, NS0-hIL-5R CDR antibody and low molecular weight markers, respectively.

Fig. 5 shows activities of three purified anti-hIL-5Rα CDR-grafted antibodies to bind to hIL-5Rα, measured by changing the antibody concentration. The ordinate and the abscissa show the binding activity with hIL-5Rα and the antibody concentration, respectively. "○", "●" and "□" show the activities of YB2/0-hIL-5R CDR antibody, CHO/d-hIL-5R CDR antibody and NS0-hIL-5R CDR antibody, respectively.

Fig. 6 show ADCC activities of three purified anti-hIL-5Rα CDR-grafted antibodies for an hIL-5R expressing mouse T cell line CTLL-2(h5R). The ordinate and the abscissas show the cytotoxic activity and the antibody concentration, respectively. "○", "●" and "□" show the activities of YB2/0-hIL-5RCDR antibody, CHO/d-hIL-5R CDR antibody and NS0-hIL-5R CDR antibody, respectively.

Fig. 7 shows inhibition activities of three purified anti-hIL-5Rα CDR-grafted antibodies in an hIL-5-induced eosinophil increasing model of *Macaca faseicularis.* The ordinate and the abscissa show the number of eosinophils in peripheral blood and the number of days (the day of the commencement of antibody and hIL-5 administration was defined as 0 day). "101 and 102", "301, 302 and 303", "401, 402 and 403" and "501, 502 and 503" show results in the antibody non-administration group, the YB2/0-hIL-5R CDR antibody administered group, the CHO/d-hIL-5R CDR antibody administered group and the NS0-hIL-5R CDR antibody administered group, respectively.

Fig. 8 shows elution patterns of reverse phase HPLC elution of a PA-treated sugar chain (left side), and an elution pattern obtained by treating the PA-treated sugar chain with α-L-fucosidase and then analyzed by reverse phase HPLC (right side), of the purified anti-hIL-5Rα CDR-grafted antibody produced by YB2/0 (Fig. 8A) and the purified anti-hIL-5Rα CDR-grafted antibody produced by NS0 (Fig. 8B). The ordinates and the abscissas show the relative fluorescence intensity and the elution time, respectively.

Fig. 9 shows an elution pattern obtained by preparing a PA-treated sugar chain from the purified anti-hIL-5Rα CDR-grafted antibody produced by CHO/d cell and analyzing it by reverse phase HPLC. The ordinate and the abscissa show the relative fluorescence intensity and the elution time, respectively.

In Fig. 10, Fig. 10A shows the GD3-binding activities of a non-adsorbed fraction and a part of an adsorbed fraction, measured by changing the antibody concentration. The ordinate and the abscissa show the binding activity with GD3 and the antibody concentration, respectively. "●" and "○" show the non-adsorbed fraction and a part of the adsorbed fraction, respectively. Fig. 10B shows the ADCC activities of the non-adsorbed fraction and a part of the adsorbed fraction for a human melanoma line G-361. The ordinate and the abscissa show the cytotoxic activity and the antibody concentration, respectively. "●" and "○" show the non-adsorbed fraction and a part of the adsorbed fraction, respectively.

Fig. 11 shows elution patterns obtained by analyzing PA-treated sugar chains prepared from a non-adsorbed fraction and a part of an adsorbed fraction by a reverse HPLC. Fig. 11A and Fig. 11B show an elution pattern of the non-adsorbed fraction and an elution pattern of a part of the adsorbed fraction, respectively. The ordinates and the abscissas show the relative fluorescence strength and the elution time, respectively.

Fig. 12 shows elution patterns of PA-treated sugar chains prepared from 6 anti-GD3 chimeric antibodies (Fig. 12A to Fig. 12F), obtained by analyzing them by reverse phase HPLC. The ordinates and the abscissas show the relative fluorescence intensity and the elution time, respectively.

Fig. 13 shows GD3-binding activities of 6 anti-GD3 chimeric antibodies having a different ratio of -1,6-fucose-free sugar chains measured by changing the antibody concentration. The ordinate and the abscissa show the binding activity with GD3 and the antibody concentration, respectively. "●", "□", "■", "Δ", "▲" and "x" show the activities of anti-GD3 chimeric antibody (50%), anti-GD3 chimeric antibody (45%), anti-GD3 chimeric antibody (29%), anti-GD3 chimeric antibody (24%), anti-GD3 chimeric antibody (13%) and anti-GD3 chimeric antibody (7%), respectively.

Fig. 14 shows ADCC activities of six kinds of anti-GD3 chimeric antibodies having a different ratio of α-1,6-fucose-free sugar chains against a human melanoma cell line G-361, using an effector cell of the donor A. The ordinate and the abscissa show the cytotoxic activity and the antibody concentration, respectively. "●", "□", "■", "Δ", "▲" and "x" show the activities of anti-GD3 chimeric antibody (50%), anti-GD3 chimeric antibody (45%), anti-GD3 chimeric antibody (29%), anti-GD3 chimeric antibody (24%), anti-GD3 chimeric antibody (13%) and anti-GD3 chimeric antibody (7%), respectively.

Fig. 15 shows ADCC activities of six kinds of anti-GD3 chimeric antibodies having a different ratio of α-1,6-fucose-free sugar chains against a human melanoma cell line G-361, using an effector cell of the donor B. The ordinate and the abscissa show the cytotoxic activity and the antibody concentration, respectively. "●", "□", "■", "Δ", "▲" and "x" show the activities of anti-GD3 chimeric antibody (50%), anti-GD3 chimeric antibody (45%), anti-GD3 chimeric antibody (29%), anti-GD3 chimeric antibody (24%), anti-GD3 chimeric antibody (13%) and anti-GD3 chimeric antibody (7%), respectively.

Fig. 16 shows elution patterns of PA-treated sugar chains prepared from six kinds of anti-GD3 chimeric antibodies, obtained by analyzing them by reverse phase HPLC. The ordinates and the abscissas show the relative fluorescence intensity and the elution time, respectively.

Fig. 17 shows CCR4-binding activities of six kinds of anti-CCR4 chimeric antibodies having a different ratio of α-1,6-fucose-free sugar chains measured by changing the antibody concentration. The ordinate and the abscissa show the binding activity with CCR4 and the antibody concentration, respectively. "■", "□", "▲", "△", "●" and "○" show the activities of anti-CCR4 chimeric antibody (46%), anti-CCR4 chimeric antibody (39%), anti-CCR4 chimeric antibody (27%), anti-CCR4 chimeric antibody (18%), anti-CCR4 chimeric antibody (9%) and anti-CCR4 chimeric antibody (8%), respectively.

Fig. 18 shows ADCC activities of anti-CCR4 chimeric antibodies having a different ratio of α-1,6-fucose-free sugar chains against CCR4/EL-4 cell, using an effector cell of the donor A. The ordinate and the abscissa show the cytotoxic activity and the antibody concentration, respectively. "■", "□", "▲", "△", "●" and "○" show the activities of anti-CCR4 chimeric antibody (46%), anti-CCR4 chimeric antibody (39%), anti-CCR4 chimeric antibody (27%), anti-CCR4 chimeric antibody (18%), anti-CCR4 chimeric antibody (9%) and anti-CCR4 chimeric antibody (8%), respectively. Also, Fig. 18A and Fig. 18B show results obtained using effector cells of the donor A and the donor B, respectively.

Fig. 19 shows ADCC activities of anti-CCR4 chimeric antibodies having a different ratio of α-1,6-fucose-free sugar chain against CCR4/EL-4 cell, using an effector cell of the donor B. The ordinate and the abscissa show the cytotoxic activity and the antibody concentration, respectively. "■", "□", "▲", "△", "●" and "○" show the activities of anti-CCR4 chimeric antibody (46%), anti-CCR4 chimeric antibody (39%), anti-CCR4 chimeric antibody (27%), anti-CCR4 chimeric antibody (18%), anti-CCR4 chimeric antibody (9%) and anti-CCR4 chimeric antibody (8%), respectively.

Fig. 20 shows construction of plasmids CHFT8-pCR2.1 and YBFT8-pCR2.1.

Fig. 21 shows construction of plasmids CHAc-pBS and YBAc-pBS.

Fig. 22 shows construction of plasmids CHFT8d-pCR2.1 and YBFT8d-pCR2.1.

Fig. 23 shows construction of plasmids CHAcd-pBS and YBAcd-pBS.

Fig. 24 shows results of determination of an FUT8 transcription product in each host cell line using competitive RT-PCR. Amounts of the FUT8 transcription product in each host cell line when rat FUT8 sequence was used as the standard and internal control are shown. "■" and "□" show results when CHO cell line and YB2/0 cell line, respectively, were used as the host cell.

Fig. 25 shows construction of a plasmid mfFUT8-pCR2.1.

Fig. 26 shows construction of a plasmid pBSmfFUT8.

Fig. 27 shows construction of a plasmid pAGEmfFUT8.

Fig. 28 shows results of analysis of expression levels of FUT8 gene by a cell line excessively expressing the gene using a competitive RT-PCR. The ordinate shows relative values of amounts of FUT8 transcription to amounts of β-actin transcription.

Fig. 29 shows ADCC activities of an anti-GD3 chimeric antibody purified from a cell line excessively expressing FUT8 gene against a human melanoma cell line G-361. The ordinate and the abscissa show the cytotoxic activity and the antibody concentration, respectively.

Fig. 30 shows elution patterns of PA-treated sugar chains prepared from antibodies produced by mfFUT8-6 and pAGE249-introduced cell lines, obtained by analyzing them by reverse phase HPLC. Fig. 30A and Fig. 30B show elution patterns of PA-treated sugar chains prepared from an antibody produced by mfFUT8-6-introduced cell line and PA-treated sugar chains prepared from an antibody produced by pAGE249-introduced cell line, respectively. The ordinate and the abscissa show the relative fluorescence intensity and the elution time, respectively.

Fig. 31 shows an elution pattern of PA-treated sugar chains prepared from Herceptin, obtained by analyzing them by reverse phase HPLC. The ordinate and the abscissa show the relative fluorescence intensity and the elution time, respectively.

Fig. 32 shows construction of a plasmid CHfFUT8-pCR2.1.

Fig. 33 shows construction of a plasmid ploxPPuro.

Fig. 34 shows construction of a plasmid pKOFUT8gE2-1.

Fig. 35 shows construction of a plasmid pKOFUT8gE2-2.

Fig. 36 shows construction of a plasmid pscFUT8gE2-3.

Fig. 37 shows construction of a plasmid pKOFUT8gE2-3.

Fig. 38 shows construction of a plasmid pKOFUT8gE2-4.

Fig. 39 shows construction of a plasmid pKOFUT8gE2-5.

Fig. 40 shows construction of a plasmid pKOFUT8Puro.

Fig. 41 shows results of genome Southern analyses of 1st.ΔFUT8 2-46-1 and 1st.ΔFUT8 2-46 as α-1,6-fucosyltransferase gene-disrupted CHO cell lines.

Fig. 42 shows ADCC activities of an anti-CCR4 chimeric antibody purified from an FUT8 allele gene-disrupted cell line. The ordinate and the abscissa show the cytotoxic activity and the antibody concentration. "▲" and "■" show the activities of a purified antibody derived from an anti-CCR4 chimeric antibody-producing CHO cell 5-03 and a purified antibody derived from 1st.ΔFUT8 2-46-1, respectively.

Fig. 43 shows ADCC activities of anti-CCR4 human chimeric antibodies produced by lectin-resistant cell lines. The ordinate and the abscissa show the cytotoxic activity and the antibody concentration. "□", "■", "♦" and "▲" show the activities of antibodies produced by the strain 5-03, CHO/CCR4-LCA, CHO/CCR4-AAL and CHO/CCR4-PHA, respectively.

Fig. 44 shows ADCC activities of anti-CCR4 human chimeric antibodies produced by lectin-resistant cell lines. The ordinate and the abscissa show the cytotoxic activity and the antibody concentration, respectively. "□", "Δ" and "●" show activities of antibodies produced by YB2/0 (KM2760 # 58-35-16), 5-03 and CHO/CCR4-LCA, respectively.

Fig.45 shows elution patterns of PA-treated sugar chains prepared from purified anti-CCR4 human chimeric antibodies, obtained by analyzing them by reverse phase HPLC. The ordinate and the abscissa show the relative fluorescence intensity and the elution time, respectively. Fig. 45A, Fig. 45B, Fig. 45C and Fig. 45D show results of analyses of an antibody produced by the strain 5-03, an antibody produced by CHO/CCR4-LCA, an antibody produced by CHO/CCR4-AAL and an antibody produced by CHO/CCR4-PHA, respectively.

Fig. 46 shows the 1st step of construction of an expression vector of CHO cell-derived GMD (6 steps in total).

Fig. 47 shows the 2nd step of construction of the expression vector of CHO cell-derived GMD (6 steps in total).

Fig. 48 shows the 3rd step of construction of the expression vector of CHO cell-derived GMD (6 steps in total).

Fig. 49 shows the 4th step of construction of the expression vector of CHO cell-derived GMD (6 steps in total).

Fig. 50 shows the 5th step of construction of the expression vector of CHO cell-derived GMD (6 steps in total).

Fig. 51 shows the 6th step of construction of the expression vector of CHO cell-derived GMD (6 steps in total).

Fig. 52 shows resistance of GMD-expressed CHO/CCR4-LCA for LCA lectin. The measurement was carried out twice by defining the survival rate of a group of cells cultured without adding LCA lectin as 100%. In the drawing, "249" shows the survival rate of the CHO/CCR4-LCA introduced with an expression vector pAGE249 for LCA lectin. GMD shows resistance of the CHO/CCR4-LCA introduced with a GMD expression vector pAGE249GMD for LCA lectin.

Fig. 53 shows ADCC activities of an anti-CCR4 chimeric antibody produced by cells of GMD-expressed CHO/CCR4-LCA cell lines. The ordinate and the abscissa show the cytotoxic activity and the antibody concentration, respectively.

Fig. 54 show a production step of a plasmid CHO-GMD in which the 5'-terminal of a clone 34-2 is introduced into the 5'-terminal of a CHO cell-derived GMD cDNA clone 22-8.

Fig. 55 shows an elution pattern of PA-treated sugar chains prepared from an anti-CCR4 human chimeric antibody purified from GMD gene-expressed CHO/CCR4-LCA, obtained by analyzing them by reverse phase HPLC. The ordinate and the abscissa show the relative fluorescence intensity and the elution time, respectively.

Example 1

Production of anti-ganglioside GD3 human chimeric antibody:

1. Construction of tandem expression vector pChiLHGM4 for anti-ganglioside GD3 human chimeric antibody

A plasmid pChi641LGM40 was constructed by ligating a fragment of about 4.03 kb containing an L chain cDNA, obtained by digesting an L chain expression vector, pChi641LGM4 [J. Immunol. Methods, 167, 271 (1994)] for anti-ganglioside GD3 human chimeric antibody (hereinafter referred to as "anti-GD3 chimeric antibody") with restriction enzymes *Mlu*I (manufactured by Takara Shuzo) and *Sal*I (manufactured by Takara Shuzo) with a fragment of about 3.40 kb containing a G418-resistant gene and a splicing signal, obtained by digesting an expression vector pAGE107 [Cytotechnology, 3, 133 (1990)] for animal cell with restriction enzymes *Mlu*I (manufactured by Takara Shuzo) and *Sal*I (manufactured by Takara Shuzo) using DNA Ligation Kit (manufactured by Takara Shuzo), and then transforming *E. coli* HB101 (Molecular Cloning, Second Edition) with the ligated product.

Next, a fragment of about 5.68 kb containing an L chain cDNA, obtained by digesting the constructed plasmid pChi641LGM40 with a restriction enzyme *Cla*I (manufactured by Takara Shuzo), blunt-terminating it using DNA Blunting Kit (manufactured by Takara Shuzo) and further digesting it with *Mlu*I (manufactured by Takara Shuzo), was ligated with a fragment of about 8.40 kb containing an H chain cDNA, obtained by digesting an anti-GD3 chimeric antibody H chain expression vector, pChi641HGM4 [J. Immunol. Methods, 167, 271 (1994)] with a restriction enzyme, *Xho*I (manufactured by Takara Shuzo), blunt-terminating it using DNA Blunting Kit (manufactured by Takara Shuzo) and further digesting it with *Mlu*I (manufactured by Takara Shuzo), using DNA Ligation Kit (manufactured by Takara Shuzo), and then *E. coli* HB101 (Molecular Cloning, Second Edition) was transformed with the ligated product to thereby construct a tandem expression vector pChi641LHGM4 for anti-GD3 chimeric antibody.

2. Preparation of cells stably producing anti-GD3 chimeric antibody

Cells capable of stably producing an anti-GD3 chimeric antibody were prepared using the tandem expression

vector pChi641LHGM4 for anti-GD3 chimeric antibody constructed in the item 1 of Example 1, as described below.

(1) Preparation of antibody-producing cell using rat myeloma YB2/0 cell

After introducing 5 μg of the anti-GD3 chimeric antibody expression vector pChi641LHGM4 into $4\times10^6$ cells of rat myeloma YB2/0 [ATCC CRL-1662, J.V. Kilmarin et al., J. Cell. Biol., 93, 576-582 (1982)] by electroporation [Cytotechnology, 3, 133 (1990)], the cells were suspended in 40 ml of RPMI1640-FBS(10) (RPMI1640 medium comprising 10% FBS (manufactured by GIBCO BRL)) and dispensed in 200 μl/well into a 96 well culture plate (manufactured by Sumitomo Bakelite). After culturing them at 37°C for 24 hours in a 5% $CO_2$ incubator, G418 was added to a concentration of 0.5 mg/ml, followed by culturing for 1 to 2 weeks. The culture supernatant was recovered from wells in which colonies of transformants showing G418 resistance were formed and growth of colonies was observed, and the antigen binding activity of the anti-GD3 chimeric antibody in the supernatant was measured by the ELISA shown in the item 3 of Example 1.

Regarding the transformants in wells in which production of the anti-GD3 chimeric antibody was observed in culture supernatants, in order to increase the amount of the antibody production using a DHFR gene amplification system, each of them was suspended in the RPMI1640-FBS(10) medium comprising 0.5 mg/ml G418 and 50 nM DHFR inhibitor, methotrexate (hereinafter referred to as "MTX"; manufactured by SIGMA) to give a density of 1 to $2\times10^5$ cells/ml, and the suspension was dispensed in 2 ml into wells of a 24 well plate (manufactured by Greiner). Transformants showing 50 nM MTX resistance were induced by culturing at 37°C for 1 to 2 weeks in a 5% $CO_2$ incubator. The antigen binding activity of the anti-GD3 chimeric antibody in culture supernatants in wells in which growth of transformants was observed was measured by the ELISA shown in the item 3 of Example 1. Regarding the transformants in wells in which production of the anti-GD3 chimeric antibody was observed in culture supernatants, the MTX concentration was increased to 100 nM and then to 200 nM, and transformants capable of growing in the RPMI1640-FBS(10) medium comprising 0.5 mg/ml G418 and 200 nM MTX and of producing the anti-GD3 chimeric antibody in a large amount were finally obtained by the same method as described above. Among the obtained transformants, suitable cell lines were selected and were made into a single cell (cloning) by limiting dilution twice. Also, using the method for determining the transcription product of an α-1,6-fucosyltransferase gene shown in Example 9, a cell line producing a relatively small amount of the transcription product was selected and used as a suitable cell line.

The obtained anti-GD3 chimeric antibody-producing transformed cell clone 7-9-51 has been deposited on April 5, 1999, as FERM BP-6691 in National Institute of Bioscience and Human Technology, Agency of Industrial Science and Technology (Higashi 1-1-3, Tsukuba, Ibaraki, Japan) (present name: International Patent Organism Depositary, National Institute of Advanced Industrial Science and Technology (AIST Tsukuba Central 6, 1-1, Higashi 1-Chome Tsukuba-shi, Ibaraki-ken, Japan)).

(2) Preparation of antibody-producing cell using CHO/DG44 cell

After introducing 4 μg of the anti-GD3 chimeric antibody expression vector, pChi641LHGM4, into $1.6\times10^6$ cells of CHO/DG44 [G. Urlaub and L.A. Chasin, Proc. Natl. Acad. Sci. USA, 77, 4216-4220 (1980)] by electroporation [Cytotechnology, 3, 133 (1990)], the cells were suspended in 10 ml of IMDM-FBS(10) [IMDM medium comprising 10% FBS and 1× concentration of HT supplement (manufactured by GIBCO BRL)] and dispensed in 200 μl/well into a 96 well culture plate (manufactured by Iwaki Glass). After culturing them at 37°C for 24 hours in a 5% $CO_2$ incubator, G418 was added to give a concentration of 0.5 mg/ml, followed by culturing for 1 to 2 weeks. The culture supernatant was recovered from wells in which colonies of transformants showing G418 resistance were formed and growth of colonies was observed, and the antigen binding activity of the anti-GD3 chimeric antibody in the supernatant was measured by the ELISA shown in the item 3 of Example 1.

Regarding the transformants in wells in which production of the anti-GD3 chimeric antibody was observed in culture supernatants, in order to increase the amount of the antibody production using a DHFR gene amplification system, each of them was suspended in an IMDM-dFBS(10) medium [IMDM medium comprising 10% dialyzed fetal bovine serum (hereinafter referred to as "dFBS"; manufactured by GIBCO BRL)] comprising 0.5 mg/ml G418 and 10 nM MTX to give a density of 1 to $2\times10^5$ cells/ml, and the suspension was dispensed in 0.5 ml into wells of a 24 well plate (manufactured by Iwaki Glass). Transformants showing 10 nM MTX resistance were induced by culturing at 37°C for 1 to 2 weeks in a 5% $CO_2$ incubator. Regarding the transformants in wells in which their growth was observed, the MTX concentration was increased to 100 nM, and transformants capable of growing in the IMDM-dFBS(10) medium comprising 0.5 mg/ml G418 and 100 nM MTX and of producing the anti-GD3 chimeric antibody in a large amount were finally obtained by the same method as described above. Among the obtained transformants, suitable cell lines were selected and were made into a single cell (cloning) by limiting dilution twice. Also, using the method for determining the transcription product of an α-1,6-fucosyltransferase gene shown in Example 9, a cell line producing a relatively small amount of the transcription product was selected and used as a suitable cell line.

(3) Preparation of antibody-producing cell using mouse myeloma NS0 cell

After introducing 5 μg of the anti-GD3 chimeric antibody expression vector pChi641LHGM4 into $4\times10^6$ cells of mouse myeloma NS0 by electroporation [Cytotechnology, 3, 133 (1990)], the cells were suspended in 40 ml of EX-CELL302-FBS(10) (EX-CELL302 medium comprising 10% FBS and 2 mM L-glutamine [hereinafter referred to as "L-Gln"; manufactured by GIBCO BRL)] and dispensed in 200 μl/well into a 96 well culture plate (manufactured by Sumitomo Bakelite). After culturing them at 37°C for 24 hours in a 5% $CO_2$ incubator, G418 was added to give a concentration of 0.5 mg/ml, followed by culturing for 1 to 2 weeks. The culture supernatant was recovered from wells in which colonies of transformants showing G418 resistance were formed and growth of colonies was observed, and the antigen binding activity of the anti-GD3 chimeric antibody in the supernatant was measured by the ELISA shown in the item 3 of Example 1.

Regarding the transformants in wells in which production of the anti-GD3 chimeric antibody was observed in culture supernatants, in order to increase the amount of the antibody production using a DHFR gene amplification system, each of them was suspended in an EX-CELL302-dFBS(10) medium (EX-CELL302 medium comprising 10% dFBS and 2 mM L-Gln) comprising 0.5 mg/ml G418 and 50 nM MTX to give a density of 1 to $2\times10^5$ cells/ml, and the suspension was dispensed in 2 ml into wells of a 24 well plate (manufactured by Greiner). Transformants showing 50 nM MTX resistance were induced by culturing at 37°C for 1 to 2 weeks in a 5% $CO_2$ incubator. The antigen binding activity of the anti-GD3 chimeric antibody in culture supernatants in wells in which growth of transformants was observed was measured by the ELISA shown in the item 3 of Example 1. Regarding the transformants in wells in which production of the anti-GD3 chimeric antibody was observed in culture supernatants, the MTX concentration was increased to 100 nM and then to 200 nM, and transformants capable of growing in the EX-CELL302-dFBS(10) medium comprising 0.5 mg/ml G418 and 200 nM MTX and of producing the anti-GD3 chimeric antibody in a large amount was finally obtained by the same method as described above. Among the obtained transformants, elite cell lines were selected and were made into a single cell (cloning) by limiting dilution twice. Also, using the method for determining the transcription product of an α-1,6-fucosyltransferase gene shown in Example 9, a cell line producing a relatively small amount of the transcription product was selected and used as a suitable cell line.

3. Measurement of binding activity of antibody to GD3 (ELISA)

The binding activity of the antibody to GD3 was measured as described below.

In 2 ml of ethanol solution containing 10 μg of dipalmitoylphosphatidylcholine (manufactured by SIGMA) and 5 μg of cholesterol (manufactured by SIGMA), 4 nmol of GD3 was dissolved. Into each well of a 96 well plate for ELISA (manufactured by Greiner), 20 μl of the solution (40 pmol/well in final concentration) was dispensed, followed by air-drying, 1% bovine serum albumin (hereinafter referred to as "BSA"; manufactured by SIGMA)-containing PBS (hereinafter referred to as "1% BSA-PBS") was dispensed in 100 μl/well, and then the reaction was carried out at room temperature for 1 hour for blocking remaining active groups. After discarding 1% BSA-PBS, a culture supernatant of a transformant or a diluted solution of a human chimeric antibody was dispensed in 50 μl/well to carry out the reaction at room temperature for 1 hour. After the reaction, each well was washed with 0.05% Tween 20 (manufactured by Wako Pure Chemical Industries)-containing PBS (hereinafter referred to as "Tween-PBS"), a peroxidase-labeled goat anti-human IgG (H & L) antibody solution (manufactured by American Qualex) diluted 3,000 times with 1% BSA-PBS was dispensed in 50 μl/well as a secondary antibody solution, and then the reaction was carried out at room temperature for 1 hour. After the reaction and subsequent washing with Tween-PBS, ABTS substrate solution [solution prepared by dissolving 0.55 g of 2,2'-azino-bis(3-ethylbenzothiazoline-6-sulfonic acid) ammonium salt in 1 liter of 0.1 M citrate buffer (pH 4.2) and adding 1 μl/ml of hydrogen peroxide to the solution just before use (hereinafter the same solution was used)] was dispensed in 50 μl/well for color development, and then absorbance at 415 nm (hereinafter referred to as "OD415") was measured.

4. Purification of anti-GD3 chimeric antibody

(1) Culturing of antibody-producing cell derived from YB2/0 cell and purification of antibody

The anti-GD3 chimeric antibody-producing transformed cell clone obtained in the item 2(1) of Example 1 was suspended in the Hybridoma-SFM medium comprising 0.2% BSA, 200 nM MTX and 100 nM triiodothyronine (hereinafter referred to as "T3"; manufactured by SIGMA) to give a density of $3\times10^5$ cells/ml and cultured using a 2.0 liter capacity spinner bottle (manufactured by Iwaki Glass) under agitating at a rate of 50 rpm. After culturing them at 37°C for 10 days in a temperature-controlling room, the culture supernatant was recovered. The anti-GD3 chimeric antibody was purified from the culture supernatant using a Prosep-A (manufactured by Bioprocessing) column in accordance with the manufacture's instructions. The purified anti-GD3 chimeric antibody was named YB2/0-GD3 chimeric antibody.

(2) Culturing of antibody-producing cell derived from CHO/DG44 cell and purification of antibody

The anti-GD3 chimeric antibody-producing transformed cell clone obtained in the item 2(2) of Example 1 was suspended in the EX-CELL302 medium comprising 3 mM L-Gln, 0.5% fatty acid concentrated solution (hereinafter referred to as "CDLC"; manufactured by GIBCO BRL) and 0.3% Pluronic F68 (hereinafter referred to as "PF68"; manufactured by GIBCO BRL) to give a density of $1\times10^6$ cells/ml, and the suspension was dispensed in 50 ml into 175 $mm^2$ flasks (manufactured by Greiner). After culturing them at 37°C for 4 days in a 5% $CO_2$ incubator, the culture supernatant was recovered. The anti-GD3 chimeric antibody was purified from the culture supernatant using a Prosep-A (manufactured by Bioprocessing) column in accordance with the manufacture's instructions. The purified anti-GD3 chimeric antibody was named CHO/DG44-GD3 chimeric antibody.

(3) Culturing of antibody-producing cell derived from NS0 cell and purification of antibody

The anti-GD3 chimeric antibody-producing transformed cell clone obtained in the item 2(3) of Example 1 was suspended in the EX-CELL302 medium comprising 2 mM L-Gln, 0.5 mg/ml G418, 200 nM MTX and 1% FBS, to give a density of $1\times10^6$ cells/ml, and the suspension was dispensed in 200 ml into 175 $mm^2$ flasks (manufactured by Greiner). After culturing them at 37°C for 4 days in a 5% $CO_2$ incubator, the culture supernatant was recovered. The anti-GD3 chimeric antibody was purified from the culture supernatant using a Prosep-A (manufactured by Bioprocessing) column in accordance with the manufacture's instructions. The purified anti-GD3 chimeric antibody was named NS0-GD3 chimeric antibody (302).

Also, the transformed cell clone was suspended in the GIT medium comprising 0.5 mg/ml G418 and 200 nM MTX to give a density of $3\times10^5$ cells/ml, and the suspension was dispensed in 200 ml into 175 $mm^2$ flasks (manufactured by Greiner). After culturing them at 37°C for 10 days in a 5% $CO_2$ incubator, the culture supernatant was recovered. The anti-GD3 chimeric antibody was purified from the culture supernatant using a Prosep-A (manufactured by Bioprocessing) column in accordance with the manufacture's instructions. The purified anti-GD3 chimeric antibody was named NS0-GD3 chimeric antibody (GIT).

(4) Culturing of antibody-producing cell derived from SP2/0 cell and purification of antibody

The anti-GD3 chimeric antibody-producing transformed cell clone (KM-871 (FERM BP-3512)) described in Japanese Published Unexamined Patent Application No. 304989/93 (EP 533199) was suspended in the GIT medium comprising 0.5 mg/ml G418 and 200 nM MTX to give a density of $3\times10^5$ cells/ml, and the suspension was dispensed in 200 ml into 175 $mm^2$ flasks (manufactured by Greiner). After culturing them at 37°C for 8 days in a 5% $CO_2$ incubator, the culture supernatant was recovered. The anti-GD3 chimeric antibody was purified from the culture supernatant using a Prosep-A (manufactured by Bioprocessing) column in accordance with the manufacture's instructions. The purified anti-GD3 chimeric antibody was named SP2/0-GD3 chimeric antibody.

5. Analysis of purified anti-GD3 chimeric antibody

In accordance with a known method [Nature, 227, 680 (1970)], 4 μg of each of the five kinds of the anti-GD3 chimeric antibodies produced by and purified from respective animal cells, obtained in the item 4 of Example 1, was subjected to SDS-PAGE to analyze the molecular weight and purification degree. The results are shown in Fig. 1. As shown in Fig. 1, a single band of about 150 kilodaltons (hereinafter referred to as "Kd") in molecular weight was found under non-reducing conditions, and two bands of about 50 Kd and about 25 Kd under reducing conditions, in each of the purified anti-GD3 chimeric antibodies. The molecular weights almost coincided with the molecular weights deduced from the cDNA nucleotide sequences of H chain and L chain of the antibody (H chain: about 49 Kd, L chain: about 23 Kd, whole molecule: about 144 Kd), and also coincided with the reports stating that the IgG antibody has a molecular weight of about 150 Kd under non-reducing conditions and is degraded into H chains having a molecular weight of about 50 Kd and L chains having a molecular weight of about 25 Kd under reducing conditions due to cutting of the disulfide bond (hereinafter referred to as "S-S bond") in the molecule [Antibodies: A Laboratory Manual, Cold Spring Harbor Laboratory, Chapter 14 (1998); Monoclonal Antibodies: Principles and Practice, Academic Press Limited (1996)], so that it was confirmed that each anti-GD3 chimeric antibody was expressed and purified as an antibody molecule having the true structure.

Example 2

Activity evaluation of anti-GD3 chimeric antibody:

1. Binding activity of anti-GD3 chimeric antibody to GD3 (ELISA)

The activity of the five kinds of the purified anti-GD3 chimeric antibodies obtained in the item 4 of Example 1 to bind to GD3 (manufactured by Snow Brand Milk Products) was measured by the ELISA shown in the item 3 of Example 1. Fig. 2 shows a result of the examination of the binding activity measured by changing the concentration of the anti-GD3 chimeric antibody to be added. As shown in Fig. 2, the five kinds of the anti-GD3 chimeric antibodies showed almost the same binding activity to GD3. The result shows that antigen binding activities of these antibodies are constant independently of the antibody-producing animal cells and their culturing methods. Also, it was suggested from the comparison of the NS0-GD3 chimeric antibody (302) with the NS0-GD3 chimeric antibody (GIT) that the antigen binding activities are constant independently of the media used in the culturing.

2. *In vitro* cytotoxic activity (ADCC activity) of anti-GD3 chimeric antibody

In order to evaluate *in vitro* cytotoxic activity of the five kinds of the purified anti-GD3 chimeric antibodies obtained in the item 4 of Example 1, the ADCC activity was measured in accordance with the following method.

(1) Preparation of target cell solution

A human melanoma cultured cell line G-361 (ATCC CRL 1424) was cultured using the RPMI1640-FBS(10) medium to prepare $1\times10^6$ cells, and the cells were radioisotope-labeled by reacting them with 3.7 MBq equivalents of a radioactive substance $Na_2{}^{51}CrO_4$ at 37°C for 1 hour. After the reaction, the cells were washed three times through their suspension in the RPMI1640-FBS(10) medium and centrifugation, re-suspended in the medium and then incubated at 4°C for 30 minutes in ice for spontaneous dissolution of the radioactive substance. After centrifugation, the precipitate was adjusted to $2\times10^5$ cells/ml by adding 5 ml of the RPMI1640-FBS(10) medium and used as the target cell solution.

(2) Preparation of effector cell solution

From a healthy person, 50 ml of venous blood was collected, and gently mixed with 0.5 ml of heparin sodium (manufactured by Takeda Pharmaceutical). The mixture was centrifuged to isolate a mononuclear cell layer using Lymphoprep (manufactured by Nycomed Pharma AS) in accordance with the manufacture's instructions. After washing with the RPMI1640-FBS(10) medium by centrifugation three times, the resulting precipitate was re-suspended to give a density of $2\times10^6$ cells/ml using the medium and used as the effector cell solution.

(3) Measurement of ADCC activity

Into each well of a 96 well U-shaped bottom plate (manufactured by Falcon), 50 $\mu$l of the target cell solution prepared in the above (1) ($1\times10^4$ cells/well) was dispensed. Next, 100 $\mu$l of the effector cell solution prepared in the above (2) was added thereto ($2\times10^5$ cells/well, the ratio of effector cells to target cells becomes 20:1). Subsequently, each of the anti-GD3 chimeric antibodies was added to give a final concentration from 0.0025 to 2.5 $\mu$g/ml, followed by reaction at 37°C for 4 hours. After the reaction, the plate was centrifuged, and the amount of $^{51}$Cr in the supernatant was measured using a $\gamma$-counter. The amount of spontaneously released $^{51}$Cr was calculated by the same operation using only the medium instead of the effector cell solution and the antibody solution, and measuring the amount of $^{51}$Cr in the supernatant. The amount of total released $^{51}$Cr was calculated by the same operation using only the medium instead of the antibody solution and adding 1 N hydrochloric acid instead of the effector cell solution, and measuring the amount of $^{51}$Cr in the supernatant. The ADCC activity was calculated from the following equation (II):

$$\text{ADCC activity (\%)} = \frac{{}^{51}\text{Cr in sample supernatant - spontaneously released } {}^{51}\text{Cr}}{\text{total released } {}^{51}\text{Cr - spontaneously released } {}^{51}\text{Cr}} \times 100 \quad \text{(II)}$$

The results are shown in Fig. 3. As shown in Fig. 3, among the five kinds of the anti-GD3 chimeric antibodies, the YB2/0-GD3 chimeric antibody showed the most potent ADCC activity, followed by the SP2/0-GD3 chimeric antibody, NS0-GD3 chimeric antibody and CHO-GD3 chimeric antibody in that order. No difference in the ADCC activity was found between the NS0-GD3 chimeric antibody (302) and NS0-GD3 chimeric antibody (GIT) prepared using different media in the culturing. The above results show that the ADCC activity of antibodies greatly varies depending on the kind of the animal cells to be used in their production. As its mechanism, since their antigen binding activities were identical, it was considered that it is caused by a difference in the structure binding to the antibody Fc region.

Example 3

Preparation of anti-human interleukin 5 receptor α chain human CDR-grafted antibody:

1. Preparation of cell stably producing anti-human interleukin 5 receptor α chain human CDR-grafted antibody

(1) Preparation of antibody-producing cell using rat myeloma YB2/0 cell

Using the anti-human interleukin 5 receptor α chain human CDR-grafted antibody (hereinafter referred to as "anti-hIL-5Rα CDR-grafted antibody") expression vector, pKANTEX1259HV3LV0, described in WO 97/10354, cells capable of stably producing anti-hIL-5Rα CDR-grafted antibody were prepared as described below.

After introducing 5 μg of the anti-hIL-5Rα CDR-grafted antibody expression vector pKANTEX1259HV3LV0 into $4\times10^6$ cells of rat myeloma YB2/0 by electroporation [Cytotechnology, 3, 133 (1990)], the cells were suspended in 40 ml of RPMI1640-FBS(10) and dispensed in 200 μl/well into a 96 well culture plate (manufactured by Sumitomo Bakelite). After culturing them at 37°C for 24 hours in a 5% $CO_2$ incubator, G418 was added to give a concentration of 0.5 mg/ml, followed by culturing for 1 to 2 weeks. The culture supernatant was recovered from wells in which colonies of transformants showing G418 resistance were formed and growth of colonies was observed, and the antigen binding activity of the anti-hIL-5Rα CDR-grafted antibody in the supernatant was measured by the ELISA shown in the item 2 of Example 3.

Regarding the transformants in wells in which production of the anti-hIL-5Rα CDR-grafted antibody was observed in culture supernatants, in order to increase amount of the antibody production using a DHFR gene amplification system, each of the them was suspended in the RPMI1640-FBS(10) medium comprising 0.5 mg/ml G418 and 50 nM MTX to give a density of 1 to $2\times10^5$ cells/ml, and the suspension was dispensed in 2 ml into wells of a 24 well plate (manufactured by Greiner). Transformants showing 50 nM MTX resistance were induced by culturing at 37°C for 1 to 2 weeks in a 5% $CO_2$ incubator. The antigen binding activity of the anti-hIL-5Rα CDR-grafted antibody in culture supernatants in wells in which growth of transformants was observed was measured by the ELISA shown in the item 2 of Example 3. Regarding the transformants in wells in which production of the anti-hIL-5Rα CDR-grafted antibody was observed in culture supernatants, the MTX concentration was increased to 100 nM and then to 200 nM, and transformants capable of growing in the RPMI1640-FBS(10) medium comprising 0.5 mg/ml G418 and 200 nM MTX and of producing the anti-hIL-5Rα CDR-grafted antibody in a large amount were finally obtained in the same manner as described above. Among the obtained transformants, elite cell lines were selected and were made into a single cell (cloning) by limiting dilution twice. Also, using the method for determining the transcription product of an α-1,6-fucosyltransferase gene shown in Example 9, a cell line producing a relatively small amount of the transcription product was selected and used as a suitable cell line. The obtained anti-hIL-5Rα CDR-grafted antibody-producing transformed cell clone No. 3 has been deposited on April 5, 1999, as FERM BP-6690 in National Institute of Bioscience and Human Technology, Agency of Industrial Science and Technology (Higashi 1-1-3, Tsukuba, Ibaraki, Japan) (present name: International Patent Organism Depositary, National Institute of Advanced Industrial Science and Technology (AIST Tsukuba Central 6, 1-1, Higashi 1-Chome Tsukuba-shi, Ibaraki-ken, Japan)).

(2) Preparation of antibody-producing cell using CHO/dhfr⁻ cell

After introducing 4 μg of the anti-hIL-5Rα CDR-grafted antibody expression vector pKANTEX1259HV3LV0 described in WO 97/10354 into $1.6\times10^6$ cells of CHO/dhfr⁻ by electroporation [Cytotechnology, 3, 133 (1990)], the cells were suspended in 10 ml of IMDM-FBS(10) and dispensed in 200 μl/well into a 96 well culture plate (manufactured by Iwaki Glass). After culturing them at 37°C for 24 hours in a 5% $CO_2$ incubator, G418 was added to give a concentration of 0.5 mg/ml, followed by culturing for 1 to 2 weeks. The culture supernatant was recovered from respective well in which colonies of transformants showing G418 resistance were formed and growth of colonies was observed, and the antigen binding activity of the anti-hIL-5Rα CDR-grafted antibody in the supernatant was measured by the ELISA shown in the item 2 of Example 3.

Regarding the transformants in wells in which production of the anti-hIL-5Rα CDR-grafted antibody was observed in culture supernatants, in order to increase amount of the antibody production using a DHFR gene amplification system, each of the transformants was suspended in an IMDM-dFBS(10) medium comprising 0.5 mg/ml G418 and 10 nM MTX to give a density of 1 to $2\times10^5$ cells/ml, and the suspension was dispensed in 0.5 ml into wells of a 24 well plate (manufactured by Iwaki Glass). Transformants showing 10 nM MTX resistance were induced by culturing at 37°C for 1 to 2 weeks in a 5% $CO_2$ incubator. Regarding the transformants in wells in which their growth was observed, the MTX concentration was increased to 100 nM and then to 500 nM, and transformants capable of growing in the IMDM-dFBS(10) medium comprising 0.5 mg/ml G418 and 500 nM MTX and of producing the anti-hIL-5Rα CDR-grafted antibody in a large amount were finally obtained in the same manner as described above. Among the obtained transformants,

elite cell lines were selected and were made into a single cell (cloning) by limiting dilution twice. Also, using the method for determining the transcription product of an α-1,6-fucosyltransferase gene shown in Example 9, a cell line producing a relatively small amount of the transcription product was selected and used as a suitable cell line.

(3) Production of antibody-producing cell using mouse myeloma NS0 cell

An anti-hIL-5Rα CDR-grafted antibody expression vector was prepared in accordance with the method of Yarranton et al. [BIO/TECHNOLOGY, 10, 169 (1992)] and using the antibody H chain cDNA and L chain cDNA on the anti-hIL-5Rα CDR-grafted antibody expression vector pKANTEX1259HV3LV0 described in WO 97/10354, and NS0 cell was transformed to obtain transformants capable of producing the anti-hIL-5Rα CDR-grafted antibody in a large amount. Among the obtained transformants, elite cell lines were selected and were made into a single cell (cloning) by limiting dilution twice. Also, using the method for determining the transcription production of an α-1,6-fucosyltransferase gene shown in Example 9, a cell line producing a relatively small amount of the transcription product was selected and used as a suitable cell line.

2. Measurement of binding activity of antibody to hIL-5Rα (ELISA)

The binding activity of the antibody to hIL-5Rα was measured as described below.

A solution was prepared by diluting the anti-hIL-5Rα mouse antibody KM1257 described in WO 97/10354 with PBS to give a concentration of 10 μg/ml, and 50 μl of the resulting solution was dispensed into each well of a 96 well plate for ELISA (manufactured by Greiner), followed by reaction at 4°C for 20 hours. After the reaction, 1% BSA-PBS was dispensed in 100 μl/well, and then the reaction was carried out at room temperature for 1 hour to block remaining active groups. After discarding 1% BSA-PBS, a solution prepared by diluting the soluble hIL-5Rα described in WO 97/10354 with 1% BSA-PBS to give a concentration of 0.5 μg/ml was dispensed in 50 μl/well, followed by reaction at 4°C for 20 hours. After the reaction, each well was washed with Tween-PBS, culture supernatants of transformants or diluted solutions of a purified human CDR-grafted antibodies were dispensed in 50 μg/well to carry out the reaction at room temperature for 2 hours. After the reaction, each well was washed with Tween-PBS, a peroxidase-labeled goat anti-human IgG (H & L) antibody solution (manufactured by American Qualex) diluted 3,000 times with 1% BSA-PBS was dispensed in 50 μl/well as a secondary antibody solution, followed by reaction at room temperature for 1 hour. After the reaction and subsequent washing with Tween-PBS, the ABTS substrate solution was dispensed in 50 μl/well for color development, and then the absorbance at OD415 was measured.

3. Purification of anti-hIL-5Rα CDR-grafted antibody

(1) Culturing of antibody-producing cell derived from YB2/0 cell and purification of antibody

The anti-hIL-5Rα CDR-grafted antibody-producing transformed cell clone obtained in the item 1(1) of Example 3 was suspended in the GIT medium comprising 0.5 mg/ml G418 and 200 nM MTX to give a density of $3\times10^5$ cells/ml and dispensed in 200 ml into 175 $mm^2$ flasks (manufactured by Greiner). After culturing them at 37°C for 8 days in a 5% $CO_2$ incubator, the culture supernatant was recovered. The anti-hIL-5Rα CDR-grafted antibody was purified from the culture supernatant using ion exchange chromatography and a gel filtration method. The purified anti-hIL-5Rα CDR-grafted antibody was named YB2/0-hIL-5R CDR antibody.

(2) Culturing of antibody-producing cell derived from CHO/dhfr- cell and purification of antibody

The anti-hIL-5Rα CDR-grafted antibody-producing transformed cell clone obtained in the item 1(2) of Example 3 was suspended in the EX-CELL302 medium comprising 3 mM L-Gln, 0.5% CDLC and 0.3% PF68 to give a density of $3\times10^5$ cells/ml and cultured using a 4.0 liter capacity spinner bottle (manufactured by Iwaki Glass) under agitating at a rate of 100 rpm. After culturing them at 37°C for 10 days in a temperature-controlling room, the culture supernatant was recovered. The anti-hIL-5Rα CDR-grafted antibody was purified from the culture supernatant using ion exchange chromatography and a gel filtration method. The purified anti-hIL-5Rα CDR-grafted antibody was named CHO/d-hIL-5R CDR antibody.

(3) Culturing of antibody-producing cell derived from NS0 cell and purification of antibody

The anti-hIL-5Rα CDR-grafted antibody-producing transformed cell clone obtained in the item 1(3) of Example 3 was cultured in accordance with the method of Yarranton et al. [BIO/TECHNOLOGY, 10, 169 (1992)] and then a culture supernatant was recovered. The anti-hIL-5Rα CDR-grafted antibody was purified from the culture supernatant

using ion exchange chromatography and the gel filtration method. The purified anti-hIL-5Rα CDR-grafted antibody was named NS0-hIL-5R CDR antibody.

4. Analysis of purified anti-hIL-5Rα CDR-grafted antibodies

In accordance with a known method [Nature, 227, 680 (1970)], 4 μg of each of the three kinds of the anti-hIL-5Rα CDR-grafted antibodies produced by and purified from each animal cells, obtained in the item 3 of Example 3, was subjected to SDS-PAGE to analyze the molecular weight and purification degree. The results are shown in Fig. 4. As shown in Fig. 4, a single band of about 150 Kd in molecular weight was found under non-reducing conditions, and two bands of about 50 Kd and about 25 Kd under reducing conditions, in each of the purified anti-hIL-5Rα CDR-grafted antibodies. The molecular weights almost coincided with the molecular weights deduced from the cDNA nucleotide sequences of H chain and L chain of the antibody (H chain: about 49 Kd, L chain: about 23 Kd, whole molecule: about 144 Kd), and also coincided with the reports stating that the IgG antibody has a molecular weight of about 150 Kd under non-reducing conditions and is degraded into H chains having a molecular weight of about 50 Kd and L chains having a molecular weight of about 25 Kd under reducing conditions due to cutting of the S-S bond in the molecule [Antibodies: A Laboratory Manual, Cold Spring Harbor Laboratory, Chapter 14 (1998); Monoclonal Antibodies: Principles and Practice, Academic Press Limited (1996)], so that it was confirmed that each anti-hIL-5Rα CDR-grafted antibody was expressed and purified as an antibody molecule having the true structure.

Example 4

Activity evaluation of anti-hIL-5Rα CDR-grafted antibody:

1. Binding activity of anti-hIL-5Rα CDR-grafted antibody to hIL-5Rα (ELISA)

The activity of the three kinds of the purified anti-hIL-5Rα CDR-grafted antibodies obtained in the item 3 of Example 3 to bind to hIL-5Rα was measured by the ELISA shown in the item 2 of Example 3. Fig. 5 shows a result of the examination of the binding activity measured by changing concentration of the anti-hIL-5Rα CDR-grafted antibody to be added. As shown in Fig. 5, the three kinds of the anti-hIL-5Rα CDR-grafted antibodies showed almost the same binding activity to hIL-5Rα. The result shows that the antigen binding activities of these antibodies are constant independently of the antibody-producing animal cells and their culturing methods, similar to the result of the item 1 of Example 2.

2. *In vitro* cytotoxic activity (ADCC activity) of anti-hIL-5Rα CDR-grafted antibody

In order to evaluate *in vitro* cytotoxic activity of the three kinds of the purified anti-hIL-5Rα CDR-grafted antibodies obtained in the item 3 of Example 3, the ADCC activity was measured in accordance with the following method.

(1) Preparation of target cell solution

A mouse T cell line CTLL-2(h5R) expressing the hIL-5Rα chain and β chain described in WO 97/10354 was cultured using the RPMI1640-FBS(10) medium to give a density of $1\times10^6$ cells/0.5 ml, and the cells were radioisotope-labeled by reacting them with 3.7 MBq equivalents of a radioactive substance $Na_2{}^{51}CrO_4$ at 37°C for 1.5 hours. After the reaction, the cells were washed three times through their suspension in the RPMI1640-FBS(10) medium and centrifugation, resuspended in the medium and then incubated at 4°C for 30 minutes in ice for spontaneous dissolution of the radioactive substance. After the centrifugation, the precipitate was adjusted to give a density of $2\times10^5$ cells/ml by adding 5 ml of the RPMI1640-FBS(10) medium and used as the target cell solution.

(2) Preparation of effector cell solution

From a healthy person, 50 ml of venous blood was collected and gently mixed with 0.5 ml of heparin sodium (manufactured by Takeda Pharmaceutical). The mixture was centrifuged to separate a mononuclear cell layer using Polymorphprep (manufactured by Nycomed Pharma AS) and in accordance with the manufacture's instructions. After washing with the RPMI1640-FBS(10) medium by centrifugation three times, the resulting cells were resuspended to give a density of $9\times10^6$ cells/ml using the medium and used as the effector cell solution.

(3) Measurement of ADCC activity

Into each well of a 96 well U-shaped bottom plate (manufactured by Falcon), 50 μl of the target cell solution prepared in the above (1) ($1\times10^4$ cells/well) was dispensed. Next, 100 μl of the effector cell solution prepared in the above (2) was dispensed ($9\times10^5$ cells/well, the ratio of effector cells to target cells becomes 90:1). Subsequently, each of the anti-hIL-5Rα CDR-grafted antibodies was added to give a final concentration of 0.001 to 0.1 μg/ml, followed by reaction at 37°C for 4 hours. After the reaction, the plate was centrifuged, and the amount of $^{51}$Cr in the supernatant was measured using a γ-counter. The amount of spontaneously released $^{51}$Cr was calculated by the same operation using only the medium instead of the effector cell solution and the antibody solution, and measuring the amount of $^{51}$Cr in the supernatant. The amount of total released $^{51}$Cr was calculated by the same operation using only the medium instead of the antibody solution and adding 1 N hydrochloric acid instead of the effector cell solution, and measuring the amount of $^{51}$Cr in the supernatant.

The ADCC activity was calculated from the above equation (II).

The results are shown in Fig. 6. As shown in Fig. 6, among the three kinds of the anti-hIL-5Rα CDR-grafted antibodies, the YB2/0-hIL-5R CDR antibody showed the most potent ADCC activity, followed by the CHO/d-hIL-5R CDR antibody and the NS0-hIL-5R CDR antibody in this order. Similar to the result of the item 2 of Example 2, the above results show that the ADCC activity of antibodies greatly varies depending on the animal cells to be used in their production. In addition, since the antibodies produced by the YB2/0 cell showed the most potent ADCC activity in both cases of the two kinds of the humanized antibodies, it was revealed that an antibody having potent ADCC activity can be produced by using the YB2/0 cell.

3. *In vivo* activity evaluation of anti-hIL-5Rα CDR-grafted antibody

In order to evaluate *in vivo* activity of the three kinds of the purified anti-hIL-5Rα CDR-grafted antibodies obtained in the item 3 of Example 3, the inhibition activity in an hIL-5-induced eosinophilia increasing model of *Macaca faseicularis* was examined in accordance with the following method.

The hIL-5 (preparation method is described in WO 97/10354) was administered to *Macaca faseicularis* under the dorsal skin at a dose of 1 μg/kg, starting on the first day and once a day for a total of 14 times. Each anti-hIL-5Rα CDR-grafted antibody was intravenously administered at a dose of 0.3 mg/kg one hour before the hIL-5 administration on the day zero. An antibody-non-added group was used as the control. In the antibody-administered groups, three animals of *Macaca faseicularis* were used in each group (No. 301, No. 302, No. 303, No. 401, No. 402, No. 403, No. 501, No. 502 and No. 503), and two animals (No. 101 and No. 102) were used in the antibody-non-added group. Starting 7 days before commencement of the administration and until 42 days after the administration, about 1 ml of blood was periodically collected from a saphena or a femoral vein, and the number of eosinophils in 1 μl of peripheral blood was measured. The results are shown in Fig. 7. As shown in Fig. 7, increase in the blood eosinophil was completely inhibited in the group to which the YB2/0-hIL-5R CDR antibody was administered. On the other hand, complete inhibition activity was found in one animal in the group to which the CHO/d-hIL-5R CDR antibody was administered, but the inhibition activity was not sufficient in two animals. In the group to which NS0-hIL-5R CDR antibody was administered, complete inhibition activity was not found and its effect was not sufficient.

The above results show that the *in vivo* activity of antibodies greatly varies depending on the animal cells to be used in their production. In addition, since a positive correlation was found between the degree of the *in vivo* activity of the anti-hIL-5Rα CDR-grafted antibody and the degree of its ADCC activity described in the item 2 of Example 4, it was indicated that the degree of ADCC activity is remarkably important for its activity expression.

Based on the above results, it is expected that an antibody having potent ADCC activity is useful also in the clinical field for various diseases in human.

Example 5

Analysis of sugar chain which enhances ADCC activity:

1. Preparation of 2-aminopyridine-labeled sugar chain (PA-treated sugar chain)

The humanized antibody of the present invention was acid-hydrolyzed with hydrochloric acid to remove sialic acid. After hydrochloric acid was completely removed, the sugar chain was cleaved from the protein by hydrazinolysis [Method of Enzymology, 83, 263 (1982)]. Hydrazine was removed, and *N*-acetylation was carried out by adding an aqueous ammonium acetate solution and acetic anhydride. After lyophilizing, fluorescence labeling with 2-aminopyridine was carried out [J. Biochem., 95, 197 (1984)]. The fluorescence-labeled sugar chain (PA-treated sugar chain) was separated from impurity using Surperdex Peptide HR 10/30 Column (manufactured by Pharmacia). The sugar chain

fraction was dried using a centrifugal concentrator and used as a purified PA-treated sugar chain.

2. Reverse phase HPLC analysis of PA-treated sugar chain of purified anti-hIL-5Rα CDR-grafted antibody

According to the method in the item 1 of Example 5, various anti-hIL-5Rα CDR-grafted antibodies produced in Example 3 were subjected to PA-treated sugar chain treatment, and reverse phase HPLC analysis was carried out by CLC-ODS column (manufactured by Shimadzu). An excess amount of α-L-fucosidase (derived from bovine kidney, manufactured by SIGMA) was added to the PA-treated sugar chain for digestion (37°C, 15 hours), and then the products were analyzed by reverse phase HPLC (Fig. 8). It was confirmed that the asparagine-linked sugar chain is eluted for 30 minutes to 80 minute using PA-treated sugar chain standards manufactured by Takara Shuzo. The ratio of sugar chains whose reverse phase HPLC elution positions were shifted (sugar chains eluted for 48 minutes to 78 minutes) by the α-L-fucosidase digestion was calculated. The results are shown in Table 1.

Table 1

| Antibody-producing cell | α-1,6-Fucose-linked sugar chain (%) |
|---|---|
| YB2/0 | 47 |
| NS0 | 73 |

About 47% of the anti-hIL-5R CDR-grafted antibody produced by the YB2/0 cell and about 73% of the anti-hIL-5R CDR-grafted antibody produced by the NS0 cell were sugar chains in which 1-position of fucose is bound to 6-position of *N*-acetylglucosamine in the reducing end through α-bond in the *N*-glycoside-linked sugar chain (hereinafter referred to as "sugar chain having α-1,6-fucose"). Thus, the ratio of sugar chains in which 1-position of fucose is not bound to 6-position of *N*-acetylglucosamine in the reducing end through α-bond in the *N*-glycoside-linked sugar chain (hereinafter referred to as "α-1,6-fucose-free sugar chain") is higher in the antibody produced by the YB2/0 cell than in the antibody produced by the NS0 cell.

3. Analysis of monosaccharide composition of purified anti-hIL-5Rα CDR-grafted antibody

Sugar chains of anti-hIL-5Rα CDR-grafted antibodies produced by the YB2/0 cell, NS0 cell and CHO/d cell were hydrolyzed into monosaccharides by acid hydrolysis with trifluoroacetic acid, and monosaccharide composition analysis was carried out using BioLC (manufactured by Dionex).

Among *N*-glycoside-linked sugar chains, there are 3 mannose units in one sugar chain in the complex type *N*-glycoside-linked sugar chain. A relative ratio of each monosaccharide obtained by calculating the number of mannose as 3 is shown in Table 2.

Table 2

| Antibody-producing cell | Fuc | GlcNAc | Gal | Man | ADCC activity (%)* |
|---|---|---|---|---|---|
| YB2/0 | 0.60 | 4.98 | 0.30 | 3.00 | 42.27 |
| NS0 | 1.06 | 3.94 | 0.66 | 3.00 | 16.22 |
| CHO/dhFr⁻ | 0.85 | 3.59 | 0.49 | 3.00 | 25.73 |
| CHO/dhFr⁻ | 0.91 | 3.80 | 0.27 | 3.00 | 25.73 |
| *: Antibody concentration: 0.01 μg/ml | | | | | |

Since the relative ratios of fucose were in an order of YB2/0 < CHO/d < NS0, the sugar chain produced in the antibody produced by YB2/0 cell showed the lowest fucose content as also shown in the present results.

4. Sugar chain analysis of antibody produced by CHO/dhfr⁻ cell

PA-treated sugar chains were prepared from purified anti-hIL-5Rα CDR-grafted antibody produced by CHO/dhfr⁻ cell, and reverse phase HPLC analysis was carried out using CLC-ODS column (manufactured by Shimadzu) (Fig. 9). In Fig. 9, an elution time from 35 to 45 minutes corresponded to sugar chains having no fucose and an elution time from 45 to 60 minutes corresponded to sugar chains having fucose. Similar to the case of the antibody produced by mouse myeloma NS0 cell, the anti-hIL-5Rα CDR-grafted antibody produced by CHO/dhfr- cell had less fucose-free sugar chain content than the antibody produced by rat myeloma YB2/0 cell.

Example 6

Separation of potent ADCC activity antibody:

The anti-hIL-5Rα CDR-grafted antibody produced by rat myeloma YB2/0 cell was separated using a lectin column which binds to sugar chains having fucose. HPLC was carried out using LC-6A manufactured by Shimadzu at a flow rate of 1 ml/min and at room temperature as the column temperature. After equilibration with 50 mM Tris-sulfate buffer (pH 7.3), the purified anti-hIL-5Rα CDR-grafted antibody was injected and then eluted by a linear density gradient (60 minutes) of 0.2 M α-methylmannoside (manufactured by Nacalai Tesque). The anti-hIL-5Rα CDR-grafted antibody was separated into non-adsorbed fraction and adsorbed fraction. When the non-adsorbed fraction and a part of the adsorbed fraction were sampled and their binding activity to hIL-5Rα was measured, they showed similar binding activity (Fig. 10A). When the ADCC activity was measured, the non-adsorbed fraction showed potent ADCC activity (100 to 1000 folds) than that of the part of adsorbed fraction (Fig. 10B). In addition, PA-treated sugar chains were prepared from the non-adsorbed fraction and a part of the adsorbed fraction, and reverse HPLC analysis was carried out using CLC-ODS column (manufactured by Shimadzu) (Fig. 11). In the non-adsorbed fraction, an antibody binding to fucose-free sugar chains was mainly present, and in the part of adsorbed fraction, an antibody binding to sugar chains having fucose was mainly present.

Example 7

Activity evaluation of anti-GD3 chimeric antibody having different ratio of α-1,6-fucose-free sugar chain:

1. Preparation of anti-GD3 chimeric antibodies having different ratio of α-1,6-fucose-free sugar chain

In accordance with the method described in the item 2(1) of Example 1, transformed clones derived from YB2/0 cell capable of producing an anti-GD3 chimeric antibody was obtained. Antibodies were prepared from the transformed clones derived from YB2/0 cell and named lot 1, lot 2 and lot 3. Each sugar chain which is bound to the anti-GD3 chimeric antibodies of lot 1, lot 2 and lot 3 was analyzed in accordance with the method of Example 11(6), and it was found that the ratios of α-1,6-fucose-free sugar chains were 50%, 45% and 29%, respectively. Herein, these samples are referred to as anti-GD3 chimeric antibody (50%), anti-GD3 chimeric antibody (45%) and anti-GD3 chimeric antibody (29%).

Also, sugar chains of the anti-GD3 chimeric antibody derived from the CHO/DG44 cell prepared in the item 2(2) of Example 1 were analyzed in accordance with the method of Example 11(6), and it was found that the ratio of α-1,6-fucose-free sugar chains was 7%. Herein, the sample is referred to as anti-GD3 chimeric antibody (7%).

The anti-GD3 chimeric antibody (45%) and anti-GD3 chimeric antibody (7%) were mixed at a ratio of anti-GD3 chimeric antibody (45%) : anti-GD3 chimeric antibody (7%) = 5:3 or 1:7. Sugar chains of the samples were analyzed in accordance with the method of Example 10(6), and it was found that samples having the ratio of α-1,6-fucose-free sugar chains of 24% and 13% (calculated value) were prepared. Herein, they are referred to as anti-GD3 chimeric antibody (24%) and anti-GD3 chimeric antibody (13%).

Results of the sugar chain analysis of each of the samples are shown in Fig. 12. The ratio of α-1,6-fucose-free sugar chains was shown as an average value of the result of two sugar chain analyses.

2. Evaluation of binding activity to GD3 (ELISA)

The binding activities of the six kinds of the anti-GD3 chimeric antibodies having a different ratio of α-1,6-fucose-free sugar chains prepared in the item 1 of Example 7 against GD3 (manufactured by Snow Brand Milk Products) were measured by the ELISA shown in the item 3 of Example 1. As a result, all of the six kinds of the anti-GD3 chimeric antibodies showed almost the same GD3-binding activity as shown in Fig. 13, and it was found that the ratio of the α-1,6-fucose-free sugar chains does not have influence on the antigen binding activity of the antibody.

3. Evaluation of ADCC activity on human melanoma cell line

The ADCC activity of anti-GD3 chimeric antibodies on a human melanoma cell line G-361 (ATCC CRL 1424) was measured as follows.

(1) Preparation of target cell suspension

$1 \times 10^6$ cells of a human melanoma cell line G-361 were prepared, a 3.7 MBq equivalent of a radioactive substance $Na_2{}^{51}CrO_4$ was added thereto and the mixture was allowed to react at 37°C for 1 hour to label the cells with

the radioisotope. After the reaction, the cells were washed three times by a procedure of their suspension in a medium and subsequent centrifugation, re-suspended in the medium and then incubated at 4°C for 30 minutes in ice to effect spontaneous dissociation of the radioactive substance. After centrifugation, the cells were adjusted to $2 \times 10^5$ cells/ml by adding 5 ml of the medium and used as a target cell suspension.

(2) Preparation of human effector cell suspension

A 50 ml portion of peripheral blood was collected from a healthy person and gently mixed with 0.5 ml of heparin sodium (manufactured by Shimizu Pharmaceutical). Using Lymphoprep (manufactured by AXIS SHIELD), this was centrifuged (800 g, 20 minutes) in accordance with the manufacture's instructions to separate a mononuclear cell layer. The cells were washed by centrifuging (1,200 rpm, 5 minutes) three times using a medium and then re-suspended in the medium to give a density of $2 \times 10^6$ cells/ml and used as a human effector cell suspension.

(3) Measurement of ADCC activity

The target cell suspension prepared in the (1) was dispensed in 50 μl ($1 \times 10^4$ cells/well) into each well of a 96 well U-bottom plate (manufactured by Falcon). Next, 100 μl of the human effector cell suspension prepared in the (2) was added thereto ($2 \times 10^5$ cells/well, ratio of the human effector cells to the target cells was 20 : 1). Each of the anti-GD3 chimeric antibodies was added thereto to give a final concentration of 0.0005 to 5 μg/ml, followed by reaction at 37°C for 4 hours. After the reaction, the plate was centrifuged and the amount of $^{51}Cr$ in the supernatant was measured using a γ-counter. An amount of the spontaneously dissociated $^{51}Cr$ was calculated by carrying out the same procedure using the medium alone instead of the human effector cell suspension and antibody solution, and measuring the amount of $^{51}Cr$ in the supernatant. An amount of the total dissociated $^{51}Cr$ was calculated by carrying out the same procedure using the medium alone instead of the antibody solution and a 1 mol/l hydrochloric acid solution instead of the human effector cell suspension, and measuring the amount of $^{51}Cr$ in the supernatant. The cytotoxic activity (%) was calculated using equation (II).

Figs. 14 and 15 show results of the measurement of ADCC activity of the six kinds of the anti-GD3 chimeric antibodies having a different ratio of α-1,6-fucose-free sugar chains at various concentrations (0.0005 to 5 μg/ml) using effector cells of two healthy donors (A and B), respectively. As shown in Figs. 14 and 15, ADCC activity of the anti-GD3 chimeric antibodies showed a tendency to increase in proportion to the ratio of α-1,6-fucose-free sugar chains at each antibody concentration. The ADCC activity decreases when the antibody concentration is low. At an antibody concentration of 0.05 μg/ml, the antibody in which the ratio of α-1,6-fucose-free sugar chains is 24%, 29%, 45% or 50% showed almost the same potent ADCC activity but the ADCC activity was low in the antibody (13%) or (7%) in which the ratio of α-1,6-fucose-free sugar chains is less that 20%. These results were the same when the effector cell donor was changed.

Example 8

Activity evaluation of anti-CCR4 chimeric antibody having different ratio of α-1,6-fucose-free sugar chain:

1. Production of cells stably producing anti-CCR4 chimeric antibody

Cells which capable of stably producing an anti-CCR4 chimeric antibody were prepared as follows using a tandem type expression vector pKANTEX2160 for an anti-CCR4 chimeric antibody described in WO 01/64754.

(1) Preparation of antibody-producing cell using rat myeloma YB2/0 cell

After introducing 10 μg of the anti-CCR4 chimeric antibody expression vector pKANTEX2160 into $4 \times 10^6$ cells of rat myeloma YB2/0 cell (ATCC CRL 1662) by electroporation [Cytotechnology, 3, 133 (1990)], the cells were suspended in 40 ml of Hybridoma-SFM-FBS(5) [Hybridoma-SFM medium (manufactured by Invitrogen) comprising 5% FBS (manufactured by PAA Laboratories)] and dispensed in 200 μl/well into a 96 well culture plate (manufactured by Sumitomo Bakelite). After culturing them at 37°C for 24 hours in a 5% $CO_2$ incubator, G418 was added to give a concentration of 1 mg/ml, followed by culturing for 1 to 2 weeks. Culture supernatant was recovered from wells in which growth of transformants showing G418 resistance was observed by the formation of colonies, and antigen binding activity of the anti-CCR4 chimeric antibody in the supernatant was measured by the ELISA described in the item 2 of Example 8.

Regarding the transformants in wells in which production of the anti-CCR4 chimeric antibody was observed in culture supernatants, in order to increase an amount of the antibody production using a DHFR gene amplification system, each of them was suspended in the Hybridoma-SFM-FBS(5) medium comprising 1 mg/ml G418 and 50 nM DHFR inhibitor MTX (manufactured by SIGMA) to give a density of 1 to $2 \times 10^5$ cells/ml, and the suspension was dispensed

in 1 ml portions into wells of a 24 well plate (manufactured by Greiner). After culturing them at 37°C for 1 to 2 weeks in a 5% $CO_2$ incubator, transformants showing 50 nM MTX resistance were induced. Antigen binding activity of the anti-CCR4 chimeric antibody in culture supernatants in wells in which growth of transformants was observed was measured by the ELISA described in the item 2 of Example 8.

Regarding the transformants in wells in which production of the anti-CCR4 chimeric antibody was observed in culture supernatants, the MTX concentration was increased by the same method, and a transformant capable of growing in the Hybridoma-SFM-FBS(5) medium comprising 200 nM MTX and of producing the anti-CCR4 chimeric antibody in a large amount was finally obtained. The obtained transformant was made into a single cell (cloning) by limiting dilution twice, and the obtained cloned cell line was named KM2760 #58-35-16. In this case, using the method for determining the transcription product of an α-1,6-fucosyltransferase gene shown in Example 9, a cell line producing a relatively small amount of the transcription product was selected and used as a suitable cell line.

(2) Preparation of antibody-producing cell using CHO/DG44 cell

After introducing 4 μg of the anti-CCR4 chimeric antibody expression vector pKANTEX2160 into $1.6 \times 10^6$ cells of CHO/DG44 cell by electroporation [Cytotechnology, 3, 133 (1990)], the cells were suspended in 10 ml of IMDM-dFBS(10)-HT(1) [IMDM medium (manufactured by Invitrogen) comprising 10% dFBS (manufactured by Invitrogen) and 1 × concentration of HT supplement (manufactured by Invitrogen)] and dispensed in 100 μl/well into a 96 well culture plate (manufactured by Iwaki Glass). After culturing them at 37°C for 24 hours in a 5% $CO_2$ incubator, the medium was changed to IMDM-dFBS(10) (IMDM medium comprising 10% of dialyzed FBS), followed by culturing for 1 to 2 weeks. Culture supernatant was recovered from wells in which the growth was observed due to formation of a transformant showing HT-independent growth, and an expression level of the anti-CCR4 chimeric antibody in the supernatant was measured by the ELISA described in the item 2 of Example 8.

Regarding the transformants in wells in which production of the anti-CCR4 chimeric antibody was observed in culture supernatants, in order to increase an amount of the antibody production using a DHFR gene amplification system, each of them was suspended in the IMDM-dFBS(10) medium comprising 50 nM MTX to give a density of 1 to $2 \times 10^5$ cells/ml, and the suspension was dispensed in 0.5 ml into wells of a 24 well plate (manufactured by Iwaki Glass). After culturing them at 37°C for 1 to 2 weeks in a 5% $CO_2$ incubator, transformants showing 50 nM MTX resistance were induced. Regarding the transformants in wells in which the growth was observed, the MTX concentration was increased to 200 nM by the same method, and a transformant capable of growing in the IMDM-dFBS(10) medium comprising 200 nM MTX and of producing the anti-CCR4 chimeric antibody in a large amount was finally obtained. The obtained transformant was named 5-03. In this case, using the method for determining the transcription product of an α-1,6-fucosyltransferase gene shown in Example 9, a cell line producing a relatively small amount of the transcription product was selected and used as a suitable cell line.

2. Antibody binding activity to CCR4 partial peptide (ELISA)

Compound 1 (SEQ ID NO:25) was selected as a human CCR4 extracellular region peptide capable of reacting with the anti-CCR4 chimeric antibody. In order to use it in the activity measurement by ELISA, a conjugate with BSA (bovine serum albumin) (manufactured by Nacalai Tesque) was prepared by the following method and used as the antigen. That is, 100 ml of a DMSO solution comprising 25 mg/ml SMCC [4-(*N*-maleimidomethyl)-cyclohexane-1-carboxylic acid *N*-hydroxysuccinimide ester] (manufactured by Sigma) was added dropwise to 900 ml of a 10 mg BSA-containing PBS solution under stirring using a vortex, followed by gently stirring for 30 minutes. A 1 ml portion of the reaction solution was applied to a gel filtration column such as NAP-10 column or the like equilibrated with 25 ml of PBS, and then eluted with 1.5 ml of PBS and the resulting eluate was used as a BSA-SMCC solution (BSA concentration was calculated based on $A_{280}$ measurement). Next, 250 ml of PBS was added to 0.5 mg of Compound 1 and then completely dissolved by adding 250 ml of DMF, and the BSA-SMCC solution was added thereto under vortex, followed by gently stirring for 3 hours. The reaction solution was dialyzed against PBS at 4°C overnight, sodium azide was added thereto to give a final concentration of 0.05%, and the mixture was filtered through a 0.22 mm filter to be used as a BSA-compound 1 solution.

The prepared conjugate was dispensed at 0.05 μg/ml and 50 μl/well into a 96 well EIA plate (manufactured by Greiner) and incubated for adhesion at 4°C overnight. After washing each well with PBS, 1% BSA-PBS was added thereto in 100 μl/well and allowed to react at room temperature to block the remaining active groups. After washing each well with PBS containing 0.05% Tween 20 (hereinafter referred to as "Tween-PBS"), a culture supernatant of a transformant was added at 50 μl/well and allowed to react at room temperature for 1 hour. After the reaction, each well was washed with Tween-PBS, and then a peroxidase-labeled goat anti-human IgG(γ) antibody solution (manufactured by American Qualex) diluted 6000 times with 1% BSA-PBS as the secondary antibody was added at 50 μl/well and allowed to react at room temperature for 1 hour. After the reaction and subsequent washing with Tween-PBS, the ABTS substrate

solution was added at 50 μl/well for color development, and 20 minutes thereafter, the reaction was stopped by adding a 5% SDS solution at 50 μl/well. Thereafter, the absorbance at $OD_{415}$ was measured. The anti-CCR4 chimeric antibody obtained in the item 1 of Example 8 showed the binding activity to CCR4.

3. Purification of anti-CCR4 chimeric antibody

(1) Culturing of antibody-producing cell derived from YB2/0 cell and purification of antibody

The anti-CCR4 chimeric antibody-expressing transformant cell clone KM2760 # 58-35-16 obtained in the item 1(1) of Example 8 was suspended in Hybridoma-SFM (manufactured by Invitrogen) medium comprising 200 nM MTX and 5% of Daigo's GF21 (manufactured by Wako Pure Chemical Industries) to give a density of $2 \times 10^5$ cells/ml and subjected to fed-batch shaking culturing using a spinner bottle (manufactured by Iwaki Glass) in a constant temperature chamber of 37°C. After culturing them for 8 to 10 days, the anti-CCR4 chimeric antibody was purified from the culture supernatant recovered using Prosep-A (manufactured by Millipore) column and gel filtration. The purified anti-CCR4 chimeric antibody was named KM2760-1.

(2) Culturing of antibody-producing cell derived from CHO-DG44 cell and purification of antibody

The anti-CCR4 chimeric antibody-producing transformant cell line 5-03 obtained in the item 1(2) of Example 8 was cultured at 37°C in a 5% $CO_2$ incubator using IMDM-dFBS(10) medium in a 182 $cm^2$ flask (manufactured by Greiner). When the cell density reached confluent after several days, the culture supernatant was discarded, and the cells were washed with 25 ml of PBS buffer and then mixed with 35 ml of EXCELL 301 medium (manufactured by JRH). After culturing them at 37°C for 7 days in a 5% $CO_2$ incubator, the culture supernatant was recovered. The anti-CCR4 chimeric antibody was purified from the culture supernatant using Prosep-A (manufactured by Millipore) column in accordance with the manufacture's instructions. The purified anti-CCR4 chimeric antibody was named KM3060.

When the binding activity to CCR4 of KM2760-1 and KM3060 was measured by ELISA, they showed equivalent binding activity.

4. Analysis of purified anti-CCR4 chimeric antibodies

Each (4 μg) of the two kinds of the anti-CCR4 chimeric antibodies produced by and purified from in different animal cells, obtained in the item 1 of this Example was subjected to SDS-PAGE in accordance with a known method [Nature, 227, 680 (1970)], and the molecular weight and purification degree were analyzed. In each of the purified anti-CCR4 chimeric antibodies, a single band corresponding to the molecular weight of about 150 Kd was found under non-reducing conditions, and two bands of about 50 Kd and about 25 Kd were found under reducing conditions. The molecular weights almost coincided with the molecular weights deduced from the cDNA nucleotide sequences of antibody H chain and L chain (H chain: about 49 Kd, L chain: about 23 Kd, whole molecule: about 144 Kd) and coincided with reports stating that an IgG type antibody has a molecular weight of about 150 Kd under non-reducing conditions and is degraded into H chain having a molecular weight of about 50 Kd and L chain having a molecular weight of about 25 Kd under reducing conditions caused by cutting an S-S bond in the molecule [Antibodies: A Laboratory Manual, Cold Spring Harbor Laboratory, Chapter 14 (1988), Monoclonal Antibodies: Principles and Practice, Academic Press Limited (1996)], thus confirming that the anti-CCR4 chimeric antibody was expressed and purified as an antibody molecule having a correct structure.

5. Preparation of anti-CCR4 chimeric antibody having different ratio of α-1,6-fucose-free sugar chain

Sugar chains which are bound to anti-CCR4 chimeric antibody KM2760-1 derived from YB2/0 cell prepared in the item 3(1) of Example 8 and the anti-CCR4 chimeric antibody KM3060 derived from CHO/DG44 cell prepared in the item 3(2) of Example 8 were analyzed in accordance with the method of Example 10(6). The ratio of α-1,6-fucose-free sugar chains was 87% and 8% in KM2760 and KM3060, respectively. Herein, the samples are referred to as anti-CCR4 chimeric antibody (87%) and anti-CCR4 chimeric antibody (8%).

The anti-CCR4 chimeric antibody (87%) and anti-CCR4 chimeric antibody (8%) were mixed at a ratio of anti-CCR4 chimeric antibody (87%) : anti-CCR4 chimeric antibody (8%) = 1 : 39, 16 : 67, 22 : 57, 32 : 47 or 42 : 37. Sugar chains of these samples were analyzed in accordance with the method of Example 10(6). The ratio of α-1,6-fucose-free sugar chains was 9%, 18%, 27%, 39% and 46%, respectively. Herein, these samples are referred to as anti-CCR4 chimeric antibody (9%), anti-CCR4 chimeric antibody (18%), anti-CCR4 chimeric antibody (27%), anti-CCR4 chimeric antibody (39%) and anti-CCR4 chimeric antibody (46%).

Results of the sugar chain analysis of each of the samples are shown in Fig. 16. The ratio of α-1,6-fucose-free

sugar chains was shown as an average value of the result of two sugar chain analyses.

6. Evaluation of binding activity to CCR4 partial peptide (ELISA)

Binding activity of the six kinds of the different anti-CCR4 chimeric antibodies having a different α-1,6-fucose-free sugar chain prepared in the item 5 of Example 8 to CCR4 partial peptide was measured in accordance with the method described in the item 2 of Example 8.

As a result, as shown in Fig. 17, the six kinds of the anti-CCR4 chimeric antibodies showed almost the same CCR4-binding activity, it was found that the ratio of α-1,6-fucose-free sugar chains does not have influence on the antigen-binding activity of the antibody.

7. Evaluation of ADCC activity on human CCR4-high expressing cell line

The ADCC activity of the anti-CCR4 chimeric antibodies against a human CCR4-high expressing cell CCR4/EL-4 cell (WO 01/64754) was measured as follows.

(1) Preparation of target cell suspension

Cells ($1.5 \times 10^6$) of a human CCR4-expressing cell, CCR4/EL-4 cell, described in WO 01/64754 were prepared and a 5.55 MBq equivalent of a radioactive substance $Na_2{}^{51}CrO_4$ was added thereto, followed by reaction at 37°C for 1.5 hours to thereby label the cells with a radioisotope. After the reaction, the cells were washed three times by suspension in a medium and subsequent centrifugation, resuspended in the medium and then incubated at 4°C for 30 minutes in ice for spontaneous dissociation of the radioactive substance. After centrifugation, the cells were adjusted to give a density of $2 \times 10^5$ cells/ml by adding 7.5 ml of the medium and used as a target cell suspension.

(2) Preparation of human effector cell suspension

From a healthy person, 60 ml of peripheral blood was collected, 0.6 ml of heparin sodium (manufactured by Shimizu Pharmaceutical) was added thereto, followed by gently mixing. The mixture was centrifuged (800 g, 20 minutes) to isolate a mononuclear cell layer using Lymphoprep (manufactured by AXIS SHIELD) in accordance with the manufacture's instructions. The cells were washed by centrifuging (1,400 rpm, 5 minutes) three times using a medium and then re-suspended in the medium to give a density of $5 \times 10^6$ cells/ml and used as a human effector cell suspension.

(3) Measurement of ADCC activity

The target cell suspension prepared in the (1) was dispensed at 50 μl ($1 \times 10^4$ cells/well) into each well of a 96 well U-bottom plate (manufactured by Falcon). Next, 100 μl of the human effector cell suspension prepared in the (2) was added thereto ($5 \times 10^5$ cells/well, ratio of the human effector cells to the target cells was 50 : 1). Furthermore, each of the anti-CCR4 chimeric antibodies was added thereto to give a final concentration of 0.0001 to 10 μg/ml, followed by reaction at 37°C for 4 hours. After the reaction, the plate was centrifuged and the amount of $^{51}Cr$ in the supernatant was measured using a γ-counter. An amount of the spontaneously dissociated $^{51}Cr$ was calculated by carrying out the same procedure using the medium alone instead of the human effector cell suspension and antibody solution, and measuring the amount of $^{51}Cr$ in the supernatant. An amount of the total dissociated $^{51}Cr$ was calculated by carrying out the same procedure using a 1 mol/L hydrochloric acid solution instead of the antibody solution and human effector cell suspension, and measuring the amount of $^{51}Cr$ in the supernatant. The ADCC activity (%) was calculated based on equation (II).

Figs. 18 and 19 show results of the measurement of ADCC activity of the anti-CCR4 chimeric antibodies having a different ratio of α-1,6-fucose-free sugar chains at various concentrations (0.001 to 10 μg/ml) using effector cells of two healthy donors (A and B), respectively. As shown in Figs. 18 and 19, the ADCC activity of the anti-CCR4 chimeric antibodies showed a tendency to increase in proportion to the ratio of α-1,6-fucose-free sugar chains at each antibody concentration. The ADCC activity decreases when the antibody concentration is low. At an antibody concentration of 0.01 μg/ml, the antibody in which the α-1,6-fucose-free sugar chains is 27%, 39% or 46% showed almost the same potent ADCC activity but the ADCC activity was low in the antibody in which the ratio of α-1,6-fucose-free sugar chains is less than 20%. The results were the same as the case when the effector cell donor was changed.

Example 9

Determination of transcription product of α-1,6-fucosyltransferase gene in host cell line:

(1) Preparation of single-stranded cDNA from various cell lines

Single-stranded cDNA samples were prepared from dihydrofolate reductase gene (dhfr)-deleted CHO/DG44 cells derived from Chinese hamster ovary and rat myeloma YB2/0 cells by the following procedure.

The CHO/DG44 cells were suspended in IMDM medium (manufactured by Life Technologies) supplemented with 10% fetal bovine serum (manufactured by Life Technologies) and 1 × concentration HT supplement (manufactured by Life Technologies), and 15 ml of the suspension was inoculated into T75 flask for adhesion cell culture use (manufactured by Greiner) at a density of $2 \times 10^5$ cells/ml. Also, the YB2/0 cells were suspended in RPMI 1640 medium (manufactured by Life Technologies) supplemented with 10% fetal bovine serum (manufactured by Life Technologies) and 4 mmol/l L-GLN (manufactured by Life Technologies), and 15 ml of the suspension was inoculated into T75 flask for suspension cell culture (manufactured by Greiner) at a density of $2 \times 10^5$ cells/ml. They were cultured at 37°C in a 5% $CO_2$ incubator, and $1 \times 10^7$ of respective host cells were recovered on the 1st, 2nd, 3rd, 4th and 5th days of the culturing to extract total RNA using RNAeasy (manufactured by QIAGEN) in accordance with the manufacture's instructions.

The total RNA was dissolved in 45 μl of sterile water, 1 μl of RQ1 RNase-Free DNase (manufactured by Promega), 5 μl of the attached 10 × DNase buffer and 0.5 μl of RNasin Ribonuclease Inhibitor (manufactured by Promega) were added thereto, followed by reaction at 37°C for 30 minutes to degrade genome DNA contaminated in the sample. After the reaction, the total RNA was purified again using RNAeasy (manufactured by QIAGEN) and dissolved in 50 μl of sterile water.

In a 20 μl of the reaction mixture using oligo(dT) as a primer, single-stranded cDNA was synthesized from 3 μg of each of the obtained total RNA samples by reverse transcription reaction using SUPERSCRIPT™ Preamplification System for First Strand cDNA Synthesis (manufactured by Life Technologies) and in accordance with the manufacture's instructions. A 1 × concentration solution of the reaction solution was used for the cloning of α-1,6-fucosyltransferase (hereinafter referred sometimes to as "FUT8") and β-actin derived from respective host cells, and 50 folds-diluted aqueous solution of the reaction solution for the determination of each gene transcription level by competitive PCR, and the solutions were stored at -80°C until use.

(2) Preparation of cDNA partial fragments of Chinese hamster FUT8 and rat FUT8

Each cDNA partial fragment of Chinese hamster FUT8 and rat FUT8 was prepared by the following procedure (Fig. 20).

First, primers (shown in SEQ ID NOs:4 and 5) specific for nucleotide sequences common to human FUT8 cDNA [J. Biochem., 121, 626 (1997)] and swine FUT8 cDNA [J. Biol. Chem., 271, 27810 (1995)] were designed.

Next, 25 μl of a reaction solution [ExTaq buffer (manufactured by Takara Shuzo), 0.2 mmol/l dNTPs and 0.5 μmol/l gene-specific primers (SEQ ID NOs:4 and 5)] containing 1 μl of each of the cDNA prepared from CHO cell and cDNA prepared from YB2/0 cell, both obtained in the item (1) 2 days after culturing, and polymerase chain reaction (PCR) was carried out using a DNA polymerase ExTaq (manufactured by Takara Shuzo). The PCR was carried out by heating at 94°C for 1 minute, subsequent 30 cycles of heating at 94°C for 30 seconds, 55°C for 30 seconds and 72°C for 2 minutes as one cycle, and final heating at 72°C for 10 minutes.

After the PCR, the reaction solution was subjected to 0.8% agarose gel electrophoresis, and a specific amplified fragment of 979 bp was purified using GENECLEAN Spin Kit (manufactured by BIO 101) and eluted with 10 μl of sterile water (hereinafter, the method was used for the purification of DNA fragments from agarose gel). Into a plasmid pCR2.1, 4 μl of the amplified fragment was employed to insert in accordance with the manufacture's instructions of TOPO TA Cloning Kit (manufactured by Invitrogen), and *E. coli* XL1-Blue was transformed with the reaction solution by the method of Cohen et al. [Proc. Natl. Acad. Sci. USA, 69, 2110 (1972)] (hereinafter, the method was used for the transformation of *E. coli).* Plasmid DNA samples were isolated in accordance with a known method [Nucleic Acids Research, 7, 1513 (1979)] (hereinafter, the method was used for the isolation of plasmid) from cDNA-inserted 6 clones among the obtained kanamycin-resistant colonies.

The nucleotide sequence of each cDNA inserted into the plasmid was determined using DNA Sequencer 377 (manufactured by Parkin Elmer) and BigDye Terminator Cycle Sequencing FS Ready Reaction kit (manufactured by Parkin Elmer) in accordance with the method of the manufacture's instructions. It was confirmed that all of the inserted cDNAs of which sequences were determined by the method encode the open reading frame (ORF) partial sequences of Chinese hamster FUT8 or rat FUT8 (shown in SEQ ID NOs:6 and 7). Among these, plasmid DNA samples containing absolutely no reading error by the PCR in the sequences were selected. Herein, these plasmids are referred to as

CHFUT8-pCR2.1 and YBFUT8-pCR2.1.

(3) Preparation of Chinese hamster β-actin and rat β-actin cDNA

Chinese hamster β-actin and rat β-actin cDNA were prepared by the following procedure (Fig. 21).

First, a forward primer specific for a common sequence containing translation initiation codon (shown in SEQ ID NO:8) and reverse primers specific for respective sequences containing translation termination codon (shown in SEQ ID NOs:9 and 10) were designed from Chinese hamster β-actin genomic sequence (GenBank, U20114) and rat β-actin genomic sequence [Nucleic Acids Research, 11, 1759 (1983).

Next, 25 μl of a reaction solution [KOD buffer #1 (manufactured by Toyobo), 0.2 mmol/l dNTPs, 1 mmol/l $MgCl_2$, 0.4 μmol/l gene-specific primers (SEQ ID NOs:8 and 9, or SEQ ID NOs:8 and 10) and 5% DMSO] containing 1 μl of each of the cDNA prepared from CHO cell and cDNA prepared from YB2/0 cell, both obtained in the item (1) 2 days after culturing was prepared, and PCR was carried out using a DNA polymerase KOD (manufactured by Toyobo). The PCR was carried out by heating at 94°C for 1 minute and subsequent 25 cycles of heating at 98°C for 15 seconds, 65°C for 2 seconds and 74°C for 30 seconds as one cycle.

After the PCR, the reaction solution was subjected to 0.8% agarose gel electrophoresis, and a specific amplified fragment of 1128 bp was purified. The DNA fragment was subjected to DNA 5'-terminal phosphorylation using MEGALABEL (manufactured by Takara Shuzo) in accordance with the manufacture's instructions. The DNA fragment was recovered from the reaction solution using ethanol precipitation method and dissolved in 10 μl of sterile water.

Separately, 3 μg of a plasmid pBluescript II KS(+) (manufactured by Stratagene) was dissolved in 35 μl of NEBuffer 2 (manufactured by New England Biolabs), and 16 units of a restriction enzyme *Eco*RV (manufactured by Takara Shuzo) were added thereto for digestion reaction at 37°C for 3 hours. To the reaction solution, 35 μl of 1 mol/l Tris-HCl buffer (pH 8.0) and 3.5 μl of *E. coli* C15-derived alkaline phosphatase (manufactured by Takara Shuzo) were added thereto, followed by reaction at 65°C for 30 minutes to thereby dephophorylate the DNA terminus. The reaction solution was extracted with phenol/chloroform, followed by ethanol precipitation, and the recovered DNA fragment was dissolved in 100 μl of sterile water.

Each 4 μl of the amplified fragment prepared from Chinese hamster cDNA or the amplified fragment (1192 bp) prepared from rat cDNA was mixed with 1 μl of the *Eco*RV-*Eco*RV fragment (about 3.0 Kb) prepared from plasmid pBluescript II KS(+) and 5 μl of Ligation High (manufactured by Toyobo) for ligation reaction at 16°C for 30 minutes. Using the reaction solution, *E. coli* XL1-Blue was transformed, and plasmid DNA samples were isolated respectively in accordance with a known method from the obtained ampiciliin-resistant clones.

The nucleotide sequence of each cDNA inserted into the plasmid was determined using DNA Sequencer 377 (manufactured by Parkin Elmer) and BigDye Terminator Cycle Sequencing FS Ready Reaction kit (manufactured by Parkin Elmer) in accordance with the method of the manufacture's instructions. It was confirmed that all of the inserted cDNAs of which sequences were determined by the method encode the ORF full sequences of Chinese hamster β-actin or rat β-actin. Among these, plasmid DNA samples containing absolutely no reading error of bases by the PCR in the sequences were selected. Herein, the plasmids are called CHAc-pBS and YBAc-pBS.

(4) Preparation of FUT8 standard and internal control

In order to measure a transcription level of FUT8 gene mRNA in each cell, CHFT8-pCR2.1 or YBFT8-pCR2.1, as plasmids in which cDNA partial fragments prepared in the item (2) from Chinese hamster FUT8 or rat FUT8 were inserted into pCR2.1, respectively, were digested with a restriction enzyme *EcoRI,* and the obtained linear DNAs were used as the standards for the preparation of a calibration curve. CHFT8d-pCR2.1 and YBFT8d-pCR2.1, which were obtained from the CHFT8-pCR2.1 and YBFT8-pCR2.1, by deleting 203 bp between *Sca*I and *Hind*III*,* an inner nucleotide sequence of Chinese hamster FUT8 and rat FUT8, respectively, were digested with a restriction enzyme *EcoRI,* and the obtained linear DNAs were used as the internal standards for FUT8 amount determination. Details thereof are described below.

Chinese hamster FUT8 and rat FUT8 standards were prepared as follows. In 40 μl of NEBuffer 2 (manufactured by New England Biolabs), 2 μg of the plasmid CHFT8-pCR2.1 was dissolved, 24 units of a restriction enzyme *Eco*RI (manufactured by Takara Shuzo) were added thereto, followed by digestion reaction at 37°C for 3 hours. Separately, 2 μg of the plasmid YBFT8-pCR2.1 was dissolved in 40 μl of NEBuffer 2 (manufactured by New England Biolabs), and 24 units of a restriction enzyme *Eco*RI (manufactured by Takara Shuzo) were added thereto, followed by digestion reaction at 37°C for 3 hours. By subjecting a portion of each of the reaction solutions to 0.8% agarose gel electrophoresis, it was confirmed that an *EcoRI*-*EcoRI* fragment (about 1 Kb) containing each of cDNA partial fragments of Chinese hamster FUT8 and rat FUT8 was separated from the plasmids CHFT8-pCR2.1 and YBFT8-pCR2.1 by the restriction enzyme digestion reactions. Each of the reaction solutions was diluted with 1 μg/ml of baker's yeast t-RNA (manufactured by SIGMA) to give a concentration of 0.02 fg/μl, 0.2 fg/μl, 1 fg/μl, 2 fg/μl, 10 fg/μl, 20 fg/μl and 100 fg/μl and used as

the Chinese hamster FUT8 and rat FUT8 standards.

Internal standards of Chinese hamster FUT8 and rat FUT8 were prepared as follows (Fig. 22). A reaction solution [KOD buffer #1 (manufactured by Toyobo), 0.2 mmol/l dNTPs, 1 mmol/l $MgCl_2$, 0.4 μmol/l gene-specific primers (SEQ ID NOs:11 and 12) and 5% DMSO] containing 5 ng of CHFT8-pCR2.1 or YBFT8-pCR2.1 was prepared, and PCR was carried out using a DNA polymerase KOD (manufactured by Toyobo). The PCR was carried out by heating at 94°C for 4 minutes and subsequent 25 cycles of heating at 98°C for 15 seconds, 65°C for 2 seconds and 74°C for 30 seconds as one cycle. After the PCR, the reaction solution was subjected to 0.8% agarose gel electrophoresis, and a specific amplified fragment of about 4.7 Kb was purified. The DNA 5'-terminal was phosphorylated using MEGALABEL (manufactured by Takara Shuzo) in accordance with the manufacture's instructions, and then the DNA fragment was recovered from the reaction solution by ethanol precipitation and dissolved in 50 μl of sterile water. The obtained DNA fragment (5 μl, about 4.7 kb) and 5 μl of Ligation High (manufactured by Toyobo) were mixed, followed by self-cyclization reaction at 16°C for 30 minutes.

Using the reaction solution, *E. coli* DH5α was transformed, and plasmid DNA samples were isolated in accordance with a known method from the obtained ampicillin-resistant clones. The nucleotide sequence of each plasmid DNA was determined using DNA Sequencer 377 (manufactured by Parkin Elmer) and BigDye Terminator Cycle Sequencing FS Ready Reaction kit (manufactured by Parkin Elmer), and it was confirmed that a 203 bp inner nucleotide sequence between *Sca*I and *Hind*III of Chinese hamster FUT8 or rat FUT8 was deleted. The obtained plasmids are referred to as CHFT8d-pCR2.1 or YBFT8d-pCR2.1, respectively.

Next, 2 μg of the plasmid CHFT8d-pCR2.1 was dissolved in 40 μl of NEBuffer 2 (manufactured by New England Biolabs), and 24 units of a restriction enzyme *Eco*RI (manufactured by Takara Shuzo) were added thereto, followed by digestion reaction at 37°C for 3 hours. Separately, 2 μg of the plasmid YBFT8d-pCR2.1 was dissolved in 40 μl of NEBuffer 2 (manufactured by New England Biolabs), and 24 units of a restriction enzyme *EcoRI* (manufactured by Takara Shuzo) were added thereto, followed by digestion reaction at 37°C for 3 hours. A portion of each of the reaction solutions was subjected to 0.8% agarose gel electrophoresis, and it was confirmed that an *EcoRI-EcoRI* fragment (about 800 bp) containing a fragment from which 203 bp of the inner nucleotide sequences of Chinese hamster FUT8 or rat FUT8 partial fragments was deleted was separated from the plasmids CHFT8d-pCR2.1 or YBFT8d-pCR2.1 by the restriction enzyme digestion reactions. Dilutions of 2 fg/μl were prepared from the reaction solutions using 1 μg/ml baker's yeast t-RNA (manufactured by SIGMA) and used as the Chinese hamster FUT8 or rat FUT8 internal controls.

(5) Preparation of β-actin standard and internal control

In order to measure the transcription level of β-actin gene mRNA in various host cells, CHAc-pBS and YBAc-pBS, as plasmids in which the ORF full length of each cDNA of Chinese hamster β-action and rat β-actin prepared in the item (3) was inserted into pBluescript II KS(+), respectively, were digested with restriction enzymes *Hind*III and *Pst*I and restriction enzymes *Hind*III and *Kpn*I, respectively, and the digested linear DNAs were used as the standards for the preparation of a calibration curve. CHAcd-pBS and YBAcd-pBS which were obtained from the CHAc-pBS and YBAc-pBS by deleting 180 bp between *Dra*III and *Dra*III of an inner nucleotide sequence of Chinese hamster β-actin and rat β-actin were digested with restriction enzymes *Hind*III and *Pst*I and restriction enzymes *Hind*III and *Kpn*I, respectively, and the digested linear DNAs were used as the internal standards for β-action amount determination. Details thereof are described below.

Chinese hamster β-actin and rat β-actin standards were prepared as follows. In 40 μl of NEBuffer 2 (manufactured by New England Biolabs), 2 μg of the plasmid CHAc-pBS was dissolved, and 25 units of a restriction enzyme *Hind*III (manufactured by Takara Shuzo) and 20 units of *Pst*I (manufactured by Takara Shuzo) were added thereto, followed by digestion reaction at 37°C for 3 hours. Separately, 2 μg of the plasmid YBAc-pBS was dissolved in 40 μl of NEBuffer 2 (manufactured by New England Biolabs), and 25 units of a restriction enzyme *Hind*III (manufactured by Takara Shuzo) and 25 units of *Kpn*I (manufactured by Takara Shuzo) were added thereto, followed by digestion reaction at 37°C for 3 hours. A portion of each of the reaction solutions was subjected to 0.8% agarose gel electrophoresis, and it was confirmed that a *Hind*III-*Pst*I fragment and a *Hind*III-*Kpn*I fragment (about 1.2 Kb) containing the full length ORF of each cDNA of Chinese hamster β-actin and rat β-actin were separated from the plasmids CHAc-pBS and YBAc-pBS by the restriction enzyme digestion reactions. Each of the reaction solutions was diluted with 1 μg/ml baker's yeast t-RNA (manufactured by SIGMA) to give a concentration 2 pg/μl, 1 pg/μl, 200 fg/μl, 100 fg/μl and 20 fg/μl and used as the Chinese hamster β-actin and or β-actin standards.

Chinese hamster β-actin and rat β-actin internal standards were prepared as follows (Fig. 23). In 100 μl of NEBuffer 3 (manufactured by New England Biolabs) containing 100 ng/μl of BSA (manufactured by New England Biolabs), 2 μg of CHAc-pBS was dissolved, and 10 units of a restriction enzyme *Dra*III (manufactured by New England Biolabs) were added thereto, followed by digestion reaction at 37°C for 3 hours. DNA fragments were recovered from the reaction solution by ethanol precipitation and the DNA termini were changed to blunt ends using DNA Blunting Kit (manufactured by Takara Shuzo) in accordance with the manufacture's instructions, and then the reaction solution was

divided into two equal parts. First, to one part of the reaction solution, 35 μl of 1 mol/l Tris-HCl buffer (pH 8.0) and 3.5 μl of *E. coli* C15-derived alkaline phosphatase (manufactured by Takara Shuzo) were added thereto, followed by reaction at 65°C for 30 minutes for dephosphorylating the DNA termini. The DNA fragment was recovered by carrying out dephosphorylation treatment, phenol/chloroform extraction treatment and ethanol precipitation treatment and then dissolved in 10 μl of sterile water. The remaining part of the reaction solution was subjected to 0.8% agarose gel electrophoresis to purify a DNA fragment of about 1.1 Kb containing the ORF partial fragment of Chinese hamster β-actin.

The dephosphorylated *Dra*III-*Dra*III fragment (4.5 μl), 4.5 μl of the *Dra*III-*Dra*III fragment of about 1.1 Kb and 5 μl of Ligation High (manufactured by Toyobo) were mixed, followed by ligation reaction at 16°C for 30 minutes. Using the reaction solution, *E. coli* DH5α was transformed, and plasmid DNAs were isolated in accordance with a known method from the obtained ampicillin-resistant clones. The nucleotide sequence of each plasmid DNA was determined using DNA Sequencer 377 (manufactured by Parkin Elmer) and BigDye Terminator Cycle Sequencing FS Ready Reaction kit (manufactured by Parkin Elmer), and it was confirmed that a Chinese hamster β-actin *Dra*III-*Dra*III 180 bp inserted into the plasmid was deleted. The plasmid is referred to as CHAcd-pBS.

Also, a plasmid in which rat β-actin *Dra*III-*Dra*III 180 bp was deleted was prepared via the same steps of CHAcd-pBS. The plasmid is referred to as YBAcd-pBS.

Next, 2 μg of the plasmid CHAcd-pBS was dissolved in 40 μl of NEBuffer 2 (manufactured by New England Biolabs), and 25 units of a restriction enzyme *Hin*dIII (manufactured by Takara Shuzo) and 20 units of *Pst*I (manufactured by Takara Shuzo) were added thereto, followed by digestion reaction at 37°C for 3 hours. Separately, 2 μg of the plasmid YBAcd-pBS was dissolved in 40 μl of NEBuffer 2 (manufactured by New England Biolabs), and 25 units of a restriction enzyme *Hin*dIII (manufactured by Takara Shuzo) and 24 units of *Kpn*I (manufactured by Takara Shuzo) were added thereto, followed by digestion reaction at 37°C for 3 hours. A portion of each of the reaction solutions was subjected to 0.8% agarose gel electrophoresis, and it was confirmed that an *Hind*III-*Pst*I fragment and *Hind*III-*Kpn*I fragment (about 1.0 Kb) containing a fragment in which 180 bp of the inner nucleotide sequence of the ORF full length of each cDNA of Chinese hamster β-actin and rat β-actin was deleted were separated from the plasmids CHAcd-pBS and YBAcd-pBS by the restriction enzyme digestion reactions. Dilutions of 200 fg/μl were prepared from the reaction solutions using 1 μg/ml baker's yeast t-RNA (manufactured by SIGMA) and used as the Chinese hamster β-actin and rat β-actin internal controls.

(6) Determination of transcription level by competitive PCR

Competitive PCR was carried out using the FUT8 internal control DNA prepared in the item (4) and the host cell-derived cDNA obtained in the item (1) as the templates, the determined value of the FUT8 transcription product in the host cell line was calculated from the relative value of the amount of the amplified product derived from each template. On the other hand, since it is considered that the β-actin gene is transcribed continuously in each cell and its transcription level is approximately the same between cells, transcription level of the β-actin gene was determined as a measure of the efficiency of synthesis reaction of cDNA in each host cell line. That is, the PCR was carried out using the β-actin internal control DNA prepared in the item (5) and the host cell-derived cDNA obtained in the item (1) as the templates, the determined value of the β-actin transcription product in the host cell line was calculated from the relative value of the amount of the amplified product derived from each template. Details thereof are described below.

The FUT8 transcription product was determined by the following procedure. First, a set of sequence-specific primers (shown in SEQ ID NOs: 13 and 14) common to the inner sequences of the ORF partial sequences of Chinese hamster FUT8 and rat FUT8 obtained in the item (2) were designed.

Next, PCR was carried out using a DNA polymerase ExTaq (manufactured by Takara Shuzo) in 20 μl in total volume of a reaction solution [ExTaq buffer (manufactured by Takara Shuzo), 0.2 mmol/l dNTPs, 0.5 μmol/l gene-specific primers (SEQ ID NOs:13 and 14) and 5% DMSO] containing 5 μl of 50 folds-diluted cDNA solution prepared from each of respective host cell line in the item (1) and 5 μl (10 fg) of the plasmid for internal control. The PCR was carried out by heating at 94°C for 3 minutes and subsequent 32 cycles of heating at 94°C for 1 minute, 60°C for 1 minute and 72°C for 1 minute as one cycle.

Also, PCR was carried out in a series of reaction in which 5 μl (0.1 fg, 1 fg, 5 fg, 10 fg, 50 fg, 100 fg, 500 fg or 1 pg) of the FUT8 standard plasmid obtained in the item (4) was added instead of the each host cell line-derived cDNA, and used in the preparation of a calibration curve for the FUT8 transcription level.

The β-actin transcription product was determined by the following procedure. First, two sets of respective gene-specific primers common to the inner sequences of the ORF full lengths of Chinese hamster β-actin and rat β-actin obtained in the item (3) were designed (the former are shown in SEQ ID NOs:15 and 16, and the latter are shown in SEQ ID NOs:17 and 18).

Next, PCR was carried out using a DNA polymerase ExTaq (manufactured by Takara Shuzo) in 20 μl in total volume of a reaction solution [ExTaq buffer (manufactured by Takara Shuzo), 0.2 mmol/l dNTPs, 0.5 μmol/l gene-specific primers (SEQ ID NOs:15 and 16, or SEQ ID NOs:17 and 18) and 5% DMSO] containing 5 μl of 50 folds-diluted cDNA

solution prepared from respective host cell line in the item (1) and 5 μl (1 pg) of the plasmid for internal control. The PCR was carried out by heating at 94°C for 3 minutes and subsequent 17 cycles of heating at 94°C for 30 seconds, 65°C for 1 minute and 72°C for 2 minutes as one cycle.

Also, PCR was carried out in a series of reaction in which 5 μl (10 pg, 5 pg, 1 pg, 500 fg or 100 fg) of the β-actin standard plasmid obtained in the item (5) was added instead of the each host cell line-derived cDNA, and used in the preparation of a calibration curve for the β-actin transcription level.

Table 3

| Target gene | Primer set | Size (bp) PCR amplification product | |
|---|---|---|---|
| | | Target | Competitor |
| FUT8 | F: 5'-GTCCATGGTGATCCTGCAGTGTGG-3'<br>R: 5'-CACCAATGATATCTCCAGGTTCC-3' | 638 | 431 |
| β-Actin (Chinese hamster) | F: 5'-GATATCGCTGCGCTCGTTGTCGAC-3'<br>R: 5'-CAGGAAGGAAGGCTGGAAAAGAGC-3' | 789 | 609 |
| β-action (Rat) | F:5'-GATATCGCTGCGCTCGTCGTCGAC-3'<br>R: 5'-CAGGAAGGAAGGCTGGAAGAGAGC-3' | 789 | 609 |

* F: forward primer, R: reverse primer

By carrying out PCR using the primer set described in Table 3, a DNA fragment having a size shown in the target column of Table 3 can be amplified from each gene transcription product and each standard, and a DNA fragment having a size shown in the competitor column of Table 3 can be amplified from each internal control.

A 7 μl portion of each of the solutions after PCR was subjected to 1.75% agarose gel electrophoresis, and then the gel was stained by soaking it for 30 minutes in 1 × concentration SYBR Green I Nucleic Acid Gel Stain (manufactured by Molecular Probes). The amount of the amplified DNA fragment was measured by calculating luminescence intensity of each amplified DNA using a fluoro-imager (FluorImager SI; manufactured by Molecular Dynamics).

The amount of an amplified product formed by PCR using a standard plasmid as the template was measured by the method, and a calibration curve was prepared by plotting the measured values against the amounts of the standard plasmid. Using the calibration curve, the amount of cDNA of a gene of interest in each cell line was calculated from the amount of the amplified product when each expression cell line-derived cDNA was used as the template, and the amount was defined as the mRNA transcription level in each cell line.

The amount of the FUT8 transcription product in each host cell line when a rat FUT8 sequence was used in the standard and internal control is shown in Fig. 24. Throughout the culturing period, the CHO cell line showed a transcription level 10 folds or higher than that of the YB2/0 cell line. The tendency was also found when a Chinese hamster FUT8 sequence was used in the standard and internal control.

Also, the FUT8 transcription levels are shown in Table 4 as relative values to the amount of the β-actin transcription product. Throughout the culturing period, the FUT8 transcription level in the YB2/0 cell line was around 0.1% of β-actin while it was 0.5% to 2% in the CHO cell line.

The results shows that the amount of the FUT8 transcription product in YB2/0 cell line was significantly smaller than that in the CHO cell line.

Table 4

| | Culture days | | | | |
|---|---|---|---|---|---|
| Cell line | 1st | 2nd | 3rd | 4th | 5th |
| CHO | 1.95 | 0.90 | 0.57 | 0.52 | 0.54 |
| YB2/0 | 0.12 | 0.11 | 0.14 | 0.08 | 0.07 |

Example 10

Determination of transcription product of α-1,6-fucosyltransferase (FUT8) gene in anti-ganglioside GD3 chimeric antibody-producing cell line:

(1) Preparation of single-stranded cDNA from various antibody-producing cell lines

Single-stranded cDNA was prepared from anti-ganglioside GD3 chimeric antibody-producing cell lines DCHI01-20 and 61-33 as follows. The DCHI01-20 is a transformant clone derived from the CHO/DG44 cell described in item 2(2) of Example 1. Also, the 61-33 is a clone obtained by carrying out serum-free adaptation of YB2/0-derived transformant cell 7-9-51 (FERM BP-6691, International Patent Organism Depositary, National Institute of Advanced Industrial Science and Technology (AIST Tsukuba Central 6, 1-1, Higashi 1-Chome Tsukuba-shi, Ibaraki-ken 305-8566 Japan)) and then carrying out single cell isolation by two limiting dilution.

Cells of the DCHI01-20 were suspended in EXCELL 302 medium (manufactured by JRH BIOSCIENCES) supplemented with 3 mmol/l L-GLN (manufactured by Life Technologies), 0.3% PLURONIC F-68 (manufactured by Life Technologies) and 0.5% fatty acid concentrate (manufactured by Life Technologies), and 15 ml of the suspension was inoculated into T75 flask for suspension cell culture use (manufactured by Greiner) at a density of $2 \times 10^5$ cells/ml. Also, cells of the 61-33 were suspended in Hybridoma-SFM medium (manufactured by Life Technologies) supplemented with 0.2% bovine serum albumin fraction V (manufactured by Life Technologies) (hereinafter referred to as "BSA"), and 15 ml of the suspension was inoculated into T75 flask for suspension cell culture (manufactured by Greiner) at a density of $2 \times 10^5$ cells/ml. They were cultured at 37°C in a 5% $CO_2$ incubator, and 1, 2, 3, 4 and 5 days after culturing, $1 \times 10^7$ of respective host cells were recovered to extract total RNA using RNAeasy (manufactured by QIAGEN) in accordance with the manufacture's instructions.

The total RNA was dissolved in 45 μl of sterile water, and 1 μl of RQ1 RNase-Free DNase (manufactured by Promega), 5 μl of the attached 10 × DNase buffer and 0.5 μl of RNasin Ribonuclease Inhibitor (manufactured by Promega) were added thereto, followed by reaction at 37°C for 30 minutes to degrade genome DNA contaminated in the sample. After the reaction, the total RNA was purified again using RNAeasy (manufactured by QIAGEN) and dissolved in 50 μl of sterile water.

In a 20 μl reaction mixture using oligo(dT) as a primer, single-stranded cDNA was synthesized from 3 μg of each of the obtained total RNA samples by reverse transcription reaction using SUPERSCRIPT™ Preamplification System for First Strand cDNA Synthesis (manufactured by Life Technologies) in accordance with the manufacture's instructions. The reaction solution was diluted 50 folds with water and stored at -80°C until use.

(2) Determination of transcription levels of each gene by competitive PCR

The transcription level of each of the genes on the cDNA derived from the antibody-producing cell line obtained in the item (1) was determined by competitive PCR in accordance with Example 9(6).

The FUT8 gene-derived mRNA transcription level in each of the antibody-producing cell lines was determined by the following procedure.

CHFT8-pCR2.1 and YBFT8-pCR2.1, as plasmids in which cDNA partial fragments prepared in Example 9(2) from Chinese hamster FUT8 and rat FUT8, respectively, were inserted into pCR2.1, were digested with a restriction enzyme *Eco*RI, and the obtained linear DNAs were used as the standards in the preparation of a calibration curve for determining the FUT8 transcription level.

CHFT8d-pCR2.1 and YBFT8d-pCR2.1, which were obtained by deleting 203 bp between *Sca*I and *Hind*III of an inner nucleotide sequence of Chinese hamster FUT8 and rat FUT8, respectively, in Example 9(4) were digested with a restriction enzyme *EcoRI,* and the obtained linear DNAs were used as the internal standards for FUT8 amount determination.

PCR was carried out using a DNA polymerase ExTaq (manufactured by Takara Shuzo) in 20 μl in total volume of a reaction solution [ExTaq buffer (manufactured by Takara Shuzo), 0.2 mmol/l dNTPs, 0.5 μmol/l FUT8 gene-specific primers (SEQ ID NOs:13 and 14) and 5% DMSO] containing 5 μl of 50 folds-diluted cDNA solution prepared from each of the antibody-producing cell line in the item (1) and 5 μl (10 fg) of the plasmid for internal control. The PCR was carried out by heating at 94°C for 3 minutes and subsequent 32 cycles of heating at 94°C for 1 minute, 60°C for 1 minute and 72°C for 1 minute as one cycle.

Also, PCR was carried out in a series of reaction in which 5 μl (0.1 fg, 1 fg, 5 fg, 10 fg, 50 fg, 100 fg, 500 fg or 1 pg) of the FUT8 standard plasmid was added instead of the each antibody-producing cell line-derived cDNA, and used in the preparation of a calibration curve for the FUT8 transcription level. In this case, 1 μg/ml of a baker's yeast t-RNA (manufactured by SIGMA) was used for the dilution of the standard plasmid.

On the other hand, since it is considered that the β-actin gene is transcribed constantly in each cell and its

transcription level is approximately the same between cells, the transcription level of the β-actin gene was determined as an index of the efficiency of synthesis reaction of cDNA in each antibody-producing cell line.

CHAc-pBS and YBAc-pBS as plasmids in which the ORF full length of each cDNA of Chinese hamster β-actin and rat β-actin prepared in Example 9(3) were inserted into pBluescript II KS(+), respectively, were digested with restriction enzymes *Hind*III and *Kpn*I, and the obtained linear DNA samples were used as the standards in the preparation of a calibration curve for determining the β-actin transcription level.

CHAcd-pBS and YBAcd-pBS which were obtained by deleting 180 bp between *Dra*I and *Dra*I of an inner nucleotide sequence of Chinese hamster β-actin and rat β-actin, respectively in Example 9(5), were digested with restriction enzymes *Hind*III and *Kpn*I, and the obtained linear DNAs were used as the internal standards for β-actin determination.

PCR was carried out using a DNA polymerase ExTaq (manufactured by Takara Shuzo) in 20 μl in total volume of a reaction solution [ExTaq buffer (manufactured by Takara Shuzo), 0.2 mmol/l dNTPs, 0.5 μmol/l β-actin-specific primers (SEQ ID NOs:17 and 18) and 5% DMSO] containing 5 μl of 50 folds-diluted cDNA solution prepared from each of the antibody-producing cell lines and 5 μl (1 pg) of the plasmid for internal control. The PCR was carried out by heating at 94°C for 3 minutes and subsequent 17 cycles of heating at 94°C for 30 seconds, 65°C for 1 minute and 72°C for 2 minutes as one cycle. Also, PCR was carried out in a series of reaction in which 10 pg, 5 pg, 1 pg, 500 fg or 100 fg of the β-actin standard plasmid was added instead of the each antibody-producing cell line-derived cDNA, and used in the preparation of a calibration curve for the β-actin transcription level. In this case, 1 μg/ml of a baker's yeast t-RNA (manufactured by SIGMA) was used for the dilution of standard plasmid.

By PCR using the primer set described in Table 3, a DNA fragment having a size shown in the target column of Table 3 can be amplified from each gene transcription product and each standard, and a DNA fragment having a size shown in the competitor column of Table 3 can be amplified from each internal control.

A 7 μl portion of each of the solutions after PCR was subjected to 1.75% agarose gel electrophoresis, and then the gel was stained by soaking it for 30 minutes in 1 × concentration SYBR Green I Nucleic Acid Gel Stain (manufactured by Molecular Probes). The amount of the amplified DNA fragment was measured by calculating luminescence intensity of each amplified DNA using a fluoro-imager (FluorImager SI; manufactured by Molecular Dynamics).

The amount of the amplified product formed by PCR which used a standard plasmid as the template was measured by the method, and a calibration curve was prepared by plotting the measured values against the amounts of the standard plasmid. Using the calibration curve, the amount of cDNA of a gene of interest in each cell line was calculated from the amount of the amplified product when each antibody-producing cell line-derived cDNA was used as the template, and the value was defined as the mRNA transcription level in each cell line.

The FUT8 transcription levels are shown in Table 5 as relative values to the amount of the β-actin transcription product. Throughout the culturing period, the FUT8 transcription level in the YB2/0 cell-derived antibody-producing 61-33 was 0.3% or less of β-actin while it was 0.7% to 1.5% in the CHO cell-derived antibody-producing cell.

The results shows that the amount of the FUT8 transcription product in the YB2/0 cell-derived antibody-producing cell line was significantly less than that in the antibody-producing cell line derived from the CHO cell.

Table 5

| | Culture days | | | | |
|---|---|---|---|---|---|
| Cell line | 1st | 2nd | 3rd | 4th | 5th |
| DCHI01-20 | 0.75 | 0.73 | 0.99 | 1.31 | 1.36 |
| 61-33 | 0.16 | 0.19 | 0.24 | 0.30 | <0.10 |

Example 11

Preparation of mouse α-1,6-fucosyltransferase (FUT8) gene over-expressing cell line:

(1) Construction of mouse α-1,6-fucosyltransferase (FUT8) expression plasmid

Total RNA was extracted from 1 × $10^7$ cells of a mouse myeloma NS0 cell (RCB0213, Cell Bank at The Institute of Physical and Chemical Research) subcultured using IMDM medium (manufactured by Life Technologies) containing 10% fetal bovine serum (manufactured by Life Technologies), using RNAeasy (manufactured by QIAGEN) in accordance with the manufacture's instructions. The total RNA was dissolved in 45 μl of sterile water, and 1 μl of RQ1 RNase-Free DNase (manufactured by Promega), 5 μl of the attached 10 × DNase buffer and 0.5 μl of RNasin Ribonuclease Inhibitor (manufactured by Promega) were added thereto, followed by reaction at 37°C for 30 minutes to degrade genome DNA contaminated in the sample. After the reaction, the total RNA was purified again using RNAeasy (manufactured by

QIAGEN) and dissolved in 50 μl of sterile water. In a 20 μl reaction mixture using oligo(dT) as a primer, single-stranded cDNA was synthesized from 3 μg of the obtained total RNA by reverse transcription reaction using SUPERSCRIPT™ Preamplification System for First Strand cDNA Synthesis (manufactured by Life Technologies) in accordance with the manufacture's instructions.

Mouse FUT8 cDNA was prepared by the following procedure (Fig. 25).

First, a forward primer specific for a sequence containing a translation initiation codon (shown in SEQ ID NO:19) and a reverse primer specific for a sequence containing translation termination codon (shown in SEQ ID NO:20) were designed from a mouse FUT8 cDNA sequence (GenBank, AB025198).

Next, 25 μl of a reaction solution [ExTaq buffer (manufactured by Takara Shuzo), 0.2 mmol/l dNTPs, 4% DMSO and 0.5 μmol/l specific primers (SEQ ID NO:19 and SEQ ID NO:20)] containing 1 μl of the NS0 cell-derived cDNA was prepared, and PCR was carried out using a DNA polymerase ExTaq (manufactured by Takara Shuzo). The PCR was carried out by heating at 94°C for 1 minute, subsequent 30 cycles of heating at 94°C for 30 seconds, 55°C for 30 seconds and 72°C for 2 minutes as one cycle, and final heating at 72°C for 10 minutes.

After the PCR, the reaction solution was subjected to 0.8% agarose gel electrophoresis, and a specific amplified fragment of 1728 bp was purified. Into a plasmid pCR2.1, 4 μl of the DNA fragment was employed to insert in accordance with the manufacturer's instructions attached to TOPO TA Cloning Kit (manufactured by Invitrogen), and *E. coli* DH5α was transformed with the reaction solution. Plasmid DNAs were isolated in accordance with a known method from cDNA-inserted 6 clones among the obtained kanamycin-resistant colonies.

The nucleotide sequence of each cDNA inserted into the plasmid was determined using DNA Sequencer 377 (manufactured by Parkin Elmer) and BigDye Terminator Cycle Sequencing FS Ready Reaction kit (manufactured by Parkin Elmer) in accordance with the method of the manufacture's instructions. It was confirmed that all of the inserted cDNAs of which sequences were determined encode the ORF full sequence of mouse FUT8. Among these, a plasmid DNA containing absolutely no reading error of bases by the PCR in the sequences were selected (its DNA sequence and amino acid sequence are shown in SEQ ID NOs:2 and 24, respectively). Also, inconsistency of 3 bases due to amino acid substitution was found in the sequence when compared with the mouse FUT8 sequence registered on GenBank. Herein, the plasmid is referred to mfFUT8-pCR2.1.

Next, a plasmid pBSmfFUT8 containing the ORF full sequence of mouse FUT8 was constructed as follows (Fig. 26). First, 1 μg of a plasmid pBluescript II KS(+) (manufactured by Stratagene) was dissolved in 35 μl of NEBuffer 2 (manufactured by New England Biolabs), and 20 units of a restriction enzyme *EcoRI* (manufactured by Takara Shuzo) were added thereto, followed by digestion reaction at 37°C for 2 hours. To the reaction solution, 35 μl of 1 mol/l Tris-HCl buffer (pH 8.0) and 3.5 μl of *E. coli* C15-derived alkaline phosphatase (manufactured by Takara Shuzo) were added, followed by reaction at 65°C for 30 minutes for dephosphorylate the DNA termini. The reaction solution was extracted with phenol/chloroform, followed by ethanol precipitated, and the recovered DNA fragment was dissolved in 10 μl of sterile water.

Separately, 1 μg of the plasmid mfFUT8-pCR2.1 was dissolved in 35 μl of NEBuffer 2 (manufactured by New England Biolabs), and 20 units of a restriction enzyme *EcoRI* (manufactured by Takara Shuzo) were added thereto, followed by digestion reaction at 37°C for 2 hours. The reaction solution was subjected to 0.8% agarose gel electrophoresis to purify a DNA fragment of about 1.7 Kb containing the ORF full sequence of mouse FUT8 cDNA.

The obtained plasmid pBluescript II KS(+)-derived *Eco*RI-*Eco*RI fragment (1 μl, 2.9 Kb), 4 μl of the *EcoRI-EcoRI* fragment (1.7 Kb) prepared from the plasmid mfFUT8-pCR2.1 and 5 μl of Ligation High (manufactured by Toyobo) were mixed, followed by ligation reaction at 16°C for 30 minutes. Using the reaction solution, *E. coli* DH5α was transformed, and plasmid DNAs were isolated in accordance with a known method from the obtained ampicillin-resistant clones. Herein, the plasmid is referred to pBSmfFUT8.

Using the pBSmfFUT8 and pAGE249, a mouse FUT8 expression vector pAGEmfFUT8 was constructed by the following procedure (Fig. 27). The pAGE249 is a derivative of pAGE248 [J. Biol. Chem., 269, 14730 (1994)], as a vector in which an *Sph*I-*SphI* fragment (2.7 Kb) containing a dihydrofolate reductase gene (dhfr) expression unit was removed from the pAGE248.

In 50 μl of Universal Buffer H (manufactured by Takara Shuzo), 1 μg of the pAGE249 was dissolved, and 20 units of a restriction enzyme *Sal*I (manufactured by New England Biolabs) were added thereto, followed by digestion reaction at 37°C for 2 hours. A DNA fragment was recovered from the reaction solution by ethanol precipitation and dissolved in 35 μl of NEBuffer 2 (manufactured by New England Biolabs), and 20 units of a restriction enzyme *Bam*HI (manufactured by New England Biolabs) were added thereto, followed by digestion reaction at 37°C for 2 hours. After the digestion reaction, to the reaction solution, 35 μl of 1 mol/l Tris-HCl buffer (pH 8.0) and 3.5 μl of *E. coli* C15-derived alkaline phosphatase (manufactured by Takara Shuzo) were added thereto, followed by reaction at 65°C for 30 minutes to dephosphorylate the DNA termini. The reaction solution was extracted with phenol/chloroform extraction, followed by ethanol precipitation, and the recovered DNA fragment was dissolved in 10 μl of sterile water.

Separately, 1 μg of pBSmfFUT8 was dissolved in 50 μl of Universal Buffer H (manufactured by Takara Shuzo), and 20 units of a restriction enzyme *Sal*I (manufactured by New England Biolabs) were added thereto, followed by

digestion reaction at 37°C for 2 hours. A DNA fragment was recovered from the reaction solution by ethanol precipitation and dissolved in 35 μl of NEBuffer 2 (manufactured by New England Biolabs), and 20 units of a restriction enzyme *Bam*HI (manufactured by New England Biolabs) were added thereto, followed by digestion reaction at 37°C for 2 hours. After the digestion reaction, the solution was subjected to 0.8% agarose gel electrophoresis to purify a DNA fragment of about 1.7 Kb containing the ORF full sequence of mouse FUT8 cDNA.

The obtained plasmid pAGE249-derived *Bam*HI-*Sal*I fragment (1 μl, 6.5 Kb), 4 μl of the *Bam*HI-*Sal*I fragment (1.7 Kb) prepared from the plasmid pBSmfFUT8 and 5 μl of Ligation High (manufactured by Toyobo) were mixed, followed by ligation reaction at 16°C for 30 minutes. Using the reaction solution, *E. coli* DH5α was transformed, and a plasmid DNA was isolated in accordance with a known method from the obtained ampicillin-resistant clones. Herein, the plasmid is referred to pAGEmfFUT8.

(2) Preparation of mouse α-1,6-fucosyltransferase (FUT8) gene over-expressing cell line

A stable FUT8 gene-expressing cell line was obtained by introducing the mouse FUT8 expression vector pAGEmfFUT8 constructed in the item (1) into 61-33. The 61-33 is a clone obtained by carrying out serum-free adaptation of a transformant cell 7-9-51 (FERM BP-6691, International Patent Organism Depositary, National Institute of Advanced Industrial Science and Technology) derived from a YB2/0 cell highly producing an anti-ganglioside GD3 chimeric antibody, and then carrying out single cell isolation by two limiting dilution.

The plasmid pAGEmfFUT8 was transferred into 61-33 by the following procedure in accordance with the electroporation *[Cytotechnology,* 3, 133 (1990)]. First, 30 μg of the plasmid pAGEmfFUT8 was dissolved in 600 μl of NEBuffer 4 (manufactured by New England Biolabs), and 100 units of a restriction enzyme *Fsp*I (manufactured by New England Biolabs) were added thereto, followed by digestion reaction at 37°C for 2 hours to obtain a linear fragment. The reaction solution was subjected to ethanol precipitation, and the recovered linear plasmid was made into a 1 μg/μl aqueous solution. Next, the 61-33 was suspended in a K-PBS buffer (137 mol/l KCl, 2.7 mol/l NaCl, 8.1 mol/l $Na_2HPO_4$, 1.5 mol/l $KH_2PO_4$, 4.0 mol/l $MgCl_2$) to give a density of $2 \times 10^7$ cells/ml, and 200 μl of the cell suspension ($4 \times 10^6$ cells) was mixed with 10 μl (10 μg) of the linear plasmid. The cell-DNA mixture was transferred into Gene Pulser Cuvette (inter-electrode distance, 2 mm) (manufactured by BIO-RAD) and then electroporation was carried out using a cell fusion apparatus Gene Pulser (manufactured by BIO-RAD) at 0.2 KV pulse voltage and 250 μF electric capacity. The cell suspension was mixed with 10 ml of Hybridoma-SFM medium (manufactured by Life Technologies) supplemented with 5% fetal bovine dialyzed serum (manufactured by Life Technologies) and 0.2% BSA (manufactured by Life Technologies) and dispensed in 100 μl portions into a 96 well plate for suspension cell use (manufactured by Greiner). After culturing them at 37°C for 24 hours in 5% $CO_2$, 50 μl of the culture supernatant was removed, and Hybridoma-SFM medium (manufactured by Life Technologies) supplemented with 0.5 mg/ml Hygromycin B (manufactured by Wako Pure Chemical Industries), 5% fetal bovine dialyzed serum (manufactured by Life Technologies) and 0.2% BSA (manufactured by Life Technologies) was dispensed at 100 μl. They were cultured for 3 weeks while repeating the medium exchange step at intervals of 3 to 4 days, and 14 cell lines showing hygromycin resistance were obtained.

On the other hand, a negative control cell line was prepared by introducing the plasmid pAGE249 as a parent vector of the pAGEmfFUT8 into the 61-33. According to the above procedure, 10 μg of the plasmid pAGE249 converted into linear form with a restriction enzyme *Fsp*I was introduced into $4 \times 10^6$ cells of the 61-33 using the electroporation. The cells were mixed with 15 ml of Hybridoma-SFM medium (manufactured by Life Technologies) supplemented with 5% fetal bovine dialyzed serum (manufactured by Life Technologies) and 0.2% BSA (manufactured by Life Technologies), transferred into a T75 flask for suspension cell (manufactured by Greiner) and then cultured at 37°C for 24 hours in 5% $CO_2$. After culturing them, a half of the culture supernatant (7.5 ml) was removed by centrifugation at 800 rpm for 4 minutes, and the cells were suspended in 7.5 ml of Hybridoma-SFM medium (manufactured by Life Technologies) supplemented with 0.5 mg/ml Hygromycin B (manufactured by Wako Pure Chemical Industries), 5% fetal bovine dialyzed serum (manufactured by Life Technologies) and 0.2% BSA (manufactured by Life Technologies) and transferred into the T75 flask for suspension cell (manufactured by Greiner). They were cultured for 3 weeks while repeating the medium exchange at intervals of 3 to 4 days, a hygromycin-resistant cell line was obtained.

(3) Analysis of expression level of α-1,6-fucosyltransferase (FUT8) gene in cell lines over-expressing the gene

Using 6 cell lines optionally selected from the 14 mouse FUT8-over expressing cell lines prepared from 61-33 in the item (2) and the negative control cell line, the FUT8 expression levels were compared using competitive RT-PCR.

Each of these over-expression cell lines was suspended in Hybridoma-SFM medium (manufactured by Life Technologies) supplemented with 0.5 mg/ml Hygromycin B (manufactured by Wako Pure Chemical Industries), 5% fetal bovine dialyzed serum (manufactured by Life Technologies) and 0.2% BSA (manufactured by Life Technologies) to give a density of $3 \times 10^5$ cells/ml and then transferred into a T75 flask for suspension cell culture use (manufactured by Greiner). After culturing them at 37°C for 24 hours in 5% $CO_2$, $1 \times 10^7$ of intact cells were recovered to extract total

RNA using RNAeasy (manufactured by QIAGEN) in accordance with the manufacture's instructions. The total RNA was dissolved in 45 μl of sterile water, and 0.5 U/μl of RQ1 RNase-Free DNase (manufactured by Promega), 5 μl of the attached 10 × DNase buffer and 0.5 μl of RNasin Ribonuclease Inhibitor (manufactured by Promega) were added thereto, followed by reaction at 37°C for 30 minutes to degrade genome DNA contaminated in the sample. After the reaction, the total RNA was purified again using RNAeasy (manufactured by QIAGEN) and dissolved in 50 μl of sterile water.

In a 20 μl reaction mixture using oligo(dT) as a primer, single-stranded cDNA was synthesized from 2.5 μg of the obtained total RNA by reverse transcription reaction using SUPERSCRIPT™ Preamplification System for First Strand cDNA Synthesis (manufactured by Life Technologies) in accordance with the manufacture's instructions. The reaction solution was diluted 50 folds with water and the transcription level of each gene was determined by the competitive PCR in accordance with Example 9(6).

The FUT8 gene-derived mRNA transcription level in each expression cell line was determined by the following procedure.

YBFT8-pCR2.1, as a plasmid in which a cDNA partial fragment prepared in Example 9(2) from rat FUT8 was inserted into pCR2.1, was digested with a restriction enzyme *EcoR*I, and the obtained linear DNA was used as the standard in the preparation of a calibration curve for determining the FUT8 transcription level.

Among the YBFT8-pCR2.1 prepared in Example 9(4), YBFT8d-pCR2.1 obtained by deleting 203 bp between *Sca*I and *Hind*III of an inner nucleotide sequence of rat FUT8 was digested with a restriction enzyme *Eco*RI, and the obtained linear DNA was used as the internal control for FUT8 determination.

PCR was carried out using a DNA polymerase ExTaq (manufactured by Takara Shuzo) in 20 μl in total volume of a reaction solution [ExTaq buffer (manufactured by Takara Shuzo), 0.2 mmol/l dNTPs, 0.5 μmol/l FUT8 gene-specific primers (SEQ ID NOs:13 and 14) and 5% DMSO] containing 5 μl of 50 folds-diluted cDNA solution prepared from respective expression cell line in the above and 5 μl (10 fg) of the plasmid for internal control. The PCR was carried out by heating at 94°C for 3 minutes and subsequent 32 cycles of heating at 94°C for 1 minute, 60°C for 1 minute and 72°C for 1 minute as one cycle.

Also, PCR was carried out in a series of reaction in which 5 μl (0.1 fg, 1 fg, 5 fg, 10 fg, 50 fg, 100 fg, 500 fg or 1 pg) of the FUT8 standard plasmid was added instead of the each expression cell line-derived cDNA, and used in the preparation of a calibration curve for the FUT8 transcription level. In this case, 1 μg/ml baker's yeast t-RNA (manufactured by SIGMA) was used for the dilution of standard plasmid.

On the other hand, since it is considered that the β-actin gene is transcribed constantly in each cell and its transcription level is approximately the same between cells, the transcription level of the β-actin gene was determined as an index of the efficiency of synthesis reaction of cDNA in each expression cell line.

YBAc-pBS, as a plasmid in which the ORF full sequence of cDNA of rat β-actin was inserted into pBluescript II KS(+) prepared in Example 9(3), was digested with restriction enzymes *Hind*III and *Kpn*I, and the obtained linear DNA was used as the standard in the preparation of a calibration curve for determining the β-actin gene transcription level.

YBAcd-pBS obtained from the YBAc-pBS by deleting 180 bp between *Dra*I and *Dra*I of an inner nucleotide sequence of rat β-actin was digested with restriction enzymes *Hind*III and *Kpn*I, and the obtained linear DNA was used as the internal standards for β-actin amount determination.

PCR was carried out using a DNA polymerase ExTaq (manufactured by Takara Shuzo) in 20 μl in total volume of a reaction solution [ExTaq buffer (manufactured by Takara Shuzo), 0.2 mmol/l dNTPs, 0.5 μmol/l β-actin-specific primers (SEQ ID NOs: 17 and 18) and 5% DMSO] containing 5 μl of 50 folds-diluted cDNA solution prepared from each of the expression cell lines and 5 μl (1 pg) of the plasmid for internal control. The PCR was carried out by heating at 94°C for 3 minutes and subsequent 17 cycles of heating at 94°C for 30 seconds, 65°C for 1 minute and 72°C for 2 minutes as one cycle.

Also, PCR was carried out in a series of reaction in which 10 pg, 5 pg, 1 pg, 500 fg or 100 fg of the β-actin standard plasmid was added instead of the each expression cell line-derived cDNA, and used in the preparation of a calibration curve for the β-actin transcription level. In this case, 1 μg/ml baker's yeast t-RNA (manufactured by SIGMA) was used for diluting the standard plasmid.

By carrying out PCR using the primer set described in Table 3, a DNA fragment having a size shown in the target column of Table 3 can be amplified from each gene transcription product and each standard, and a DNA fragment having a size shown in the competitor column of Table 3 can be amplified from each internal control.

Each (7 μl) of the solutions after PCR was subjected to a 1.75% agarose gel electrophoresis, and then the gel was stained by soaking it for 30 minutes in 1 × concentration SYBR Green I Nucleic Acid Gel Stain (manufactured by Molecular Probes). By calculating luminescence intensity of each amplified DNA fragment using a fluoro-imager (Fluor-Imager SI; manufactured by Molecular Dynamics), the amount of the amplified DNA fragment was measured.

The amount of an amplified product formed by PCR using the standard plasmid as the template was measured by the method, and a calibration curve was prepared by plotting the measured values against the amounts of the standard plasmid. Using the calibration curve, the amount of cDNA of a gene of interest in each cell line was calculated from the

amount of an amplified product when each expression cell line-derived cDNA was used as the template, and the amount was defined as the mRNA transcription level in each cell line.

Fig. 28 shows the FUT8 transcription levels as relative values to the amount of β-actin transcription product. Three cell lines mfFUT8-1, mfFUT8-2 and mfFUT8-4 and the pAGE249-introduced cell line were cell lines having a relatively small FUT8 transcription level, which was equivalent to 0.3 to 10% of a β-actin transcription level. On the other hand, other three cell lines mfFUT8-3, mfFUT8-6 and mfFUT8-7 were cell lines having a relatively large FUT8 transcription level, which was equivalent to 20 to 40% of a β-actin transcription level.

(4) Purification of antibody produced by α-1,6-fucosyltransferase (FUT8) gene over-expressing cell line

Each of the six FUT8 gene over-expressing cell lines and one negative control cell line obtained in the item (2) was suspended in Hybridoma-SFM medium (manufactured by Life Technologies) supplemented with 200 nmol/l MTX, 0.5 mg/ml Hygromycin B (manufactured by Wako Pure Chemical Industries) and 0.2% BSA (manufactured by Life Technologies) to give a density of $2 \times 10^5$ cells/ml, and then 100 ml in total of the suspension was inoculated into three T225 flasks for suspension cell culture use (manufactured by IWAKI). After culturing them at 37°C for 7 to 9 days in a 5% $CO_2$ incubator, the number of intact cells was counted to confirm that their viability was almost the same (each 30% or less), and then each cell suspension was recovered. Each of the cell suspensions was centrifuged at 3,000 rpm at 4°C for 10 minutes, and the recovered supernatant was centrifuged at 10,000 rpm at 4°C for 1 hour and then filtered using PES Filter Unit (manufactured by NALGENE) having a pore diameter of 0.22 μm with 150 ml capacity.

Prosep-A HighCapacity (manufactured by bioPROCESSING) was packed in a 0.8 cm diameter column to a thickness of 2 cm and washed with 10 ml of 0.1 mol/l citrate buffer (pH 3.0) and 10 ml of 1 mol/l glycine/NaOH-0.1 mol/l NaCl buffer (pH 8.6) in that order to effect equilibration the carrier. Next, 100 ml of each of the culture supernatant was passed through the column and washed with 50 ml of 1 mol/l glycine/NaOH-0.15 mol/l NaCl buffer (pH 8.6). After washing them, the antibody absorbed to Prosep-A was eluted using 2.5 ml of a 0.1 mol/l citrate buffer (pH 3.0), the eluate was fractionated at 500 μl and each fraction was neutralized by mixing with 100 μl of 2 mol/l Tris-HCl (pH 8.5). Two fractions containing the antibody at a high concentration (1.2 ml in total) were selected by the BCA method [Anal. Biochem., 150, 76 (1985)], combined and then dialyzed against 10 mol/l citrate buffer (pH 6.0) at 4°C for a whole day and night. After the dialysis, the antibody solution was recovered and subjected to sterile filtration using a 0.22 μm pore size Millex GV (manufactured by MILLIPORE).

*(5) In vitro* cytotoxic activity (ADCC activity) of antibody produced by mouse α-1,6-fucosyltransferase (FUT8) gene over-expressing cell line

In order to evaluate *in vitro* cytotoxic activity of the anti-GD3 antibodies purified in the item (4), ADCC activity was measured using a GD3-positive cell, human melanoma cultured cell line G-361 (RCB0991, Cell Bank at The Institute of Physical and Chemical Research).

The G-361 cells subcultured in RPMI1640 medium (manufactured by Life Technologies) containing 10% fetal bovine serum (manufactured by Life Technologies) (hereinafter referred to as "RPMI1640-FBS(10)") were suspended in 500 μl of RPMI1640-FBS(10) at a density of $1 \times 10^6$ cells, and 3.7 MBq of $Na_2{}^{51}CrO_4$ was added thereto, followed by culturing at 37°C for 30 minutes for labeling the cells with a radioisotope. After centrifugation at 1,200 rpm for 5 minutes, the supernatant was discarded and the target cells were suspended in 5 ml of RPMI1640-FBS(10). The washing step was repeated three times and then the cell suspension was incubated for 30 minutes on ice for spontaneous dissociation of the radioactive substance. The washing step was again repeated twice and then the cells were suspended in 5 ml of RPMI1640-FBS(10) to thereby prepare $2 \times 10^5$ cells/ml of a target cell suspension.

On the other hand, 30 ml of peripheral blood was collected from a healthy person and gently mixed with 0.5 ml of heparin sodium (manufactured by Shimizu Pharmaceutical) and then mixed with 30 ml of physiological saline (manufactured by Otsuka Pharmaceutical). After the mixing, 10 ml of the mixture was gently overlaid on 4 ml of Lymphoprep (manufactured by NYCOMED PHARMA AS) and centrifuged at room temperature at 2,000 rpm for 30 minutes. The separated mononuclear cell fractions were collected from the centrifugation tubes, combined and then suspended in 30 ml of RPMI1640-FBS(10). After centrifugation at room temperature at 1,200 rpm for 5 minutes, the supernatant was discarded and the cells were suspended in 20 ml of RPMI1640-FBS(10). The washing step was repeated twice and then $2 \times 10^6$ cells/ml of an effector cell suspension was prepared using RPMI1640-FBS(10).

The target cell suspension was dispensed at 50 μl ($1 \times 10^4$ cells/well) into each well of a 96 well U-bottom plate (manufactured by Falcon). Subsequently, the effector cell suspension was dispensed at 100 μl ($2 \times 10^5$ cells/well) into each well to thereby adjust the ratio of the effector cells to the target cells to 20 : 1. Next, using a 10 M citrate buffer (pH 6.0), a series of dilution solution of 0.01 μg/ml, 0.1 μg/ml, 1 μg/ml and 10 μg/ml of each anti-GD3 antibody obtained in the item (4) was prepared, and the diluted solutions were dispensed at 50 μl into the wells to give final concentrations of 0.0025 μg/ml, 0.025 μg/ml, 0.25 μg/ml and 2.5 μg/ml, respectively. After carrying out the reaction at 37°C for 4 hours

in 5% $CO_2$, the plate was centrifuged at 1,200 rpm for 5 minutes. Into a 12 mm diameter RIA tube (manufactured by IWAKI), 50 μl of the supernatant in each well was transferred and, and the amount of the dissociated $^{51}Cr$ was measured using MINAX-γ auto-gamma counter 5550 (manufactured by PACKARD).

Also, the amount of the spontaneously dissociated $^{51}Cr$ was calculated by carrying out the same reaction in a reaction mixture in which 150 μl of RPMI1640-FBS(10) was added instead of the effector cell suspension and antibody solution. The amount of the total dissociated $^{51}Cr$ was calculated by carrying out the same reaction in a reaction mixture in which 100 μl of 1 N hydrochloric acid and 50 μl of RPMI1640-FBS(10) were added instead of the effector cell suspension and antibody solution. Using these values, the ADCC activity was calculated based on the formula (II) described in the item 2(3) of Example 2.

Fig. 29 shows ADCC activity of each of the anti-GD3 antibodies for G-361 cell. Three cell lines mfFUT8-1, mfFUT8-2 and mfFUT8-4 having a low FUT8 expression level as shown in Fig. 28 showed potent ADCC activity equivalent to that of the negative control pAGE249-introduced cell line. On the other hand, other three cell lines mfFUT8-3, mfFUT8-6 and mfFUT8-7 having a high FUT8 expression level as shown in Fig. 28 showed low ADCC activity equivalent to that of the anti-GD3 antibody produced from CHO cell. Based on these results, it was shown that the ADCC activity of produced antibodies can be controlled by regulating the expression level of FUT8 in host cells.

(6) Sugar chain analysis of antibody produced by mouse α-1,6-fucosyltransferase (FUT8) gene over-expressing cell line

Sugar chains of the anti-GD3 antibodies purified in the item (4) were analyzed. The sugar chains binding to the antibodies produced by mfFUT8-6 and pAGE249-introduced cell lines were cleaved from proteins by subjecting the antibodies to hydrazinolysis [Method of Enzymology, 83, 263 (1982)]. After removing hydrazine by evaporation under a reduced pressure, *N*-acetylation was carried out by adding an aqueous ammonium acetate solution and acetic anhydride. After freeze-drying, fluorescence labeling by 2-aminopyridine [J. Biochem., 95, 197 (1984)] was carrying out. A fluorescence-labeled sugar chain group (PA-treated sugar chain group) was separated from excess reagents using Superdex Peptide HR 10/30 column (manufactured by Pharmacia). The sugar chain fractions were dried using a centrifugation concentrator and used as a purified PA-treated sugar chain group. Next, the purified PA-treated sugar chain group was subjected to reverse phase HPLC analysis using a CLC-ODS column (manufactured by Shimadzu) (Fig. 30). When calculated from the peak area, the content of α-1,6-fucose-free sugar chains in mfFUT8-6 was 10%, and the content of α-1,6-fucose-bound sugar chains was 90%. The content of α-1,6-fucose-free sugar chains in pAGE249 was 20%, and the content of α-1,6-fucose-bound sugar chains was 80%. Based on these results, it was found that the content of α-1,6-fucose-bound sugar chains of a produced antibody is increased by over-expressing the FUT8 gene.

Fig. 30 shows elution patterns obtained by carrying out reverse phase HPLC analysis of each of PA-treated sugar chains prepared from antibodies produced by mfFUT8-6 and pAGE249-introduced cell lines. Figs. 30A and 30B show elution patterns of mfFUT8-6 and pAGE249, respectively. The relative fluorescence intensity and the elution time are plotted as the ordinate and the abscissa, respectively. Using a sodium phosphate buffer (pH 3.8) as buffer A and a sodium phosphate buffer (pH 3.8) + 0.5% 1-butanol as buffer B, the analysis was carried out by the following gradient.

| Time (minute) | 0 | 80 | 90 | 90.1 | 120 |
|---|---|---|---|---|---|
| Buffer B (%) | 0 | 60 | 60 | 0 | 0 |

Peaks (i) to (ix) shown in Fig. 30 and Fig. 31 show the following structures.

(i)

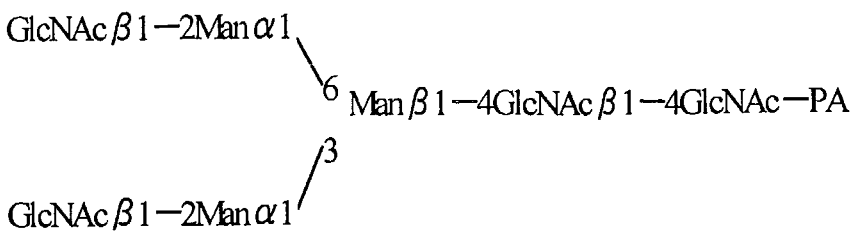

(ii)

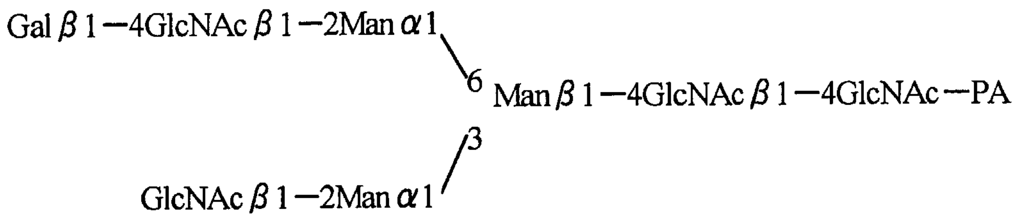

(iii)

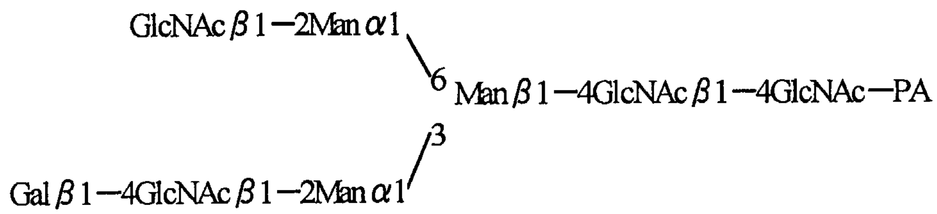

(iv)

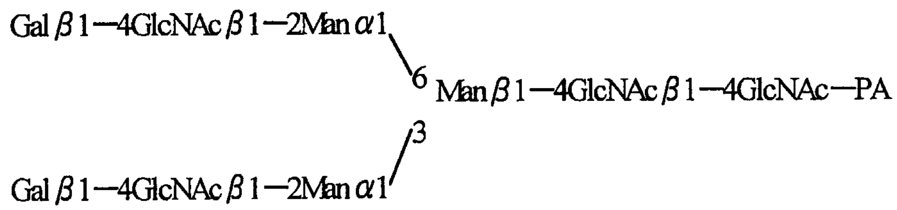

(v)

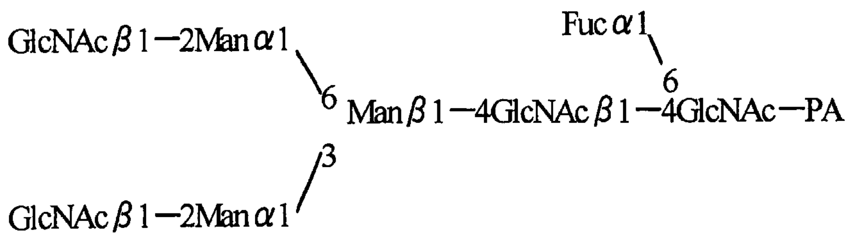

(vi)

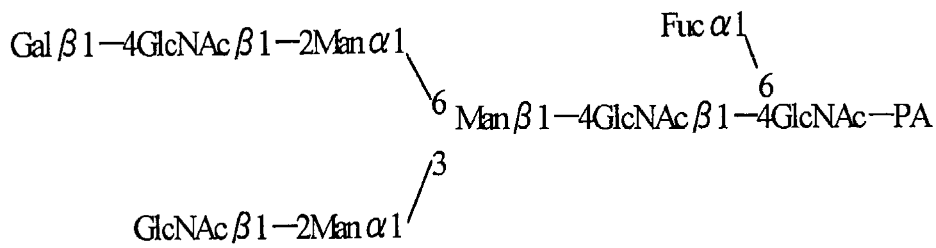

(vii)

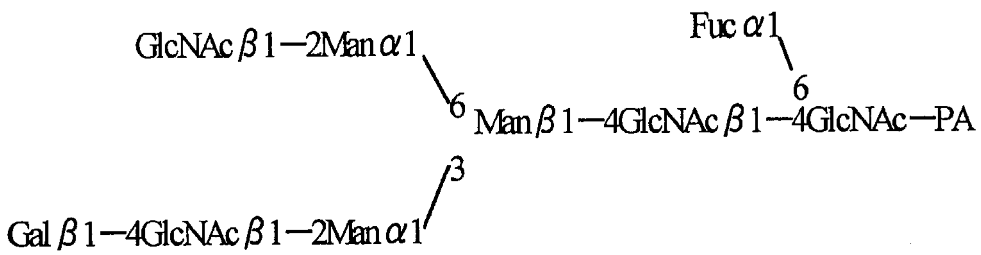

(viii)

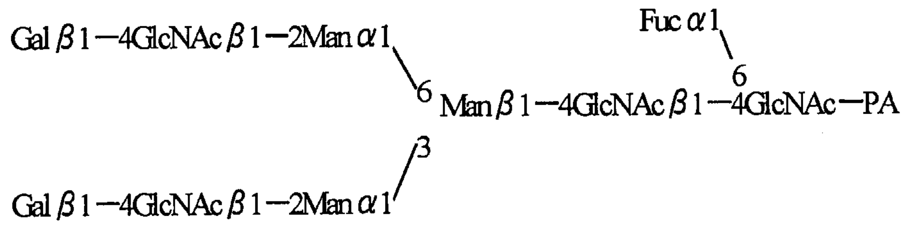

(ix)

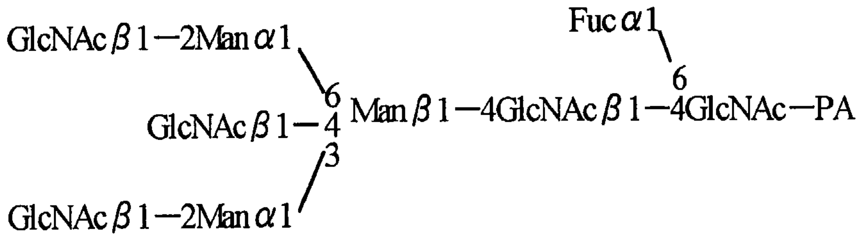

GlcNAc, Gal, Man, Fuc and PA indicate *N*-acetylglucosamine, galactose, mannose, fucose and a pyridylamino group, respectively. In Figs. 30 and 31, the ratio of the α-1,6-fucose-free sugar chain group was calculated from the area occupied by the peaks (i) to (iv) among (i) to (ix), and the ratio of the α-1,6-fucose-bound sugar chain group from the area occupied by the peaks (v) to (ix) among (i) to (ix).

Example 12

Preparation of CHO cell α-1,6-fucosyltransferase (FUT8) gene:

(1) Preparation of CHO cell α-1,6-fucosyltransferase (FUT8) cDNA sequence

From a single-stranded cDNA prepared from CHO/DG44 cells on the 2nd day of culturing in Example 9(1), Chinese hamster FUT8 cDNA was obtained by the following procedure (Fig. 32).

First, a forward primer specific for a 5'-terminal non-translation region (shown in SEQ ID NO:21) and a reverse primer specific for a 3'-terminal non-translation region (shown in SEQ ID NO:22) were designed from a mouse FUT8 cDNA sequence (GenBank, AB025198).

Next, 25 μl of a reaction solution [ExTaq buffer (manufactured by Takara Shuzo), 0.2 mmol/l dNTPs, 4% DMSO and 0.5 μmol/l specific primers (SEQ ID NOs:21 and 22)] containing 1 μl of the CHO/DG44 cell-derived cDNA was prepared and PCR was carried out using a DNA polymerase ExTaq (manufactured by Takara Shuzo). The PCR was carried out by heating at 94°C for 1 minute, subsequent 30 cycles of heating at 94°C for 30 seconds, 55°C for 30 seconds and 72°C for 2 minutes as one cycle, and final heating at 72°C for 10 minutes.

After the PCR, the reaction solution was subjected to 0.8% agarose gel electrophoresis, and a specific amplified fragment of about 2 Kb was purified. Into a plasmid pCR2.1, 4 μl of the DNA fragment was employed to insert in accordance with the instructions attached to TOPO TA Cloning Kit (manufactured by Invitrogen), and *E. coli* DH5α was transformed with the reaction solution. Plasmid DNAs were isolated in accordance with a known method from cDNA-inserted 8 clones among the obtained kanamycin-resistant colonies.

The nucleotide sequence of each cDNA inserted into the plasmid was determined using DNA Sequencer 377 (manufactured by Parkin Elmer) and BigDye Terminator Cycle Sequencing FS Ready Reaction Kit (manufactured by Parkin Elmer) in accordance with the method of the manufacture's instructions. It was confirmed by the method that all of the inserted cDNAs encode a sequence containing the full ORF of CHO cell FUT8. Among these, a plasmid DNA containing absolutely no reading error of bases by the PCR in the sequences was selected. Herein, the plasmid is referred to as CHfFUT8-pCR2.1. The determined nucleotide sequence and the amino acid sequence of the cDNA of CHO FUT8 are shown in SEQ ID NOs:1 and 23, respectively.

(2) Preparation of CHO cell α-1,6-fucosyltransferase (FUT8) genomic sequence

Using the ORF full length cDNA fragment of CHO cell FUT8 obtained in the item (1) as a probe, a CHO cell FUT8 genomic clone was obtained in accordance with a known genome screening method described, e.g., in Molecular Cloning, Second Edition, Current Protocols in Molecular Biology, A Laboratory Manual, Second Edition (1989). Next, after digesting the obtained genomic clone using various restriction enzymes, the Southern hybridization was carried

out using an *Afa*I-*Sau*3AI fragment (about 280 bp) containing initiation codon of the CHO cell FUT8 cDNA as a probe, and then a *Xba*I-*Xba*I fragment (about 2.5 Kb) and a *Sac*I-*Sac*I fragment (about 6.5 Kb) were selected from restriction enzyme fragments showing positive reaction, inserted into pBluescript II KS(+) (manufactured by Stratagene), respectively.

The nucleotide sequence of each of the obtained genomic fragments was determined using DNA Sequencer 377 (manufactured by Parkin Elmer) and BigDye Terminator Cycle Sequencing FS Ready Reaction Kit (manufactured by Parkin Elmer) in accordance with the method of the manufacture's instructions. Thereby, it was confirmed that the *Xba*I-*Xba*I fragment encodes a sequence of an upstream intron of about 2.5 Kb containing exon 2 of the CHO cell FUT8, and the *Sac*I-*Sac*I fragment encodes a sequence of a downstream intron of about 6.5 Kb containing exon 2 of the CHO cell FUT8. Herein, the plasmid containing *Xba*I-*Xba*I fragment is referred to as pFUT8fgE2-2, and the plasmid containing *Sac*I-*Sac*I fragment is referred to as pFUT8fgE2-4. The determined nucleotide sequence (about 9.0 Kb) of the genome region containing exon 2 of the CHO cell FUT8 is shown in SEQ ID NO:3.

Example 13

Preparation of CHO cell in which α-1,6-fucose transferase gene is disrupted and production of antibody using the cell:

A CHO cell from which the genome region comprising the CHO cell α-1,6-fucosyltransferase (FUT8) gene exon 2 was deleted was prepared and the ADCC activity of an antibody produced by the cell was evaluated.

1. Construction of Chinese hamster α-1,6-fucosyltransferase (FUT8) gene exon 2 targeting vector plasmid pKOFUT8Puro

(1) Construction of plasmid ploxPPuro

A plasmid ploxPPuro was constructed by the following procedure (Fig. 33).

In 35 μl of NEBuffer 4 (manufactured by New England Biolabs), 1.0 μg of a plasmid pKOSelectPuro (manufactured by Lexicon) was dissolved, and 20 units of a restriction enzyme *Asc*I (manufactured by New England Biolabs) were added thereto, followed by digestion reaction at 37°C for 2 hours. After the digestion reaction, the solution was subjected to 0.8% (w/v) agarose gel electrophoresis to purify a DNA fragment of about 1.5 Kb containing a puromycin resistance gene expression unit.

On the other hand, 1.0 μg of a plasmid ploxP described in Japanese Published Examined Patent Application No. 314512/99 was dissolved in 35 μl of NEBuffer 4 (manufactured by New England Biolabs), and 20 units of a restriction enzyme *Asc*I (manufactured by New England Biolabs) were added thereto, followed by digestion reaction at 37°C for 2 hours. After the digestion reaction, the solution was subjected to 0.8% (w/v) agarose gel electrophoresis to purify a DNA fragment of about 2.0 Kb.

The obtained *Asc*I-*Asc*I fragment (4.5 μl, about 1.5 Kb) derived from the plasmid pKOSelectPuro, 0.5 μl of the *Asc*I-*Asc*I fragment (about 2.0 Kb) derived from the plasmid ploxP and 5.0 μl of Ligation High (manufactured by Toyobo) were mixed, followed by ligation reaction at 16°C for 30 minutes. *E. coli* DH5α was transformed using the reaction solution, and a plasmid DNA was isolated in accordance with a known method from the obtained ampicillin-resistant clones. Herein, the plasmid is referred to as ploxPPuro.

(2) Construction of plasmid pKOFUT8gE2-1

A plasmid pKOFUT8gE2-1 was constructed by the following procedure (Fig. 34), using the plasmid pFUT8fgE2-2 obtained in Example 12(2) having a genome region comprising exon 2 of Chinese hamster FUT8.

In 35 μl of NEBuffer 1 (manufactured by New England Biolabs) containing 100 μg/ml of BSA (manufactured by New England Biolabs), 2.0 μg of the plasmid pFUT8fgE2-2 was dissolved, and 20 units of a restriction enzyme *Sac*I (manufactured by New England Biolabs) were added thereto, followed by digestion reaction at 37°C for 2 hours. A DNA fragment was recovered from the reaction solution by ethanol precipitation and dissolved in 35 μl of NEBuffer 2 (manufactured by New England Biolabs) containing 100 μg/ml of BSA (manufactured by New England Biolabs), and 20 units of a restriction enzyme *EcoRV* (manufactured by New England Biolabs) were added thereto, followed by digestion reaction at 37°C for 2 hours. After the digestion reaction, the solution was subjected to 0.8% (w/v) agarose gel electrophoresis to purify a DNA fragment of about 1.5 Kb.

Separately, 1.0 μg of a plasmid LITMUS28 (manufactured by New England Biolabs) was dissolved in 35 μl of NEBuffer 1 (manufactured by New England Biolabs) containing 100 μg/ml of BSA (manufactured by New England Biolabs), and 20 units of a restriction enzyme *Sac*I (manufactured by New England Biolabs) were added thereto, followed by digestion reaction at 37°C for 2 hours. A DNA fragment was recovered from the reaction solution by ethanol precipitation and dissolved in 35 μl of NEBuffer 2 (manufactured by New England Biolabs) containing 100 μg/ml of BSA (manufactured

by New England Biolabs), and 20 units of a restriction enzyme *Eco*RV (manufactured by New England Biolabs) were added thereto, followed by digestion reaction at 37°C for 2 hours. After the digestion reaction, the solution was subjected to 0.8% (w/v) agarose gel electrophoresis to purify a DNA fragment of about 2.8 Kb.

The obtained *Eco*RV-*Sac*I fragment (4.5 μl, about 1.5 Kb) derived from the plasmid pFUT8fgE2-2, 0.5 μl of the *Eco*RV-*Sac*I fragment (about 2.8 Kb) derived from the plasmid LITMUS28 and 5.0 μl of Ligation High (manufactured by Toyobo) were mixed, followed by ligation reaction at 16°C for 30 minutes. *E. coli* DH5α was transformed using the reaction solution, and a plasmid DNA was isolated in accordance with a known method from the obtained ampicillin-resistant clones. Herein, the plasmid is referred to as pKOFUT8gE2-1.

(3) Construction of plasmid pKOFUT8gE2-2

A plasmid pKOFUT8gE2-2 was constructed by the following procedure (Fig. 35), using the plasmid pKOFUT8gE2-1 obtained in the item (2).

In 30 μl of NEBuffer 2 (manufactured by New England Biolabs) containing 100 μg/ml of BSA (manufactured by New England Biolabs), 2.0 μg of the plasmid pKOFUT8gE2-1 was dissolved, and 20 units of a restriction enzyme *Eco*RV (manufactured by New England Biolabs) were added thereto, followed by digestion reaction at 37°C for 2 hours. A DNA fragment was recovered from the reaction solution by ethanol precipitation and dissolved in 30 μl of NEBuffer 1 (manufactured by New England Biolabs) containing 100 μg/ml of BSA (manufactured by New England Biolabs), and 20 units of a restriction enzyme *Kpn*I (manufactured by New England Biolabs) were added thereto, followed by digestion reaction at 37°C for 2 hours. After the digestion reaction, the solution was subjected to 0.8% (w/v) agarose gel electrophoresis to purify a DNA fragment of about 1.5 Kb.

Separately, 1.0 μg of the plasmid ploxPPuro was dissolved in 30 μl of NEBuffer 4 (manufactured by New England Biolabs), and 20 units of a restriction enzyme *Hpa*I (manufactured by New England Biolabs) were added thereto, followed by digestion reaction at 37°C for 2 hours. A DNA fragment was recovered from the reaction solution by ethanol precipitation and dissolved in 30 μl of NEBuffer 1 (manufactured by New England Biolabs) containing 100 μg/ml of BSA (manufactured by New England Biolabs), and 20 units of a restriction enzyme *Kpn*I (manufactured by New England Biolabs) were added thereto, followed by digestion reaction at 37°C for 2 hours. After the digestion reaction, the solution was subjected to 0.8% (w/v) agarose gel electrophoresis to purify a DNA fragment of about 3.5 Kb.

A 4.0 μl portion of the obtained *Eco*RV-*Kpn*I fragment (about 1.5 Kb) derived from the plasmid pKOFUT8gE2-1, 1.0 μl of the *Hpa*I-*Kpn*I fragment (about 3.5 Kb) derived from the plasmid ploxPPuro and 5.0 μl of Ligation High (manufactured by Toyobo) were mixed and allowed to undergo the ligation reaction at 16°C for 30 minutes. *E. coli* DH5α was transformed using the reaction solution, and a plasmid DNA was isolated in accordance with a known method from the obtained ampicillin-resistant clones. Herein, the plasmid is referred to pKOFUT8gE2-2.

(4) Construction of plasmid pscFUT8gE2-3

A plasmid pscFUT8gE2-3 was constructed by the following procedure (Fig. 36), using the plasmid pFUT8fgE2-4 obtained in Example 12(2) having a genome region comprising exon 2 of Chinese hamster FUT8.

In 35 μl of NEBuffer 1 (manufactured by New England Biolabs), 2.0 μg of the plasmid pFUT8fgE2-4 was dissolved, and 20 units of a restriction enzyme *Hpa*II (manufactured by New England Biolabs) were added thereto, followed by digestion reaction at 37°C for 2 hours. A DNA fragment was recovered from the reaction solution by ethanol precipitation, and then the DNA termini were changed to blunt ends using Blunting High (manufactured by Toyobo) in accordance with the manufacture's instructions. The DNA fragment was recovered by carrying out phenol/chloroform extraction and ethanol precipitation and dissolved in 35 μl of NEBuffer 2 (manufactured by New England Biolabs), and 20 units of a restriction enzyme *Hind*III (manufactured by New England Biolabs) were added thereto, followed by digestion reaction at 37°C for 2 hours. After the digestion reaction, the solution was subjected to 0.8% (w/v) agarose gel electrophoresis to purify a DNA fragment of about 3.5 Kb.

On the other hand, 1.0 μg of a plasmid LITMUS39 (manufactured by New England Biolabs) was dissolved in 35 μl of NEBuffer 2 (manufactured by New England Biolabs), and the solution was mixed with 20 units of a restriction enzyme *Eco*RV (manufactured by New England Biolabs) and 20 units of a restriction enzyme *Hind*III (manufactured by New England Biolabs) and subjected to the digestion reaction at 37°C for 2 hours. After the digestion reaction, the solution was subjected to 0.8% (w/v) agarose gel electrophoresis to purify a DNA fragment of about 2.8 Kb.

The obtained *Hpa*II-*Hind*III fragment (4.0 μl, about 3.5 Kb) derived from the plasmid pFUT8fgE2-4, 1.0 μl of the *Eco*RV-*Hind*III fragment (about 2.8 Kb) derived from the plasmid LITMUS39 and 5.0 μl of Ligation High (manufactured by Toyobo) were mixed, followed by ligation reaction at 16°C for 30 minutes. *E. coli* DH5α was transformed using the reaction solution, and a plasmid DNA was isolated in accordance with a known method from the obtained ampicillin-resistant clones. Herein, the plasmid is referred to pscFUT8gE2-3.

(5) Construction of plasmid pKOFUT8gE2-3

A plasmid pKOFUT8gE2-3 was constructed by the following procedure (Fig. 37), using the plasmid pFUT8fgE2-4 obtained in Example 12(2) having a genome region comprising exon 2 of Chinese hamster FUT8.

In 35 μl of NEBuffer for *Eco*RI (manufactured by New England Biolabs), 2.0 μg of the plasmid pFUT8fgE2-4 was dissolved, and 20 units of a restriction enzyme *Eco*RI (manufactured by New England Biolabs) and 20 units of a restriction enzyme *Hind*III (manufactured by New England Biolabs) were added thereto, followed by digestion reaction at 37°C for 2 hours. After the digestion reaction, the solution was subjected to 0.8% (w/v) agarose gel electrophoresis to purify a DNA fragment of about 1.8 Kb.

Separately, 1.0 μg of a plasmid pBluescript II KS(+) (manufactured by Stratagene) was dissolved in 35 μl of NEBuffer for *EcoRI* (manufactured by New England Biolabs), and 20 units of a restriction enzyme *Eco*RI (manufactured by New England Biolabs) and 20 units of a restriction enzyme *Hind*III (manufactured by New England Biolabs) were added thereto, followed by digestion reaction at 37°C for 2 hours. After the digestion reaction, the solution was subjected to 0.8% (w/v) agarose gel electrophoresis to purify a DNA fragment of about 3.0 Kb.

The obtained *Hind*III-*Eco*RI fragment (4.0 μl about 1.8 Kb) derived from the plasmid pFUT8fgE2-4, 1.0 μl of the *Hind*III-*Eco*RI fragment (about 3.0 Kb) derived from the plasmid pBluescript II KS(+) and 5.0 μl of Ligation High (manufactured by Toyobo) were mixed, followed by ligation reaction at 16°C for 30 minutes. *E. coli* DH5α was transformed using the reaction solution, and a plasmid DNA was isolated in accordance with a known method from the obtained ampicillin-resistant clones. Herein, the plasmid is referred to pKOFUT8gE2-3.

(6) Construction of plasmid pKOFUT8gE2-4

A plasmid pKOFUT8gE2-4 was constructed by the following procedure (Fig. 38), using the plasmids pscFUT8fgE2-3 and pKOFUT8gE2-3 obtained in the items (4) and (5).

In 35 μl of NEBuffer for *Sal*I (manufactured by New England Biolabs) containing 100 μg/ml of BSA (manufactured by New England Biolabs), 1.0 μg of the plasmid pscFUT8gE2-3 was dissolved, and 20 units of a restriction enzyme *Sal*I (manufactured by New England Biolabs) were added thereto, followed by digestion reaction at 37°C for 2 hours. A DNA fragment was recovered from the reaction solution by ethanol precipitation and dissolved in 30 μl of NEBuffer 2 (manufactured by New England Biolabs) containing 100 μg/ml of BSA (manufactured by New England Biolabs), and 20 units of a restriction enzyme *Hind*III (manufactured by New England Biolabs) were added thereto, followed by digestion reaction at 37°C for 2 hours. After the digestion reaction, the solution was subjected to 0.8% (w/v) agarose gel electrophoresis to purify a DNA fragment of about 3.6 Kb.

Separately, 1.0 μg of the plasmid pKOFUT8gE2-3 was dissolved in 35 μl of NEBuffer for *Sal*I (manufactured by New England Biolabs), and 20 units of a restriction enzyme *Sal*I (manufactured by New England Biolabs) were added thereto, followed by digestion reaction at 37°C for 2 hours. A DNA fragment was recovered from the reaction solution by ethanol precipitation and dissolved in 35 μl of NEBuffer 2 (manufactured by New England Biolabs), and 20 units of a restriction enzyme *Hind*III (manufactured by New England Biolabs) were added thereto, followed by digestion reaction at 37°C for 2 hours. After the digestion reaction, 35 μl of 1 mol/l Tris-HCl buffer (pH 8.0) and 3.5 μl of *E. coli* C15-derived alkaline phosphatase (manufactured by Takara Shuzo) were added thereto, followed by reaction at 65°C for 30 minutes to dephosphorylate the DNA termini. After the dephosphorylation treatment, a DNA fragment was recovered by carrying out phenol/chloroform extraction and ethanol precipitation and dissolved in 10 μl of sterile water.

The obtained *Sal*I-*Hind*III fragment (4.0 μl, about 3.1 Kb) derived from the plasmid pscFUT8gE2-3, 1.0 μl of the *Sal*I-*Hind*III fragment (about 4.8 Kb) derived from the plasmid pKOFUT8gE2-3 and 5.0 μl of Ligation High (manufactured by Toyobo) were mixed, followed by ligation reaction at 16°C for 30 minutes. *E. coli* DH5α was transformed using the reaction solution, and a plasmid DNA was isolated in accordance with a known method from the obtained ampicillin-resistant clones. Herein, the plasmid is referred to pKOFUT8gE2-4.

(7) Construction of plasmid pKOFUT8gE2-5

A plasmid pKOFUT8gE2-5 was constructed by the following procedure (Fig. 39), using the plasmids pKOFUT8gE2-2 and pKOFUT8gE2-4 obtained in the items (3) and (6).

In 30 μl of NEBuffer 4 (manufactured by New England Biolabs), 1.0 μg of the plasmid pKOFUT8gE2-2 was dissolved, and 20 units of a restriction enzyme *Sma*I (manufactured by New England Biolabs) were added thereto, followed by digestion reaction at 25°C for 2 hours. A DNA fragment was recovered from the reaction solution by ethanol precipitation and dissolved in 30 μl of NEBuffer 2 (manufactured by New England Biolabs), and 20 units of a restriction enzyme *Bam*HI (manufactured by New England Biolabs) were added thereto, followed by digestion reaction at 37°C for 2 hours. After the digestion reaction, 30 μl of 1 mol/l Tris-HCl buffer (pH 8.0) and 3.0 μl of *E. coli* C15-derived alkaline phosphatase (manufactured by Takara Shuzo) were added thereto, followed by reaction at 65°C for 1 hour to dephosphorylate the DNA termini. After the dephosphorylation treatment, the DNA fragment was recovered by carrying out phenol/chloroform extraction and ethanol precipitation and dissolved in 10 μl of sterile water.

Separately, 1.0 μg of the plasmid pKOFUT8gE2-4 was dissolved in 30 μl of NEBuffer 4 (manufactured by New England Biolabs), and 20 units of a restriction enzyme *Sma*I (manufactured by New England Biolabs) were added thereto, followed by digestion reaction at 25°C for 2 hours. A DNA fragment was recovered from the reaction solution by ethanol precipitation and dissolved in 30 μl of NEBuffer 2 (manufactured by New England Biolabs), and 20 units of a restriction enzyme *BamH*I (manufactured by New England Biolabs) were added thereto, followed by digestion reaction at 37°C for 2 hours. After the digestion reaction, the solution was subjected to 0.8% (w/v) agarose gel electrophoresis to purify a DNA fragment of about 5.2 Kb.

The obtained *Sma*I-*Bam*HI fragment (0.5 μl, about 5.0 Kb) derived from the plasmid pKOFUT8gE2-2, 4.5 μl of the *Sma*I-*Bam*HI fragment (about 5.4 Kb) derived from the plasmid pKOFUT8gE2-4 and 5.0 μl of Ligation High (manufactured by Toyobo) were mixed, followed by ligation reaction at 16°C for 15 hours. *E. coli* DH5α was transformed using the reaction solution, and a plasmid DNA was isolated in accordance with a known method from the obtained ampicillin-resistant clones. Herein, the plasmid is referred to pKOFUT8gE2-5.

(8) Construction of plasmid pKOFUT8Puro

A plasmid pKOFUT8Puro was constructed by the following procedure (Fig. 40), using the plasmid pKOFUT8gE2-5 obtained in the item (7).

In 50 μl of NEBuffer 4 (manufactured by New England Biolabs), 1.0 μg of a plasmid pKOSelectDT (manufactured by Lexicon) was dissolved, and 16 units of a restriction enzyme *Rsr*II (manufactured by New England Biolabs) were added thereto, followed by digestion reaction at 37°C for 2 hours. After the digestion reaction, the solution was subjected to 0.8% (w/v) agarose gel electrophoresis to purify a DNA fragment of about 1.2 Kb comprising a diphtheria toxin expression unit.

Separately, 1.0 μg of the plasmid pKOFUT8gE2-5 was dissolved in 50 μl of NEBuffer 4 (manufactured by New England Biolabs), and 16 units of a restriction enzyme *Rsr*II (manufactured by New England Biolabs) were added thereto, followed by digestion reaction at 37°C for 2 hours. After the digestion reaction, 30 μl of 1 mol/l Tris-HCl buffer (pH 8.0) and 3.0 μl of *E. coli* C15-derived alkaline phosphatase (manufactured by Takara Shuzo) were added thereto, followed by reaction at 65°C for 1 hour to dephosphorylate the DNA termini. After the dephosphorylation treatment, the DNA fragment was recovered by carrying out phenol/chloroform extraction and ethanol precipitation and dissolved in 10 μl of sterile water.

The obtained *Rsr*II-*Rsr*II fragment (1.0 μl, about 1.2 Kb) derived from the plasmid pKOSelectDT, 1.0 μl of the *Rsr*II-*Rsr*II fragment (about 10.4 Kb) derived from the plasmid pKOFUT8gE2-5, 3.0 μl of sterile water and 5.0 μl of Ligation High (manufactured by Toyobo) were mixed, followed by ligation reaction at 16°C for 30 minutes. *E. coli* DH5α was transformed using the reaction solution, and a plasmid DNA was isolated in accordance with a known method from the obtained ampicillin-resistant clones. Herein, the plasmid is referred to pKOFUT8Puro.

2. Preparation of CHO cell in which one copy of the genome region containing α-1,6-fucosyltransferase (FUT8) gene exon 2 was disrupted

(1) Introduction of targeting vector

A Chinese hamster FUT8 genome region targeting vector pKOFUT8Puro constructed in the item 1 of this Example was introduced into the strain 5-03 prepared in the item 1(2) of Example 8.

A gene of the plasmid pKOFUT8Puro was introduced into the strain 5-03 as described below in accordance with the electroporation method [Cytotechnology, 3, 133 (1990)]. First, 150 μg of the plasmid pKOFUT8Puro was dissolved in 1.8 ml of NEBuffer for *Sal*I (manufactured by New England Biolabs), and 600 units of a restriction enzyme *Sal*I (manufactured by New England Biolabs) were added thereto, followed by digestion reaction at 37°C for 5 hours to obtain a linear fragment. The reaction solution was extracted with phenol/chloroform extraction, followed by ethanol precipitation, and the recovered linear plasmid was made into a 1 μg/μl aqueous solution. Separately, the strain 5-03 was suspended in a K-PBS buffer (137 mmol/l KCl, 2.7 mmol/l NaCl, 8.1 mmol/l $Na_2HPO_4$, 1.5 mmol/l $KH_2PO_4$, 4.0 mmol/l $MgCl_2$) to give a density of $8 \times 10^7$ cells/ml. After mixing 200 μl of the cell suspension ($1.6 \times 10^6$ cells) with 4 μl (4 μg) of the linear plasmid, an entire volume of the cell-DNA mixture was transferred into Gene Pulser Cuvette (inter-electrode distance, 2 mm) (manufactured by BIO-RAD) and then the electroporation was carried out using a cell fusion apparatus Gene Pulser (manufactured by BIO-RAD) at 350 V pulse voltage and 250 μF electric capacity. After carrying out the electroporation using 30 cuvettes in the same manner, the cell suspension was suspended in IMDM medium (manufactured by Life Technologies) supplemented with 10% fetal bovine serum (manufactured by Life Technologies) and 1 × concentration HT supplement (manufactured by Life Technologies) and inoculated into 30 adhesion cell culture dishes

of 10 cm in diameter (manufactured by Falcon). After culturing them at 37°C for 24 hours in 5% $CO_2$, the culture supernatant was removed, and IMDM medium (manufactured by Life Technologies) supplemented with 15 μg/ml puromycin (manufactured by SIGMA) and 10% fetal bovine dialyzed serum (manufactured by Life Technologies) was dispensed in 10 ml portions. After culturing them for 10 days while repeating the medium exchange at intervals of 3 to 4 days, puromycin-resistant cell lines were obtained.

(2) Preparation of targeting vector-introduced cell lines

Arbitrary 900 colonies were obtained as follows from the puromycin-resistant cell lines obtained in the item (1).
First, culture supernatant was removed from the 10 cm dish in which colony of puromycin-resistant cell lines were formed and 7 ml of a phosphate buffer was added to the dish which was subsequently set under a stereoscopic microscope. Next, each colony was scratched and sucked up using Pipetteman (manufactured by GILSON) and transferred into a 96 well round-bottom plate (manufactured by Falcon). After a trypsin-treatment, each clone was inoculated into a 96 well flat-bottom plate for adhesion cell culture use (manufactured by Iwaki Glass) and cultured for 1 week using IMDM medium (manufactured by Life Technologies) supplemented with 15 μg/ml puromycin (manufactured by SIGMA) and 10% fetal bovine dialyzed serum (manufactured by Life Technologies).
After culturing them, each clone in the plate was subjected to trypsin treatment and then mixed with two volumes of a freeze drying medium (20% DMSO, 40% fetal bovine serum, 40% IMDM). A half of the mixture was inoculated into a 96 well flat-bottom plate for adhesion cell culture (manufactured by Iwaki Glass) as a replica plate, while the remaining half of the mixture was subjected to cryopreservation as master plates. The replica plate was cultured for 1 week using IMDM medium (manufactured by Life Technologies) supplemented with 15 μg/ml of puromycin (manufactured by SIGMA) and 10% fetal bovine dialyzed serum (manufactured by Life Technologies).

(3) Diagnosis of homologous recombination by genomic PCR

Diagnosis of homologous recombination in the 900 clones obtained in the item

(2) was carried out by genomic PCR.

First, genome DNA of each clone was prepared from the replica plate prepared in the item (2) in accordance with a known method *[*Analytical Biochemistry, 201, 331 (1992)] and dissolved overnight in 30 μl of a TE-RNase buffer (pH 8.0) (10 mmol/l Tris-HCl, 1 mmol/l EDTA, 200 μg/ml RNase A). Also, a primer (shown in SEQ ID NO:26) which binds to a sequence outside the targeting vector homologous region among the FUT8 genome region obtained in Example 12 and a primer (shown in SEQ ID NO:27) which binds to the loxP sequence in the vector were designed.
Using a DNA polymerase ExTaq (manufactured by Takara Shuzo), 25 μl of a reaction solution [ExTaq buffer (manufactured by Takara Shuzo), 0.2 mmol/l dNTPs and 0.5 μmol/l gene-specific primers (SEQ ID NO:26 and SEQ ID NO:27)] containing 10 μl of each the above-prepared genome DNA solution were prepared, and polymerase chain reaction (PCR) was carried out. The PCR was carried out by heating at 94°C for 3 minutes and subsequent 38 cycles of heating using the reaction at 94°C for 1 minute, 60°C for 1 minute and 72°C for 2 minutes as one cycle.
After the PCR, the reaction solution was subjected to 0.8% (w/v) agarose gel electrophoresis, and a specifically amplifying fragment of about 1.7 Kb containing a border region between the CHO cell genome region and the targeting vector homologous region was identified as a positive clone. One positive clone was found by the method.

(4) Diagnosis of homologous recombination by genome Southern blotting

Diagnosis of homologous recombination in the 1 clone, whose positive signal was confirmed in the item (3), was carried out by genome Southern blotting.
Among the master plates cryo-preserved in the item (2), a 96 well plate containing the positive clone found in the item (3) was selected and incubated at 37°C for 10 minutes in 5% $CO_2$. After the incubation, cells were collected from a well corresponding to the positive clone and inoculated into a 24 well flat bottom plate for adhesion cell (manufactured by Greiner). After culturing them for 1 week using IMDM medium (manufactured by Life Technologies) supplemented with 15 μg/ml of puromycin (manufactured by SIGMA) and 10% fetal bovine dialyzed serum (manufactured by Life Technologies), the cells were inoculated into a 6 well flat bottom plate for adhesion cell (manufactured by Greiner). Genome DNA was prepared from the clone in the plate in accordance with a known method *[*Nucleic Acids Research, 3, 2303 (1976)] and dissolved overnight in 150 μl of a TE-RNase buffer (pH 8.0) (10 mmol/l Tris-HCl, 1 mmol/l EDTA, 200 μg/ml RNase A).
In 120 μl of NEBuffer 3 (manufactured by New England Biolabs), 12 μg of the obtained genome DNA was dissolved, and 25 unites of a restriction enzyme *Pst*I (manufactured by New England Biolabs) were added thereto,

followed by digestion reaction at 37°C overnight. A DNA fragment was recovered from the reaction solution by ethanol precipitation, dissolved in 20 μl of TE buffer (pH 8.0) (10 mmol/l Tris-HCl, 1 mmol/l EDTA) and then subjected to 0.8% (w/v) agarose gel electrophoresis. After the electrophoresis, the genome DNA was transferred onto a nylon membrane in accordance with a known method [Proc. Natl. Acad. Sci. USA, 76, 3683 (1979)]. After completion of the transfer, the nylon membrane was heated at 80°C for 2 hours.

Separately, a probe used in the Southern blotting was prepared as follows. First, primers (SEQ ID NOs:28 and 29) which bind to a sequence outside the targeting vector homologous region with the FUT8 genome region obtained in Example 12 were designed. Next, using a DNA polymerase ExTaq (manufactured by Takara Shuzo), 20 μl of a reaction solution [ExTaq buffer (manufactured by Takara Shuzo), 0.2 mmol/l dNTPs and 0.5 μmol/l gene-specific primers (SEQ ID NOs:28 and 29)] containing 4.0 ng of the plasmid pFUT8fgE2-2 obtained in Example 12(2) was prepared, and polymerase chain reaction (PCR) was carried out. The PCR was carried out by heating at 94°C for 1 minute and subsequent 25 cycles of heating at 94°C for 30 seconds, 55°C for 30 seconds and 74°C for 1 minute as one cycle. After the PCR, the reaction solution was subjected to 1.75% (w/v) agarose gel electrophoresis to purify a probe DNA fragment of about 230 bp. The obtained probe DNA solution (5 μl) was labeled with a radioisotope using 1.75 MBq of [$\alpha$-$^{32}$P]dCTP and Megaprime DNA Labeling System, dCTP (manufactured by Amersham Pharmacia Biotech).

The hybridization was carried out as follows. First, the nylon membrane was sealed in a roller bottle, and pre-hybridization was carried out at 65°C for 3 hours by adding 15 ml of a hybridization solution [5 × SSPE, 50 × Denhaldt's solution, 0.5% (w/v) SDS, 100 μg/ml salmon sperm DNA]. Next, the $^{32}$P-labeled probe DNA was heat-denatured and put into the bottle. Then, the nylon membrane was heated at 65°C overnight.

After the hybridization, the nylon membrane was soaked in 50 ml of 2 × SSC-0.1% (w/v) SDS and heated at 65°C for 15 minutes. After repeating the washing step twice, the membrane was soaked in 50 ml of 0.2 × SSC-0.1% (w/v) SDS and heated at 65°C for 15 minutes. After the washing, the nylon membrane was exposed to an X-ray film at -80°C for two nights for development.

By the restriction enzyme *Pst*I treatment, a DNA fragment of about 4.4 Kb is formed from a wild type FUT8 allele. On the other hand, a DNA fragment of about 6.0 Kb is formed from an allele in which homologous recombination with a targeting vector was generated.

By the method, such specific fragments of about 4.4 Kb and about 6.0 Kb were found from the positive clone genome DNA in the item (3). Since the quantitative ratio of both fragments was 1 : 1, it was confirmed that the clone is a clone in which 1 copy of the FUT8 allele was disrupted. Hereinafter, the clone is referred to as the strain 1st.ΔFUT8 2-46.

3. Deletion of drug resistance gene from CHO cell in which 1 copy of the $\alpha$-1,6-fucosyltransferase (FUT8) gene was disrupted

(1) Introduction of Cre recombinase expression vector

A Cre recombinase expression vector pBS185 (manufactured by Life Technologies) was introduced into the strain 1st.ΔFUT8 2-46 prepared in the item 2 of this Example.

A gene of the plasmid pBS185 was introduced into the strain 1st.ΔFUT8 2-46 as follows in accordance with the electroporation method [Cytotechnology, 3, 133 (1990)]. First, the strain lst.ΔFUT8 2-46 was suspended in a K-PBS buffer (137 mmol/l KCl, 2.7 mmol/l NaCl, 8.1 mmol/l $Na_2HPO_4$, 1.5 mmol/l $KH_2PO_4$, 4.0 mmol/l $MgCl_2$) to give a density of 8 x $10^7$ cells/ml. After mixing 200 μl of the cell suspension (1.6 ×$10^6$ cells) with 4 μg of the plasmid pBS185, an entire volume of the cell-DNA mixture was transferred into Gene Pulser Cuvette (inter-electrode distance, 2 mm) (manufactured by BIO-RAD) and then the gene transfer was carried out using a cell fusion apparatus Gene Pulser (manufactured by BIO-RAD) at 350 V pulse voltage and 250 μF electric capacity. After the gene transfer, the cell suspension was suspended in 10 ml of IMDM medium (manufactured by Life Technologies) supplemented with 10% fetal bovine serum (manufactured by Life Technologies) and 1 × concentration HT supplement (manufactured by Life Technologies) and further diluted 20,000 folds using the same medium. The cells were inoculated into 7 adhesion cell culture dishes of 10 cm in diameter (manufactured by Falcon) and then cultured at 37°C for 24 hours in 5% $CO_2$. After culturing them, the culture supernatant was removed and IMDM medium (manufactured by Life Technologies) supplemented with 10% fetal bovine dialyzed serum (manufactured by Life Technologies) was dispensed at 10 ml. Culturing was carried out for 10 days while repeating the medium exchange at intervals of 3 to 4 days.

(2) Preparation of Cre recombinase expression vector-introduced cell lines

Arbitrary 400 colonies were obtained as follows from the cell line obtained in the item (1).

First, culture supernatant was removed from the 10 cm dish and 7 ml of a phosphate buffer was added to the dish which was subsequently set under a stereoscopic microscope. Next, each colony was scratched and sucked up using Pipetteman (manufactured by GILSON) and transferred into a 96 well round-bottom plate (manufactured by Falcon).

After a trypsin-treatment, each clone was inoculated into a 96 well flat-bottom plate for adhesion cell culture (manufactured by Iwaki Glass) and cultured for 1 week using IMDM medium (manufactured by Life Technologies) supplemented with 10% fetal bovine dialyzed serum (manufactured by Life Technologies).

After the culturing, each clone in the plate was subjected to trypsin treatment and then mixed with two volumes of a freeze drying medium (20% DMSO, 40% fetal bovine serum, 40% IMDM). A half of the mixture was inoculated into a 96 well flat-bottom plate for adhesion cell culture use (manufactured by Iwaki Glass) to prepare a replica plate, while the remaining half was subjected to cryopreservation as a master plate.

Next, the replica plate was cultured for 6 days using IMDM medium (manufactured by Life Technologies) supplemented with 15 μg/ml of puromycin (manufactured by SIGMA) and 10% fetal bovine dialyzed serum (manufactured by Life Technologies). A positive clone from which the puromycin resistance gene interposed between loxP sequences was eliminated by the expression of Cre recombinase dies out in the presence of puromycin. By the selection method, 91 positive clones were found.

(3) Diagnosis of drug resistance gene elimination by genome Southern blotting

Diagnosis of drug resistance gene elimination by the genome Southern blotting was carried out on optional 6 clones among the positive clones found in the item (2).

Among the master plates cryo-preserved in the item (2), 96 well plates containing the 6 positive clones were selected and incubated at 37°C for 10 minutes in 5% $CO_2$. After the incubation, cells were collected from a well corresponding to each positive clone and inoculated into a 24 well flat bottom plate for adhesion cell use (manufactured by Greiner). After culturing them for 1 week using IMDM medium (manufactured by Life Technologies) supplemented with 10% fetal bovine dialyzed serum (manufactured by Life Technologies), the cells were inoculated into a 6 well flat bottom plate for adhesion cell use (manufactured by Greiner). Genome DNAs were prepared from each clone in the plate in accordance with a known method [Nucleic Acids Research, 3, 2303 (1976)] and dissolved overnight in 150 μl of a TE-RNase buffer (pH 8.0) (10 mmol/l Tris-HCl, 1 mmol/l EDTA, 200 μg/ml RNase A).

In 120 μl of NEBuffer for *Bam*HI (manufactured by New England Biolabs), 12 μg of the obtained genome DNA was dissolved, and 20 unites of a restriction enzyme *Bam*HI (manufactured by New England Biolabs) were mixed, followed by digestion reaction at 37°C overnight. A DNA fragment was recovered from the reaction solution by ethanol precipitation, dissolved in 20 μl of TE buffer (pH 8.0) (10 mmol/l Tris-HCl, 1 mmol/l EDTA) and then subjected to 0.4% (w/v) agarose gel electrophoresis. After the electrophoresis, the genome DNA was transferred onto a nylon membrane in accordance with a known method [Proc. Natl. Acad. Sci. USA, 76, 3683 (1979)]. After completion of the transfer, the nylon membrane was heated at 80°C for 2 hours.

On the other hand, a probe used in the Southern blotting was prepared as follows. First, primers (SEQ ID NOs:30 and 31) which bind to a sequence outside the targeting vector homologous region among the FUT8 genome region obtained in Example 12 were designed. Next, polymerase chain reaction (PCR) was carried out using a DNA polymerase ExTaq (manufactured by Takara Shuzo), by preparing 20 μl of a reaction solution [ExTaq buffer (manufactured by Takara Shuzo), 0.2 mmol/l dNTPs and 0.5 μmol/l gene-specific primers (SEQ ID NOs:30 and 31)] containing 4.0 ng of the plasmid pFUT8fgE2-2 obtained in Example 12(2). The PCR was carried out by heating at 94°C for 1 minute and subsequent 25 cycles of heating at 94°C for 30 seconds, 55°C for 30 seconds and 74°C for 1 minute as one cycle. After the PCR, the reaction solution was subjected to 1.75% (w/v) agarose gel electrophoresis to purify a probe DNA fragment of about 230 bp. A 5 μl portion of the obtained probe DNA solution was radioisotope-labeled using 1.75 MBq of [$\alpha$-$^{32}$P]dCTP and Megaprime DNA Labeling System, dCTP (manufactured by Amersham Pharmacia Biotech).

The hybridization was carried out as follows. First, the nylon membrane was sealed in a roller bottle, and pre-hybridization was carried out at 65°C for 3 hours by adding 15 ml of a hybridization solution [5 × SSPE, 50 × Denhaldt's solution, 0.5% (w/v) SDS, 100 μg/ml salmon sperm DNA]. Next, the $^{32}$P-labeled probe DNA was heat-denatured and put into the bottle and the nylon membrane was heated overnight at 65°C.

After the hybridization, the nylon membrane was soaked in 50 ml of 2 × SSC-0.1% (w/v) SDS and heated at 65°C for 15 minutes. After repeating the washing step twice, the membrane was soaked in 50 ml of 0.2 × SSC-0.1% (w/v) SDS and heated at 65°C for 15 minutes. After washing the nylon membrane, it was exposed to an X-ray film two nights at -80°C for development.

By the restriction enzyme *Bam*HI treatment, a DNA fragment of about 19.0 Kb was formed from a wild type FUT8 allele. Also, a DNA fragment of about 12.5 Kb was formed from an allele in which homologous recombination with a targeting vector was generated. In addition, when the puromycin resistance gene (about 1.5 Kb) was deleted from the allele in which homologous recombination was generated, a DNA fragment of about 11.0 Kb was formed by the same treatment.

By the method, such specific fragments of about 19.0 Kb and about 11.0 Kb were found from the genome DNA of 5 clones among the 6 clones. Since the quantitative ratio of both fragments was 1 : 1, it was shown that the puromycin resistance gene was deleted from the cell lines in which 1 copy of the FUT8 genome region was disrupted. Hereinafter,

one of the clone is called 1st.ΔFUT8 2-46-1. Also, results of the genome Southern blotting of the strain 1st.ΔFUT8 2-46-1, 1st.ΔFUT8 2-46 and 5-03 are shown in Fig. 41. Also, the strain 1st.ΔFUT8 2-46-1, as a name of 2-46-1, has been deposited on September 26, 2001, as FERM BP-7755 in International Patent Organism Depositary, National Institute of Advanced Industrial Science and Technology (AIST Tsukuba Central 6, 1-1, Higashi 1-Chome Tsukuba-shi, Ibaraki-ken 305-8566 Japan)).

4. Purification of antibody produced by α-1,6-fucosyltransferase (FUT8) gene-disrupted cell line

1st.ΔFUT8 2-46-1 obtained in the item 3 of this Example by disrupting one copy of the FUT8 allele was suspended in IMDM medium (manufactured by Life Technologies) supplemented with 15 μg/ml of puromycin (manufactured by SIGMA) and 10% fetal bovine dialyzed serum (manufactured by Life Technologies) to give a density of $3 \times 10^5$ cells/ml, and then 60 ml in total of the suspension was inoculated into two T182 flasks for adhesion cell culture use (manufactured by Greiner). After culturing them for 3 days, the supernatant was discarded and changed to a total of 60 ml of EXCELL301 medium (manufactured by JRH Biosciences).

After culturing them at 37°C for 7 days in a 5% $CO_2$ incubator, the number of intact cells was counted to confirm that their viability was almost the same (each 30% or less), and then each cell suspension was recovered. The cell suspension was centrifuged at 3,000 rpm at 4°C for 10 minutes, and the recovered supernatant was centrifuged at 10,000 rpm at 4°C for 1 hour and then filtered using 150 ml capacity PES Filter Unit (manufactured by NALGENE) having a pore diameter of 0.22 μm.

Prosep-A HighCapacity (manufactured by bioPROCESSING) was packed in a 0.8 cm diameter column to a thickness of 2 cm and washed with 10 ml of 0.1 mol/l citrate buffer (pH 3.0) and 10 ml of 1 mol/l glycine/NaOH-0.15 mol/l NaCl buffer (pH 8.6) in this order to effect equilibrate the carrier. Next, 100 ml of each of the culture supernatant was passed through the column and washed with 50 ml of 1 mol/l glycine/NaOH-0.15 mol/l NaCl buffer (pH 8.6). After washing it, the antibody absorbed to Prosep-A was eluted using 2.5 ml of 0.1 mol/l citrate buffer (pH 3.0), the eluate was fractionated in 500 μl portions and each fraction was neutralized by mixing with 100 μl of 2 mol/l Tris-HCl (pH 8.5). Two fractions containing the antibody at a high concentration (1.2 ml in total) were selected by the BCA method [Anal. Biochem., 150, 76 (1985)], combined and then dialyzed against 10 mol/l citrate-0.15 mol/l NaCl buffer (pH 6.0) at 4°C for a whole day and night. After the dialysis, the antibody solution was recovered and subjected to sterile filtration using a 0.22 μm pore size Millex GV (manufactured by MILLIPORE).

5. *In vitro* cytotoxic activity (ADCC activity) of antibody produced by α-1,6-fucosyltransferase (FUT8) gene-disrupted cell line

In order to evaluate *in vitro* cytotoxic activity of the anti-CCR4 antibody purified in the item 4 of this Example, ADCC activity was measured using the CCR4-positive cell line CCR4/EL-4 described in Example 8.

The CCR4/EL-4 cells subcultured in RPMI1640 medium (manufactured by Life Technologies) containing 10% fetal bovine serum (manufactured by Life Technologies) (hereinafter referred to as "RPMI1640-FBS(10)") were suspended in 500 μl of RPMI1640-FBS(10) at a density of $1 \times 10^6$ cells, and 3.7 MBq of $Na_2{}^{51}CrO_4$ was added thereto, followed by culturing at 37°C for 90 minutes to label the cells with a radioisotope. After centrifugation at 1,200 rpm for 5 minutes, the supernatant was discarded and the target cells were suspended in 5 ml of RPMI1640-FBS(10). The washing step was repeated three times and then the cell suspension was incubated for 30 minutes on ice for spontaneous dissociation of the radioactive substance. The washing step was again repeated twice and then the cells were suspended in 5 ml of RPMI1640-FBS(10) to thereby prepare $2.0 \times 10^5$ cells/ml of a target cell suspension.

Separately, 30 ml of venous blood was collected from a healthy person, gently mixed with 0.5 ml of heparin sodium (manufactured by Shimizu Pharmaceutical) and then mixed with 30 ml of physiological saline (manufactured by Otsuka Pharmaceutical). After mixing them, 10 ml of the mixture was gently overlaid on 4 ml of Lymphoprep (manufactured by NYCOMED PHARMA AS) and centrifuged at room temperature at 2,000 rpm for 30 minutes. The separated mononuclear cell fractions were collected from the centrifugation tubes, combined and then suspended in 30 ml of RPMI1640-FBS(10). After centrifugation at room temperature at 1,200 rpm for 15 minutes, the supernatant was discarded and the cells were suspended in 20 ml of RPMI1640-FBS(10). The washing step was repeated twice and then $2.5 \times 10^6$ cells/ml of an effector cell suspension was prepared using RPMI1640-FBS(10).

The target cell suspension was dispensed at 50 μl ($1 \times 10^4$ cells/well) into each well of a 96 well U-bottom plate (manufactured by Falcon). Subsequently, the effector cell suspension was dispensed at 100 μl ($2.5 \times 10^5$ cells/well) into each well to thereby adjust the ratio of the effector cells to the target cells to 25 : 1. Next, using RPMI1640-FBS(10), a series of dilution solution of 0.01 μg/ml, 0.1 μg/ml, 1 μg/ml and 10 μg/ml was prepared from each of the anti-CCR4 antibodies obtained in the item 5 of Example 13, and the diluted solutions were dispensed in 50 μl portions into the wells to give final concentrations of 0.0025 μg/ml, 0.025 μg/ml, 0.25 μg/ml and 2.5 μg/ml, respectively. After the reaction at 37°C for 4 hours in 5% $CO_2$, the plate was centrifuged at 1,200 rpm for 5 minutes. Into a 12 mm diameter RIA tube

(manufactured by IWAKI), 75 μl of the supernatant in each well was batched off and the amount of the dissociated $^{51}Cr$ was measured using MINAX-γ auto-gamma counter 5550 (manufactured by PACKARD).

Also, the amount of the spontaneously dissociated $^{51}Cr$ was calculated by carrying out the same reaction in a reaction mixture in which 150 μl of RPMI1640-FBS(10) was added instead of the effector cell suspension and antibody solution. The amount of the total dissociated $^{51}Cr$ was calculated by carrying out the same reaction in a reaction mixture in which 100 μl of 1 N hydrochloric acid and 50 μl of RPMI1640-FBS(10) were added instead of the effector cell suspension and antibody solution. Using these values, the ADCC activity was calculated based on equation (II).

Fig. 42 shows ADCC activity of each of the anti-CCR4 antibodies. The antibody obtained from the strain 1st.ΔFUT8 2-46-1 in which 1 copy of the FUT8 allele was disrupted showed a significantly more potent ADCC activity than the antibody produced by the strain 5-03 which is the CHO cell line before gene disruption. Also, changes in the antigen binding activity of these antibodies were not observed. Based on the results, it was confirmed that the ADCC activity of produced antibodies can be improved by disrupting the FUT8 allele in host cells.

Example 14

Preparation of lectin-resistant CHO/DG44 cell and production of antibody using the cell:

(1) Preparation of lectin-resistant CHO/DG44

CHO/DG44 cells were grown until they reached a stage of just before confluent, by culturing in a 75 $cm^2$ flask for adhesion culture (manufactured by Greiner) using IMDM-FBS(10) medium [IMDM medium comprising 10% of fetal bovine serum (FBS) and 1 × concentration of HT supplement (manufactured by GIBCO BRL)]. After washing the cells with 5 ml of Dulbecco PBS (manufactured by Invitrogen), 1.5 ml of 0.05% trypsin (manufactured by Invitrogen) diluted with Dulbecco PBS was added thereto and incubated at 37°C for 5 minutes for peel the cells from the flask bottom. The peeled cells were recovered by a centrifugation operation generally used in cell culture and suspended in IMDM-FBS(10) medium to give a density of $1 \times 10^5$ cells/ml, and then 0.1 μg/ml of an alkylating agent *N*-methyl-N'-nitro-*N*-nitrosoguanidine (hereinafter referred to as "MNNG", manufactured by Sigma) was added or not added thereto. After incubating them at 37°C for 3 days in a $CO_2$ incubator (manufactured by TABAI), the culture supernatant was discarded, and the cells were again washed, peeled and recovered by the same operations, suspended in IMDM-FBS(10) medium and then inoculated into an adhesion culture 96 well plate (manufactured by IWAKI Glass) to give a density of 1,000 cells/well. To each well, as the final concentration in medium, 1 mg/ml *Lens culinaris* agglutinin (hereinafter referred to as "LCA", manufactured by Vector), 1 mg/ml *Aleuria aurantia* agglutinin (*Aleuria aurantia* lectin; hereinafter referred to as "AAL", manufactured by Vector) or 1 mg/ml kidney bean agglutinin (*Phaseolus vulgaris* leucoagglutinin; hereinafter referred to as "L-PHA", manufactured by Vector) was added. After culturing them at 37°C for 2 weeks in a $CO_2$ incubator, the appeared colonies were obtained as lectin-resistant CHO/DG44. Regarding the obtained lectin-resistant CHO/DG44, an LCA-resistant cell line was named CHO-LCA, an AAL-resistant cell line was named CHO-AAL and an L-PHA-resistant cell line was named CHO-PHA. When the resistance of these cell lines to various kinds of lectin was examined, it was found that the CHO-LCA was also resistant to AAL and the CHO-AAL was also resistant LCA. In addition, the CHO-LCA and CHO-AAL also showed a resistance to a lectin which recognizes a sugar chain structure identical to the sugar chain structure recognized by LCA and AAL, namely a lectin which recognizes a sugar chain structure in which 1-position of fucose is bound to 6-position of *N*-acetylglucosamine residue in the reducing end through α-bond in the *N*-glycoside-linked sugar chain. Specifically, it was found that the CHO-LCA and CHO-AAL can show resistance and survive even in a medium supplemented with 1 mg/ml at a final concentration of a pea agglutinin (*Pisum sativum* agglutinin; hereinafter referred to as "PSA", manufactured by Vector). In addition, even when the alkylating agent MNNG was not added, it was able to obtain lectin-resistant cell lines by increasing the number of cells to be treated. Hereinafter, these cell lines were used in analyses.

(2) Preparation of anti-CCR4 human chimeric antibody-producing cell

An anti-CCR4 human chimeric antibody expression plasmid pKANTEX2160 was introduced into the three lectin-resistant cell lines obtained in the (1) by the method described in Example 8, and gene amplification by a drug MTX was carried out to prepare an anti-CCR4 human chimeric antibody-producing cell line. By measuring an amount of antibody expression by the ELISA described in Example 8-2, antibody-expressing transformants were obtained from each of the CHO-LCA, CHO-AAL and CHO-PHA. Regarding each of the obtained transformants, a transformant derived from CHO-LCA was named CHO/CCR4-LCA, a transformant derived from CHO-AAL was named CHO/CCR4-AAL and a transformant derived from CHO-PHA was named CHO/CCR4-PHA. Also, the CHO/CCR4-LCA, as a name of Nega-13, has been deposited on September 26, 2001, as FERM BP-7756 in International Patent Organism Depositary, National Institute of Advanced Industrial Science and Technology (AIST Tsukuba Central 6, 1-1, Higashi 1-Chome Tsukuba-shi,

Ibaraki-ken 305-8566 Japan)).

(3) Production of potent ADCC activity antibody by lectin-resistant CHO cell

Using the three transformants obtained in the (2), purified antibodies were obtained by the method described in Example 8-3. The antigen binding activity of each of the purified anti-CCR4 human chimeric antibodies was evaluated using the ELISA described in Example 8-2. The antibodies produced by all transformants showed an antigen binding activity similar to that of the antibody produced by a recombinant cell line (strain 5-03) prepared in Example 8 using general CHO/DG44 cell as the host. Using these purified antibodies, ADCC activity of each of the purified anti-CCR4 human chimeric antibodies was evaluated in accordance with the method described in Example 8-7. The results are shown in Fig. 43. In comparison with the antibody produced by the strain 5-03, about 100 folds-increased ADCC activity was observed in the antibodies produced by the CHO/CCR4-LCA and CHO/CCR4-AAL. On the other hand, no significant increase in the ADCC activity was observed in the antibody produced by the CHO/CCR4-PHA. Also, when ADCC activities of the antibodies produced by the CHO/CCR4-LCA and YB2/0 were compared in accordance with the method described in Example 8-7, it was found that the antibody produced by the CHO/CCR4-LCA shows more potent ADCC activity than the antibody produced by the strain 5-03, similar to the case of the antibody KM2760-1 produced by the YB2/0 cell line prepared in Example 8-1 (Fig. 44).

(4) Sugar chain analysis of antibodies produced by lectin-resistant CHO cell

Sugar chains of the anti-CCR4 human chimeric antibodies purified in the (3) were analyzed. The solution of each of the purified antibodies was exchanged to 10 mM $KH_2PO_4$ using Ultra Free 0.5-10K (manufactured by Millipore). The exchange was carried out in such a manner that the exchanging ratio became 80 folds or more. The concentration of the antibodies after the solution exchange was measured using UV-1600 (manufactured by Shimadzu). The molar absorption coefficient was calculated from the amino acid sequence of each antibody based on the following equation (III) [Advances in Protein Chemistry, 12, 303 (1962)], and the concentration was determined by defining the absorbance at 280 nm as 1.38 mg/ml.

$$E_{1mol/l} = A \times n1 + B \times n2 + C \times n3 \quad (III)$$

$$E_{1mol/ml} = E_{1mol/l}/MW$$

| | |
|---|---|
| $E_{1mol/l}$: | absorption coefficient at 280 nm ($mg^{-1}$ ml $cm^{-1}$) |
| $E_{1mol/ml}$: | molar absorption coefficient at 280 nm ($M^{-1}$ $cm^{-1}$) |
| A: | molar absorption coefficient of tryptophan at 280 nm = 5550 ($M^{-1}$ $cm^{-1}$) |
| B: | molar absorption coefficient of tyrosine at 280 nm = 1340 ($M^{-1}$ $cm^{-1}$) |
| C: | molar absorption coefficient of cystine at 280 nm = 200 ($M^{-1}$ $cm^{-1}$) |
| n1: | the number of tryptophan per 1 antibody molecule |
| n2: | the number of tyrosine per 1 antibody molecule |
| n3: | the number of cystine per 1 antibody molecule |
| MW: | molecular weight of antibody (g/mol) |

Into Hydraclub S-204 test tube, 100 μg of each antibody was put and dried using a centrifugal evaporator. The dried sample in the test tube was subjected to hydrazinolysis using Hydraclub manufactured by Hohnen. The sample was allowed to react with hydrazine at 110°C for 1 hour using a hydrazinolysis reagent manufactured by Hohnen hydrazinolysis [Method of Enzymology, 83, 263 (1982)]. After the reaction, hydrazine was evaporated under a reduced pressure, and the reaction tube was returned to room temperature by allowing it to stand for 30 minutes. Next, 250 μl of an acetylation reagent manufactured by Hohnen and 25 μl of acetic anhydride were added thereto, followed by thoroughly stirred for reaction at room temperature for 30 minutes. Then, 250 μl of the acetylation reagent and 25 μl of acetic anhydride were further added thereto, followed by thoroughly stirring for reaction at room temperature for 1 hour. The sample was frozen at -80°C in a freezer and freeze-dried for about 17 hours. Sugar chains were recovered from the freeze-dried sample using Cellulose Cartridge Glycan Preparation Kit manufactured by Takara Shuzo Co., Ltd. The sample sugar chain solution was dried using a centrifugal evaporator and then subjected to fluorescence labeling with 2-aminopyridine [J. Biochem., 95, 197 (1984)]. The 2-aminopyridine solution was prepared by adding 760 μl of HCl per 1 g of 2-aminopyridine (1 × PA solution) and diluting the solution 10 folds with reverse osmosis purified water (10-folds

diluted PA solution). The sodium cyanoborohydride solution was prepared by adding 20 μl of 1 x PA solution and 430 μl of reverse osmosis purified water per 10 mg of sodium cyanoborohydride. To the sample, 67 μl of a 10 folds-diluted PA solution was added, followed by reaction at 100°C for 15 minutes and spontaneously cooled, and 2 μl of sodium cyanoborohydride was further added thereto, followed by reaction at 90°C for 12 hours for fluorescence labeling of the sample sugar chains. The fluorescence-labeled sugar chain group (PA-treated sugar chain group) was separated from excess reagent using Superdex Peptide HR 10/30 column (manufactured by Pharmacia). This step was carried out using 10 mM ammonium bicarbonate as the eluent at a flow rate of 0.5 ml/min and at a column temperature of room temperature, and using a fluorescence detector of 320 nm excitation wavelength and 400 nm fluorescence wavelength. The eluate was recovered 20 to 30 minutes after addition of the sample and dried using a centrifugal evaporator to be used as purified PA-treated sugar chains. Next, reverse phase HPLC analysis of the purified PA-treated sugar chains was carried out using CLC-ODS column (manufactured by Shimadzu, ϕ 6.0 nm × 159 nm). The step was carried out at a column temperature of 55°C and at a flow rate of 1 ml/min and using a fluorescence detector of 320 nm excitation wavelength and 400 nm fluorescence wavelength. The column was equilibrated with a 10 mM sodium phosphate buffer (pH 3.8) and elution was carried out for 80 minutes by a 0.5% 1-butanol linear density gradient. Each of the PA-treated sugar chain was identified by post source decay analysis of each peak of the separated PA-treated sugar chains using matrix-assisted laser ionization time of flight mass spectrometry (MALDI-TOF-MS analysis), comparison of elution positions with standards of PA-treated sugar chain manufactured by Takara Shuzo, and reverse phase HPLC analysis after digestion of each PA-treated sugar chain using various enzymes (Fig. 45). Each of the sugar chain content was calculated from each of the peak area of PA-treated sugar chain by reverse HPLC analysis. A PA-treated sugar chain whose reducing end is not *N*-acetylglucosamine was excluded from the peak area calculation, because it is an impurity or a by-product during preparation of PA-treated sugar chain.

The analysis was carried out in the same manner as in Example 11(6) using a sodium phosphate buffer (pH 3.8) as buffer A and a sodium phosphate buffer (pH 3.8) + 0.5% 1-butanol as buffer B.

In Fig. 45, the ratio of the α-1,6-fucose-free sugar chain group was calculated from the area occupied by the peaks (i) to (iv) among (i) to (viii), and the ratio of the α-1,6-fucose-bound sugar chain group from the area occupied by the peaks (v) to (viii) among (i) to (viii).

Results of the sugar chain structure analysis of the purified anti-CCR4 human chimeric antibodies produced by lectin-resistant cell lines are shown in Table 6. The result shows the analysis of sugar chains of the anti-CCR4 human chimeric antibody produced by lectin-resistant cell lines. The ratio of α-1,6-fucose-free sugar chains (%) calculated from peak areas by analyzing by the method described in Example d(4) is shown in the table.

Table 6

| Antibody producing cells | α-1,6-Fucose-free complex double-chain sugar chain (%) |
|---|---|
| Strain 5-03 | 9 |
| Strain CHO/CCR4-LCA | 48 |
| Strain CHO/CCR4-AAL | 27 |
| Strain CHO/CCR4-PHA | 8 |

In comparison with the antibody produced by the strain 5-03, the ratio of the α-1,6-fucose-free sugar chains was increased from 9% to 48% in the antibody produced by the CHO/CCR4-LCA when calculated from the analyzed peak area. The ratio of α-1,6-fucose-free sugar chains was increased from 9% to 27% in the antibody produced by the CHO/CCR4-AAL. On the other hand, changes in the sugar chain pattern and ratio of the α-1,6-fucose-free sugar chains were hardly found in the PHA-resistant cell line when compared with the strain 5-03.

Example 15

Analysis of lectin-resistant CHO cell line:

1. Analysis of expression level of GMD enzyme in an anti-CCR4 human chimeric antibody-producing cell line CHO/CCR4-LCA

The expression level of each of the genes of GMD (GDP-mannose 4,6-dehydratase), GFPP (GDP-keto-6-deoxymannose 3,5-epimerase, 4-reductase) and FX (GDP-beta-L-fucose pyrophosphorylase) known as fucose biosynthesis enzymes and FUT8 (α-1,6-fucosyltransferase) as a fucose transferase, in the anti-CCR4 human chimeric antibody-producing cell line CHO/CCR4-LCA obtained in Example 14, was analyzed using RT-PCR method.

(1) Preparation of RNA from various cell lines

Each of the CHO/DG44 cell, the anti-CCR4 human chimeric antibody-producing cell line 5-03 obtained in Example 8-1(2) and the anti-CCR4 human chimeric antibody-producing cell line CHO/CCR4-LCA obtained in Example 14(2) was subcultured at 37°C in a 5% $CO_2$ incubator and then cultured for 4 days. After culturing them, total RNA was prepared from $1 \times 10^7$ cells of each cell line using RNeasy Protect Mini Kit (manufactured by QIAGEN) in accordance with the manufacture's instructions. Subsequently, single-stranded cDNA was synthesized from 5 μg of each RNA in a 20 μl of a reaction solution using SUPER SCRIPT First-Strand Synthesis System for RT-PCR (manufactured by GIBCO BRL) in accordance with the manufacture's instructions.

(2) Analysis of expression level of GMD gene using RT-PCR

In order to amplify GMD cDNA by PCR, a 24 mer synthetic DNA primer having the nucleotide sequence shown by SEQ ID NO:32 and a 26 mer synthetic DNA primer having the nucleotide sequence shown by SEQ ID NO:33 were prepared based on the CHO cell-derived GMD cDNA sequence shown in Example 17-1.

Next, 20 μl of a reaction solution [1 × Ex Taq buffer (manufactured by Takara Shuzo), 0.2 mM dNTPs, 0.5 unit of Ex Taq polymerase (manufactured by Takara Shuzo) and 0.5 μM of the synthetic DNA primers of SEQ ID NOs:32 and 33] containing 0.5 μl of the single-stranded cDNA prepared from each cell line in the item (1) as the template was prepared, and PCR was carried out using DNA Thermal Cycler 480 (manufactured by Perkin Elmer) by heating at 94°C for 5 minutes and subsequent 30 cycles of heating of 94°C for 1 minute and 68°C for 2 minutes as one cycle. After subjecting 10 μl of the PCR reaction solution to agarose electrophoresis, DNA fragments were stained using Cyber Green (manufactured by BMA) and then the amount of the DNA fragment of about 350 bp was measured using Fluor Imager SI (manufactured by Molecular Dynamics).

(3) Analysis of expression level of GFPP gene using RT-PCR

In order to amplify GFPP cDNA by PCR, a 27 mer synthetic DNA primer having the nucleotide sequence shown by SEQ ID NO:34 and a 23 mer synthetic DNA primer having the nucleotide sequence shown by SEQ ID NO:35 were prepared based on the CHO cell-derived GFPP cDNA sequence obtained in Example 16-2.

Next, 20 μl of a reaction solution [1 × Ex Taq buffer (manufactured by Takara Shuzo), 0.2 mM dNTPs, 0.5 unit of Ex Taq polymerase (manufactured by Takara Shuzo) and 0.5 μM of the synthetic DNA primers of SEQ ID NOs:34 and 35] containing 0.5 μl of the single-stranded cDNA prepared from each cell line in the item (1) as the template was prepared, and PCR was carried out using DNA Thermal Cycler 480 (manufactured by Perkin Elmer) by heating at 94°C for 5 minutes and subsequent 24 cycles of heating at 94°C for 1 minute and 68°C for 2 minutes as one cycle. After subjecting 10 μl of the PCR reaction solution to agarose electrophoresis, DNA fragments were stained using Cyber Green (manufactured by BMA) and then the amount of the DNA fragment of about 600 bp was measured using Fluor Imager SI (manufactured by Molecular Dynamics).

(4) Analysis of expression level of FX gene using RT-PCR

In order to amplify FX cDNA by PCR, a 28 mer synthetic DNA primer having the nucleotide sequence shown by SEQ ID NO:36 and a 28 mer synthetic DNA primer having the nucleotide sequence shown by SEQ ID NO:37 were prepared based on the CHO cell-derived FX cDNA sequence shown in Example 16-1.

Next, 20 μl of a reaction solution [1 × Ex Taq buffer (manufactured by Takara Shuzo), 0.2 mM dNTPs, 0.5 unit of Ex Taq polymerase (manufactured by Takara Shuzo) and 0.5 μM of the synthetic DNA primers of SEQ ID NO:36 and SEQ ID NO:37] containing 0.5 μl of the single-stranded cDNA prepared from each cell line in the item (1) as the template was prepared, and PCR was carried out using DNA Thermal Cycler 480 (manufactured by Perkin Elmer) by heating at 94°C for 5 minutes and subsequent 22 cycles of heating at 94°C for 1 minute and 68°C for 2 minutes as one cycle. After subjecting 10 μl of the PCR reaction solution to agarose electrophoresis, DNA fragments were stained using Cyber Green (manufactured by BMA) and then the amount of the DNA fragment of about 300 bp was measured using Fluor Imager SI (manufactured by Molecular Dynamics).

(5) Analysis of expression level of FUT8 gene using RT-PCR

In order to amplify FUT8 cDNA by PCR, 20 μl of a reaction solution [1 × Ex Taq buffer (manufactured by Takara Shuzo), 0.2 mM dNTPs, 0.5 unit of Ex Taq polymerase (manufactured by Takara Shuzo) and 0.5 μM of the synthetic DNA primers of SEQ ID NOs:13 and 14] containing 0.5 μl of the single-stranded cDNA prepared from each cell line in the item (1) as the template was prepared, and PCR was carried out using DNA Thermal Cycler 480 (manufactured by

Perkin Elmer) by heating at 94°C for 5 minutes and subsequent 20 cycles of heating at 94°C for 1 minute and 68°C for 2 minutes as one cycle. After subjecting 10 μl of the PCR reaction solution to agarose electrophoresis, DNA fragments were stained using Cyber Green (manufactured by BMA) and then amount of the DNA fragment of about 600 bp was measured using Fluor Imager SI (manufactured by Molecular Dynamics).

(6) Analysis of expression level of β-actin gene using RT-PCR

In order to amplify β-actin cDNA by PCR, 20 μl of a reaction solution [1 × Ex Taq buffer (manufactured by Takara Shuzo), 0.2 mM dNTPs, 0.5 unit of Ex Taq polymerase (manufactured by Takara Shuzo) and 0.5 μM of the synthetic DNA primers of SEQ ID NOs:15 and 16] containing 0.5 μl of the single-stranded cDNA prepared from each cell line in the item (1) as the template was prepared, and the reaction was carried out using DNA Thermal Cycler 480 (manufactured by Perkin Elmer) by heating at 94°C for 5 minutes and subsequent 14 cycles of heating at 94°C for 1 minute and 68°C for 2 minutes as one cycle. After subjecting 10 μl of the PCR reaction solution to agarose electrophoresis, DNA fragments were stained using Cyber Green (manufactured by BMA) and then the amount of the DNA fragment of about 800 bp was measured using Fluor Imager SI (manufactured by Molecular Dynamics).

(7) Expression levels of GMD, GFPP, FX and FUT8 genes in each cell line

The amount of the PCR-amplified fragment of each gene in the strain 5-03 and the CHO/CCR4-LCA was calculated by dividing values of the amounts of PCR-amplified fragments derived from GMD, GFPP, FX and FUT cDNA in each cell line measured in the items (2) to (6) by the value of the amount of PCR-amplified fragment derived from β-actin cDNA in each cell line, and defining the amount of the PCR-amplified fragments in CHO/DG44 cell as 1. The results are shown in Table 7.

Table 7

| | GMD | GEPP | FX | FUT8 |
|---|---|---|---|---|
| Strain CHO/DG44 | 1 | 1 | 1 | 1 |
| Strain 5-03 | 1.107 | 0.793 | 1.093 | 0.901 |
| Strain 5-03-derived LCA-resistant cell CHO/CCR4-LCA | 0.160 | 0.886 | 0.920 | 0.875 |

As shown in Table 7, the expression level of GMD gene in the CHO/CCR4-LCA was decreased to about 1/10 in comparison with other cell lines. In this case, the test was independently carried out twice, and the average value was used.

2. Analysis using anti-CCR4 human chimeric antibody-producing CHO/CCR4-LCA in which GMD gene was forced to express

(1) Construction of CHO cell-derived GMD gene expression vector pAGE249GMD

Based on the CHO cell-derived GMD cDNA sequence obtained in Example 17-1, a 28 mer primer having the nucleotide sequence shown by SEQ ID NO:38 and a 29 mer primer having the nucleotide sequence shown by SEQ ID NO:39 were prepared. Next, 20 μl of a reaction solution [1 × Ex Taq buffer (manufactured by Takara Shuzo), 0.2 mM dNTPs, 0.5 unit of Ex Taq polymerase (manufactured by Takara Shuzo) and 0.5 μM of the synthetic DNA primers of SEQ ID NOs:38 and 39] containing 0.5 μl of the CHO cell-derived GMD single-stranded cDNA prepared in the item 1(1) of this Example as the template was prepared, and PCR was carried out using DNA Thermal Cycler 480 (manufactured by Perkin Elmer) by heating at 94°C for 5 minutes and subsequently 8 cycles of heating at 94°C for 1 minute, 58°C for 1 minute and 72°C for 1 minute as one cycle, and then 22 cycles of heating at 94°C for 1 minute and 68°C as one cycle. After completion of the reaction, the PCR reaction solution was fractionated by agarose electrophoresis, and then a DNA fragment of about 600 bp was recovered using Gene Clean II Kit (manufactured by BIO 101) in accordance with the manufacture's instructions. The recovered DNA fragment was connected to pT7Blue(R) vector (manufactured by Novagen) using DNA Ligation Kit (manufactured by Takara Shuzo), and *E. coli* DH5α (manufactured by Toyobo) was transformed using the obtained recombinant plasmid DNA to obtain a plasmid mt-C (cf. Fig. 46).

Next, based on the CHO cell-derived GMD cDNA sequence obtained in Example 17-1, a 45 mer primer having the nucleotide sequence shown by SEQ ID NO:40 and a 31 mer primer having the nucleotide sequence shown by SEQ ID NO:41 were prepared. Next, 20 μl of a reaction solution [1 × Ex Taq buffer (manufactured by Takara Shuzo), 0.2

mM dNTPs, 0.5 unit of Ex Taq polymerase (manufactured by Takara Shuzo) and 0.5 μM of the synthetic DNA primers of SEQ ID NOs:40 and 41] containing 0.5 μl of the CHO cell-derived GMD single-stranded cDNA prepared in the item 1(1) of this Example as the template was prepared, and PCR was carried out using DNA Thermal Cycler 480 (manufactured by Perkin Elmer) by heating at 94°C for 5 minutes and subsequently 8 cycles of heating at 94°C for 1 minute, 57°C for 1 minute and 72°C for 1 minute as one cycle, and then 22 cycles of heating at 94°C for 1 minute and 68°C for 2 minutes as one cycle. After completion of the reaction, the PCR reaction solution was fractionated by agarose electrophoresis, and then a DNA fragment of about 150 bp was recovered using Gene Clean II Kit (manufactured by BIO 101) in accordance with the manufacture's instructions. The recovered DNA fragment was connected to pT7Blue(R) vector (manufactured by Novagen) using DNA Ligation Kit (manufactured by Takara Shuzo), and *E. coli* DH5α (manufactured by Toyobo) was transformed using the obtained recombinant plasmid DNA to obtain a plasmid ATG (cf. Fig. 47).

Next, 3 μg of the plasmid CHO-GMD prepared in Example 17-1 was allowed to react with a restriction enzyme *Sac*I (manufactured by Takara Shuzo) at 37°C for 16 hours, a DNA was recovered by carrying out phenol/chloroform extraction and ethanol precipitation and allowed to react with a restriction enzyme *EcoRI* (manufactured by Takara Shuzo) at 37°C for 16 hours, a digest DNA was fractionated by agarose electrophoresis and then a DNA fragment of about 900 bp was recovered using Gene Clean II Kit (manufactured by BIO 101) in accordance with the manufacture's instructions. The plasmid mt-C (1.4 μg) was allowed to react with a restriction enzyme *Sac*I (manufactured by Takara Shuzo) at 37°C for 16 hours, DNA was recovered by carrying out phenol/chloroform extraction and ethanol precipitation and allowed to react with a restriction enzyme *Eco*RI (manufactured by Takara Shuzo) at 37°C for 16 hours, the digest was fractionated by agarose electrophoresis and then a DNA fragment of about 3.1 kbp was recovered using Gene Clean II Kit (manufactured by BIO 101) in accordance with the manufacture's instructions. The recovered DNA fragments were ligated using DNA Ligation Kit (manufactured by Takara Shuzo), and *E. coli* DH5α was transformed using the obtained recombinant plasmid DNA to obtain a plasmid WT-N(-) (cf. Fig. 48).

Next, 2 μg of the plasmid WT-N(-) was allowed to react with a restriction enzyme *Bam*HI (manufactured by Takara Shuzo) at 37°C for 16 hours, DNA was recovered by carrying out phenol/chloroform extraction and ethanol precipitation and allowed to react with a restriction enzyme *Eco*RI (manufactured by Takara Shuzo) at 37°C for 16 hours, the digest was fractionated by agarose electrophoresis and then a DNA fragment of about 1 kbp was recovered using Gene Clean II Kit (manufactured by BIO 101) in accordance with the manufacture's instructions. The plasmid pBluescript SK(-) (3 μg; manufactured by Stratagene) was allowed to react with a restriction enzyme *Bam*HI (manufactured by Takara Shuzo) at 37°C for 16 hours, DNA was recovered by carrying out phenol/chloroform extraction and ethanol precipitation and allowed to react with a restriction enzyme *Eco*RI (manufactured by Takara Shuzo) at 37°C for 16 hours, the digest was fractionated by agarose electrophoresis and then a DNA fragment of about 3 kbp was recovered using Gene Clean II Kit (manufactured by BIO 101) in accordance with the manufacture's instructions. The recovered respective DNA fragments were ligated using DNA Ligation Kit (manufactured by Takara Shuzo), and *E. coli* DH5α was transformed using the obtained recombinant plasmid DNA to obtain a plasmid WT-N(-) in pBS (cf. Fig. 49).

Next, 2 μg of the plasmid WT-N(-) in pBS was allowed to react with a restriction enzyme *Hind*III (manufactured by Takara Shuzo) at 37°C for 16 hours, DNA was recovered by carrying out phenol/chloroform extraction and ethanol precipitation and allowed to react with a restriction enzyme *Eco*RI (manufactured by Takara Shuzo) at 37°C for 16 hours, the digest was fractionated by agarose electrophoresis and then a DNA fragment of about 4 kbp was recovered using Gene Clean II Kit (manufactured by BIO 101) in accordance with the manufacture's instructions. A 2 μg portion of the plasmid ATG was allowed to react with a restriction enzyme *Hind*III (manufactured by Takara Shuzo) at 37°C for 16 hours, DNA was recovered by carrying out phenol/chloroform extraction and ethanol precipitation and allowed to react with a restriction enzyme *Eco*RI (manufactured by Takara Shuzo) at 37°C for 16 hours, the digest was fractionated by agarose electrophoresis and then a DNA fragment of about 150 bp was recovered using Gene Clean II Kit (manufactured by BIO 101) in accordance with the manufacture's instructions. The recovered respective DNA fragments were ligated using DNA Ligation Kit (manufactured by Takara Shuzo), and *E. colι* DH5α was transformed using the obtained recombinant plasmid DNA to obtain a plasmid WT in pBS (cf. Fig. 50).

Next, 2 μg of the plasmid pAGE249 was allowed to react with restriction enzymes *Hind*III and *Bam*HI (both manufactured by Takara Shuzo) at 37°C for 16 hours, the digest was fractionated by agarose electrophoresis and then a DNA fragment of about 6.5 kbp was recovered using Gene Clean II Kit (manufactured by BIO 101) in accordance with the manufacture's instructions. The plasmid WT (2 μg) in pBS was allowed to react with restriction enzymes *Hind*III and *Bam*HI (both manufactured by Takara Shuzo) at 37°C for 16 hours, the digest was fractionated by agarose electrophoresis and then a DNA fragment of about 1.2 kbp was recovered using Gene Clean II Kit (manufactured by BIO 101) in accordance with the manufacture's instructions. The recovered respective DNA fragments were ligated using DNA Ligation Kit (manufactured by Takara Shuzo), and *E. coli* DH5α was transformed using the obtained recombinant plasmid DNA to obtain a plasmid pAGE249GMD (cf. Fig. 51).

(2) Stable expression of GMD gene in CHO/CCR4-LCA

The CHO cell-derived GMD gene expression vector pAGE249GMD (5 μg) made into linear form by digesting it with a restriction enzyme *Fsp*I (manufactured by NEW ENGLAND BIOLABS), which was introduced into 1.6 × $10^6$ cells of CHO/CCR4-LCA by electroporation [Cytotechnology, 3, 133 (1990)]. Then, the cells were suspended in 30 ml of IMDM-dFBS(10) medium [IMDM medium (manufactured by GIBCO BRL) supplemented with 10% of dFBS] containing 200 nM MTX (manufactured by SIGMA), and cultured using a 182 $cm^2$ flask (manufactured by Greiner) at 37°C for 24 hours in a 5% $CO_2$ incubator. After culturing them, the medium was changed to IMDM-dFBS(10) medium containing 0.5 mg/ml hygromycin and 200 nM MTX (manufactured by SIGMA), followed by culturing for 19 days to obtain colonies of hygromycin-resistant transformants.

In the same manner, the pAGE249 vector was introduced into the CHO/CCR4-LCA by the same method to obtain colonies of hygromycin-resistant transformants.

(3) Culturing of GMD gene-expressed CHO/CCR4-LCA and purification of antibody

Using IMDM-dFBS(10) medium comprising 200 nM MTX (manufactured by SIGMA) and 0.5 mg/ml hygromycin, the GMD-expressing transformant cells obtained in the item (2) were cultured using a 182 $cm^2$ flask (manufactured by Greiner) at 37°C in a 5% $CO_2$ incubator. Several days thereafter, when the cell density reached confluent, the culture supernatant was discarded and the cells were washed with 25 ml of PBS buffer (manufactured by GIBCO BRL) and mixed with 35 ml of EXCELL301 medium (manufactured by JRH). After culturing them at 37°C in a 5% $CO_2$ incubator for 7 days, the culture supernatant was recovered. An anti-CCR4 chimeric antibody was purified from the culture supernatant using Prosep-A (manufactured by Millipore) in accordance with the manufacture's instructions.

In the same manner, the pAGE249 vector-introduced transformant cells were cultured by the same method and then anti-CCR4 chimeric antibody was recovered and purified from the culture supernatant.

(4) Measurement of lectin resistance in transformed cells

The GMD-expressing transformant cells obtained in the item (2) were suspended in IMDM-dFBS(10) medium comprising 200 nM MTX (manufactured by SIGMA) and 0.5 mg/ml hygromycin to give a density of 6 × $10^4$ cells/ml, and the suspension was dispensed in 50 μl/well portions into a 96 well culture plate (manufactured by Iwaki Glass). Next, a medium prepared by suspending at concentrations of 0 mg/ml, 0.4 mg/ml, 1.6 mg/ml or 4 mg/ml LCA (*Lens culinaris* agglutinin: manufactured by Vector Laboratories) in IMDM-dFBS(10) medium containing 200 nM MTX (manufactured by SIGMA) and 0.5 mg/ml hygromycin was added to the plate at 50 μl/well, followed by culturing at 37°C for 96 hours in a 5% $CO_2$ incubator. After culturing them, WST-I (manufactured by Boehringer) was added at 10 μl/well and incubated at 37°C for 30 minutes in a 5% $CO_2$ incubator to effect color development, and then the absorbance at 450 nm and 595 nm (hereinafter referred to as "OD450" and "OD595", respectively) was measured using Microplate Reader (manufactured by BIO-RAD). In the same manner, the pAGE249 vector-introduced transformant cells were measured by the same method. The test was carried out twice independently.

Fig. 52 shows the number of survived cells in each well by percentage when a value calculated by subtracting OD595 from OD450 measured in the above is used as the survived number of each cell group and the number of survived cells in each of the LCA-free wells is defined as 100%. As shown in Fig. 52, decrease in the LCA-resistance was observed in the GMD-expressed CHO/CCR4-LCA, and the survival ratio was about 40% in the presence of 0.2 mg/ml LCA and the survival ratio was about 20% in the presence of 0.8 mg/ml LCA. On the other hand, in the pAGE249 vector-introduced CHO/CCR4-LCA, the survival ratio was 100% in the presence of 0.2 mg/ml LCA and the survival ratio was about 80% even in the presence of 0.8 mg/ml LCA. Based on these results, it was suggested that expression level of GMD gene in the CHO/CCR4-LCA was decreased and, as a result, the resistance against LCA was obtained.

*(5) In vitro* cytotoxic activity (ADCC activity) of anti-CCR4 chimeric antibody obtained from GMD-expressed CHO/CCR4-LCA

In order to evaluate *in vitro* cytotoxic activity of the purified anti-CCR4 chimeric antibody obtained in the item (3), the ADCC activity was measured in accordance with the following methods.

i) Preparation of target cell suspension

A 3.7 MBq equivalent of a radioactive substance $Na_2{}^{51}CrO_4$ was added to 1 × $10^6$ cells of the CCR4-EL4 (cf. Example 8-7) cultured using a medium prepared by adding 500 μg/ml G418 sulfate (manufactured by Nacalai Tesque) to the RPMI1640-FBS(10) medium, followed by reaction at 37°C for 90 minutes to thereby label the cells with a radioisotope. After the reaction, the cells were washed three times by suspension in the RPMI1640-FBS(10) medium and subsequent centrifugation, re-suspended in the medium and then incubated at 4°C for 30 minutes in ice for spontaneous dissociation of the radioactive substance. After centrifugation, the cells were adjusted to $2.5 \times 10^5$ cells/ml by adding 5 ml of the RPMI1640-FBS(10) medium and used as a target cell suspension.

ii) Preparation of effector cell suspension

From a healthy person, 50 ml of venous blood was collected and gently mixed with 0.5 ml of heparin sodium (manufactured by Takeda Pharmaceutical). Using Lymphoprep (manufactured by Nycomed Pharma AS), the mixture was centrifuged in accordance with the manufacture's instructions to separate a mononuclear cell layer. The cells were washed three times by centrifuging using the RPMI1640-FBS(10) medium and then resuspended in the medium to give a density of $2 \times 10^6$ cells/ml and used as a effector cell suspension.

iii) Measurement of ADCC activity

The target cell suspension prepared in the 1) was dispensed at 50 μl ($1 \times 10^4$ cells/well) into each well of a 96 well U-bottom plate (manufactured by Falcon). Next, 100 μl of the effector cell suspension prepared in the 2) was added thereto ($2 \times 10^5$ cells/well, ratio of the effector cells to the target cells was 25 : 1). Each of various anti-CCR4 chimeric antibodies (the anti-CCR4 chimeric antibody purified in the item (3), and KM2760-1 and KM3060) was further added thereto to give a final concentration of 0.0025 to 2.5 μg/ml, followed by reaction at 37°C for 4 hours. After the reaction, the plate was centrifuged and the amount of $^{51}Cr$ in the supernatant was measured using a γ-counter. The amount of the spontaneously dissociated $^{51}Cr$ was calculated by carrying out the same procedure using the medium alone instead of the effector cell suspension and antibody solution, and measuring the amount of $^{51}Cr$ in the supernatant. The amount of the total dissociated $^{51}Cr$ was calculated by carrying out the same procedure using the medium alone instead of the antibody solution and adding 1 N hydrochloric acid instead of the effector cell suspension and measuring the amount of $^{51}Cr$ in the supernatant. The ADCC activity was calculated based on the formula (II).

Results of the measurement of ADCC activity are shown in Fig. 53. As shown in Fig. 53, ADCC activity of the purified anti-CCR4 chimeric antibody obtained from the GMD-expressed CHO/CCR4-LCA was decreased to a similar degree to that of the KM3060 obtained in Example 8. On the other hand, ADCC activity of the purified anti-CCR4 chimeric antibody obtained from the pAGE249 vector-introduced CHO/CCR4-LCA showed a similar degree of ADCC activity to that of the purified anti-CCR4 chimeric antibody obtained from the CHO/CCR4-LCA. Based on the results, it was suggested that expression level of GMD gene in the CHO/CCR4-LCA is decreased and, as a result, an antibody having potent ADCC activity can be produced

(6) Sugar chain analysis of anti-CCR4 chimeric antibody derived from GMD-expressed CHO/CCR4-LCA

Sugar chains binding to the purified anti-CCR4 chimeric antibody obtained in the item (3) were analyzed in accordance with the method shown in Example 14(4), with the analyzed results shown in Fig. 55. In comparison with the purified anti-CCR4 chimeric antibody prepared from CHO/CCR4-LCA in Example 14, the ratio of sugar chain having no α-1,6-fucose in the purified anti-CCR4 chimeric antibody derived from GMD-expressed CHO/CCR4-LCA was decreased to 9% when calculated from the peak area. Thus, it was shown that the ratio of sugar chain having no α-1,6-fucose in the antibody produced by the cell is decreased to similar level of the antibody produced by the strain 5-03, by expressing GMD gene in the CHO/CCR4-LCA

Example 16

Preparation of various genes encoding enzymes relating to the sugar chain synthesis in CHO cell:

1. Determination of CHO cell-derived FX cDNA sequence

(1) Extraction of total RNA from CHO/DG44 cell

CHO/DG44 cells were suspended in IMDM medium containing 10% fetal bovine serum (manufactured by Life Technologies) and 1 × concentration HT supplement (manufactured by Life Technologies), and 15 ml of the suspension was inoculated into a T75 flask for adhesion cell culture use (manufactured by Greiner) to give a density of $2 \times 10^5$ cells/ml. On the second day after culturing them at 37°C in a 5% $CO_2$ incubator, $1 \times 10^7$ of the cells were recovered and total RNA was extracted therefrom using RNAeasy (manufactured by QIAGEN) in accordance with the manufacture's instructions.

(2) Preparation of total single-stranded cDNA from CHO/DG44 cell

The total RNA prepared in the (1) was dissolved in 45 μl of sterile water, and 1 μl of RQ1 RNase-Free DNase (manufactured by Promega), 5 μl of the attached 10 × DNase buffer and 0.5 μl of RNasin Ribonuclease Inhibitor (manufactured by Promega) were added thereto, followed by reaction at 37°C for 30 minutes to degrade genome DNA contaminated in the sample. After the reaction, the total RNA was purified again using RNAeasy (manufactured by QIAGEN) and dissolved in 50 μl of sterile water.

In a 20 μl of reaction mixture using oligo(dT) as a primer, single-stranded cDNA was synthesized from 3 μg of the obtained total RNA samples by carrying out reverse transcription reaction using SUPERSCRIPT™ Preamplification System for First Strand cDNA Synthesis (manufactured by Life Technologies) in accordance with the manufacture's instructions. A 50 folds-diluted aqueous solution of the reaction solution was used in the cloning of GFPP and FX. This was stored at -80°C until use.

(3) Preparation of cDNA partial fragment of Chinese hamster-derived FX

FX cDNA partial fragment derived from Chinese hamster was prepared by the following procedure.

First, primers (shown in SEQ ID NOs:42 and 43) specific for common nucleotide sequences registered at a public data base, namely a human FX cDNA (Genebank Accession No. U58766) and a mouse cDNA (Genebank Accession No. M30127), were designed.

Next, 25 μl of a reaction solution [ExTaq buffer (manufactured by Takara Shuzo), 0.2 mmol/l dNTPs and 0.5 μmol/l gene-specific primers (SEQ ID NOs:42 and 43)] containing 1 μl of the CHO/DG44-derived single-stranded cDNA prepared in the item (2) was prepared, and polymerase chain reaction (PCR) was carried out using a DNA polymerase ExTaq (manufactured by Takara Shuzo). The PCR was carried out by heating at 94°C for 5 minutes, subsequent 30 cycles of heating at 94°C for 1 minute, 58°C for 2 minutes and 72°C for 3 minutes as one cycle, and final heating at 72°C for 10 minutes.

After the PCR, the reaction solution was subjected to 2% agarose gel electrophoresis, and a specific amplified fragment of 301 bp was purified using QuiaexII Gel Extraction Kit (manufactured by Quiagen) and eluted with 20 μl of sterile water (hereinafter, the method was used for the purification of DNA fragments from agarose gel). Into a plasmid pCR2.1, 4 μl of the amplified fragment was employed to insert in accordance with the instructions attached to TOPO TA Cloning Kit (manufactured by Invitrogen), and *E coli* DH5α was transformed with the reaction solution by the method of Cohen et al. [Proc. Natl. Acad. Sci. USA, 69, 2110 (1972)] (hereinafter, the method was used for the transformation of *E. coli*). Plasmid DNA was isolated in accordance with a known method *[*Nucleic Acids Research, 7, 1513 (1979)] (hereinafter, the method was used for the isolation of plasmid) from the obtained several kanamycin-resistant colonies to obtain 2 clones into which FX cDNA partial fragments were respectively inserted. They are referred to as pCRFX clone 8 and pCRFX clone 12.

The nucleotide sequence of the cDNA inserted into each of the FX clone 8 and FX clone 12 was determined using DNA Sequencer 377 (manufactured by Parkin Elmer) and BigDye Terminator Cycle Sequencing FS Ready Reaction kit (manufactured by Parkin Elmer) in accordance with the method of the manufacture's instructions. It was confirmed that each of the inserted cDNA whose sequence was determined encodes open reading frame (ORF) partial sequence of the Chinese hamster FX.

(4) Synthesis of single-stranded cDNA for RACE

Single-stranded cDNA samples for 5' and 3' RACE were prepared from the CHO/DG44 total RNA extracted in the item (1) using SMART™ RACE cDNA Amplification Kit (manufactured by CLONTECH) in accordance with the manufacture's instructions. In the case, PowerScript™ Reverse Transcriptase (manufactured by CLONTECH) was used as the reverse transcriptase. Each single-stranded cDNA after the preparation was diluted 10 folds with the Tricin-EDTA buffer attached to the kit and used as the template of PCR.

(5) Determination of Chinese hamster-derived FX full length cDNA by RACE method

Based on the FX partial sequence derived from Chinese hamster determined in the item (3), primers FXGSP1-1 (SEQ ID NO:44) and FXGSP1-2 (SEQ ID NO:45) for the Chinese hamster FX-specific 5' RACE and primers FXGSP2-1 (SEQ ID NO:46) and FXGSP2-2 (SEQ ID NO:47) for the Chinese hamster FX-specific 3' RACE were designed.

Next, polymerase chain reaction (PCR) was carried out using Advantage2 PCR Kit (manufactured by CLONTECH), by preparing 50 μl of a reaction solution [Advantage2 PCR buffer (manufactured by CLONTECH), 0.2 mM dNTPs, 0.2 μmol/l Chinese hamster FX-specific primers for RACE and 1 × concentration of common primers (manufactured by CLONTECH)] containing 1 μl of the CHO/DG44-derived single-stranded cDNA for RACE prepared in the

item (4).

The PCR was carried out by repeating 20 cycles of heating at 94°C for 5 seconds, 68°C for 10 seconds and 72°C for 2 minutes as one cycle.

After completion of the reaction, 1 μl of the reaction solution was diluted 50-folds with the Tricin-EDTA buffer, and 1 μl of the diluted solution was used as a template. The reaction solution was again prepared and the PCR was carried out under the same conditions. The templates, the combination of primers used in the first and second PCRs and the length of amplified DNA fragments by the PCRs are shown in Table 8.

Table 8

Combination of primers used in Chinese hamster FX cDNA RACE PCR and the size of PCR products

| 5' RACE | FX-specific primers | Common primers | PCR-amplified product size |
|---|---|---|---|
| First | FXGSP1-1 | UPM (Universal primer mix) | |
| Second | FXGSP1-2 | NUP (Nested Universal primer) | 300 bp |

| 3' RACE | FX-specific primers | Common primers | PCR-amplified product size |
|---|---|---|---|
| First | FXGSP2-1 | UPM (Universal primer mix) | |
| Second | FXGSP2-2 | NUP (Nested Universal primer) | 1,100 bp |

After the PCR, the reaction solution was subjected to 1% agarose gel electrophoresis, and the specific amplified fragment of interest was purified using QiaexII Gel Extraction Kit (manufactured by Qiagen) and eluted with 20 μl of sterile water. Into a plasmid pCR2.1, 4 μl of the amplified fragment was inserted, and *E. coli* DH5α was transformed using the reaction solution in accordance with the instructions attached to TOPO TA Cloning Kit (manufactured by Invitrogen).

Plasmid DNAs were isolated from the appeared several kanamycin-resistant colonies to obtain 5 cDNA clones containing Chinese hamster FX 5' region. They are referred to as FX5' clone 25, FX5' clone 26, FX5' clone 27, FX5' clone 28, FX5' clone 31 and FX5' clone 32.

In the same manner, 5 cDNA clones containing Chinese hamster FX 3' region were obtained. These FX3' clones are referred to as FX3' clone 1, FX3' clone 3, FX3' clone 6, FX3' clone 8 and FX3' clone 9.

The nucleotide sequence of the cDNA moiety of each of the clones obtained by the 5' and 3' RACE was determined using DNA Sequencer 377 (manufactured by Parkin Elmer) in accordance with the method described in the manufacture's instructions. By comparing the cDNA nucleotide sequences determined by the method, reading errors of nucleotide bases due to PCR were excluded and the full length nucleotide sequence of Chinese hamster FX cDNA was determined. The determined sequence is shown in SEQ ID NO:48.

2. Determination of CHO cell-derived GFPP cDNA sequence

(1) Preparation of GFPP cDNA partial fragment derived from Chinese hamster

GFPP cDNA partial fragment derived from Chinese hamster was prepared by the following procedure.

First, nucleotide sequences of a human GFPP cDNA (Genebank Accession No. number AF017445), mouse EST sequences having high homology with the sequence (Genebank Accession Nos. AI467195, AA422658, BE304325 and AI466474) and rat EST sequences (Genebank Accession Nos. BF546372, AI058400 and AW144783), registered at public data bases, were compared, and primers GFPP FW9 and GFPP RV9 (SEQ ID NOs:49 and 50) specific for rat GFPP were designed on a highly preserved region among these three species.

Next, polymerase chain reaction (PCR) was carried out using a DNA polymerase ExTaq (manufactured by Takara Shuzo), by preparing 25 μl of a reaction solution [ExTaq buffer (manufactured by Takara Shuzo), 0.2 mM dNTPs and 0.5 μmol/l GFPP-specific primers GFPP FW9 and GFPP RV9 (SEQ ID NOs:49 and 50)] containing 1 μl of the CHO/DG44-derived single-stranded cDNA prepared in the item 1(2). The PCR was carried out by heating at 94°C for 5 minutes, subsequent 30 cycles of heating at 94°C for 1 minute, 58°C for 2 minutes and 72°C for 3 minutes as one cycle, and final heating at 72°C for 10 minutes.

After the PCR, the reaction solution was subjected to 2% agarose gel electrophoresis, and a specific amplified fragment of 1.4 Kbp was purified using QuiaexII Gel Extraction Kit (manufactured by Quiagen) and eluted with 20 μl of sterile water. Into a plasmid pCR2.1, 4 μl of the amplified fragment was employed to insert in accordance with the instructions attached to TOPO TA Cloning Kit (manufactured by Invitrogen), and *E. coli* DH5α was transformed using

the reaction solution.

Plasmid DNAs were isolated from the appeared several kanamycin-resistant colonies to obtain 3 clones into which GFPP cDNA partial fragments were respectively integrated. They are referred to as GFPP clone 8, GFPP clone 11 and GFPP clone 12.

The nucleotide sequence of the cDNA inserted into each of the GFPP clone 8, GFPP clone 11 and GFPP clone 12 was determined using DNA Sequencer 377 (manufactured by Parkin Elmer) and BigDye Terminator Cycle Sequencing FS Ready Reaction kit (manufactured by Parkin Elmer) in accordance with the method described in the manufacture's instructions. It was confirmed that the inserted cDNA whose sequence was determined encodes open reading frame (ORF) partial sequence of the Chinese hamster GFPP.

(2) Determination of Chinese hamster GFPP full length cDNA by RACE method

Based on the Chinese hamster FX partial sequence determined in the item 2(1), primers GFPP GSP1-1 (SEQ ID NO:52) and GFPP GSP1-2 (SEQ ID NO:53) for the Chinese hamster FX-specific 5' RACE and primers GFPP GSP2-1 (SEQ ID NO:54) and GFPP GSP2-2 (SEQ ID NO:55) for the Chinese hamster GFPP-specific 3' RACE were designed.

Next, polymerase chain reaction (PCR) was carried out using Advantage2 PCR Kit (manufactured by CLONTECH), by preparing 50 μl of a reaction solution [Advantage2 PCR buffer (manufactured by CLONTECH), 0.2 mM dNTPs, 0.2 μmol/l Chinese hamster GFPP-specific primers for RACE and 1 × concentration of common primers (manufactured by CLONTECH)] containing 1 μl of the CHO/DG44-derived single-stranded cDNA for RACE prepared in the item (4).

The PCR was carried out by repeating 20 cycles of heating at 94°C for 5 seconds, 68°C for 10 seconds and 72°C for 2 minutes as one cycle.

After completion of the reaction, 1 μl of the reaction solution was diluted 50-folds with the Tricin-EDTA buffer, and 1 μl of the diluted solution was used as a template. The reaction solution was again prepared and the PCR was carried out under the same conditions. The templates, the combination of primers used in the first and second PCRs and the size of amplified DNA fragments by the PCRs are shown in Table 9.

Table 9

| Combination of primers used in Chinese hamster GFPP cDNA RACE PCR and the size of PCR products | | | |
|---|---|---|---|
| 5' RACE | GFPP-specific primers | Common primers | PCR-amplified product size |
| First | GFPPGSP1-1 | UPM (Universal primer mix) | |
| Second | GFPPGSP1-2 | NUP (Nested Universal primer) | 1,100 bp |
| 3' RACE | GFPP-specific primers | Common primers | PCR-amplified product size |
| First | GFPPGSP2-1 | UPM (Universal primer mix) | |
| Second | GFPPGSP2-2 | NUP (Nested Universal primer) | 1,400 bp |

After the PCR, the reaction solution was subjected to 1% agarose gel electrophoresis, and the specific amplified fragment of interest was purified using QiaexII Gel Extraction Kit (manufactured by Qiagen) and eluted with 20 μl of sterile water. Into a plasmid pCR2.1, 4 μl of the amplified fragment was employed to insert and *E. coli* DH5α was transformed with the reaction solution in accordance with the instructions attached to TOPO TA Cloning Kit (manufactured by Invitrogen).

Plasmid DNAs were isolated from the obtained several kanamycin-resistant colonies to obtain 4 cDNA clones containing Chinese hamster GFPP 5' region. They are referred to as GFPP5' clone 1, GFPP5' clone 2, GFPP5' clone 3 and GFPP5' clone 4.

In the same manner, 5 cDNA clones containing Chinese hamster GFPP 3' region were obtained. They are referred to as GFPP3' clone 10, GFPP3' clone 16 and GFPP3' clone 20.

The nucleotide sequence of the cDNA of each of the clones obtained by the 5' and 3' RACE was determined using DNA Sequencer 377 (manufactured by Parkin Elmer) in accordance with the method described in the manufacture's instructions. By comparing the cDNA nucleotide sequences after the nucleotide sequence determination, reading errors of bases due to PCR were excluded and the full length nucleotide sequence of Chinese hamster GFPP cDNA was determined. The determined sequence is shown in SEQ ID NO:51.

Example 17

Preparation of CHO cell-derived GMD gene:

1. Determination of CHO cell-derived GMD cDNA sequence

(1) Preparation of CHO cell-derived GMD gene cDNA (preparation of partial cDNA excluding 5'- and 3'-terminal sequences)

Rodents-derived GMD cDNA was searched in a public data base (BLAST) using a human-derived GMD cDNA sequence (GenBank Accession No. AF042377) registered at GenBank as a query, and three kinds of mouse EST sequences were obtained (GenBank Accession Nos. BE986856, BF158988 and BE284785). By ligating these EST sequences, a deduced mouse GMD cDNA sequence was determined.

On the base of the mouse-derived GMD cDNA sequence, a 28 mer primer having the sequence represented by SEQ ID NO:56, a 27 mer primer having the sequence represented by SEQ ID NO:57, a 25 mer primer having the sequence represented by SEQ ID NO:58, a 24 mer primer having the sequence represented by SEQ ID NO:59 and a 25 mer primer having the sequence represented by SEQ ID NO:60 were generated.

Next, in order to amplify the CHO cell-derived GMD cDNA, PCR was carried out by the following method. A 20 μl portion of a reaction solution [1 × Ex Taq buffer (manufactured by Takara Shuzo), 0.2 mM dNTPs, 0.5 unit of Ex Taq polymerase (manufactured by Takara Shuzo) and 0.5 μM of two synthetic DNA primers] containing 0.5 μl of the CHO cell-derived single-stranded cDNA prepared in Example 15-1(1) as the template was prepared. In this case, combinations of SEQ ID NO:56 with SEQ ID NO:57, SEQ ID NO:58 with SEQ ID NO:57, SEQ ID NO:56 with SEQ ID NO:59 and SEQ ID NO:56 with SEQ ID NO:60 were used as the synthetic DNA primers. The reaction was carried out using DNA Thermal Cycler 480 (manufactured by Perkin Elmer) by heating at 94°C for 5 minutes and subsequent 30 cycles of heating at 94°C for 1 minute and 68°C for 2 minutes as one cycle.

The PCR reaction solution was fractionated by agarose electrophoresis to find that a DNA fragment of about 1.2 kbp was amplified in the PCR product when synthetic DNA primers of SEQ ID NOs:56 and 57 were used, a fragment of about 1.1 kbp was amplified in the PCR product when synthetic DNA primers of SEQ ID NOs:57 and 59 were used, a fragment of about 350 bp was amplified in the PCR product when synthetic DNA primers of SEQ ID NOs:56 and 59 were used and a fragment of about 1 kbp was amplified in the PCR product when synthetic DNA primers of SEQ ID NOs:56 and 60 were used. The DNA fragments were recovered using Gene Clean II Kit (manufactured by BIO 101) in accordance with the manufacture's instructions. The recovered DNA fragments were ligated to a pT7Blue(R) vector (manufactured by Novagen) using DNA Ligation Kit (manufactured by Takara Shuzo), and *E. coli* DH (manufactured by Toyobo) was transformed using the obtained recombinant plasmid DNA samples to thereby obtain plasmids 22-8 (having a DNA fragment of about 1.2 kbp amplified from synthetic DNA primers of SEQ ID NO:56 and SEQ ID NO:57), 23-3 (having a DNA fragment of about 1.1 kbp amplified from synthetic DNA primers of SEQ ID NO:58 and SEQ ID NO:57), 31-5 (a DNA fragment of about 350 bp amplified from synthetic DNA primers of SEQ ID NO:56 and SEQ ID NO:59) and 34-2 (having a DNA fragment of about 1 kbp amplified from synthetic DNA primers of SEQ ID NO:56 and SEQ ID NO:60). The CHO cell-derived GMD cDNA sequence contained in these plasmids was determined in the usual way using a DNA sequencer ABI PRISM 377 (manufactured by Parkin Elmer) (since a sequence of 28 bases in downstream of the initiation codon methionine in the 5'-terminal side and a sequence of 27 bases in upstream of the termination codon in the 3'-terminal side are originated from synthetic oligo DNA sequences, they are mouse GMD cDNA sequences).

In addition, the following steps were carried out in order to prepare a plasmid in which the CHO cell-derived GMD cDNA fragments contained in the plasmids 22-8 and 34-2 are combined. The plasmid 22-8 (1 μg) was allowed to react with a restriction enzyme *Eco*RI (manufactured by Takara Shuzo) at 37°C for 16 hours, the digest was subjected to agarose electrophoresis and then a DNA fragment of about 4 kbp was recovered using Gene Clean II Kit (manufactured by BIO 101) in accordance with the manufacture's instructions. The plasmid 34-2 (2 μg) was allowed to react with a restriction enzyme *Eco*RI at 37°C for 16 hours, the digest was subjected to agarose electrophoresis and then a DNA fragment of about 150 bp was recovered using Gene Clean II Kit (manufactured by BIO 101) in accordance with the manufacture's instructions. The recovered DNA fragments were respectively subjected to terminal dephosphorylation using Calf Intestine Alkaline Phosphatase (manufactured by Takara Shuzo) and then ligated using DNA Ligation Kit (manufactured by Takara Shuzo), and *E. coli* DH5α (manufactured by Toyobo) was transformed using the obtained recombinant plasmid DNA to obtain a plasmid CHO-GMD (cf. Fig. 54).

(2) Determination of 5'-terminal sequence of CHO cell-derived GMD cDNA

A 24 mer primer having the nucleotide sequence represented by SEQ ID NO:61 was prepared from 5'-terminal side non-coding region nucleotide sequences of CHO cell-derived human and mouse GMD cDNA, and a 32 mer primer

having the nucleotide sequence represented by SEQ ID NO:62 from CHO cell-derived GMD cDNA sequence were prepared, and PCR was carried out by the following method to amplify cDNA. Then, 20 μl of a reaction solution [1 × Ex Taq buffer (manufactured by Takara Shuzo), 0.2 mM dNTPs, 0.5 unit of Ex Taq polymerase (manufactured by Takara Shuzo) and 0.5 μM of the synthetic DNA primers of SEQ ID NO:61 and SEQ ID NO:62] containing 0.5 μl of the single-stranded cDNA prepared in Example 15-1(1) was prepared as the template, and the reaction was carried out therein using DNA Thermal Cycler 480 (manufactured by Perkin Elmer) by heating at 94°C for 5 minutes, subsequent 20 cycles of heating at 94°C for 1 minute, 55°C for 1 minute and 72°C for 2 minutes as one cycle and further 18 cycles of heating at 94°C for 1 minute and 68°C for 2 minutes as one cycle. After fractionation of the PCR reaction solution by agarose electrophoresis, a DNA fragment of about 300 bp was recovered using Gene Clean II Kit (manufactured by BIO 101) in accordance with the manufacture's instructions. The recovered DNA fragment was ligated to a pT7Blue(R) vector (manufactured by Novagen) using DNA Ligation Kit (manufactured by Takara Shuzo), and *E. coli* DH5α (manufactured by Toyobo) was transformed using the obtained recombinant plasmid DNA samples to thereby obtain a plasmid 5'GMD. Using DNA Sequencer 377 (manufactured by Parkin Elmer), the nucleotide sequence of 28 bases in downstream of the initiation methionine of CHO cell-derived GMD cDNA contained in the plasmid was determined.

(3) Determination of 3'-terminal sequence of CHO cell-derived GMD cDNA

In order to obtain 3'-terminal cDNA sequence of CHO cell-derived GMD, RACE method was carried out by the following method. A single-stranded cDNA for 3' RACE was prepared from the CHO cell-derived RNA obtained in Example 15-1(1) using SMART™ RACE cDNA Amplification Kit (manufactured by CLONTECH) in accordance with the manufacture's instructions. In the case, PowerScript™ Reverse Transcriptase (manufactured by CLONTECH) was used as the reverse transcriptase. The single-stranded cDNA after the preparation was diluted 10 folds with the Tricin-EDTA buffer attached to the kit and used as the template of PCR.

Next, 20 μl of a reaction solution [ExTaq buffer (manufactured by Takara Shuzo), 0.2 mM dNTPs, 0.5 unit of EX Taq polymerase (manufactured by Takara Shuzo), 0.5 μM of the 25 mer synthetic DNA primer shown in SEQ ID NO:63 [generated on the base of the CHO cell-derived GMD cDNA sequence determined in the item (1)] and 1 × concentration of Universal Primer Mix (attached to SMART™ RACE cDNA Amplification Kit; manufactured by CLONTECH] containing 1 μl of the cDNA for 3' RACE as the template was prepared, and PCR was carried out using DNA Thermal Cycler 480 (manufactured by Perkin Elmer) by heating at 94°C for 5 minutes and subsequent 30 cycles of heating at 94°C for 1 minute and 68°C for 2 minutes as one cycle.

After completion of the reaction, 1 μl of the PCR reaction solution was diluted 20 folds with Tricin-EDTA buffer (manufactured by CLONTECH). Then, 20 μl of a reaction solution [ExTaq buffer (manufactured by Takara Shuzo), 0.2 mM dNTPs, 0.5 unit of EX Taq polymerase (manufactured by Takara Shuzo), 0.5 μM of the 25 mer synthetic DNA primer shown in SEQ ID NO:64 [generated on the base of the CHO cell-derived GMD cDNA sequence determined in the item (1)] and 0.5 μM of Nested Universal Primer (attached to SMART™ RACE cDNA Amplification Kit; manufactured by CLONTECH) containing 1 μl of the 20 folds-diluted aqueous solution as the template] was prepared, and the reaction was carried out using DNA Thermal Cycler 480 (manufactured by Perkin Elmer) by heating at 94°C for 5 minutes and subsequent 30 cycles at 94°C for 1 minute and 68°C for 2 minutes as one cycle.

After completion of the reaction, the PCR reaction solution was fractionated by agarose electrophoresis and then a DNA fragment of about 700 bp was recovered using Gene Clean II Kit (manufactured by BIO 101) in accordance with the manufacture's instructions. The recovered DNA fragment was ligated to a pT7Blue(R) vector (manufactured by Novagen) using DNA Ligation Kit (manufactured by Takara Shuzo), and *E. coli* DH5α (manufactured by Toyobo) was transformed using the obtained recombinant plasmid DNA, thereby obtaining a plasmid 3'GMD. Using DNA Sequencer 377 (manufactured by Parkin Elmer), the nucleotide sequence of 27 bases in upstream of the termination codon of CHO cell-derived GMD cDNA contained in the plasmid was determined.

The full length cDNA sequence of the CHO-derived GMD gene determined by the items (1), (2) and (3) and the corresponding amino acid sequence are shown in SEQ ID NOs:65 and 71, respectively.

2. Determination of genomic sequence containing CHO/DG44-derived cell GMD gene

A 25 mer primer having the nucleotide sequence represented by SEQ ID NO:66 was prepared from the mouse GMD cDNA sequence determined in Example 17-1. Next, a CHO cell-derived genome DNA was obtained by the following method. A CHO/DG44 cell-derived KC861 was suspended in IMDM-dFBS(10)-HT(1) medium [IMDM-dFBS(10) medium comprising 1 × concentration of HT supplement (manufactured by Invitrogen)] to give a density of 3 × $10^5$ cells/ml, and the suspension was dispensed at 2 ml/well into a 6 well flat bottom plate for adhesion cell use (manufactured by Greiner). After culturing them at 37°C in a 5% $CO_2$ incubator until the cells became confluent on the plate, genome DNA was prepared from the cells on the plate by a known method [Nucleic Acids Research, 3, 2303 (1976)] and dissolved overnight in 150 μl of TE-RNase buffer (pH 8.0) (10 mmol/l Tris-HCl, 1 mmol/l EDTA, 200 μg/ml RNase A).

A reaction solution (20 μl) [1 × Ex Taq buffer (manufactured by Takara Shuzo), 0.2 mM dNTPs, 0.5 unit of EX Taq polymerase (manufactured by Takara Shuzo) and 0.5 μM of synthetic DNA primers of SEQ ID NO:59 and SEQ ID NO:66] containing 100 ng of the obtained CHO/DG44 cell-derived genome DNA was prepared, and PCR was carried out using DNA Thermal Cycler 480 (manufactured by Perkin Elmer) by heating at 94°C for 5 minutes and subsequent 30 cycles of heating at 94°C for 1 minute and 68°C for 2 minutes as one cycle. After completion of the reaction, the PCR reaction solution was fractionated by agarose electrophoresis and then a DNA fragment of about 100 bp was recovered using Gene Clean II Kit (manufactured by BIO 101) in accordance with the manufacture's instructions. The recovered DNA fragment was ligated to a pT7Blue(R) vector (manufactured by Novagen) using DNA Ligation Kit (manufactured by Takara Shuzo), and *E. coli* DH5α (manufactured by Toyobo) was transformed using the obtained recombinant plasmid DNA, thereby obtaining a plasmid ex3. Using DNA Sequencer 377 (manufactured by Parkin Elmer), the nucleotide sequence of CHO cell-derived genome DNA contained in the plasmid was determined. The result is shown in SEQ ID NO:67.

Next, a 25 mer primer having the nucleotide sequence represented by SEQ ID NO:68 and a 25 mer primer having the nucleotide sequence represented by SEQ ID NO:69 were generated on the base of the CHO cell-derived GMD cDNA sequence determined in Example 17-1. Next, 20 μl of a reaction solution [1 × Ex Taq buffer (manufactured by Takara Shuzo), 0.2 mM dNTPs, 0.5 unit of EX Taq polymerase (manufactured by Takara Shuzo) and 0.5 μM of the synthetic DNA primers of SEQ ID NO:68 and SEQ ID NO:69] containing 100 ng of the CHO/DG44-derived genome DNA was prepared, and PCR was carried out using DNA Thermal Cycler 480 (manufactured by Perkin Elmer) by heating at 94°C for 5 minutes and subsequent 30 cycles of heating at 94°C for 1 minute and 68°C for 2 minutes as one cycle.

After completion of the reaction, the PCR reaction solution was fractionated by agarose electrophoresis and then a DNA fragment of about 200 bp was recovered using Gene Clean II Kit (manufactured by BIO 101) in accordance with the manufacture's instructions. The recovered DNA fragment was ligated to a pT7Blue(R) vector (manufactured by Novagen) using DNA Ligation Kit (manufactured by Takara Shuzo), and *E. coli* DH5α (manufactured by Toyobo) was transformed using the obtained recombinant plasmid DNA, thereby obtaining a plasmid ex4. Using DNA Sequencer 377 (manufactured by Parkin Elmer), the nucleotide sequence of CHO cell-derived genome DNA contained in the plasmid was determined. The result is shown in SEQ ID NO: 70.

Example 18

Sugar chain analysis of conventionally available antibodies:

Sugar chains binding to a conventionally available anti-HER2/neu antibody Herceptin (manufactured by GENENTECH and Roche) produced by CHO cell as the host cell was analyzed in accordance with the method of Example 10(6) (Fig. 31). When calculated from each peak area of elution diagram, the content of α-1,6-fucose-free sugar chains of Herceptin was 16%, and the content of α-1,6-fucose-bound sugar chains was 84%. The same analysis was carried out on other commercially available antibodies, Rituxan (manufactured by GENENTECH, Roche and IDEC) and Zenapax (manufactured by Roche and PDL), and the α-1,6-fucose-free sugar chain content of was less than that in Herceptin.

Fig. 31 is a graph showing elution pattern of PA-treated sugar chains prepared from Herceptin, obtained by analyzing them by reverse phase HPLC. The relative fluorescence intensity and the elution time are plotted as the ordinate and the abscissa, respectively. The reverse phase HPLC analysis conditions, sugar chain structure analysis and calculation of the ratio of sugar chain group containing no α-1,6-fucose sugar chain were carried out by the same methods of Example 11(6).

## INDUSTRIAL APPLICABILITY

The present invention provides a cell capable of producing an antibody composition and, a process for producing an antibody composition using the cell. Free Text of Sequence Listing

SEQ ID NO:4 - Explanation of synthetic sequence: Synthetic DNA
SEQ ID NO:5 - Explanation of synthetic sequence: Synthetic DNA
SEQ ID NO:8 - Explanation of synthetic sequence: Synthetic DNA
SEQ ID NO:9 - Explanation of synthetic sequence: Synthetic DNA
SEQ ID NO:10 - Explanation of synthetic sequence: Synthetic DNA
SEQ ID NO:11 - Explanation of synthetic sequence: Synthetic DNA
SEQ ID NO:12 - Explanation of synthetic sequence: Synthetic DNA
SEQ ID NO: 13 - Explanation of synthetic sequence: Synthetic DNA
SEQ ID NO: 14 - Explanation of synthetic sequence: Synthetic DNA
SEQ ID NO:15 - Explanation of synthetic sequence: Synthetic DNA

SEQ ID NO: 16 - Explanation of synthetic sequence: Synthetic DNA
SEQ ID NO:17 - Explanation of synthetic sequence: Synthetic DNA
SEQ ID NO: 18 - Explanation of synthetic sequence: Synthetic DNA
SEQ ID NO:19 - Explanation of synthetic sequence: Synthetic DNA
SEQ ID NO:20 - Explanation of synthetic sequence: Synthetic DNA
SEQ ID NO:21 - Explanation of synthetic sequence: Synthetic DNA
SEQ ID NO:22 - Explanation of synthetic sequence: Synthetic DNA
SEQ ID NO:26 - Explanation of synthetic sequence: Synthetic DNA
SEQ ID NO:27 - Explanation of synthetic sequence: Synthetic DNA
SEQ ID NO:28 - Explanation of synthetic sequence: Synthetic DNA
SEQ ID NO:29 - Explanation of synthetic sequence: Synthetic DNA
SEQ ID NO:30 - Explanation of synthetic sequence: Synthetic DNA
SEQ ID NO:31 - Explanation of synthetic sequence: Synthetic DNA
SEQ ID NO:32 - Explanation of synthetic sequence: Synthetic DNA
SEQ ID NO:33 - Explanation of synthetic sequence: Synthetic DNA
SEQ ID NO:34 - Explanation of synthetic sequence: Synthetic DNA
SEQ ID NO:35 - Explanation of synthetic sequence: Synthetic DNA
SEQ ID NO:36 - Explanation of synthetic sequence: Synthetic DNA
SEQ ID NO:37 - Explanation of synthetic sequence: Synthetic DNA
SEQ ID NO:38 - Explanation of synthetic sequence: Synthetic DNA
SEQ ID NO:39 - Explanation of synthetic sequence: Synthetic DNA
SEQ ID NO:40 - Explanation of synthetic sequence: Synthetic DNA
SEQ ID NO:41 - Explanation of synthetic sequence: Synthetic DNA
SEQ ID NO:42 - Explanation of synthetic sequence: Synthetic DNA
SEQ ID NO:43 - Explanation of synthetic sequence: Synthetic DNA
SEQ ID NO:44 - Explanation of synthetic sequence: Synthetic DNA
SEQ ID NO:45 - Explanation of synthetic sequence: Synthetic DNA
SEQ ID NO:46 - Explanation of synthetic sequence: Synthetic DNA
SEQ ID NO:47 - Explanation of synthetic sequence: Synthetic DNA
SEQ ID NO:49 - Explanation of synthetic sequence: Synthetic DNA
SEQ ID NO:50 - Explanation of synthetic sequence: Synthetic DNA
SEQ ID NO:52 - Explanation of synthetic sequence: Synthetic DNA
SEQ ID NO:53 - Explanation of synthetic sequence: Synthetic DNA
SEQ ID NO:54 - Explanation of synthetic sequence: Synthetic DNA
SEQ ID NO:55 - Explanation of synthetic sequence: Synthetic DNA
SEQ ID NO:56 - Explanation of synthetic sequence: Synthetic DNA
SEQ ID NO:57 - Explanation of synthetic sequence: Synthetic DNA
SEQ ID NO:58 - Explanation of synthetic sequence: Synthetic DNA
SEQ ID NO:59 - Explanation of synthetic sequence: Synthetic DNA
SEQ ID NO:60 - Explanation of synthetic sequence: Synthetic DNA
SEQ ID NO:61 - Explanation of synthetic sequence: Synthetic DNA
SEQ ID NO:62 - Explanation of synthetic sequence: Synthetic DNA
SEQ ID NO:63 - Explanation of synthetic sequence: Synthetic DNA
SEQ ID NO:64 - Explanation of synthetic sequence: Synthetic DNA
SEQ ID NO:66 - Explanation of synthetic sequence: Synthetic DNA
SEQ ID NO:68 - Explanation of synthetic sequence: Synthetic DNA
SEQ ID NO:69 - Explanation of synthetic sequence: Synthetic DNA

## Claims

1. A cell in which

   (i) the activity of an enzyme relating to the synthesis of GDP-fucose is deleted, or
   (ii) the activity of an enzyme relating to the synthesis of GDP-fucose and the activity of α1,6-fucosyltransferase are deleted,

   by a genetic engineering technique, wherein said enzyme relating to the synthesis of GDP-fucose is an enzyme selected from the group consisting of the following (a), (b) and (c) :

   (a) GMD (GDP-mannose 4,6-dehydratase);
   (b) Fx (GDP-keto-6-deoxymannose 3,5-epimerase, 4-reductase);
   (c) GFPP (GDP-beta-L-fucose pyrophosphorylase),

   and wherein the genetic engineering technique is a technique selected from the group consisting of the following (A), (B), (C) and (D):

   (A) a gene disruption technique targeting a gene encoding the enzyme;
   (B) a technique for introducing a dominant negative mutant of a gene encoding the enzyme;
   (C) a technique for introducing mutation into the enzyme;
   (D) a technique for inhibiting transcription and/or translation of a gene encoding the enzyme.

2. A transgenic non-human animal or plant or the progenies thereof, comprising a genome which is modified such that the activity of an enzyme relating to the synthesis of GDP-fucose and the activity of α1,6-fucosyltransferase are

decreased,
by a genetic engineering technique, wherein said enzyme relating to the synthesis of GDP-fucose is an enzyme selected from the group consisting of the following (a), (b) and (c):

(a) GMD (GDP-mannose 4,6-dehydratase);
(b) Fx (GDP-keto-6-deoxymannose 3,5-epimerase, 4-reductase);
(c) GFPP (GDP-beta-L-fucose pyrophosphorylase),

and wherein the genetic engineering technique is a technique selected from the group consisting of the following (A), (B), (C) and (D):

(A) a gene disruption technique targeting a gene encoding the enzyme;
(B) a technique for introducing a dominant negative mutant of a gene encoding the enzyme;
(C) a technique for introducing mutation into the enzyme;
(D) a technique for inhibiting transcription and/or translation of a gene encoding the enzyme

wherein in said transgenic non-human animal or plant or progenies thereof a gene encoding an antibody has been introduced and the transgenic non-human animal or plant or progenies thereof is capable of producing an antibody composition in which fucose is not bound to N-acetylglucosamine at the reducing end of the sugar chain in 20% or more among the total complex N-glycoside-linked sugar chains binding to the Fc region contained in the antibody composition.

**3.** The transgenic non-human animal or plant or the progenies thereof according to claim 2, comprising a genome which is modified such that a gene encoding an enzyme relating to the synthesis of GDP-fucose or $\alpha$1,6-fucosyltransferase is knocked out.

**4.** The cell according to claim 1 or the transgenic non-human animal or plant or the progenies thereof according to claim 2 or 3, wherein the GMD is a protein encoded by a DNA of the following (a) or (b):

(a) a DNA comprising the nucleotide sequence represented by SEQ ID NO:65;
(b) a DNA which hybridizes with the DNA comprising the nucleotide sequence represented by SEQ ID NO:65 under stringent conditions and encodes a protein having GMD activity.

**5.** The cell according to claim 1 or the transgenic non-human animal or plant or the progenies thereof according to claim 2 or 3, wherein the GMD is a protein selected from the group consisting of the following (a), (b) and (c) :

(a) a protein comprising the amino acid sequence represented by SEQ ID NO:71;
(b) a protein which comprises an amino acid sequence in which 1 to 20 amino acids are deleted, substituted, inserted and/or added in the amino acid sequence represented by SEQ ID NO:71 and has GMD activity;
(c) a protein which comprises an amino acid sequence having a homology of at least 80 % with the amino acid sequence represented by SEQ ID NO:71 and has GMD activity.

**6.** The cell according to claim 1 or the transgenic non-human animal or plant or the progenies thereof according to claim 2 or 3, wherein the Fx is a protein encoded by a DNA of the following (a) or (b):

(a) a DNA comprising the nucleotide sequence represented by SEQ ID NO:48;
(b) a DNA which hybridizes with the DNA comprising the nucleotide sequence represented by SEQ ID NO:48 under stringent conditions and encodes a protein having Fx activity.

**7.** The cell according to claim 1 or the transgenic non-human animal or plant or the progenies thereof according to claim 2 or 3, wherein the Fx is a protein selected from the group consisting of the following (a), (b) and (c) :

(a) a protein comprising the amino acid sequence represented by SEQ ID NO:72;
(b) a protein which comprises an amino acid sequence in which 1 to 20 amino acids are deleted, substituted, inserted and/or added in the amino acid sequence represented by SEQ ID NO:72 and has Fx activity.
(c) a protein which comprises an amino acid sequence having a homology of at least 80 % with the amino acid sequence represented by SEQ ID NO:72 and has Fx activity.

8. The cell according to claim 1 or the transgenic non-human animal or plant or the progenies thereof according to claim 2 or 3, wherein the GFPP is a protein encoded by a DNA of the following (a) or (b):

(a) a DNA comprising the nucleotide sequence represented by SEQ ID NO:51;
(b) a DNA which hybridizes with the DNA comprising the nucleotide sequence represented by SEQ ID NO:51 under stringent conditions and encodes a protein having GFPP activity.

9. The cell according to claim 1 or the transgenic non-human animal or plant or the progenies thereof according to claim 2 or 3, wherein the GFPP is a protein selected from the group consisting of the following (a), (b) and (c) :

(a) a protein comprising the amino acid sequence represented by SEQ ID NO:73;
(b) a protein which comprises an amino acid sequence in which 1 to 20 amino acids are deleted, substituted, inserted and/or added in the amino acid sequence represented by SEQ ID NO:73 and has GFPP activity;
(c) a protein which comprises an amino acid sequence having a homology of at least 80 % with the amino acid sequence represented by SEQ ID NO:73 and has GFPP activity.

10. The cell, transgenic non-human animal or plant or the progenies thereof according to any one of claims 1 to 3, wherein the α1,6-fucosyltransferase is a protein encoded by a DNA selected from the group consisting of the following (a), (b), (c) and (d):

(a) a DNA comprising the nucleotide sequence represented by SEQ ID NO:1;
(b) a DNA comprising the nucelotide sequence represented by SEQ ID NO:2;
(c) a DNA which hybridizes with the DNA comprising the nucleotide sequence represented by SEQ ID NO:1 under stringent conditions and encodes a protein having α1,6-fucosyltransferase activity;
(d) a DNA which hybridizes with the DNA comprising the nucleotide sequence represented by SEQ ID NO:2 under stringent conditions and encodes a protein having α1,6-fucosyltransferase activity.

11. The cell, transgenic non-human animal or plant or the progenies thereof according to any one of claims 1 to 3, wherein the α1,6-fucosyltransferase is a protein selected from the group consisting of the following (a), (b), (c), (d), (e) and (f) :

(a) a protein comprising the amino acid sequence represented by SEQ ID:23;
(b) a protein comprising the amino acid sequence represented by SEQ ID:24;
(c) a protein which comprises an amino acid sequence in which 1 to 20 amino acids are deleted, substituted, inserted and/or added in the amino acid sequence represented by SEQ ID NO:23 and has a α1,6-fucosyltransferase activity;
(d) a protein which comprises an amino acid sequence in which 1 to 20 amino acids are deleted, substituted, inserted and/or added in the amino acid sequence represented by SEQ ID NO:24 and has a α1,6-fucosyltransferase activity;
(e) a protein which comprises an amino acid sequence having a homology of at least 80 % with the amino acid sequence represented by SEQ ID NO:23 and has a α1,6-fucosyltransferase activity;
(f) a protein which comprises an amino acid sequence having a homology of at least 80% with the amino acid sequence represented by SEQ ID NO:24 and has a α1,6-fucosyltransferase activity.

12. The cell according to any one of claims 1 and 4 to 11, which is resistant to at least a lectin which recognizes a sugar chain in which 1-position of fucose is bound to 6-position of N-acetylglucosamine in the reducing end through α-bond in the complex N-glycoside-linked sugar chain.

13. The cell according to any one of claims 1 and 4 to 12, which is a cell selected from the group consisting of the following (a) to (i):

(a) a CHO cell derived from a Chinese hamster ovary tissue;
(b) a rat myeloma cell line, YB2/3HL.P2.G11.16.AG.20 cell;
(c) a mouse myeloma cell line, NS0 cell;
(d) a mouse myeloma cell line, SP2/0-Ag14 cell;
(e) a BHK cell derived from a syrian hamster kidney tissue;
(f) an antibody-producing hybridoma cell;
(g) a human leukemia cell line Namalwa cell;

(h) a non-human embryonic stem cell;
(i) a non-human fertilized egg cell.

**14.** The cell according to any one of claims 1 or 4 to 13 into which a gene encoding an antibody molecule is introduced.

**15.** The cell according to claim 14, wherein the antibody molecule belongs to an IgG class.

**16.** A process for producing an antibody composition, which comprises culturing the cell according to claim 14 or 15 in a medium to produce and accumulate the antibody composition in the culture; and recovering the antibody composition from the culture.

**17.** The process according to claim 16, which produces an antibody composition having higher antibody-dependent cell-mediated cytotoxic activity than an antibody composition obtained from its parent cell line.

**18.** The transgenic non-human animal or plant or the progenies thereof according to any one of claims 2 to 11, wherein the transgenic non-human animal is an animal selected from the group consisting of cattle, sheep, goat, pig, horse, mouse, rat, fowl, monkey and rabbit.

**19.** A process for producing an antibody composition, which comprises
recovering an antibody composition from tissue or body fluid comprising the antibody, which tissue or body fluid has been isolated from a transgenic non-human animal or plant or the progenies thereof according to any one of claims 2 to 11 and 18, into which a gene encoding an antibody molecule has been introduced.

**20.** The process according to claim 19, wherein the antibody molecule belongs to an IgG class.

**21.** The process according to claim 19 or 20, which produces an antibody composition having higher antibody-dependent cell-mediated cytotoxic activity than an antibody composition obtained from a non-human animal or plant or the progenies thereof whose genome is not modified.

**22.** The cell according to any one of claims 1 or 4 to 15 or the transgenic non-human animal or plant or the progenies thereof according to any one of claims 2 to 11 or 18, wherein said technique according to (D) is targeting a gene of an enzyme relating to the synthesis of GDP-fucose or a gene of $\alpha$1,6-fucosyltransferase with an RNAi gene construct, wherein said enzyme relating to the synthesis of GDP-fucose is an enzyme selected from the group consisting of the following (a), (b) and (c):

(a) GMD (GDP-mannose 4,6-dehydratase);
(b) Fx (GDP-keto-6-deoxymannose 3,5-epimerase, 4-reductase);
(c) GFPP (GDP-beta-L-fucose pyrophosphorylase).

**Patentansprüche**

**1.** Zelle, in welcher mittels eines gentechnischen Verfahrens

(i) die Aktivität eines Enzyms, das in Zusammenhang mit der Synthese von GDP-Fucose steht, ausgeschaltet ist oder
(ii) die Aktivität eines Enzyms, das im Zusammenhang mit der Synthese von GDP-Fucose steht, und die Aktivität von $\alpha$1,6-Fucosyltransferase ausgeschaltet sind,

wobei das Enzym, das in Zusammenhang mit der Synthese von GDP-Fucose steht, ein Enzym ist, das ausgewählt wird aus der Gruppe, bestehend aus den folgenden (a), (b) und (c) :

(a) GMD (GDP-Mannose-4,6-Dehydratase);
(b) Fx (GDP-Keto-6-deoxymannose-3,5-Epimerase, 4-Reductase);
(c) GFPP (GDP-beta-L-Fucosepyrophosphorylase),

und wobei das gentechnische Verfahren ein Verfahren ist, das ausgewählt wird aus der Gruppe, bestehend aus den folgenden (A), (B), (C) und (D):

(A) einem Gendisruptionsverfahren, das auf ein Gen abzielt, welches das Enzym kodiert;
(B) einem Verfahren zum Einführen eines dominant negativen Mutanten eines Gens, das das Enzym kodiert;
(C) einem Verfahren zum Einführen einer Mutation in das Enzym;
(D) einem Verfahren zum Inhibieren der Transkription und/oder Translation eines Gens, das das Enzym kodiert.

**2.** Transgene(s) nicht-humane(s) Tier oder Pflanze oder dessen/deren Nachkommen, umfassend ein Genom, das mittels eines gentechnischen Verfahrens so modifiziert ist, dass die Aktivität eines Enzyms, das in Zusammenhang mit der Synthese von GDP-Fucose steht, und die Aktivität von $\alpha$1,6-Fucosyltransferase vermindert sind,
wobei das Enzym, das in Zusammenhang mit der Synthese von GDP-Fucose steht, ein Enzym ist, das ausgewählt wird aus der Gruppe, bestehend aus den folgenden (a), (b) und (c) :

(a) GMD (GDP-Mannose-4,6-Dehydratase);
(b) Fx (GDP-Keto-6-deoxymannose-3,5-Epimerase, 4-Reduktase);
(c) GFPP (GDP-beta-L-Fucosepyrophosphorylase),

und wobei das gentechnische Verfahren ein Verfahren ist, das ausgewählt wird aus der Gruppe, bestehend aus den folgenden (A), (B), (C) und (D):

(A) einem Gendisruptionsverfahren, das auf ein Gen abzielt, welches das Enzym kodiert;
(B) einem Verfahren zum Einführen eines dominant negativen Mutanten eines Gens, das das Enzym kodiert;
(C) einem Verfahren zum Einführen einer Mutation in das Enzym;
(D) einem Verfahren zum Inhibieren der Transkription und/oder Translation eines Gens, das das Enzym kodiert,

wobei ein Gen, das einen Antikörper kodiert, in das/die transgene nicht-humane Tier oder Pflanze oder dessen/deren Nachkommen eingeführt wurde und das/die transgene nicht-humane Tier oder Pflanze oder dessen/deren Nachkommen imstande ist/sind, eine Antikörperzusammensetzung zu produzieren, in der Fucose nicht gebunden ist an N-Acetylglucosamin am reduzierenden Ende der Zuckerkette bei 20 % oder mehr unter den gesamten komplexen N-Glycosid-gebundenen Zuckerketten, die an die in der Antikörperzusammensetzung enthaltene Fc-Region binden.

**3.** Transgene(s) nicht-humane(s) Tier oder Pflanze oder dessen/deren Nachkommen gemäß Anspruch 2, umfassend ein Genom, das so modifiziert ist, dass ein Gen, das ein Enzym, das in Zusammenhang mit der Synthese von GDP-Fucose steht, oder $\alpha$1,6-Fucosyltransferase kodiert, abgeschaltet [knocked out] ist.

**4.** Zelle gemäß Anspruch 1 oder transgene(s) nicht-humane(s) Tier oder Pflanze oder dessen/deren Nachkommen gemäß Anspruch 2 oder 3, wobei die GMD ein Protein ist, das durch eine DNA aus den folgenden (a) oder (b) kodiert wird:

(a) einer DNA, umfassend die durch SEQ ID NO: 65 dargestellte Nukleotidsequenz;
(b) einer DNA, die mit der DNA, umfassend die durch SEQ ID NO: 65 dargestellte Nukleotidsequenz, unter stringenten Bedingungen hybridisiert und ein Protein mit GMD-Aktivität kodiert.

**5.** Zelle gemäß Anspruch 1 oder transgene(s) nicht-humane(s) Tier oder Pflanze oder dessen/deren Nachkommen gemäß Anspruch 2 oder 3, wobei die GMD ein Protein ist, das ausgewählt wird aus der Gruppe, bestehend aus den folgenden (a), (b) und (c):

(a) einem Protein, umfassend die durch SEQ ID NO: 71 dargestellte Aminosäuresequenz;
(b) einem Protein, das eine Aminosäuresequenz, in welcher 1 bis 20 Aminosäuren in der/die durch SEQ ID NO: 71 dargestellte(n) Aminosäuresequenz gelöscht, substituiert, inseriert und/oder addiert sind, umfasst und GMD-Aktivität aufweist;
(c) einem Protein, das eine Aminosäuresequenz mit einer Homologie von wenigstens 80 % zu der durch SEQ ID NO: 71 dargestellten Aminosäuresequenz umfasst und GMD-Aktivität aufweist.

**6.** Zelle gemäß Anspruch 1 oder transgene(s) nicht-humane(s) Tier oder Pflanze oder dessen/deren Nachkommen gemäß Anspruch 2 oder 3, wobei die Fx ein Protein ist, das durch eine DNA aus den folgenden (a) oder (b) kodiert wird:

(a) einer DNA, umfassend die durch SEQ ID NO: 48 dargestellte Nukleotidsequenz;
(b) einer DNA, die mit der DNA, umfassend die durch SEQ ID NO: 48 dargestellte Nukleotidsequenz, unter stringenten Bedingungen hybridisiert und ein Protein mit Fx-Aktivität kodiert.

7. Zelle gemäß Anspruch 1 oder transgene(s) nicht-humane(s) Tier oder Pflanze oder dessen/deren Nachkommen gemäß Anspruch 2 oder 3, wobei die Fx ein Protein ist, das ausgewählt wird aus der Gruppe, bestehend aus den folgenden (a), (b) und (c):

(a) einem Protein, umfassend die durch SEQ ID NO: 72 dargestellte Aminosäuresequenz;
(b) einem Protein, das eine Aminosäuresequenz, in welcher 1 bis 20 Aminosäuren in der/die durch SEQ ID NO: 72 dargestellte(n) Aminosäuresequenz gelöscht, substituiert, inseriert und/oder addiert sind, umfasst und Fx-Aktivität aufweist;
(c) einem Protein, das eine Aminosäuresequenz mit einer Homologie von wenigstens 80 % zu der durch SEQ ID NO: 72 dargestellten Aminosäuresequenz umfasst und Fx-Aktivität aufweist.

8. Zelle gemäß Anspruch 1 oder transgene(s) nicht-humane(s) Tier oder Pflanze oder dessen/deren Nachkommen gemäß Anspruch 2 oder 3, wobei die GFPP ein Protein ist, das durch eine DNA aus den folgenden (a) oder (b) kodiert wird:

(a) einer DNA, umfassend die durch SEQ ID NO: 51 dargestellte Nukleotidsequenz;
(b) einer DNA, die mit der DNA, umfassend die durch SEQ ID NO: 51 dargestellte Nukleotidsequenz, unter stringenten Bedingungen hybridisiert und ein Protein mit GFPP-Aktivität kodiert.

9. Zelle gemäß Anspruch 1 oder transgene(s) nicht-humane(s) Tier oder Pflanze oder dessen/deren Nachkommen gemäß Anspruch 2 oder 3, wobei die GFPP ein Protein ist, das ausgewählt wird aus der Gruppe, bestehend aus den folgenden (a), (b) und (c):

(a) einem Protein, umfassend die durch SEQ ID NO: 73 dargestellte Aminosäuresequenz;
(b) einem Protein, das eine Aminosäuresequenz, in welcher 1 bis 20 Aminosäuren in der/die durch SEQ ID NO: 73 dargestellte(n) Aminosäuresequenz gelöscht, substituiert, inseriert und/oder addiert sind, umfasst und GFPP-Aktivität aufweist;
(c) einem Protein, das eine Aminosäuresequenz mit einer Homologie von wenigstens 80 % zu der durch SEQ ID NO: 73 dargestellten Aminosäuresequenz umfasst und GFPP-Aktivität aufweist.

10. Zelle, transgene(s) nicht-humane(s) Tier oder Pflanze oder dessen/deren Nachkommen gemäß einem der Ansprüche 1 bis 3, wobei die $\alpha$1,6-Fucosyltransferase ein Protein ist, das durch eine DNA, ausgewählt aus der Gruppe, bestehend aus den folgenden (a), (b), (c) und (d), kodiert wird:

(a) einer DNA, umfassend die durch SEQ ID NO: 1 dargestellte Nukleotidsequenz;
(b) einer DNA, umfassend die durch SEQ ID NO: 2 dargestellte Nukleotidsequenz;
(c) einer DNA, die mit der DNA, umfassend die durch SEQ ID NO: 1 dargestellte Nukleotidsequenz, unter stringenten Bedingungen hybridisiert und ein Protein mit $\alpha$1,-6-Fucosyltransferaseaktivität kodiert;
(d) einer DNA, die mit der DNA, umfassend die durch SEQ ID NO: 2 dargestellte Nukleotidsequenz, unter stringenten Bedingungen hybridisiert und ein Protein mit $\alpha$1,6-Fucosyltransferaseaktivität kodiert.

11. Zelle, transgene(s) nicht-humane(s) Tier oder Pflanze oder dessen/deren Nachkommen gemäß einem der Ansprüche 1 bis 3, wobei die $\alpha$1,6-Fucosyltransferase ein Protein ist, das ausgewählt wird aus der Gruppe, bestehend aus den folgenden (a), (b), (c), (d), (e) und (f) :

(a) einem Protein, umfassend die durch SEQ ID NO: 23 dargestellte Aminosäuresequenz;
(b) einem Protein, umfassend die durch SEQ ID NO: 24 dargestellte Aminosäuresequenz;
(c) einem Protein, das eine Aminosäuresequenz, in welcher 1 bis 20 Aminosäuren in der/die durch SEQ ID NO: 23 dargestellte(n) Aminosäuresequenz gelöscht, substituiert, inseriert und/oder addiert sind, umfasst und eine $\alpha$1,6-Fucosyltransferaseaktivität aufweist;
(d) einem Protein, das eine Aminosäuresequenz, in welcher 1 bis 20 Aminosäuren in der/die durch SEQ ID NO: 24 dargestellte(n) Aminosäuresequenz gelöscht, substituiert, inseriert und/oder addiert sind, umfasst und eine $\alpha$1,6-Fucosyltransferaseaktivität aufweist;
e) einem Protein, das eine Aminosäuresequenz mit einer Homologie von wenigstens 80 % zu der durch SEQ ID NO: 23 dargestellten Aminosäuresequenz umfasst und eine $\alpha$1,6-Fucosyltransferaseaktivität aufweist;
(f) einem Protein, das eine Aminosäuresequenz mit einer Homologie von wenigstens 80 % zu der durch SEQ ID NO: 24 dargestellten Aminosäuresequenz umfasst und eine $\alpha$1,6-Fucosyltransferaseaktivität aufweist.

**12.** Zelle gemäß einem der Ansprüche 1 und 4 bis 11, welche gegenüber wenigstens einem Lectin resistent ist, welches eine Zuckerkette erkennt, in der die 1-Position von Fucose an die 6-Position von N-Acetylglucosamin im reduzierenden Ende durch $\alpha$-Bindung in der komplexen N-Glycosid-verknüpften Zuckerkette gebunden ist.

**13.** Zelle gemäß einem der Ansprüche 1 und 4 bis 12, die eine Zelle, ausgewählt aus der Gruppe, bestehend aus den folgenden (a) bis (i), ist:

(a) einer CHO-Zelle, die von Ovariengewebe eines chinesischen Hamsters stammt;
(b) einer Ratten-Myelom-Zelllinie, YB2/3HL.P2.G11.16.AG.20-Zelle;
(c) einer Maus-Myelom-Zelllinie, NS0-Zelle;
(d) einer Maus-Myelom-Zelllinie, SP2/0-Ag14-Zelle;
(e) einer BHK-Zelle, die von Nierengewebe eines syrischen Hamsters stammt;
(f) einer Antikörper-produzierenden Hybridomzelle;
(g) einer Zelle der humanen Leukämie-Zelllinie Namalwa;
(h) einer nicht-humanen embryonalen Stammzelle;
(i) einer nicht-humanen befruchteten Eizelle.

**14.** Zelle gemäß einem der Ansprüche 1 oder 4 bis 13, in welcher ein Gen, das ein Antikörpermolekül kodiert, eingebracht ist.

**15.** Zelle gemäß Anspruch 14, wobei das Antikörpermolekül zu einer IgG-Klasse gehört.

**16.** Verfahren zur Herstellung einer Antikörperzusammensetzung, das das Kultivieren der Zelle gemäß Anspruch 14 oder 15 in einem Medium, so dass die Antikörperzusammensetzung in der Kultur produziert und akkumuliert wird; und die Gewinnung der Antikörperzusammensetzung aus der Kultur umfasst.

**17.** Verfahren gemäß Anspruch 16, das eine Antikörperzusammensetzung produziert, die eine höhere Antikörper-abhängige zellvermittelte cytotoxische Aktivität aufweist als eine aus ihrer Parental-Zelllinie gewonnenen Antikörperzusammensetzung.

**18.** Transgene(s) nicht-humane(s) Tier oder Pflanze oder dessen/deren Nachkommen gemäß einem der Ansprüche 2 bis 11, wobei das transgene nicht-humane Tier ein Tier ist, das ausgewählt wird aus der Gruppe, bestehend aus Rindern, Schafen, Ziege, Schwein, Pferd, Maus, Ratte, Geflügel, Affen und Kaninchen.

**19.** Verfahren zur Herstellung einer Antikörperzusammensetzung, umfassend
Gewinnung einer Antikörperzusammensetzung aus Gewebe- oder Körperflüssigkeit, die den Antikörper umfasst, wobei die Gewebe- oder Körperflüssigkeit aus einem(r) transgenen nicht-humanen Tier oder Pflanze oder dessen/deren Nachkommen gemäß einem der Ansprüche 2 bis 11 und 18 isoliert wurde, in das/die ein ein Antikörpermolekül-kodierendes Gen eingeführt wurde.

**20.** Verfahren gemäß Anspruch 19, wobei das Antikörpermolekül zu einer IgG-Klasse gehört.

**21.** Verfahren gemäß Anspruch 19 oder 20, welches eine Antikörperzusammensetzung produziert, die eine höhere Antikörper-abhängige zellvermittelte cytotoxische Aktivität aufweist als eine Antikörperzusammensetzung, die aus einem(r) nicht-humanen Tier oder Pflanze oder dessen/deren Nachkommen, dessen Genom nicht modifiziert ist, gewonnen wurde.

**22.** Zelle gemäß einem der Ansprüche 1 oder 4 bis 15 oder transgene(s) nicht-Humane(s) Tier oder Pflanze oder dessen/deren Nachkommen gemäß einem der Ansprüche 2 bis 11 oder 18, wobei das Verfahren gemäß (D) auf ein Gen eines Enzyms, das in Zusammenhang mit der Synthese von GDP-Fucose steht, oder ein Gen von $\alpha$1,6-Fucosyltransferase mittels eines RNAi-Genkonstrukts abzielt, wobei das Enzym, das in Zusammenhang mit der Synthese von GDP-Fucose steht, ein Enzym ist, das ausgewählt wird aus der Gruppe, bestehend aus den folgenden (a), (b) und (c):

(a) GMD (GDP-Mannose-4,6-Dehydratase);
(b) Fx (GDP-Keto-6-deoxymannose-3,5-Epimerase, 4-Reductase);
(c) GFPP (GDP-beta-L-Fucosepyrophosphorylase).

## Revendications

1. Cellule dans laquelle

   (i) l'activité d'une enzyme en rapport avec la synthèse du GDP-fucose est supprimée, ou
   (ii) l'activité d'une enzyme en rapport avec la synthèse du GDP-fucose et l'activité de la α1,6-fucosyltransférase sont supprimées,

   par une technique de génie génétique, dans laquelle ladite enzyme en rapport avec la synthèse du GDP-fucose est une enzyme choisie dans le groupe constitué par (a), (b) et (c) suivants :

   (a) GMD (GDP-mannose 4,6-déshydratase) ;
   (b) Fx (GDP-céto-6-désoxymannose 3,5-épimérase, 4-réductase) ;
   (c) GFPP (GDP-bêta-L-fucose pyrophosphorylase),

   et dans laquelle la technique de génie génétique est une technique choisie dans le groupe constitué par (A), (B), (C) et (D) suivants :

   (A) une technique de disruption génique ciblant un gène codant pour l'enzyme ;
   (B) une technique d'introduction d'un mutant négatif dominant d'un gène codant pour l'enzyme ;
   (C) une technique d'introduction d'une mutation dans l'enzyme ;
   (D) une technique d'inhibition de la transcription et/ou de traduction d'un gène codant pour l'enzyme.

2. Animal non humain ou plante transgénique ou leurs descendances, comprenant un génome qui est modifié de sorte que l'activité d'une enzyme en rapport avec la synthèse du GDP-fucose et l'activité de la α1,6-fucosyltransférase sont réduites,
   par une technique de génie génétique, dans laquelle ladite enzyme en rapport avec la synthèse du GDP-fucose est une enzyme choisie dans le groupe constitué par (a), (b) et (c) suivants :

   (a) GMD (GDP-mannose 4,6-déshydratase) ;
   (b) Fx (GDP-céto-6-désoxymannose 3,5-épimérase, 4-réductase) ;
   (c) GFPP (GDP-bêta-L-fucose pyrophosphorylase),

   et dans laquelle la technique de génie génétique est une technique choisie dans le groupe constitué par (A), (B), (C) et (D) suivants :

   (A) une technique de disruption génique ciblant un gène codant pour l'enzyme ;
   (B) une technique d'introduction d'un mutant négatif dominant d'un gène codant pour l'enzyme ;
   (C) une technique d'introduction d'une mutation dans l'enzyme ;
   (D) une technique d'inhibition de la transcription et/ou de traduction d'un gène codant pour l'enzyme

   dans laquelle dans ledit animal non humain ou ladite plante transgénique ou leurs descendances, un gène codant pour un anticorps est introduit et l'animal non humain ou la plante transgénique ou leurs descendances est/sont capable(s) de produire une composition d'anticorps dans laquelle le fucose n'est pas lié à la N-acétylglucosamine à l'extrémité de réduction de la chaîne de sucre à hauteur de 20 % ou plus dans les chaînes de sucre complexes totales liées au N-glycoside se liant à la région Fc contenue dans la composition d'anticorps.

3. Animal non humain ou plante transgénique ou leurs descendances selon la revendication 2, comprenant un génome qui est modifié de sorte qu'un gène codant pour une enzyme en rapport avec la synthèse du GDP-fucose ou la α1,6-fucosyltransférase est supprimé.

4. Cellule selon la revendication 1 ou animal non humain ou plante transgénique ou leurs descendances selon la revendication 2 ou 3, dans laquelle/lequel/lesquelles la GMD est une protéine codée par un ADN de (a) ou (b) suivant :

   (a) un ADN comprenant la séquence nucléotidique représentée par la SEQ ID N° 65 ;
   (b) un ADN qui s'hybride avec l'ADN comprenant la séquence nucléotidique représentée par la SEQ ID N° 65 dans des conditions rigoureuses et code pour une protéine ayant une activité de GMD.

**5.** Cellule selon la revendication 1 ou animal non humain ou plante transgénique ou leurs descendances selon la revendication 2 ou 3, dans laquelle/lequel/lesquelles la GMD est une protéine choisie dans le groupe constitué par (a), (b) et (c) suivants :

(a) une protéine comprenant la séquence d'acides aminés représentée par SEQ ID n° 71 ;
(b) une protéine qui comprend une séquence d'acides aminés dans laquelle 1 à 20 acides aminés sont supprimés, substitués, insérés et/ou ajoutés dans la séquence d'acides aminés représentée par SEQ ID n° 71 et possède une activité de GMD ;
(c) une protéine qui comprend une séquence d'acides aminés ayant une homologie d'au moins 80 % avec la séquence d'acides aminés représentée par SEQ ID n° 71 et possède une activité de GMD.

**6.** Cellule selon la revendication 1 ou animal non humain ou plante transgénique ou leurs descendances selon la revendication 2 ou 3, dans laquelle/lequel/lesquelles la Fx est une protéine codée par un ADN de (a) ou (b) suivant :

(a) un ADN comprenant la séquence nucléotidique représentée par la SEQ ID N° 48 ;
(b) un ADN qui s'hybride avec l'ADN comprenant la séquence nucléotidique représentée par la SEQ ID N° 48 dans des conditions rigoureuses et code pour une protéine ayant une activité de Fx.

**7.** Cellule selon la revendication 1 ou animal non humain ou plante transgénique ou leurs descendances selon la revendication 2 ou 3, dans laquelle/lequel/lesquelles la Fx est une protéine choisie dans le groupe constitué par (a), (b) et (c) suivants :

(a) une protéine comprenant la séquence d'acides aminés représentée par SEQ ID n° 72 ;
(b) une protéine qui comprend une séquence d'acides aminés dans laquelle 1 à 20 acides aminés sont supprimés, substitués, insérés et/ou ajoutés dans la séquence d'acides aminés représentée par SEQ ID n° 72 et possède une activité de Fx ;
(c) une protéine qui comprend une séquence d'acides aminés ayant une homologie d'au moins 80 % avec la séquence d'acides aminés représentée par SEQ ID n° 72 et possède une activité de Fx.

**8.** Cellule selon la revendication 1 ou animal non humain ou plante transgénique ou leurs descendances selon la revendication 2 ou 3, dans laquelle/lequel/lesquelles la GFPP est une protéine codée par un ADN de (a) ou (b) suivant :

(a) un ADN comprenant la séquence nucléotidique représentée par la SEQ ID N° 51 ;
(b) un ADN qui s'hybride avec l'ADN comprenant la séquence nucléotidique représentée par la SEQ ID N° 51 dans des conditions rigoureuses et code pour une protéine ayant une activité de GFPP.

**9.** Cellule selon la revendication 1 ou animal non humain ou plante transgénique ou leurs descendances selon la revendication 2 ou 3, dans laquelle/lequel/lesquelles la GFPP est une protéine choisie dans le groupe constitué par (a), (b) et (c) suivants :

(a) une protéine comprenant la séquence d'acides aminés représentée par SEQ ID n° 73 ;
(b) une protéine qui comprend une séquence d'acides aminés dans laquelle 1 à 20 acides aminés sont supprimés, substitués, insérés et/ou ajoutés dans la séquence d'acides aminés représentée par SEQ ID n° 73 et possède une activité de GFPP ;
(c) une protéine qui comprend une séquence d'acides aminés ayant une homologie d'au moins 80 % avec la séquence d'acides aminés représentée par SEQ ID n° 73 et possède une activité de GFPP.

**10.** Cellule, animal non humain ou plante transgénique ou leurs descendances selon l'une quelconque des revendications 1 à 3, dans laquelle/lequel/lesquelles la $\alpha$1,6-fucosyltransférase est une protéine codée par un ADN choisi dans le groupe constitué par (a), (b), (c) et (d) suivants :

(a) un ADN comprenant la séquence nucléotidique représentée par la SEQ ID N° 1 ;
(b) un ADN comprenant la séquence nucléotidique représentée par la SEQ ID N° 2 ;
(c) un ADN qui s'hybride avec l'ADN comprenant la séquence nucléotidique représentée par la SEQ ID N° 1 dans des conditions rigoureuses et code pour une protéine ayant une activité de $\alpha$1,6-fucosyltransférase ;
(d) un ADN qui s'hybride avec l'ADN comprenant la séquence nucléotidique représentée par la SEQ ID N° 2 dans des conditions rigoureuses et code pour une protéine ayant une activité $\alpha$1,6-fucosyltransférase.

**11.** Cellule, animal non humain ou plante transgénique ou leurs descendances selon l'une quelconque des revendications 1 à 3, dans laquelle/lequel/lesquelles la α1,6-fucosyltransférase est une protéine choisie dans le groupe constitué par (a), (b), (c), (d), (e) et (f) suivants :

(a) une protéine comprenant la séquence d'acides aminés représentée par SEQ ID n° 23 ;
(b) une protéine comprenant la séquence d'acides aminés représentée par SEQ ID n° 24 ;
(c) une protéine qui comprend une séquence d'acides aminés dans laquelle 1 à 20 acides aminés sont supprimés, substitués, insérés et/ou ajoutés dans la séquence d'acides aminés représentée par SEQ ID n° 23 et possède une activité de α1,6-fucosyltransférase ;
(d) une protéine qui comprend une séquence d'acides aminés dans laquelle 1 à 20 acides aminés sont supprimés, substitués, insérés et/ou ajoutés dans la séquence d'acides aminés représentée par SEQ ID n° 24 et possède une activité de α1,6-fucosyltransférase ;
(e) une protéine qui comprend une séquence d'acides aminés ayant une homologie d'au moins 80 % avec la séquence d'acides aminés représentée par SEQ ID n° 23 et possède une activité de α1,6-fucosyltransférase ;
(f) une protéine qui comprend une séquence d'acides aminés ayant une homologie d'au moins 80 % avec la séquence d'acides aminés représentée par SEQ ID n° 24 et possède une activité de α1,6-fucosyltransférase.

**12.** Cellule selon l'une quelconque des revendications 1 et 4 à 11, qui est résistante à au moins une lectine qui reconnait une chaîne de sucre dans laquelle la position 1 du fucose est liée à la position 6 de la N-acétylglycosamine dans l'extrémité de réduction par le biais d'une liaison α dans la chaîne de sucre complexe liée au N-glycoside.

**13.** Cellule selon l'une quelconque des revendications 1 et 4 à 12, qui est une cellule choisie dans le groupe constitué par (a) à (i) suivants :

(a) une cellule CHO dérivée d'un tissu d'ovaire de hamster chinois ;
(b) une lignée cellulaire de myélome de rat, cellule YB2/3HL.P2.G11.16.AG.20 ;
(c) une lignée cellulaire de myélome de souris, cellule NS20 ;
(d) une lignée cellulaire de myélome de souris, cellule SP2/0-Agl4 ;
(e) une cellule BHK dérivée d'un tissu de reins de hamster syrien ;
(f) une cellule d'hybridome produisant un anticorps ;
(g) une cellule d'une lignée cellulaire de leucémie humaine Namalwa;
(h) une cellule souche d'embryon non humain ;
(i) une cellule d'oeuf fertilisé non humain.

**14.** Cellule selon l'une quelconque des revendications 1 ou 4 à 13, dans laquelle un gène codant pour une molécule d'anticorps est introduit.

**15.** Cellule selon la revendication 14, dans laquelle la molécule d'anticorps appartient à une classe d'IgG.

**16.** Procédé de production d'une composition d'anticorps, qui comprend la mise en culture de la cellule selon la revendication 14 ou 15 dans un milieu pour produire et accumuler la composition d'anticorps dans la culture ; et la récupération de la composition d'anticorps de la culture.

**17.** Procédé selon la revendication 16, qui produit une composition d'anticorps ayant une activité cytotoxique à médiation cellulaire dépendante des anticorps supérieure à celle d'une composition d'anticorps obtenue à partir de sa lignée cellulaire mère.

**18.** Animal non humain ou plante transgénique ou leurs descendances selon l'une quelconque des revendications 2 à 11, dans lequel/laquelle/lesquelles l'animal non humain transgénique est un animal choisi dans le groupe constitué par le bétail, les moutons, les chèvres, les cochons, les chevaux, les souris, les rats, la volaille, les singes et les lapins.

**19.** Procédé de production d'une composition d'anticorps, qui comprend la récupération d'une composition d'anticorps provenant d'un tissu ou d'un fluide corporel comprenant l'anticorps, lequel tissu ou fluide corporel a été isolé à partir d'un animal non humain ou d'une plante transgénique ou de leurs descendances selon l'une quelconque des revendications 2 à 11 et 18, dans lequel/laquelle/lesquelles un gène codant pour une molécule d'anticorps a été introduit.

**20.** Procédé selon la revendication 19, dans lequel la molécule d'anticorps appartient à une classe d'IgG.

**21.** Procédé selon la revendication 19 ou 20, qui produit une composition d'anticorps ayant une activité cytotoxique à médiation cellulaire dépendante des anticorps supérieure à celle d'une composition d'anticorps obtenue à partir d'un animal non humain ou d'une plante transgénique ou de leurs descendances dont le génome n'est pas modifié.

**22.** Cellule selon l'une quelconque des revendications 1 ou 4 à 15 ou animal non humain ou plante transgénique ou leurs descendances selon l'une quelconque des revendications 2 à 11 ou 18, dans laquelle/lequel/lesquelles ladite technique (D) cible un gène d'une enzyme en rapport avec la synthèse du GDP-fucose ou un gène de la $\alpha$1,6-fucosyltransférase avec une construction génique d'ARNi, dans laquelle/lequel/lesquelles ladite enzyme en rapport avec la synthèse du GDP-fucose est une enzyme choisie dans le groupe constitué par (a), (b) et (c) suivants :

(a) GMD (GDP-mannose 4,6-déshydratase) ;
(b) Fx (GDP-céto-6-désoxymannose 3,5-épimérase, 4-réductase) ;
(c) GFPP (GDP-bêta-L-fucose pyrophosphorylase).

FIG. 1A

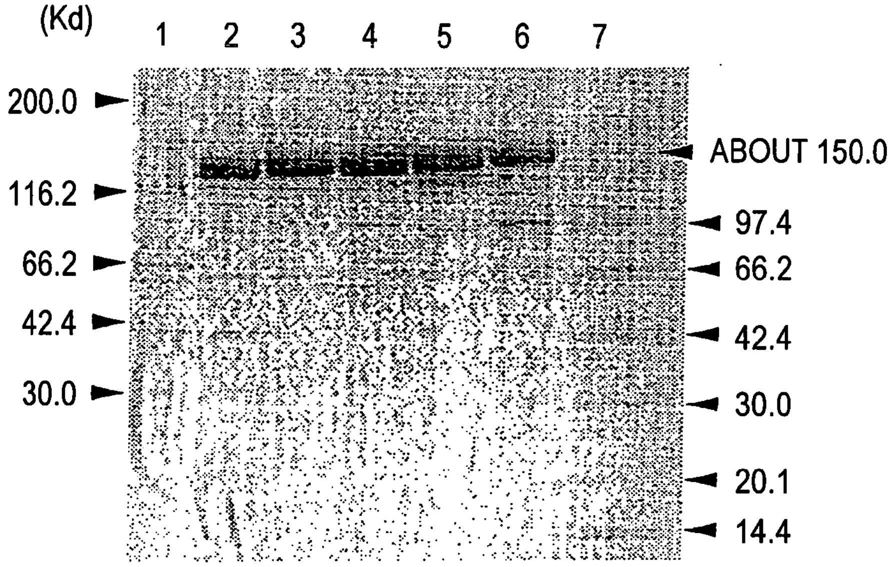
(Kd)
1
2
3
4
5
6
7
200.0
116.2
66.2
42.4
30.0
ABOUT 150.0
97.4
66.2
42.4
30.0
20.1
14.4

FIG. 1B

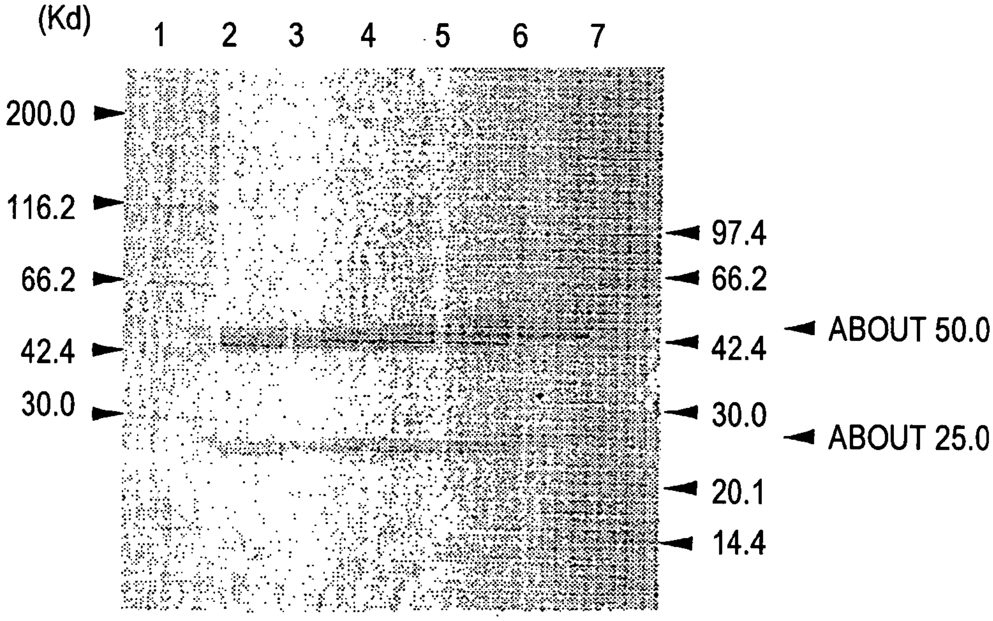
(Kd)
1
2
3
4
5
6
7
200.0
116.2
66.2
42.4
30.0
97.4
66.2
ABOUT 50.0
42.4
30.0
ABOUT 25.0
20.1
14.4

FIG. 2

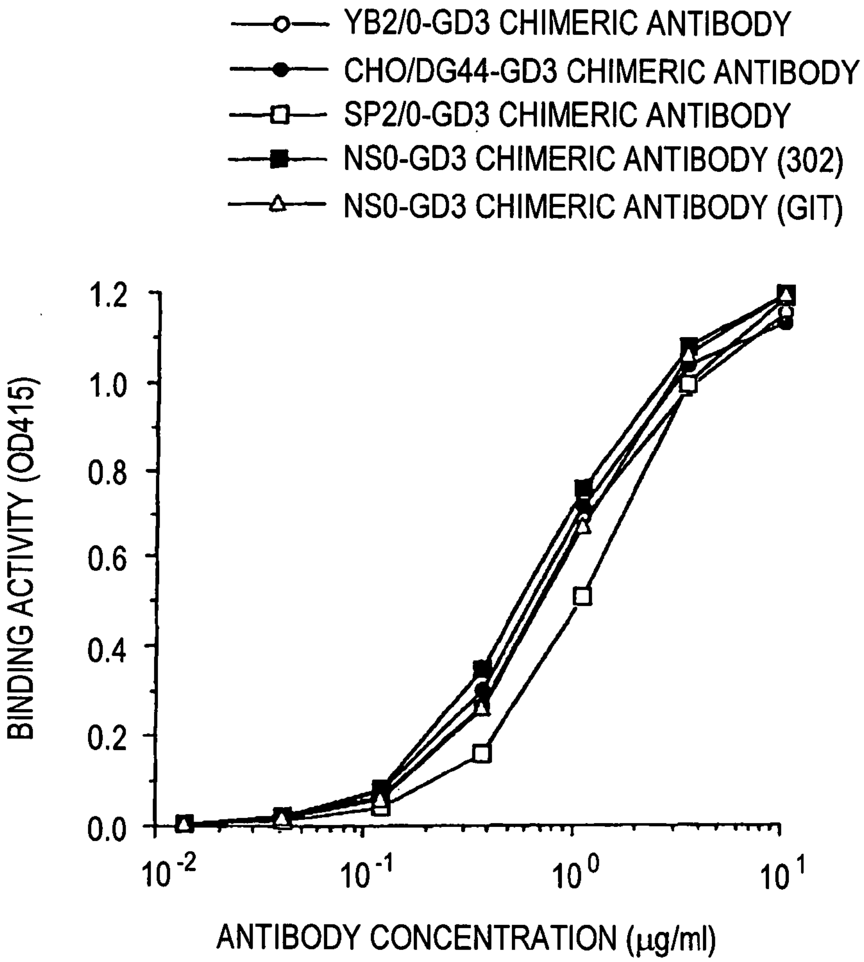
YB2/0-GD3 CHIMERIC ANTIBODY
CHO/DG44-GD3 CHIMERIC ANTIBODY
SP2/0-GD3 CHIMERIC ANTIBODY
NS0-GD3 CHIMERIC ANTIBODY (302)
NS0-GD3 CHIMERIC ANTIBODY (GIT)
BINDING ACTIVITY (OD415)
1.2
1.0
0.8
0.6
0.4
0.2
0.0
10 -2
10 -1
10 0
10 1
ANTIBODY CONCENTRATION (μg/ml)

FIG. 3

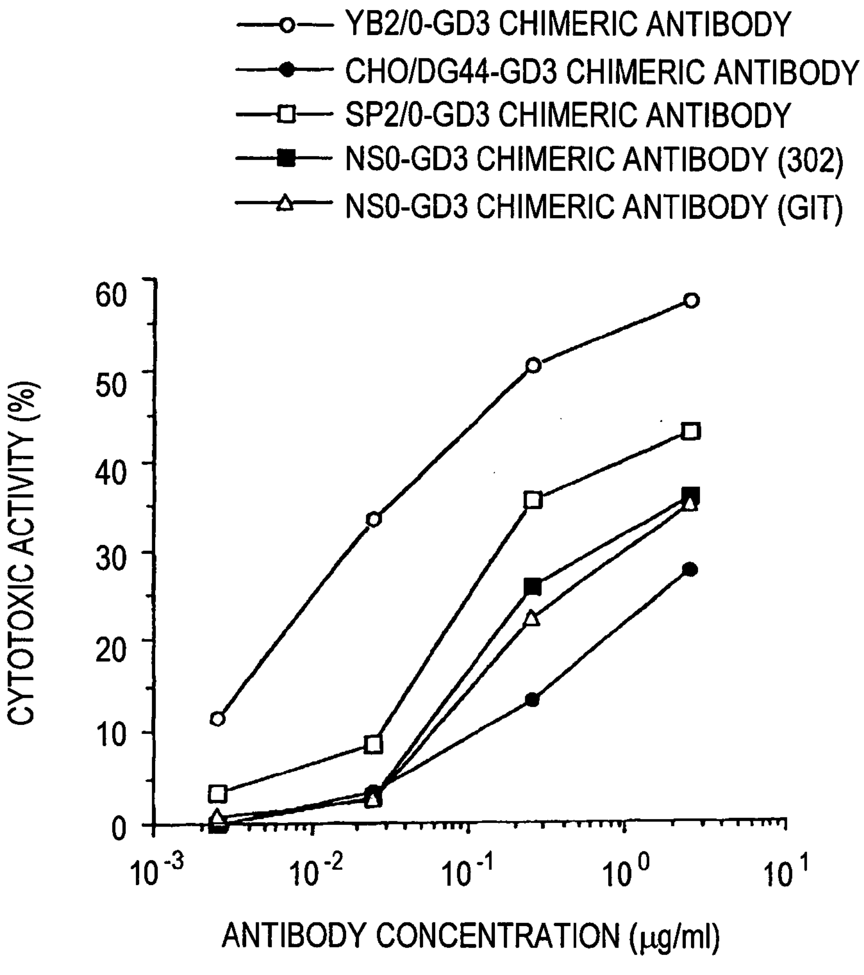
YB2/0-GD3 CHIMERIC ANTIBODY
CHO/DG44-GD3 CHIMERIC ANTIBODY
SP2/0-GD3 CHIMERIC ANTIBODY
NS0-GD3 CHIMERIC ANTIBODY (302)
NS0-GD3 CHIMERIC ANTIBODY (GIT)
CYTOTOXIC ACTIVITY (%)
0
10
20
30
40
50
60
$10^{-3}$
$10^{-2}$
$10^{-1}$
$10^{0}$
$10^{1}$
ANTIBODY CONCENTRATION (μg/ml)

FIG. 4A

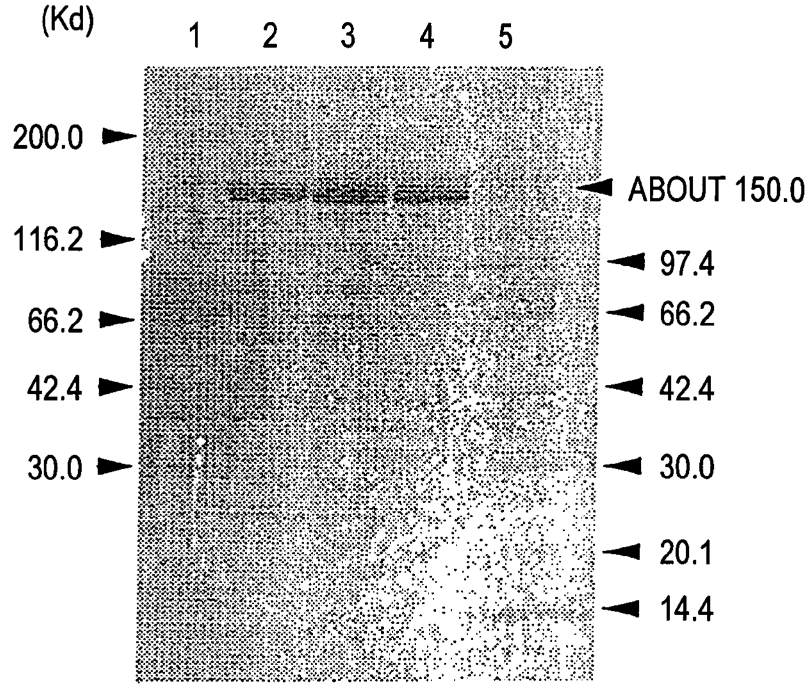
(Kd)
1
2
3
4
5
200.0
116.2
66.2
42.4
30.0
ABOUT 150.0
97.4
66.2
42.4
30.0
20.1
14.4

FIG. 4B

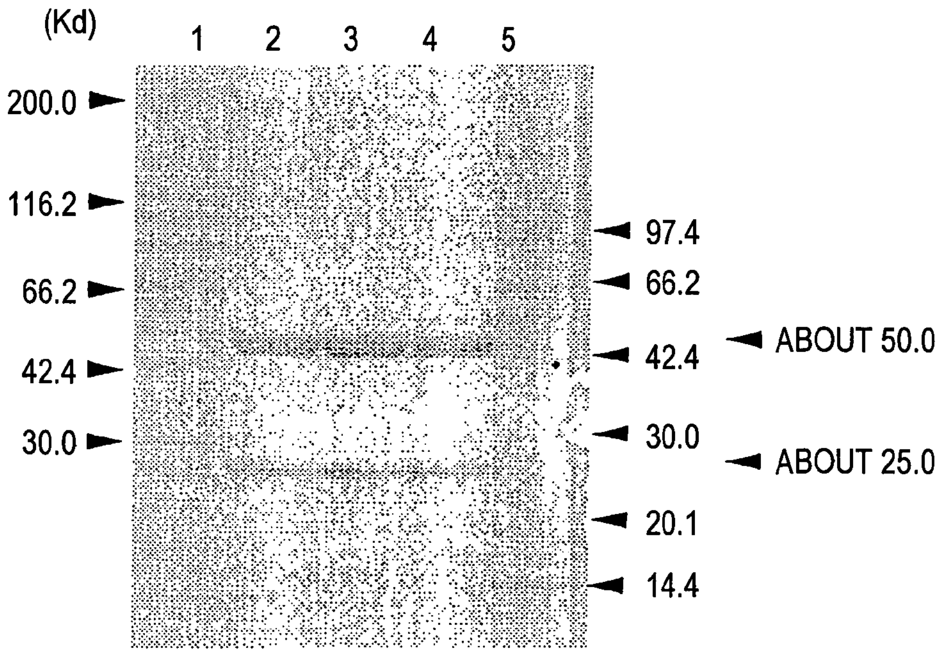
(Kd)
1
2
3
4
5
200.0
116.2
66.2
42.4
30.0
97.4
66.2
42.4
30.0
20.1
14.4
ABOUT 50.0
ABOUT 25.0

FIG. 5

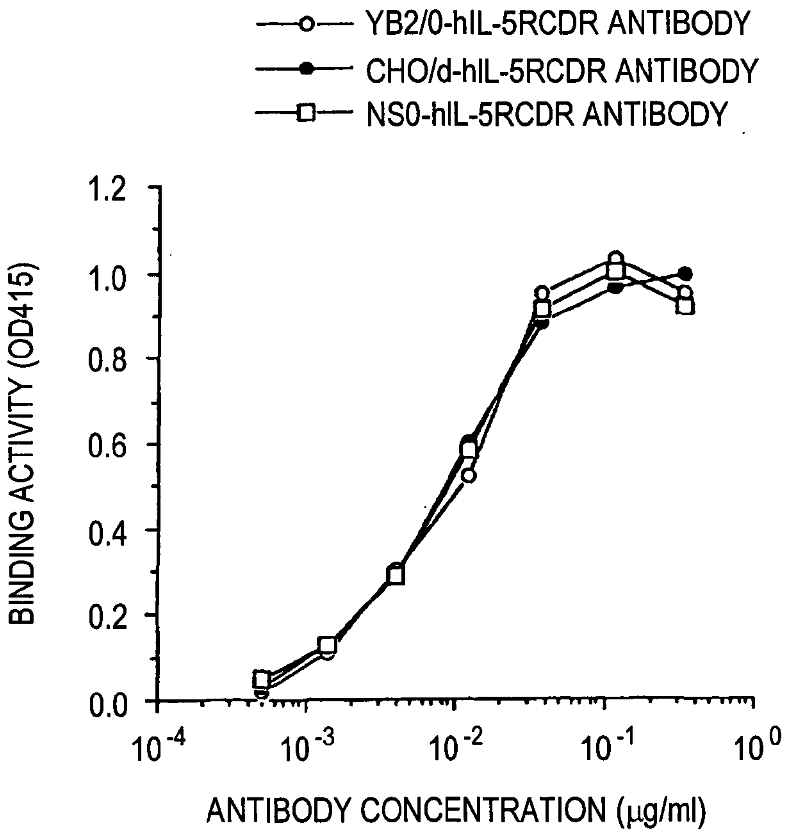
YB2/0-hIL-5RCDR ANTIBODY
CHO/d-hIL-5RCDR ANTIBODY
NS0-hIL-5RCDR ANTIBODY
BINDING ACTIVITY (OD415)
1.2
1.0
0.8
0.6
0.4
0.2
0.0
10 -4
10 -3
10 -2
10 -1
10 0
ANTIBODY CONCENTRATION (μg/ml)

FIG. 6

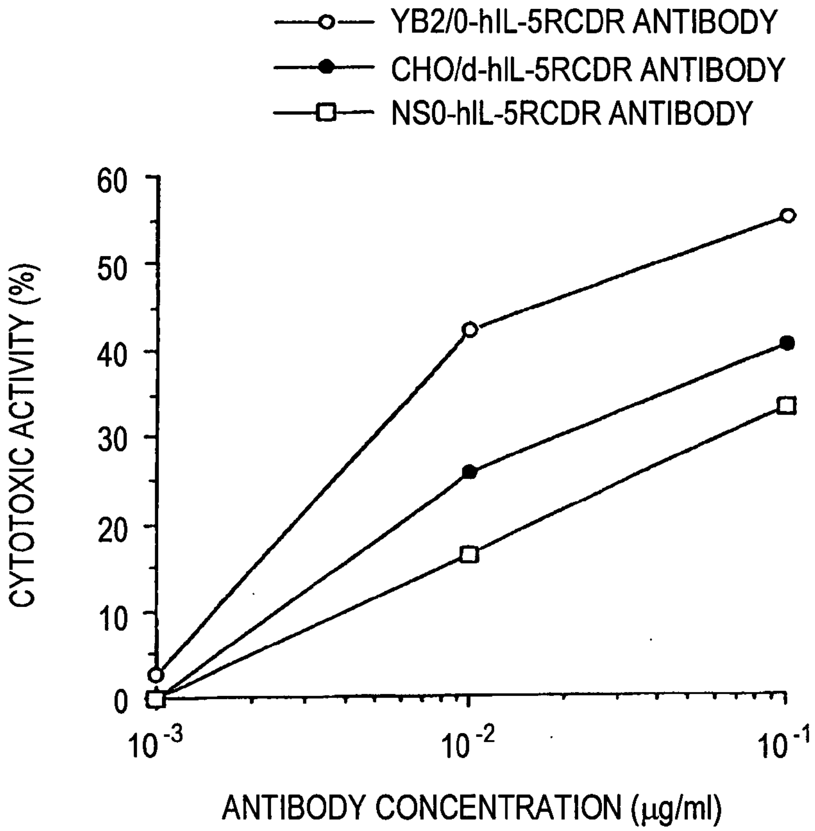
YB2/0-hIL-5RCDR ANTIBODY
CHO/d-hIL-5RCDR ANTIBODY
NS0-hIL-5RCDR ANTIBODY
CYTOTOXIC ACTIVITY (%)
60
50
40
30
20
10
0
$10^{-3}$
$10^{-2}$
$10^{-1}$
ANTIBODY CONCENTRATION (μg/ml)

FIG. 7

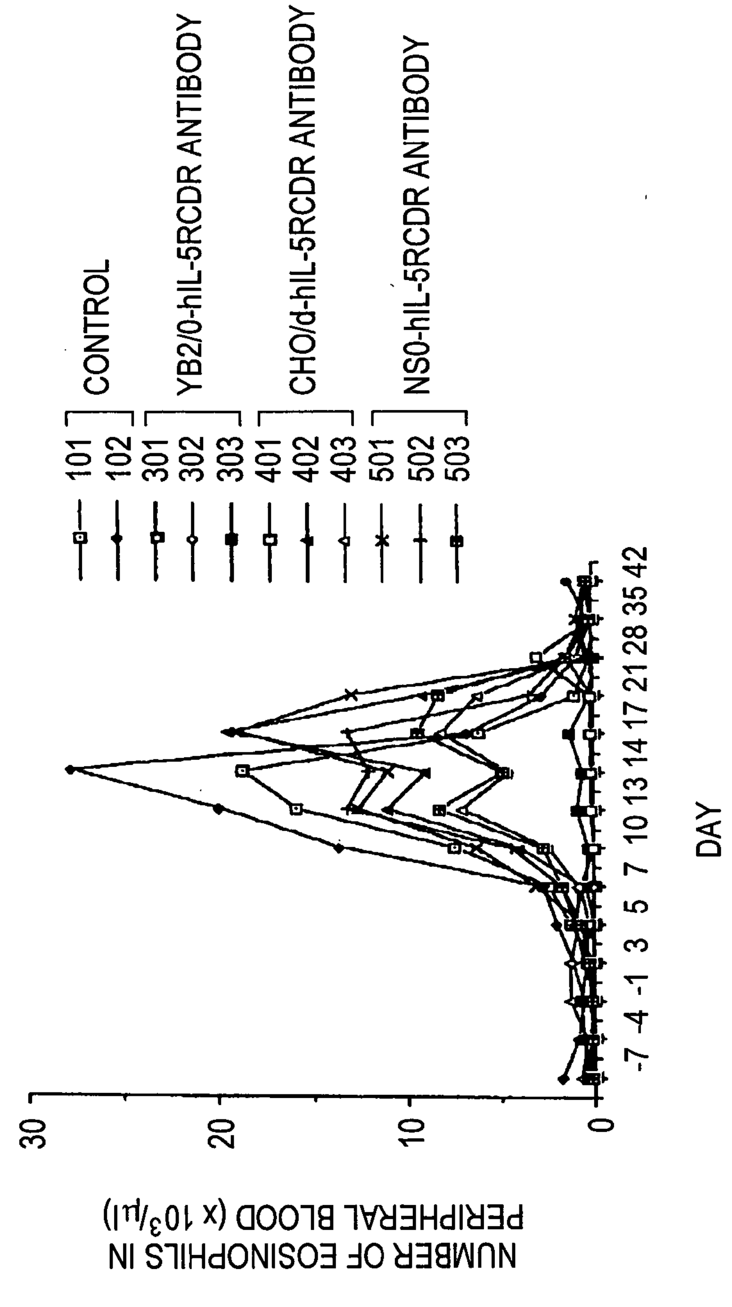
101
102
CONTROL
301
302
303
YB2/0-hIL-5RCDR ANTIBODY
401
402
403
CHO/d-hIL-5RCDR ANTIBODY
501
502
503
NS0-hIL-5RCDR ANTIBODY
NUMBER OF EOSINOPHILS IN PERIPHERAL BLOOD (x 10³/μl)
30
20
10
0
-7 -4 -1 3 5 7 10 13 14 17 21 28 35 42
DAY

# FIG. 8A

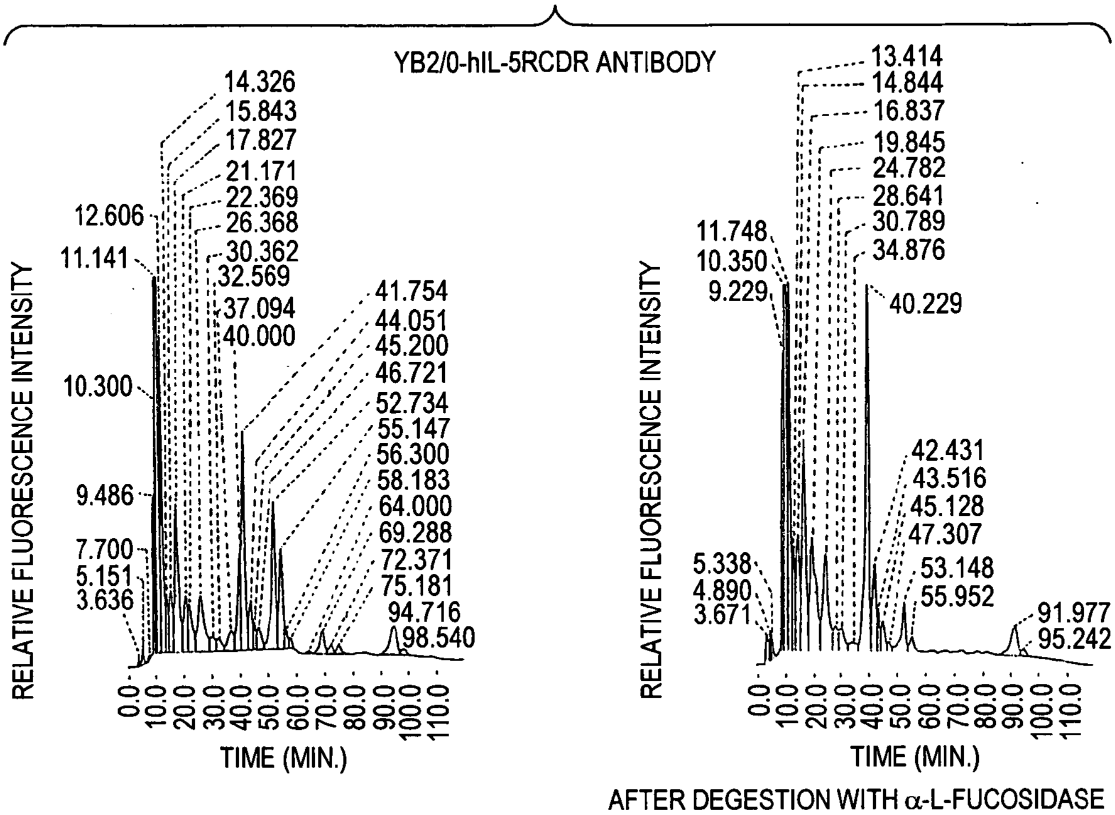
YB2/0-hIL-5RCDR ANTIBODY
RELATIVE FLUORESCENCE INTENSITY
14.326
15.843
17.827
21.171
22.369
26.368
30.362
32.569
37.094
40.000
12.606
11.141
10.300
9.486
7.700
5.151
3.636
41.754
44.051
45.200
46.721
52.734
55.147
56.300
58.183
64.000
69.288
72.371
75.181
94.716
98.540
0.0 10.0 20.0 30.0 40.0 50.0 60.0 70.0 80.0 90.0 100.0 110.0
TIME (MIN.)
13.414
14.844
16.837
19.845
24.782
28.641
30.789
34.876
11.748
10.350
9.229
40.229
42.431
43.516
45.128
47.307
5.338
4.890
3.671
53.148
55.952
91.977
95.242
AFTER DEGESTION WITH α-L-FUCOSIDASE

# FIG. 8B

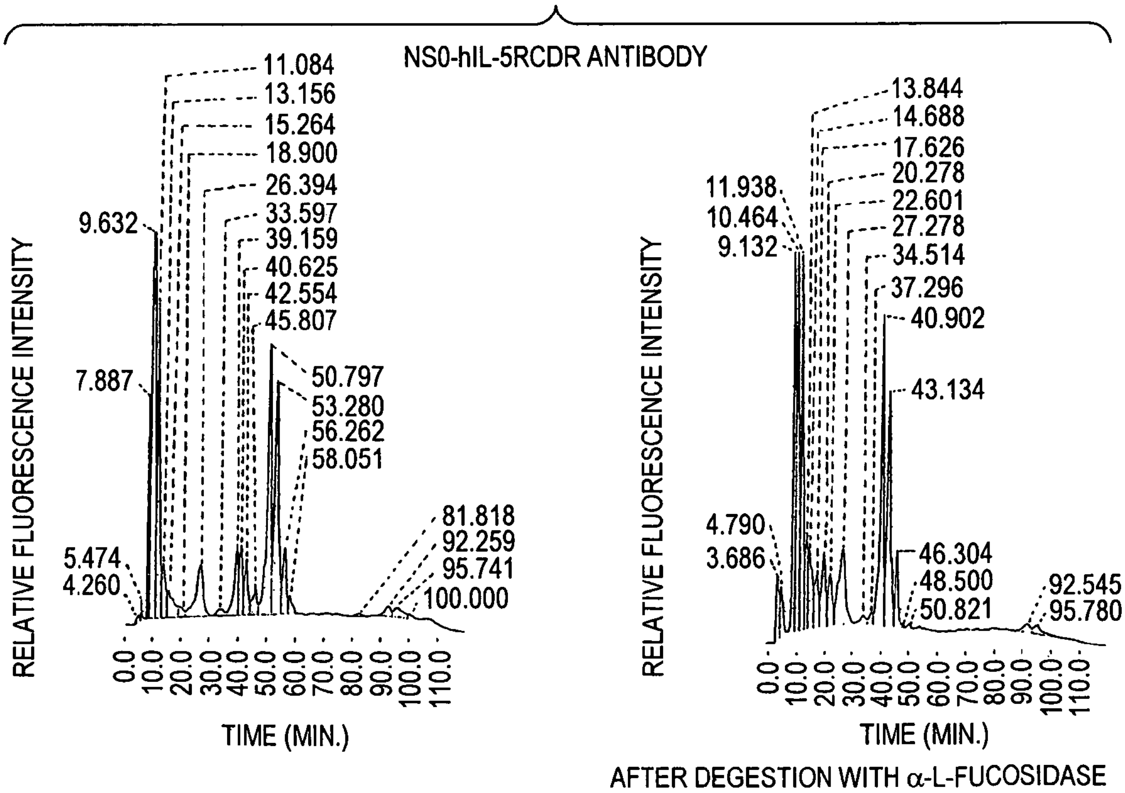
NS0-hIL-5RCDR ANTIBODY
RELATIVE FLUORESCENCE INTENSITY
11.084
13.156
15.264
18.900
26.394
33.597
39.159
40.625
42.554
45.807
9.632
7.887
50.797
53.280
56.262
58.051
5.474
4.260
81.818
92.259
95.741
100.000
0.0 10.0 20.0 30.0 40.0 50.0 60.0 70.0 80.0 90.0 100.0 110.0
TIME (MIN.)
13.844
14.688
17.626
20.278
22.601
27.278
34.514
37.296
40.902
11.938
10.464
9.132
43.134
4.790
3.686
46.304
48.500
50.821
92.545
95.780
AFTER DEGESTION WITH α-L-FUCOSIDASE

*FIG. 9*

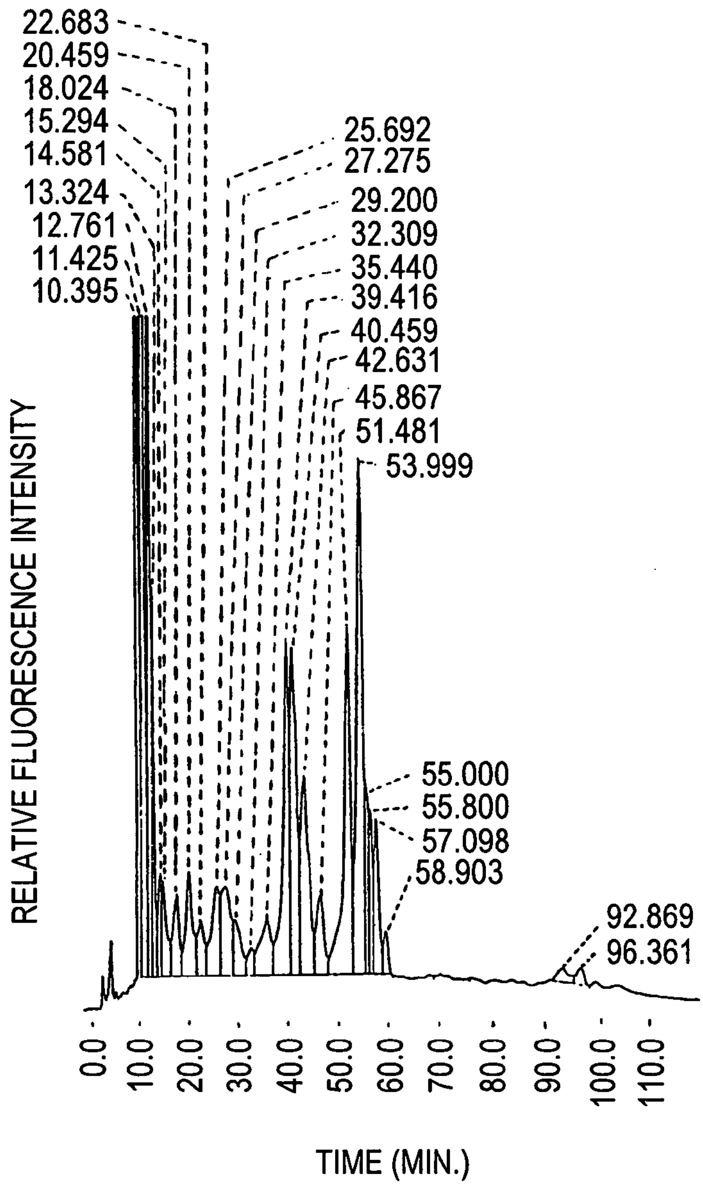
22.683
20.459
18.024
15.294
14.581
13.324
12.761
11.425
10.395
25.692
27.275
29.200
32.309
35.440
39.416
40.459
42.631
45.867
51.481
53.999
55.000
55.800
57.098
58.903
92.869
96.361
RELATIVE FLUORESCENCE INTENSITY
0.0
10.0
20.0
30.0
40.0
50.0
60.0
70.0
80.0
90.0
100.0
110.0
TIME (MIN.)

FIG. 10

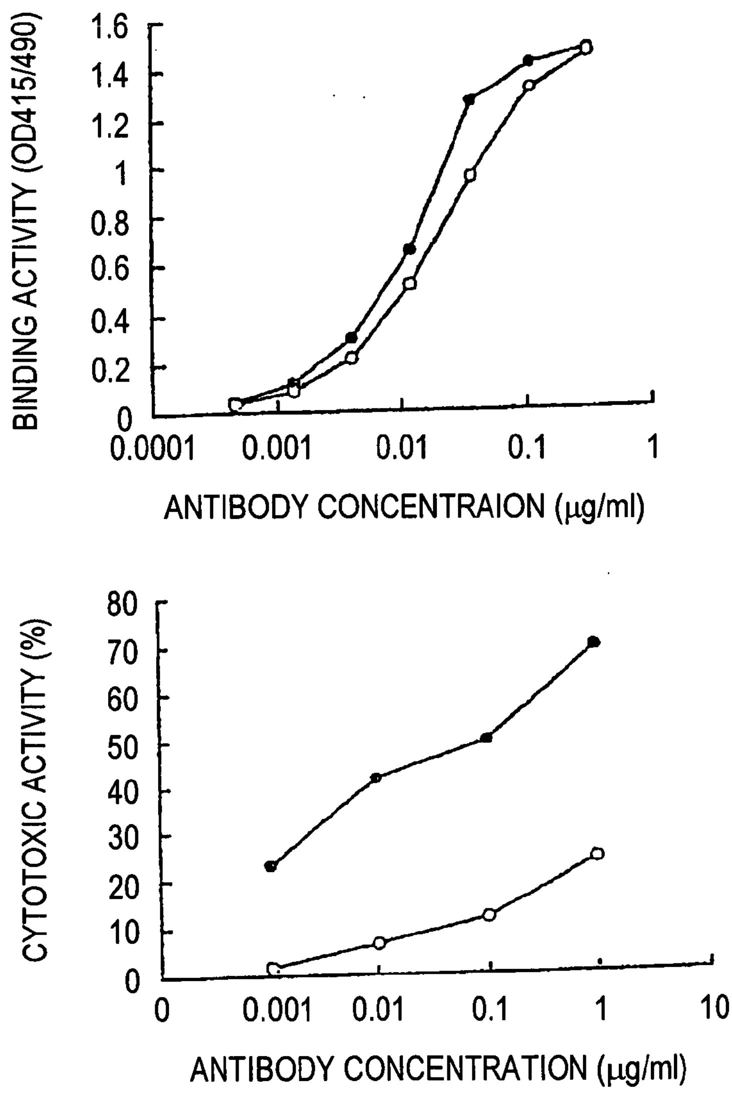
BINDING ACTIVITY (OD415/490)
1.6
1.4
1.2
1
0.8
0.6
0.4
0.2
0
0.0001
0.001
0.01
0.1
1
ANTIBODY CONCENTRAION (µg/ml)
CYTOTOXIC ACTIVITY (%)
80
70
60
50
40
30
20
10
0
0
0.001
0.01
0.1
1
10
ANTIBODY CONCENTRATION (µg/ml)

FIG. 11A

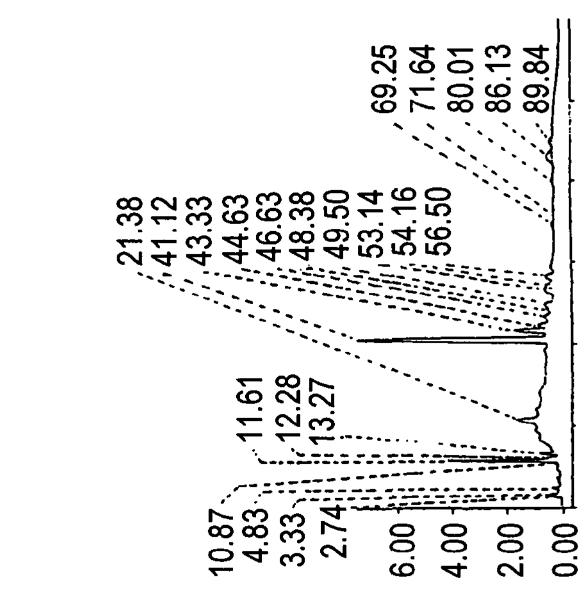


FIG. 11B

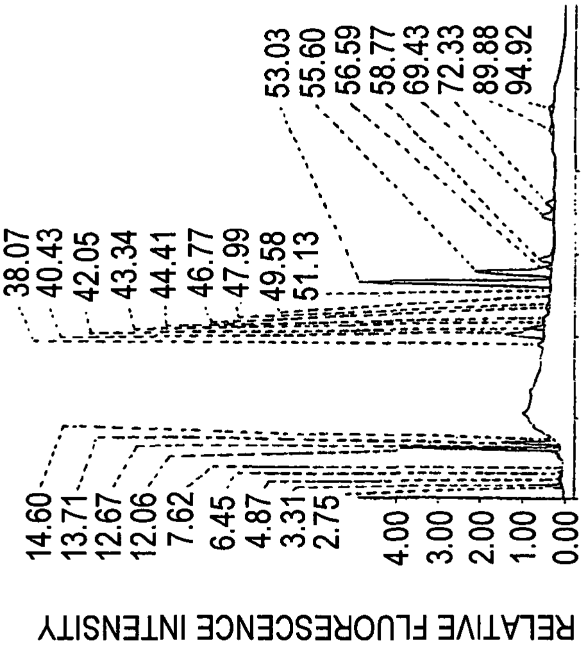

FIG. 12A

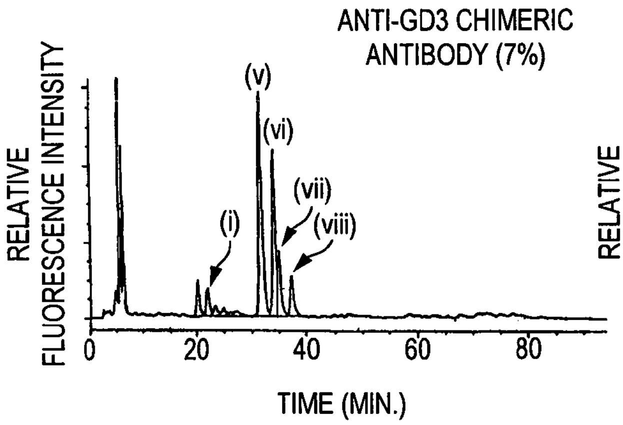


FIG. 12B

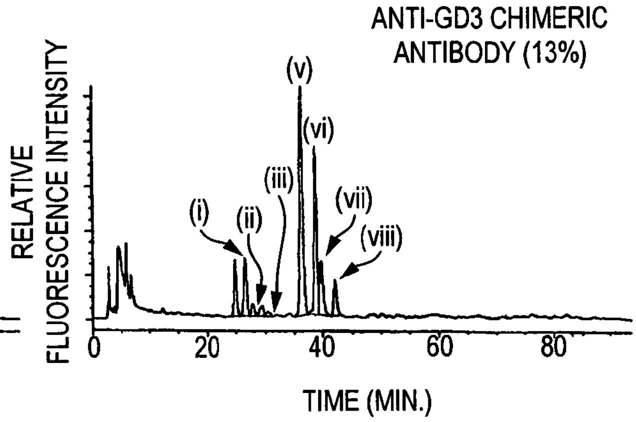


FIG. 12C

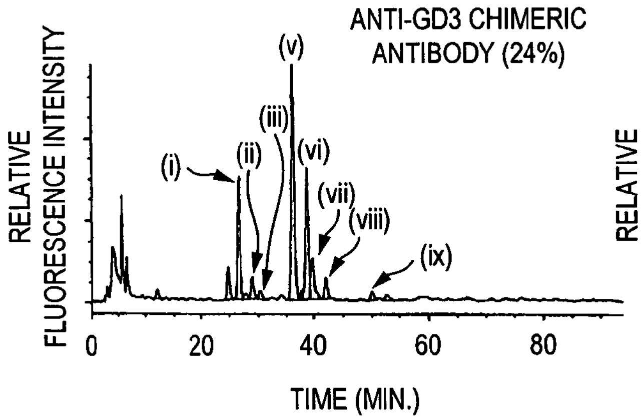


FIG. 12D

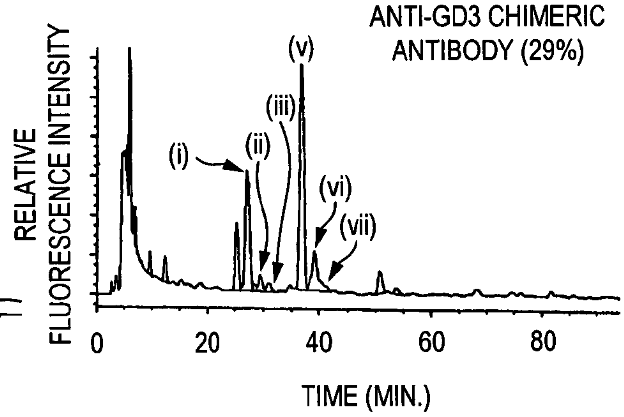


FIG. 12E

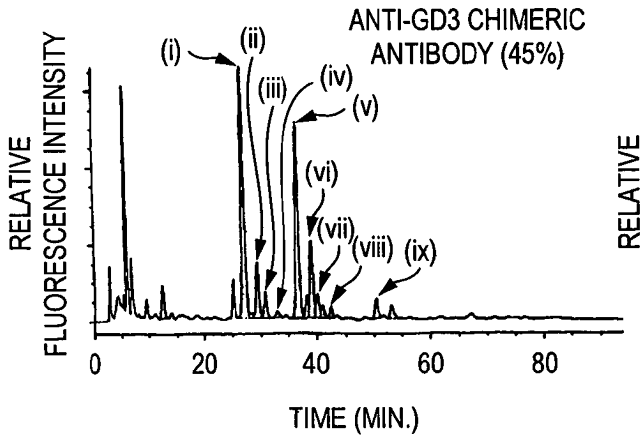


FIG. 12F

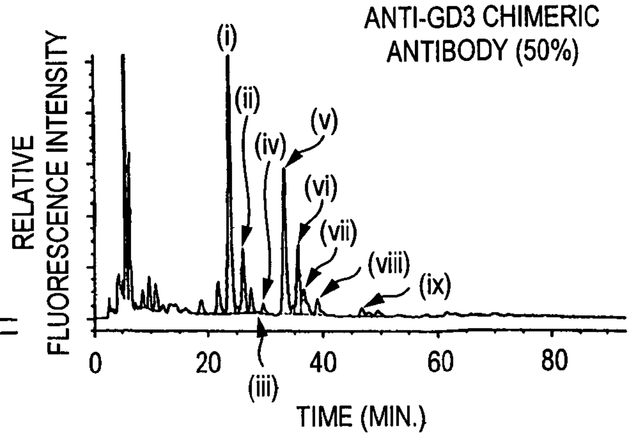

FIG. 13

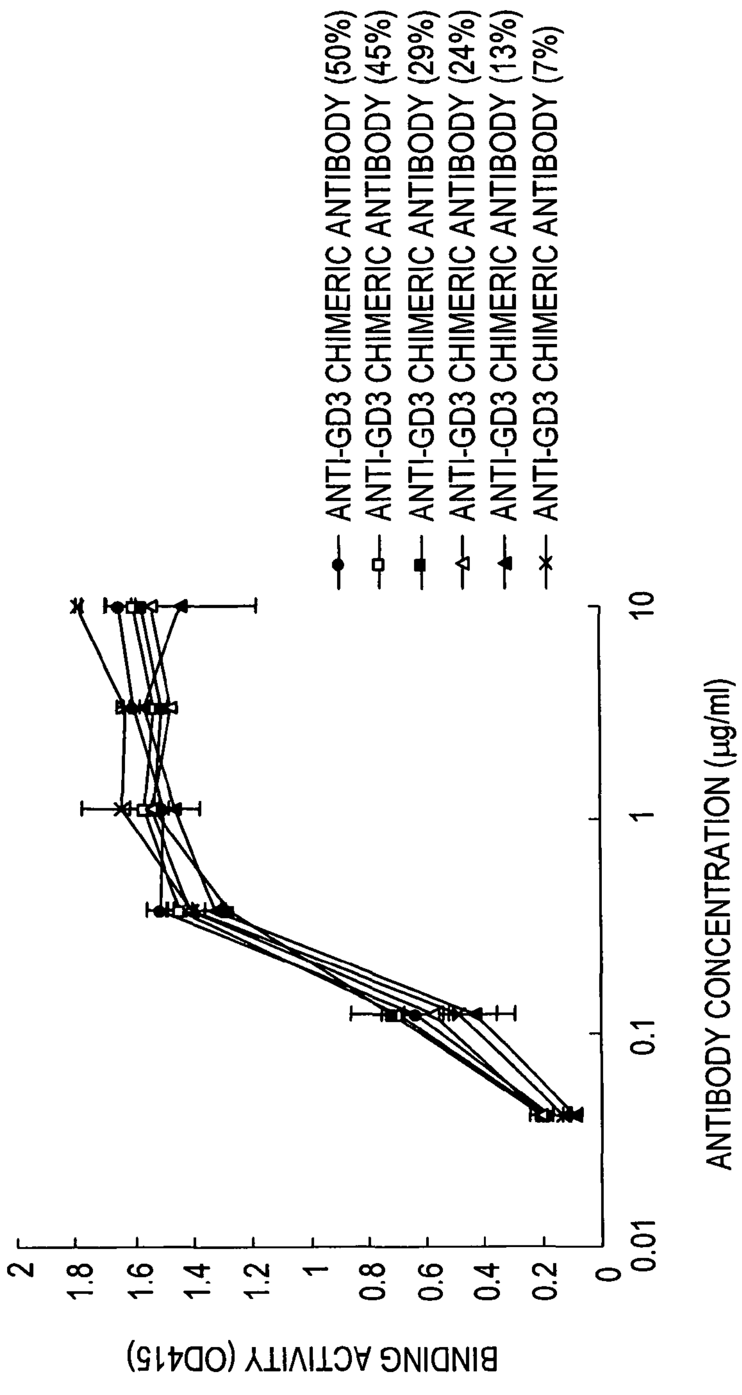
BINDING ACTIVITY (OD415)
2
1.8
1.6
1.4
1.2
1
0.8
0.6
0.4
0.2
0
0.01
0.1
1
10
ANTIBODY CONCENTRATION (μg/ml)
ANTI-GD3 CHIMERIC ANTIBODY (50%)
ANTI-GD3 CHIMERIC ANTIBODY (45%)
ANTI-GD3 CHIMERIC ANTIBODY (29%)
ANTI-GD3 CHIMERIC ANTIBODY (24%)
ANTI-GD3 CHIMERIC ANTIBODY (13%)
ANTI-GD3 CHIMERIC ANTIBODY (7%)

FIG. 14

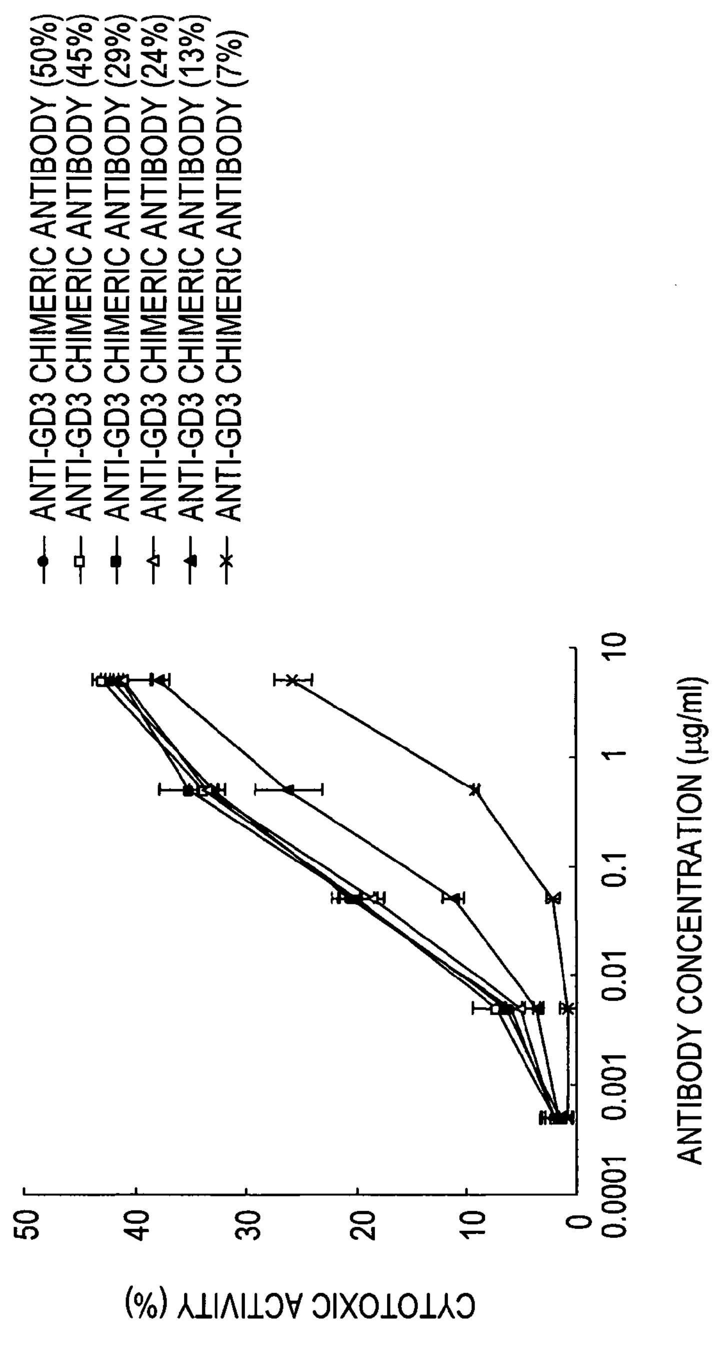
CYTOTOXIC ACTIVITY (%)
0
10
20
30
40
50
0.0001
0.001
0.01
0.1
1
10
ANTIBODY CONCENTRATION (μg/ml)
ANTI-GD3 CHIMERIC ANTIBODY (50%)
ANTI-GD3 CHIMERIC ANTIBODY (45%)
ANTI-GD3 CHIMERIC ANTIBODY (29%)
ANTI-GD3 CHIMERIC ANTIBODY (24%)
ANTI-GD3 CHIMERIC ANTIBODY (13%)
ANTI-GD3 CHIMERIC ANTIBODY (7%)

FIG. 15

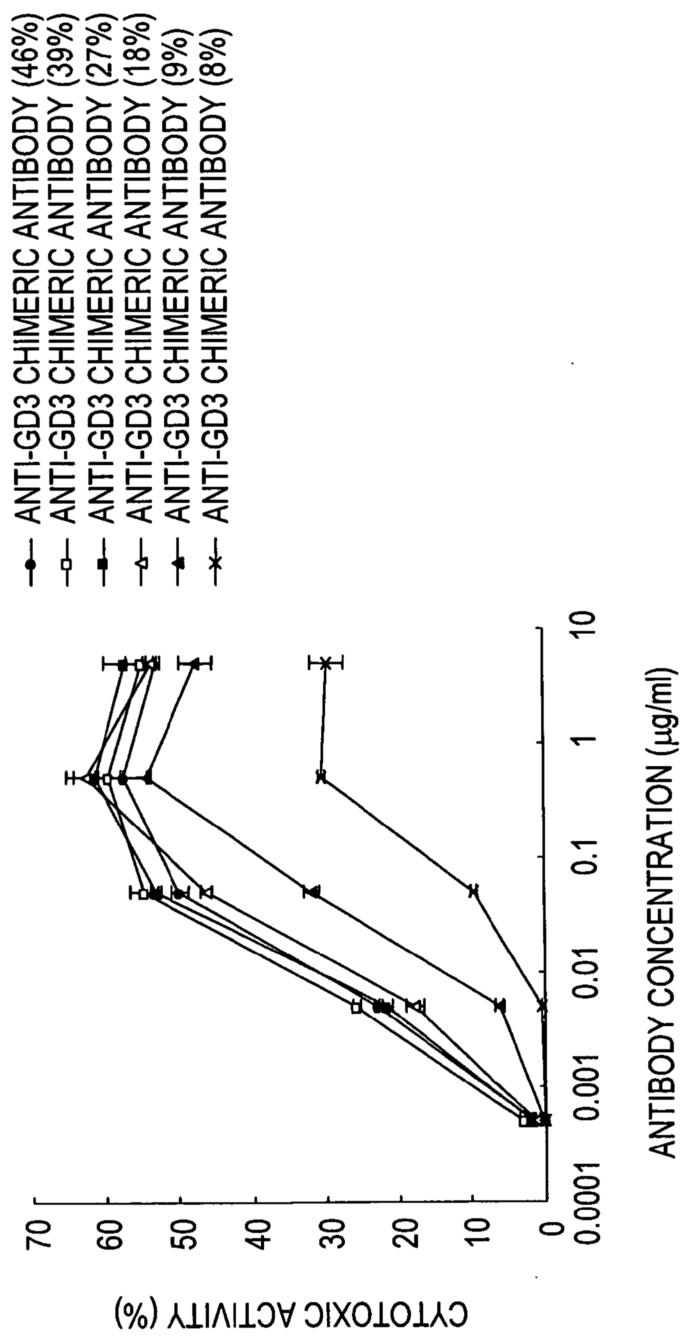
ANTI-GD3 CHIMERIC ANTIBODY (46%)
ANTI-GD3 CHIMERIC ANTIBODY (39%)
ANTI-GD3 CHIMERIC ANTIBODY (27%)
ANTI-GD3 CHIMERIC ANTIBODY (18%)
ANTI-GD3 CHIMERIC ANTIBODY (9%)
ANTI-GD3 CHIMERIC ANTIBODY (8%)
CYTOTOXIC ACTIVITY (%)
70
60
50
40
30
20
10
0
0.0001
0.001
0.01
0.1
1
10
ANTIBODY CONCENTRATION (μg/ml)

*FIG. 16A*

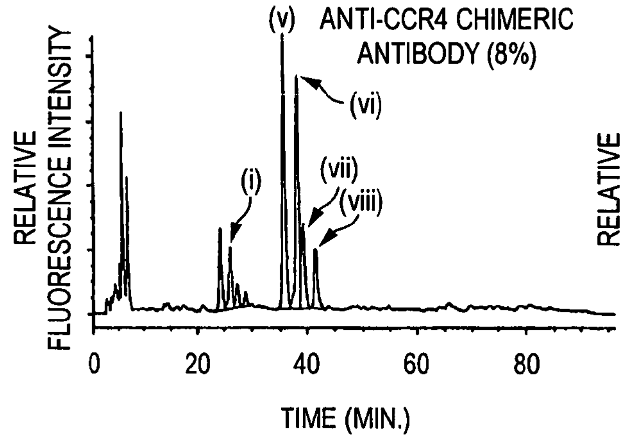


*FIG. 16B*

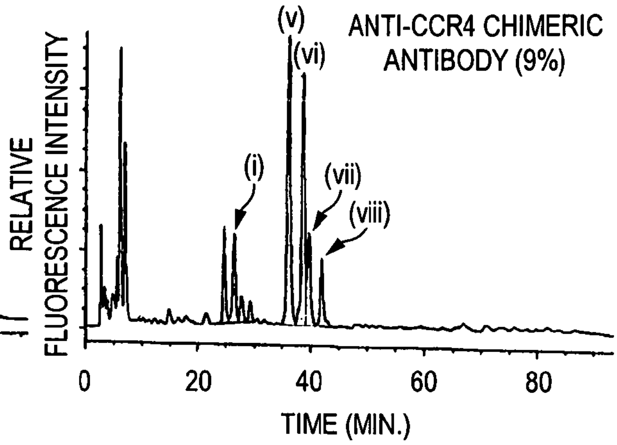


*FIG. 16C*

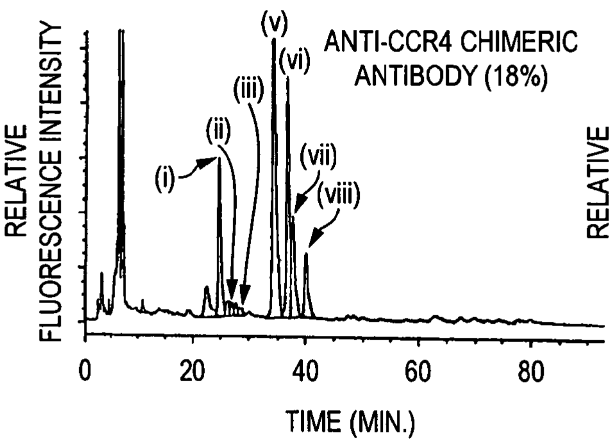


*FIG. 16D*

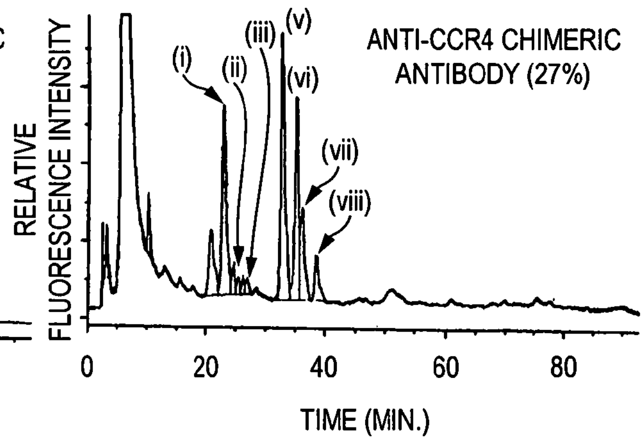


*FIG. 16E*

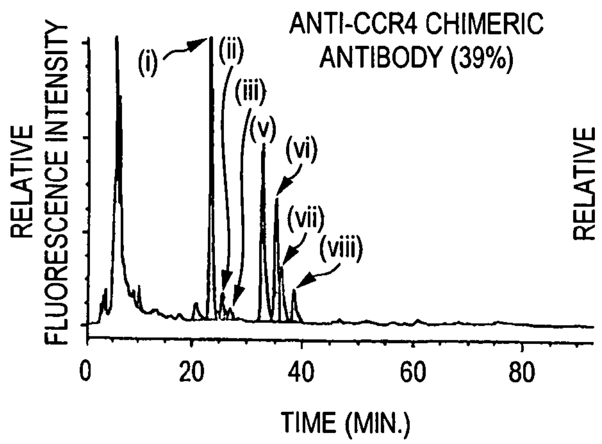


*FIG. 16F*

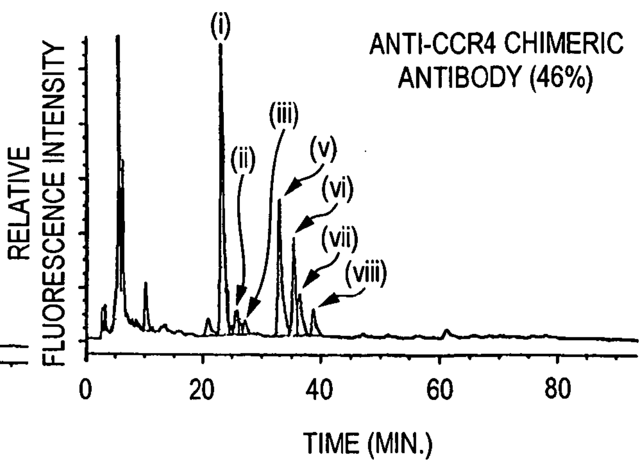

FIG. 17

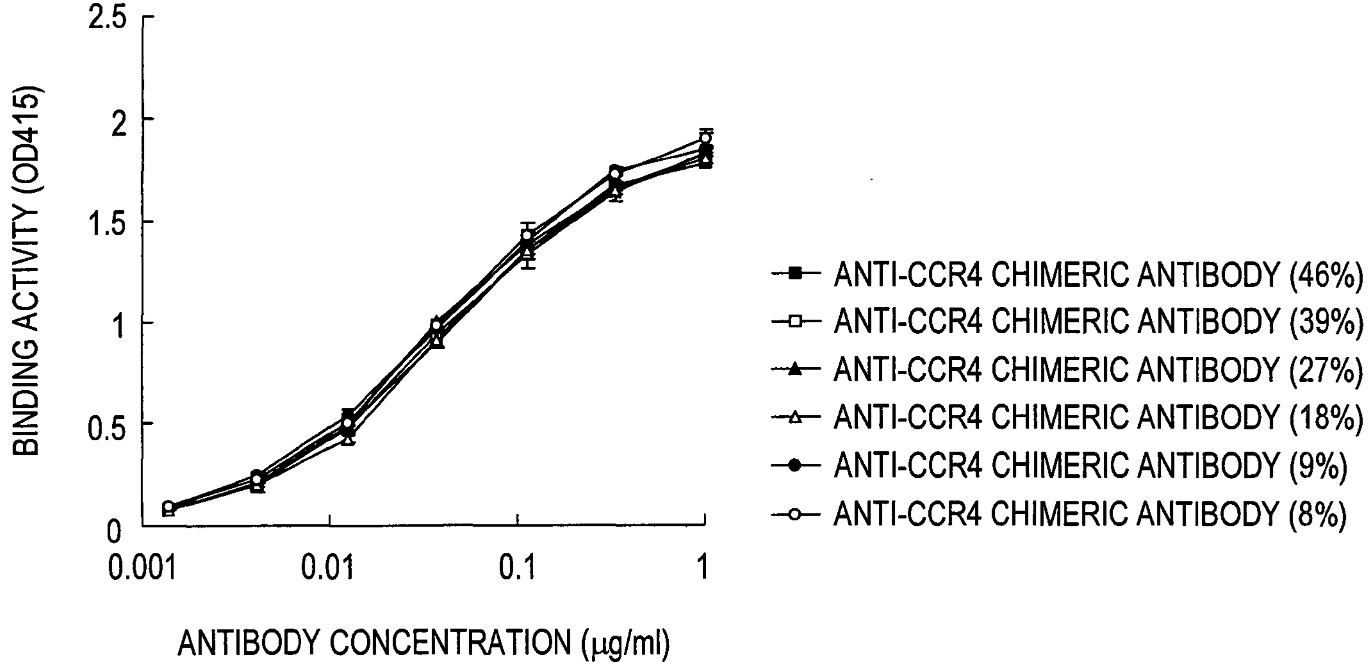
2.5
2
1.5
1
0.5
0
BINDING ACTIVITY (OD415)
0.001
0.01
0.1
1
ANTIBODY CONCENTRATION (μg/ml)
ANTI-CCR4 CHIMERIC ANTIBODY (46%)
ANTI-CCR4 CHIMERIC ANTIBODY (39%)
ANTI-CCR4 CHIMERIC ANTIBODY (27%)
ANTI-CCR4 CHIMERIC ANTIBODY (18%)
ANTI-CCR4 CHIMERIC ANTIBODY (9%)
ANTI-CCR4 CHIMERIC ANTIBODY (8%)

*FIG. 18*

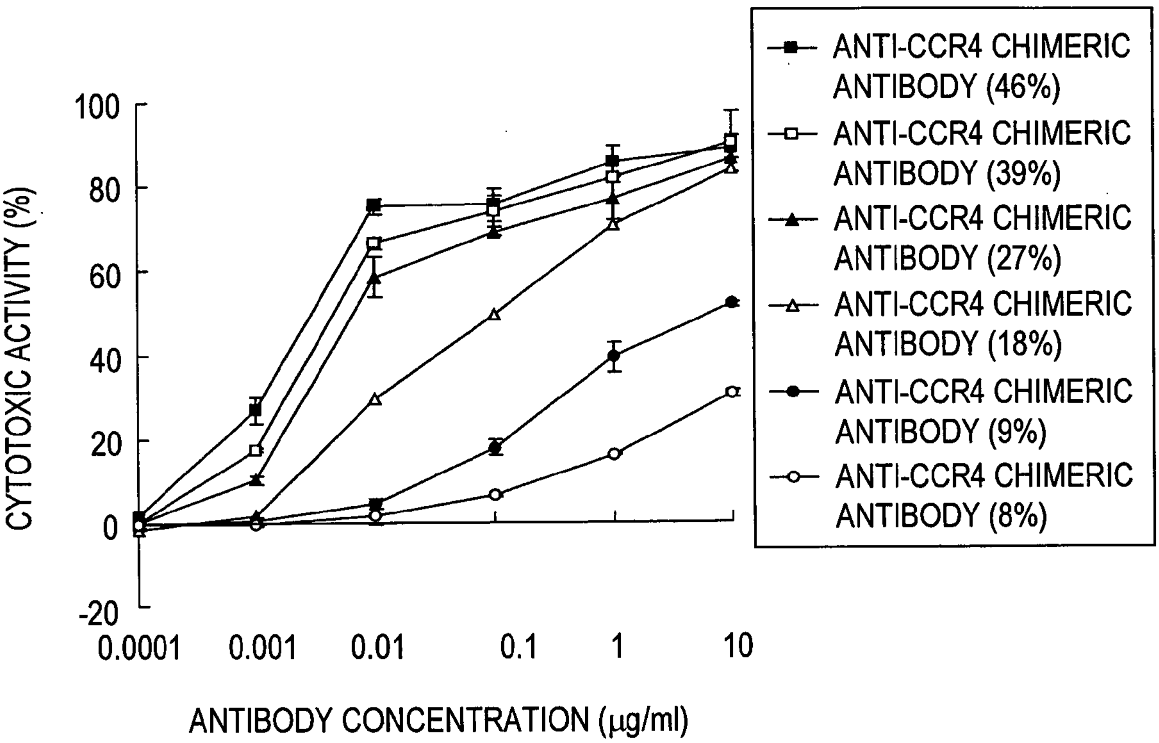
CYTOTOXIC ACTIVITY (%)
100
80
60
40
20
0
-20
0.0001
0.001
0.01
0.1
1
10
ANTIBODY CONCENTRATION (μg/ml)
ANTI-CCR4 CHIMERIC ANTIBODY (46%)
ANTI-CCR4 CHIMERIC ANTIBODY (39%)
ANTI-CCR4 CHIMERIC ANTIBODY (27%)
ANTI-CCR4 CHIMERIC ANTIBODY (18%)
ANTI-CCR4 CHIMERIC ANTIBODY (9%)
ANTI-CCR4 CHIMERIC ANTIBODY (8%)

FIG. 19

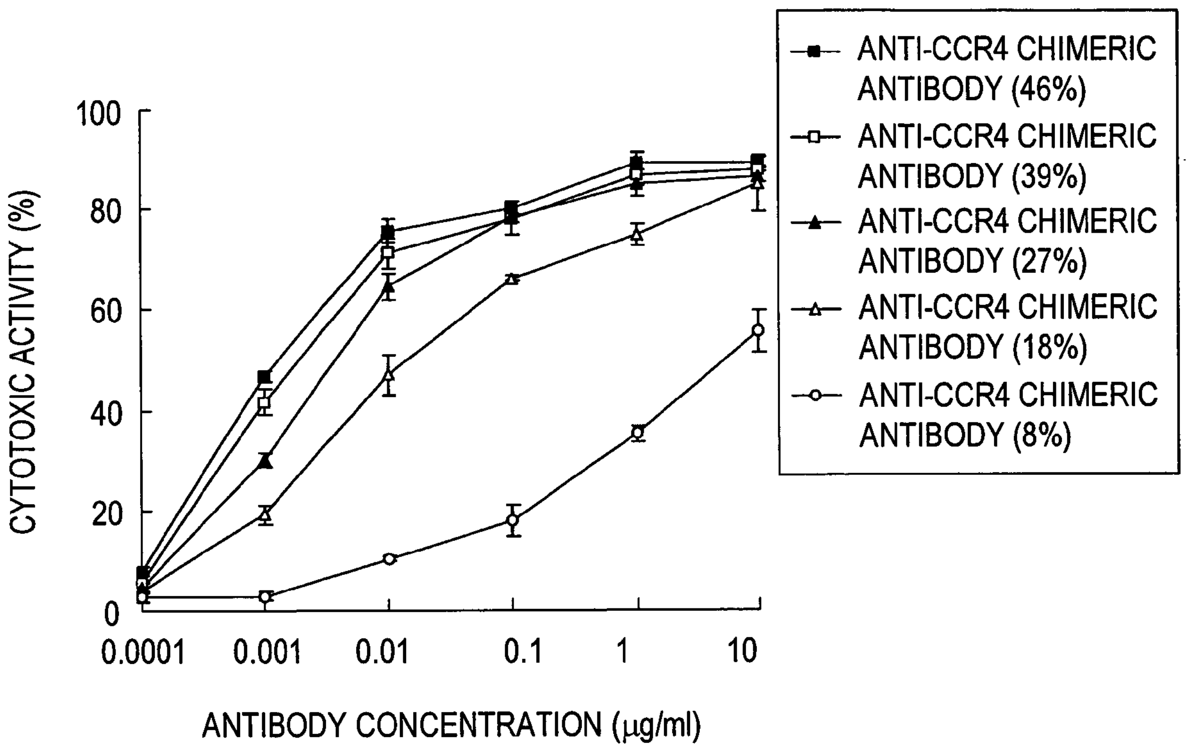
ANTI-CCR4 CHIMERIC ANTIBODY (46%)
ANTI-CCR4 CHIMERIC ANTIBODY (39%)
ANTI-CCR4 CHIMERIC ANTIBODY (27%)
ANTI-CCR4 CHIMERIC ANTIBODY (18%)
ANTI-CCR4 CHIMERIC ANTIBODY (8%)
CYTOTOXIC ACTIVITY (%)
100
80
60
40
20
0
0.0001
0.001
0.01
0.1
1
10
ANTIBODY CONCENTRATION (μg/ml)

FIG. 20

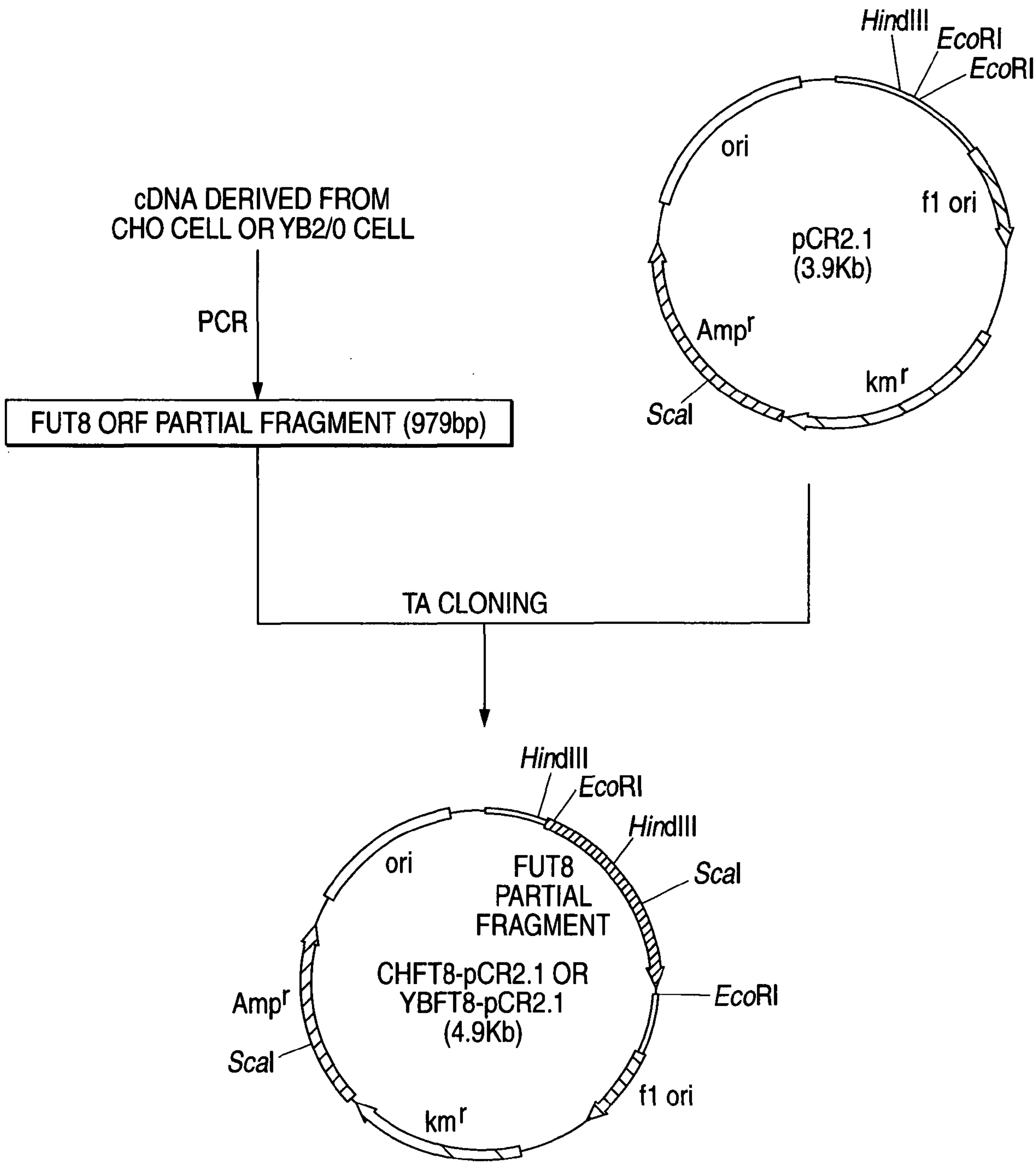

cDNA DERIVED FROM CHO CELL OR YB2/0 CELL
PCR
FUT8 ORF PARTIAL FRAGMENT (979bp)
HindIII
EcoRI
EcoRI
ori
f1 ori
pCR2.1 (3.9Kb)
Amp^r
km^r
ScaI
TA CLONING
HindIII
EcoRI
HindIII
ScaI
ori
FUT8 PARTIAL FRAGMENT
CHFT8-pCR2.1 OR YBFT8-pCR2.1 (4.9Kb)
Amp^r
EcoRI
ScaI
km^r
f1 ori

*FIG. 21*

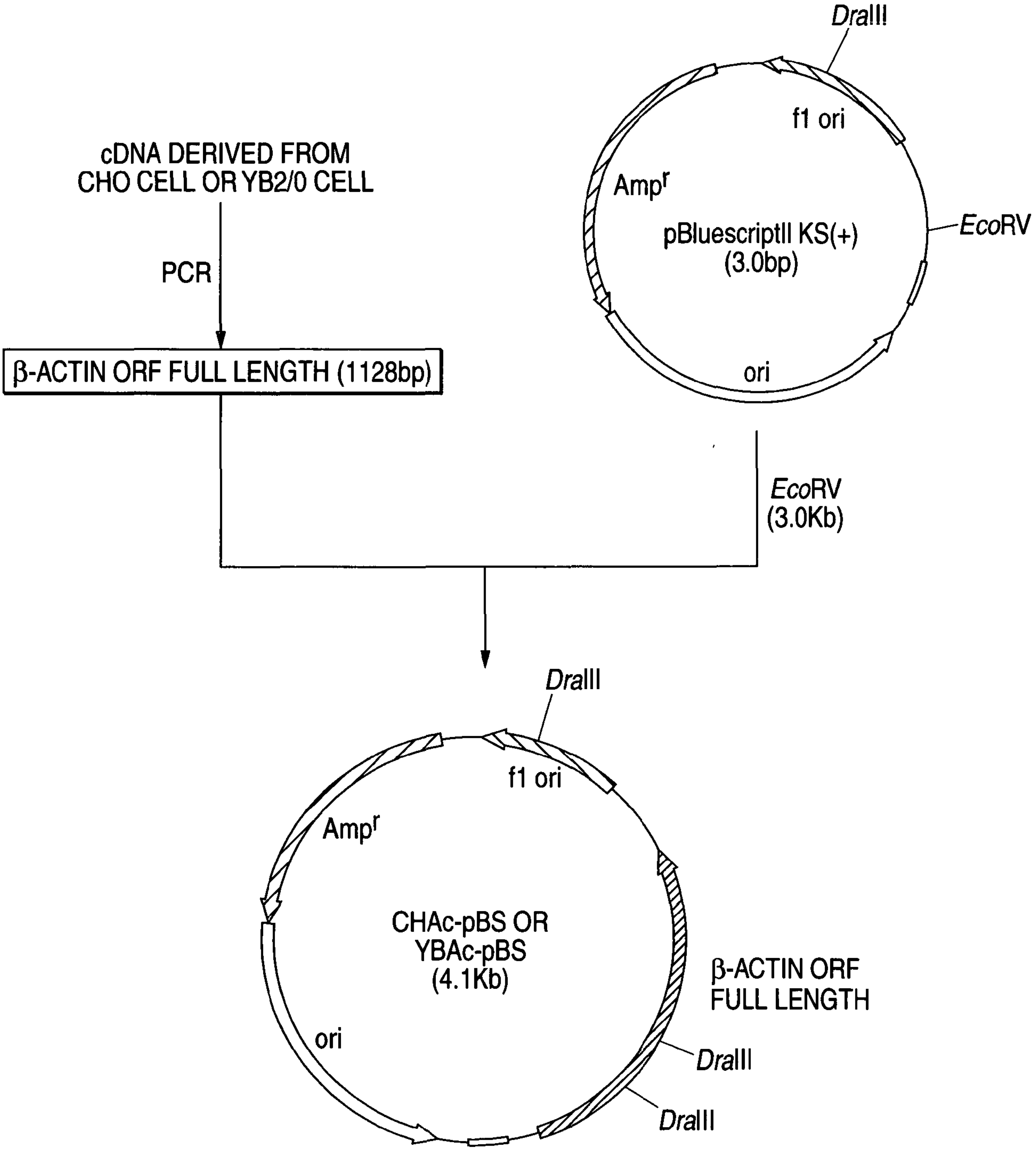
cDNA DERIVED FROM
CHO CELL OR YB2/0 CELL
PCR
β-ACTIN ORF FULL LENGTH (1128bp)
DraIII
f1 ori
Amp$^r$
pBluescriptII KS(+)
(3.0bp)
EcoRV
ori
EcoRV
(3.0Kb)
DraIII
f1 ori
Amp$^r$
CHAc-pBS OR
YBAc-pBS
(4.1Kb)
β-ACTIN ORF
FULL LENGTH
ori
DraIII
DraIII

*FIG. 22*

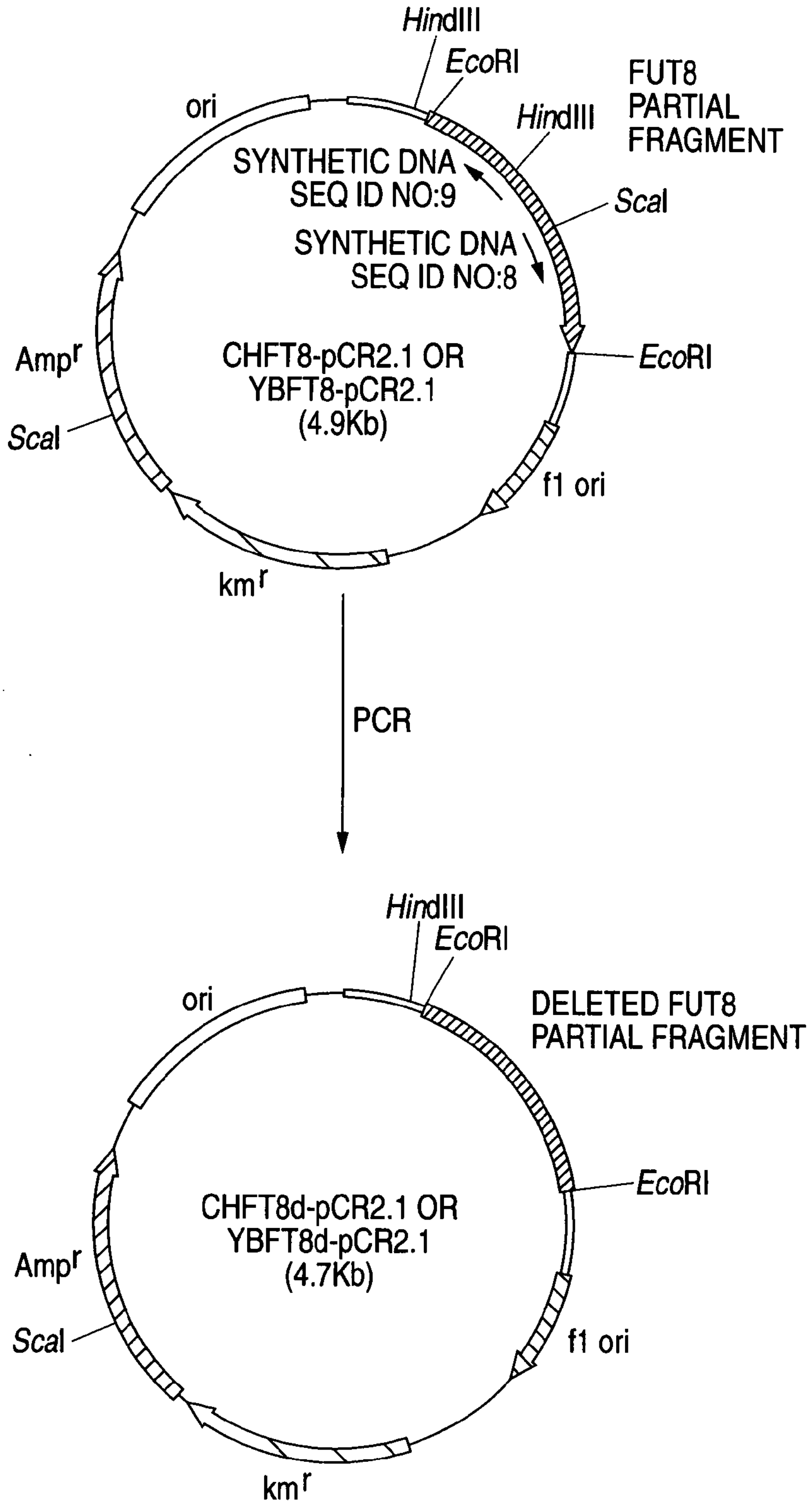
HindIII
EcoRI
FUT8
PARTIAL
FRAGMENT
ori
HindIII
SYNTHETIC DNA
SEQ ID NO:9
ScaI
SYNTHETIC DNA
SEQ ID NO:8
Amp^r
CHFT8-pCR2.1 OR
YBFT8-pCR2.1
(4.9Kb)
EcoRI
ScaI
f1 ori
km^r
PCR
HindIII
EcoRI
ori
DELETED FUT8
PARTIAL FRAGMENT
EcoRI
CHFT8d-pCR2.1 OR
YBFT8d-pCR2.1
(4.7Kb)
Amp^r
ScaI
f1 ori
km^r

*FIG. 23*

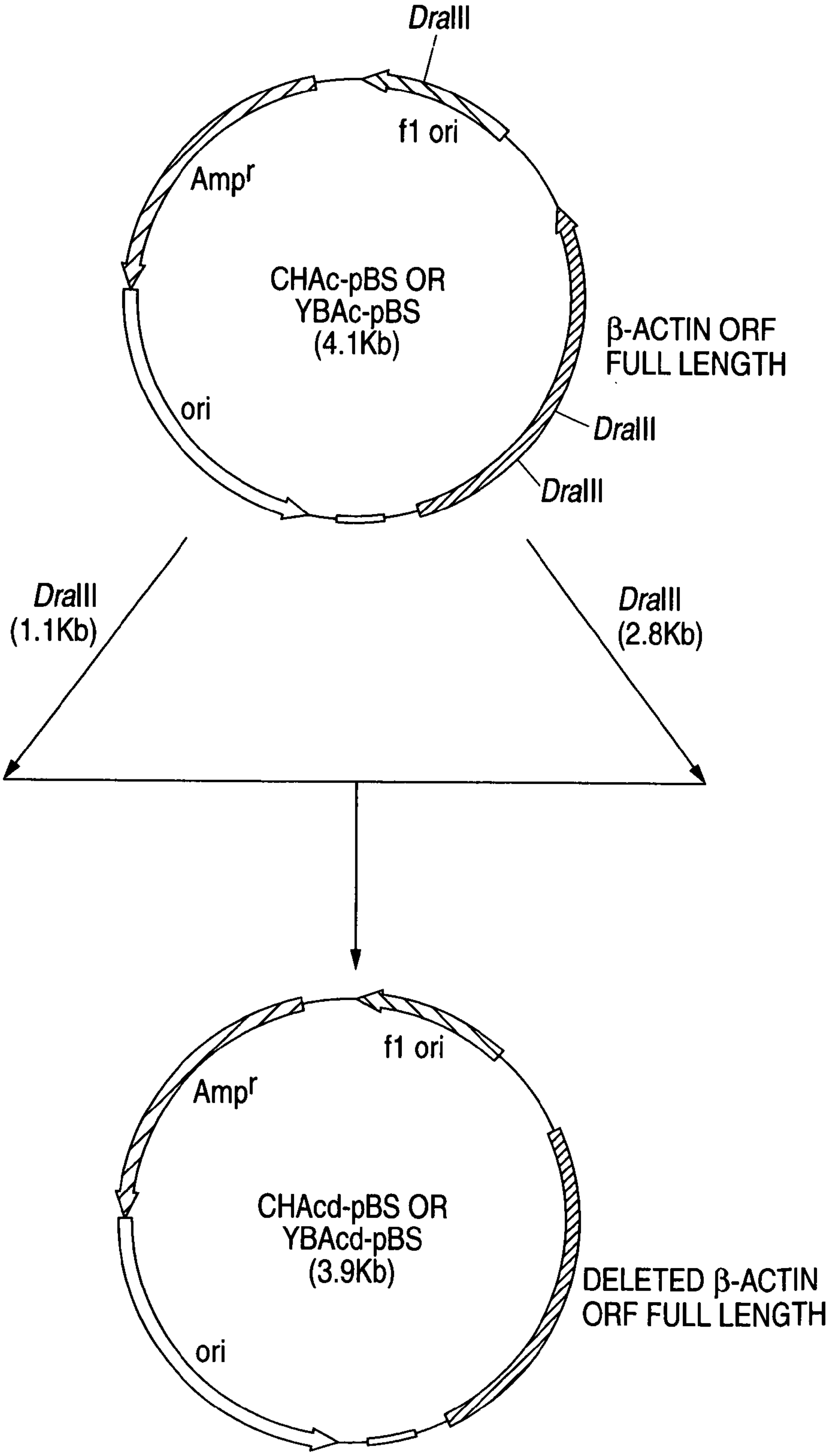
*Dra*III
f1 ori
Amp$^r$
CHAc-pBS OR
YBAc-pBS
(4.1Kb)
β-ACTIN ORF
FULL LENGTH
ori
*Dra*III
*Dra*III
*Dra*III
(1.1Kb)
*Dra*III
(2.8Kb)
f1 ori
Amp$^r$
CHAcd-pBS OR
YBAcd-pBS
(3.9Kb)
DELETED β-ACTIN
ORF FULL LENGTH
ori

FIG. 24

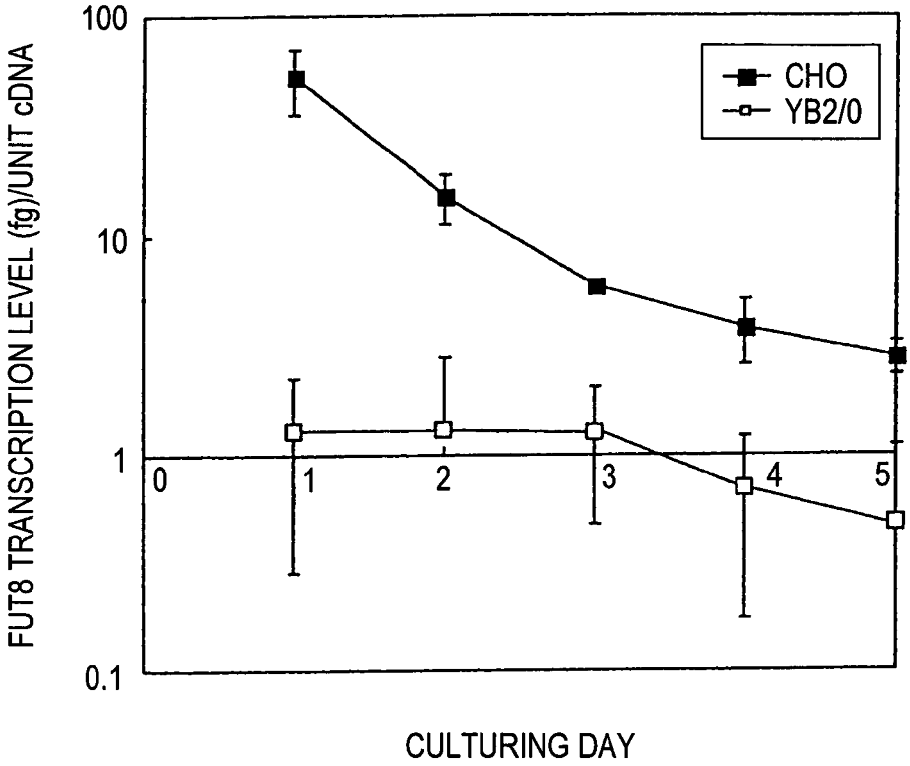
FUT8 TRANSCRIPTION LEVEL (fg)/UNIT cDNA
100
10
1
0.1
0
1
2
3
4
5
CHO
YB2/0
CULTURING DAY

FIG. 25

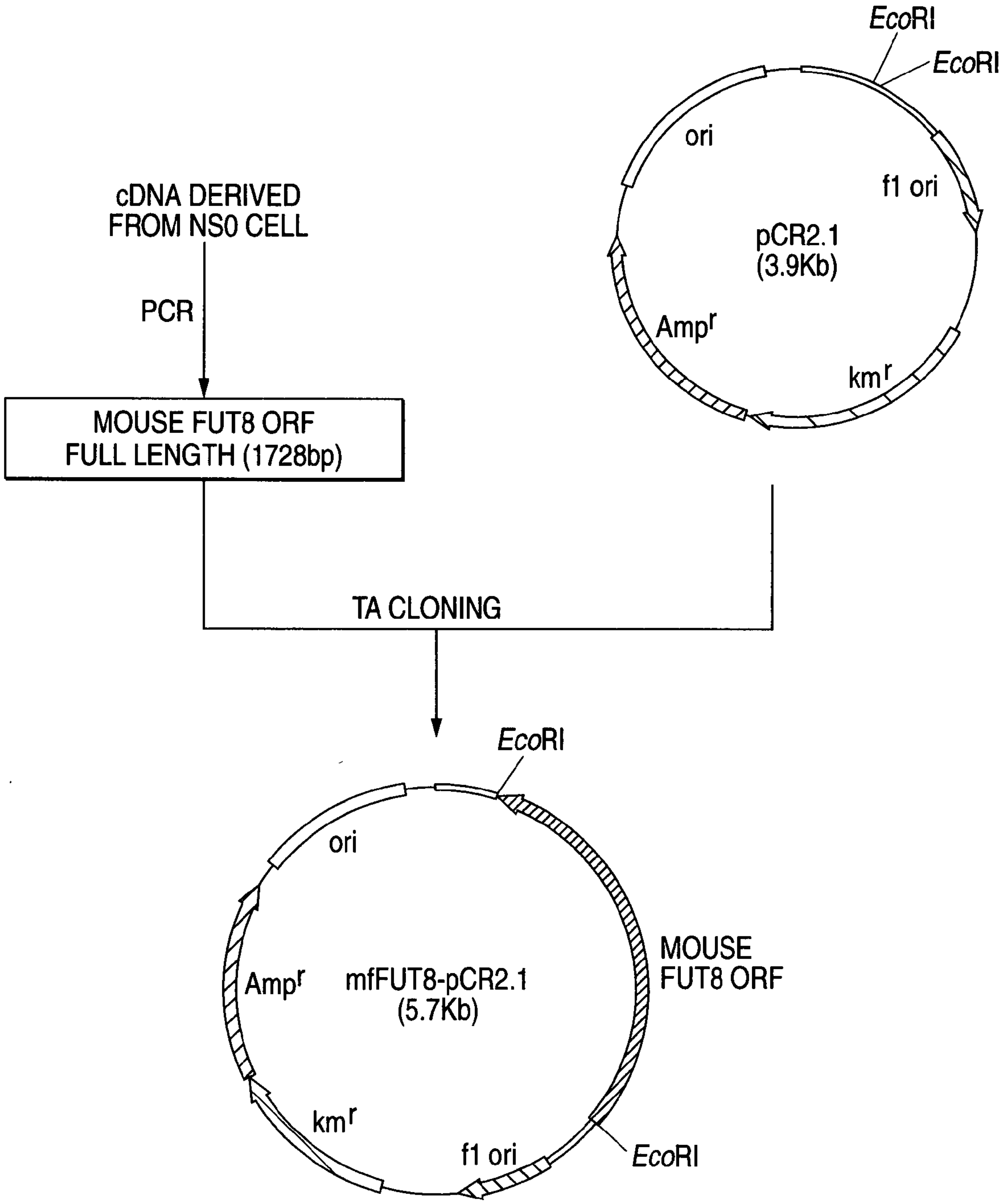

cDNA DERIVED
FROM NS0 CELL
PCR
MOUSE FUT8 ORF
FULL LENGTH (1728bp)
EcoRI
EcoRI
ori
f1 ori
pCR2.1
(3.9Kb)
Amp$^r$
km$^r$
TA CLONING
EcoRI
ori
Amp$^r$
mfFUT8-pCR2.1
(5.7Kb)
MOUSE
FUT8 ORF
km$^r$
f1 ori
EcoRI

*FIG. 26*

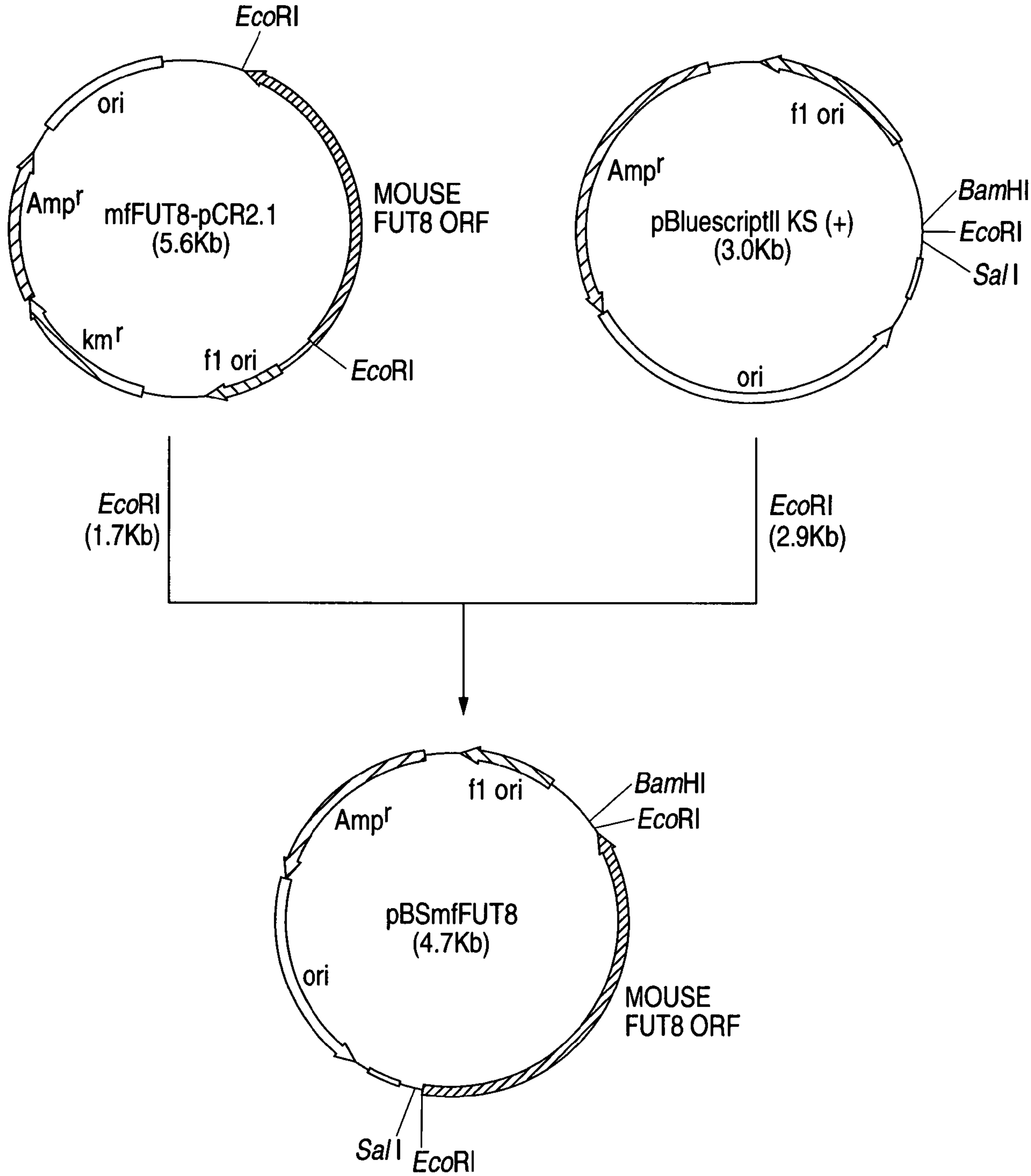
EcoRI
ori
Amp^r
mfFUT8-pCR2.1
(5.6Kb)
MOUSE
FUT8 ORF
km^r
f1 ori
EcoRI
f1 ori
Amp^r
pBluescriptII KS (+)
(3.0Kb)
BamHI
EcoRI
SalI
ori
EcoRI
(1.7Kb)
EcoRI
(2.9Kb)
f1 ori
Amp^r
BamHI
EcoRI
pBSmfFUT8
(4.7Kb)
ori
MOUSE
FUT8 ORF
SalI
EcoRI

FIG. 27

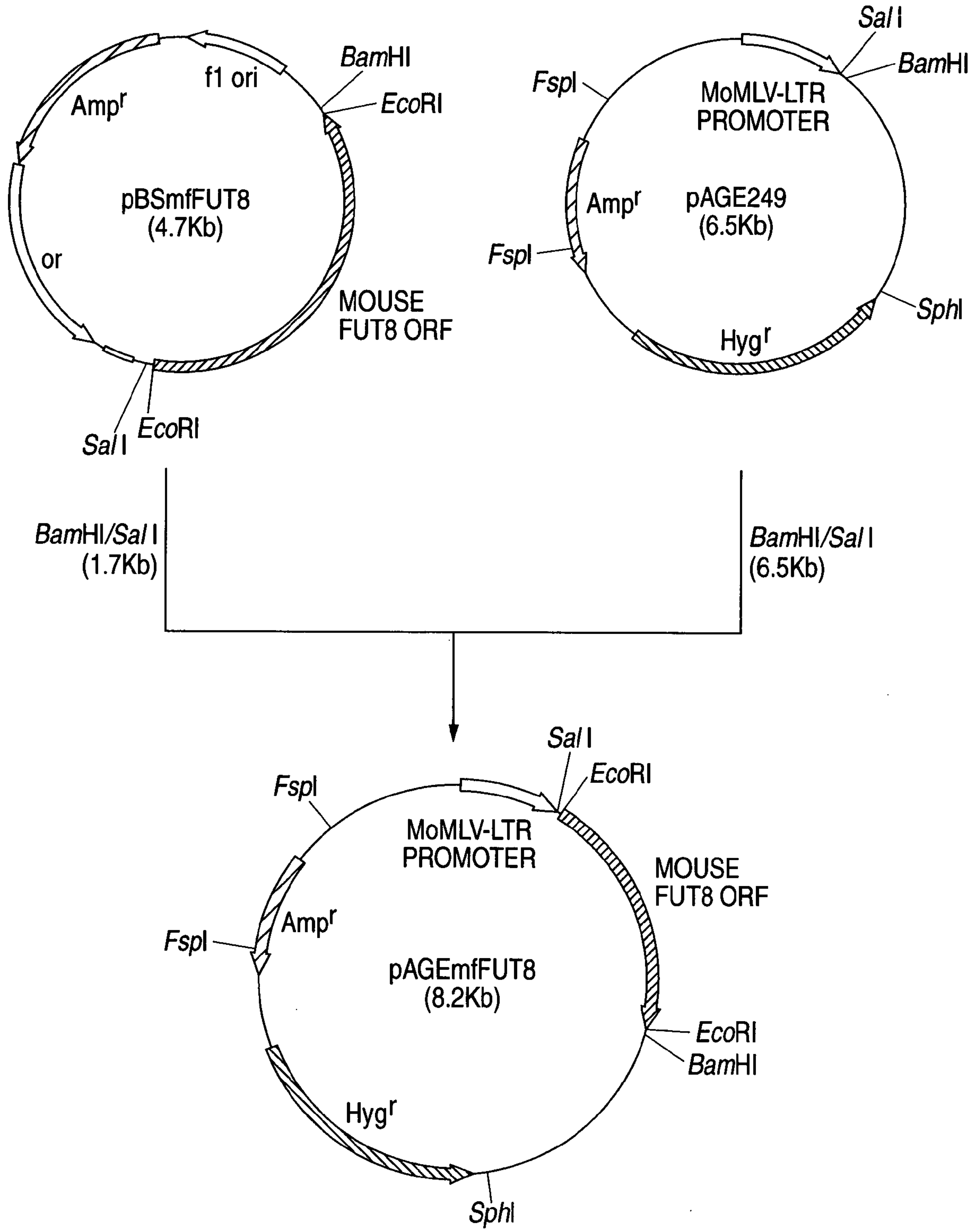
f1 ori
BamHI
EcoRI
Ampr
pBSmfFUT8
(4.7Kb)
or
MOUSE
FUT8 ORF
Sal I
EcoRI
Sal I
BamHI
FspI
MoMLV-LTR
PROMOTER
Ampr
pAGE249
(6.5Kb)
FspI
SphI
Hygr
BamHI/Sal I
(1.7Kb)
BamHI/Sal I
(6.5Kb)
Sal I
EcoRI
FspI
MoMLV-LTR
PROMOTER
MOUSE
FUT8 ORF
Ampr
FspI
pAGEmfFUT8
(8.2Kb)
EcoRI
BamHI
Hygr
SphI

FIG. 28

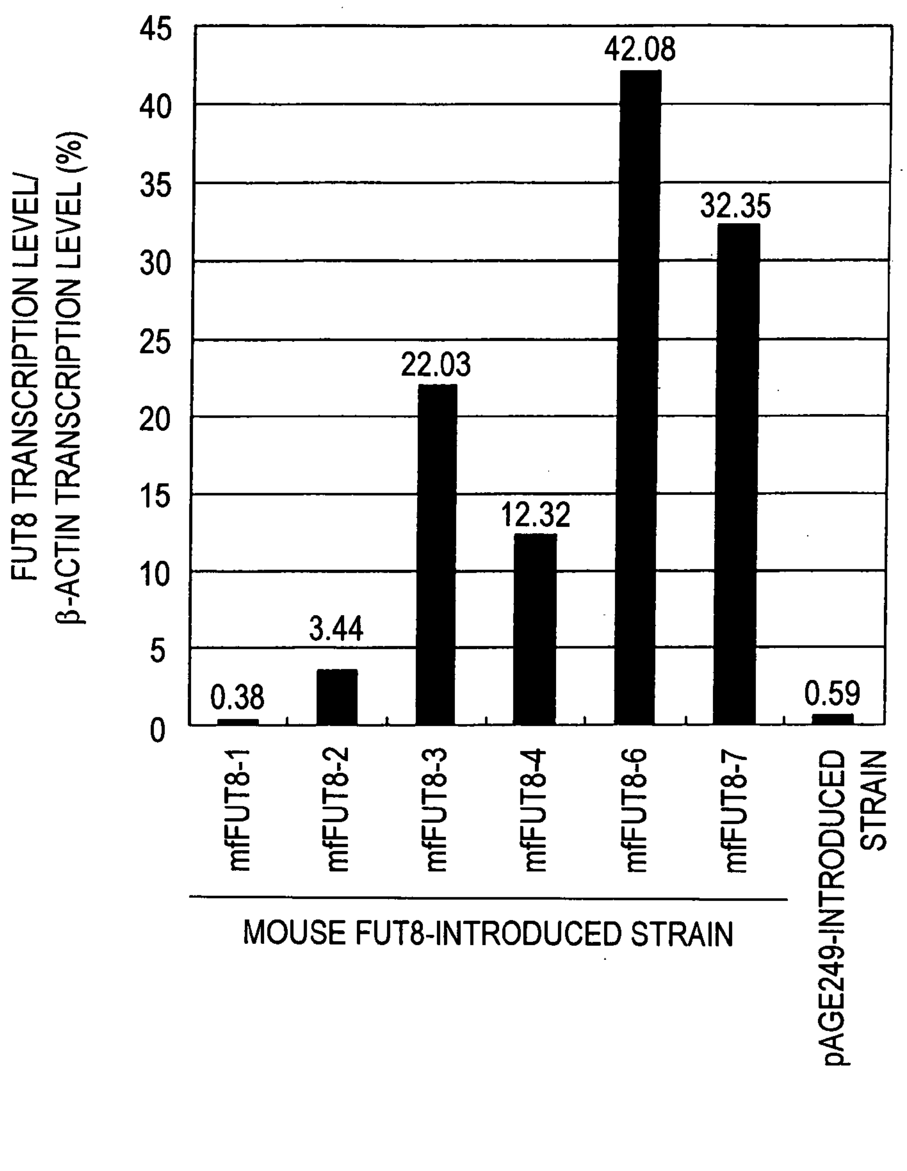
FUT8 TRANSCRIPTION LEVEL/
β-ACTIN TRANSCRIPTION LEVEL (%)
45
40
35
30
25
20
15
10
5
0
0.38
3.44
22.03
12.32
42.08
32.35
0.59
mfFUT8-1
mfFUT8-2
mfFUT8-3
mfFUT8-4
mfFUT8-6
mfFUT8-7
pAGE249-INTRODUCED STRAIN
MOUSE FUT8-INTRODUCED STRAIN

FIG. 29

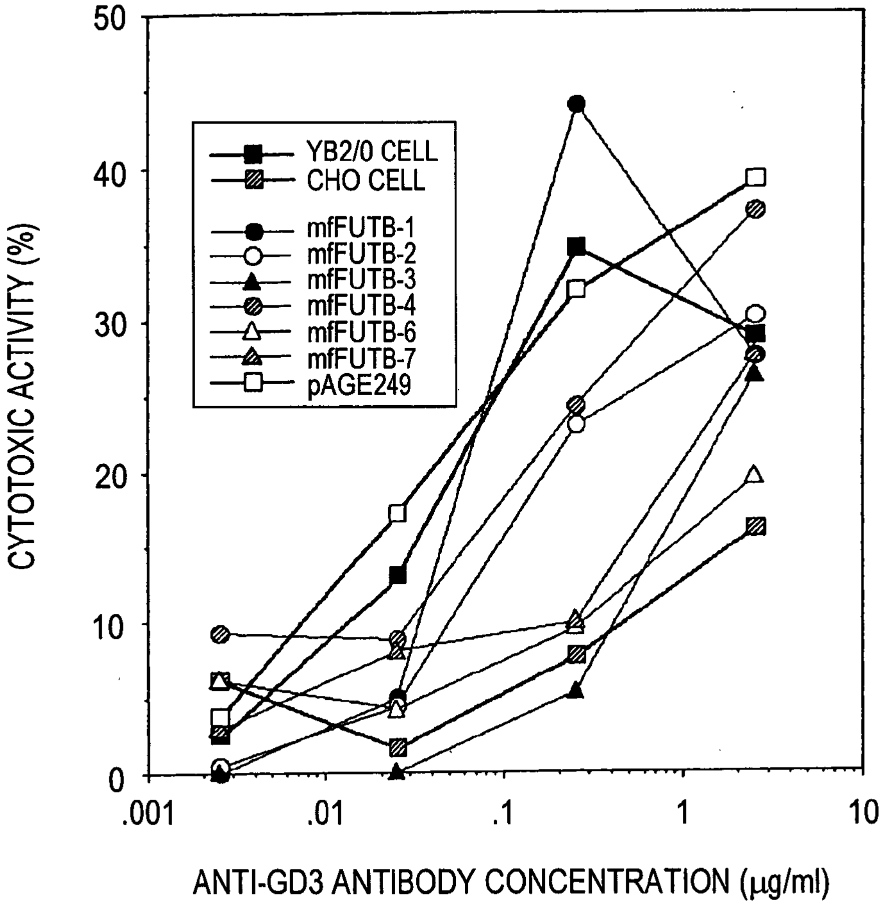
50
40
30
20
10
0
.001
.01
.1
1
10
CYTOTOXIC ACTIVITY (%)
ANTI-GD3 ANTIBODY CONCENTRATION (μg/ml)
YB2/0 CELL
CHO CELL
mfFUTB-1
mfFUTB-2
mfFUTB-3
mfFUTB-4
mfFUTB-6
mfFUTB-7
pAGE249

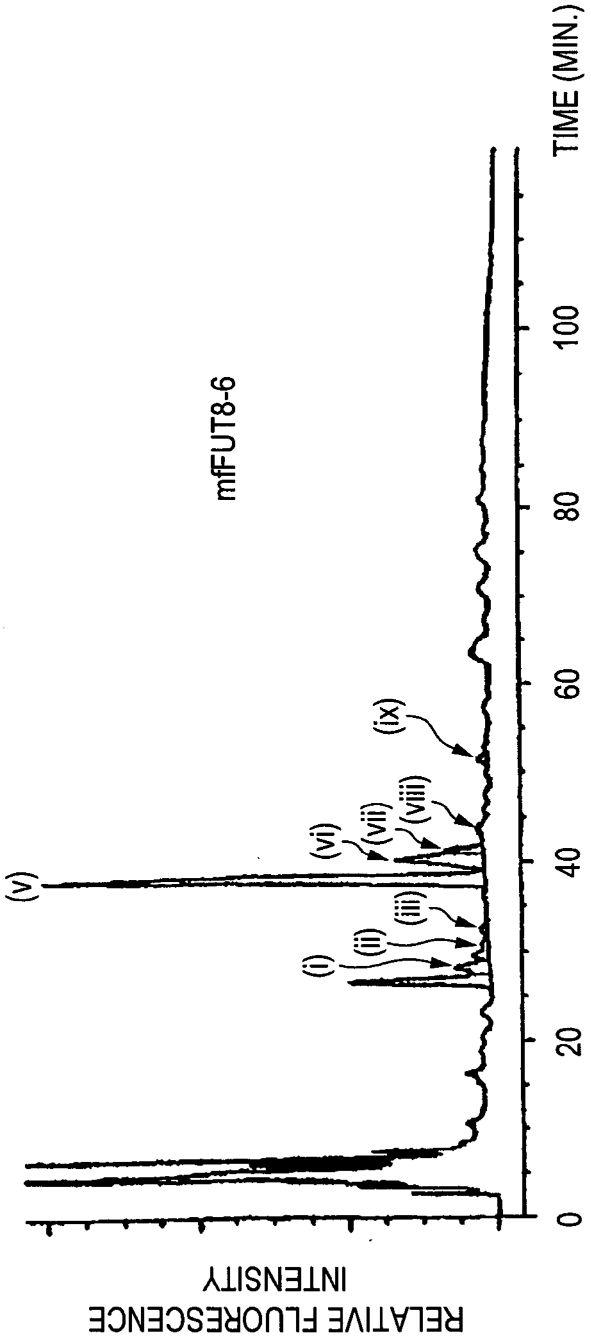


FIG. 30A

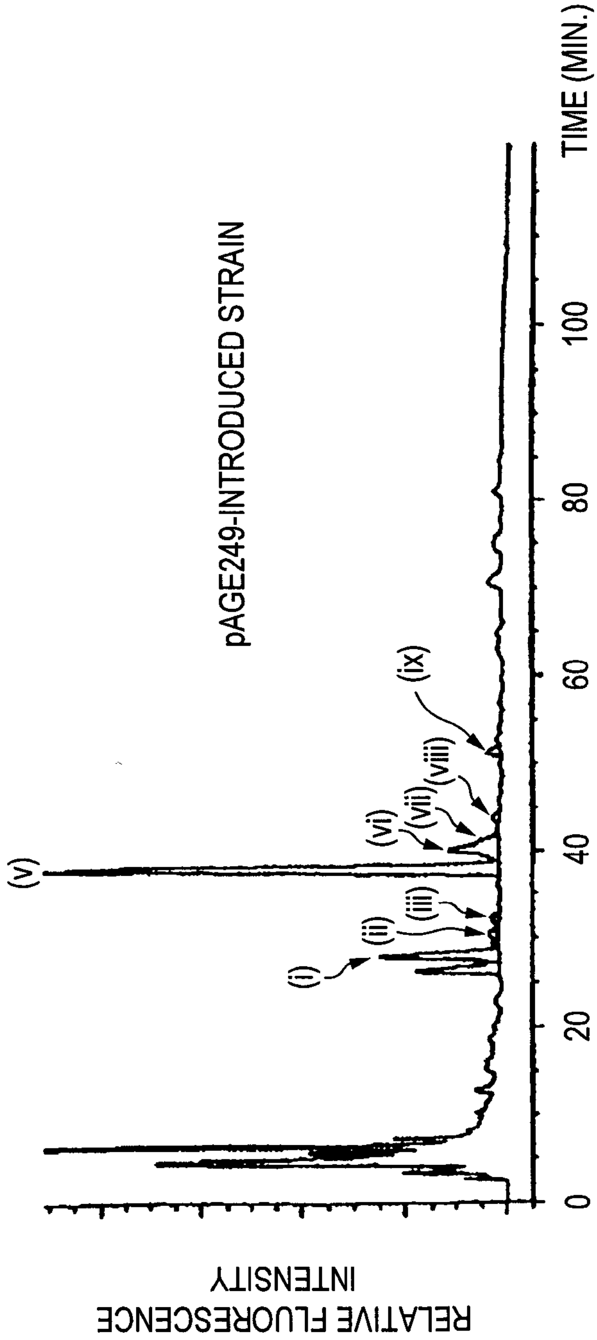


FIG. 30B

FIG. 31

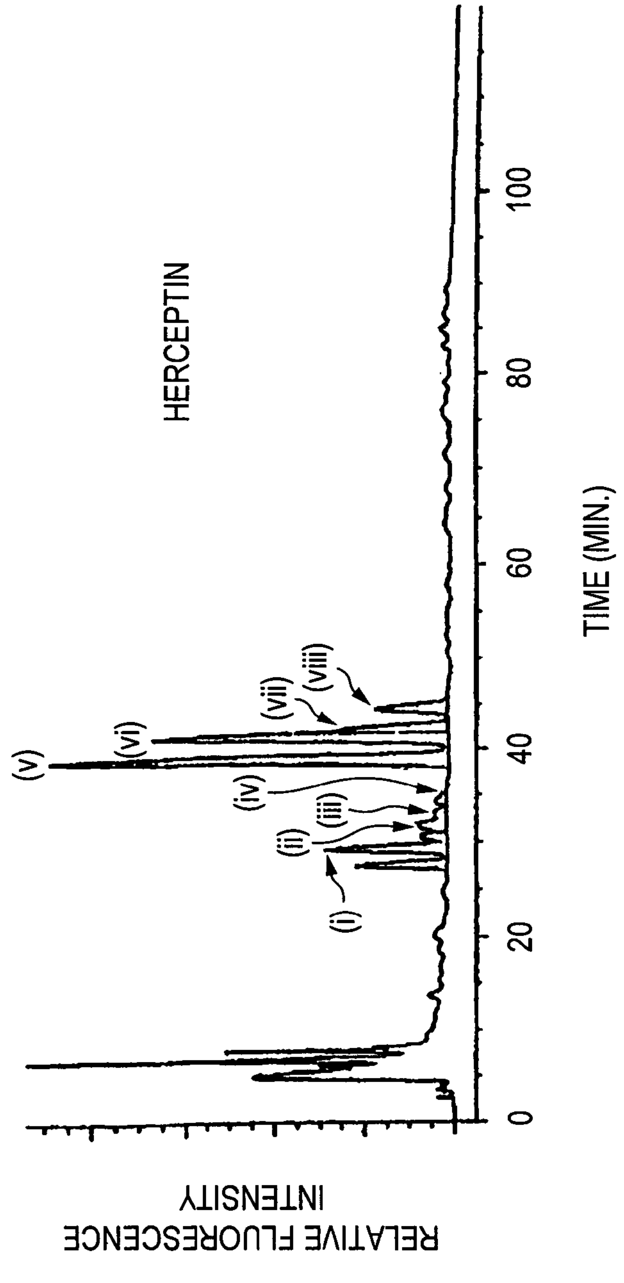
HERCEPTIN
(i)
(ii)
(iii)
(iv)
(v)
(vi)
(vii)
(viii)
0
20
40
60
80
100
TIME (MIN.)
RELATIVE FLUORESCENCE INTENSITY

*FIG. 32*

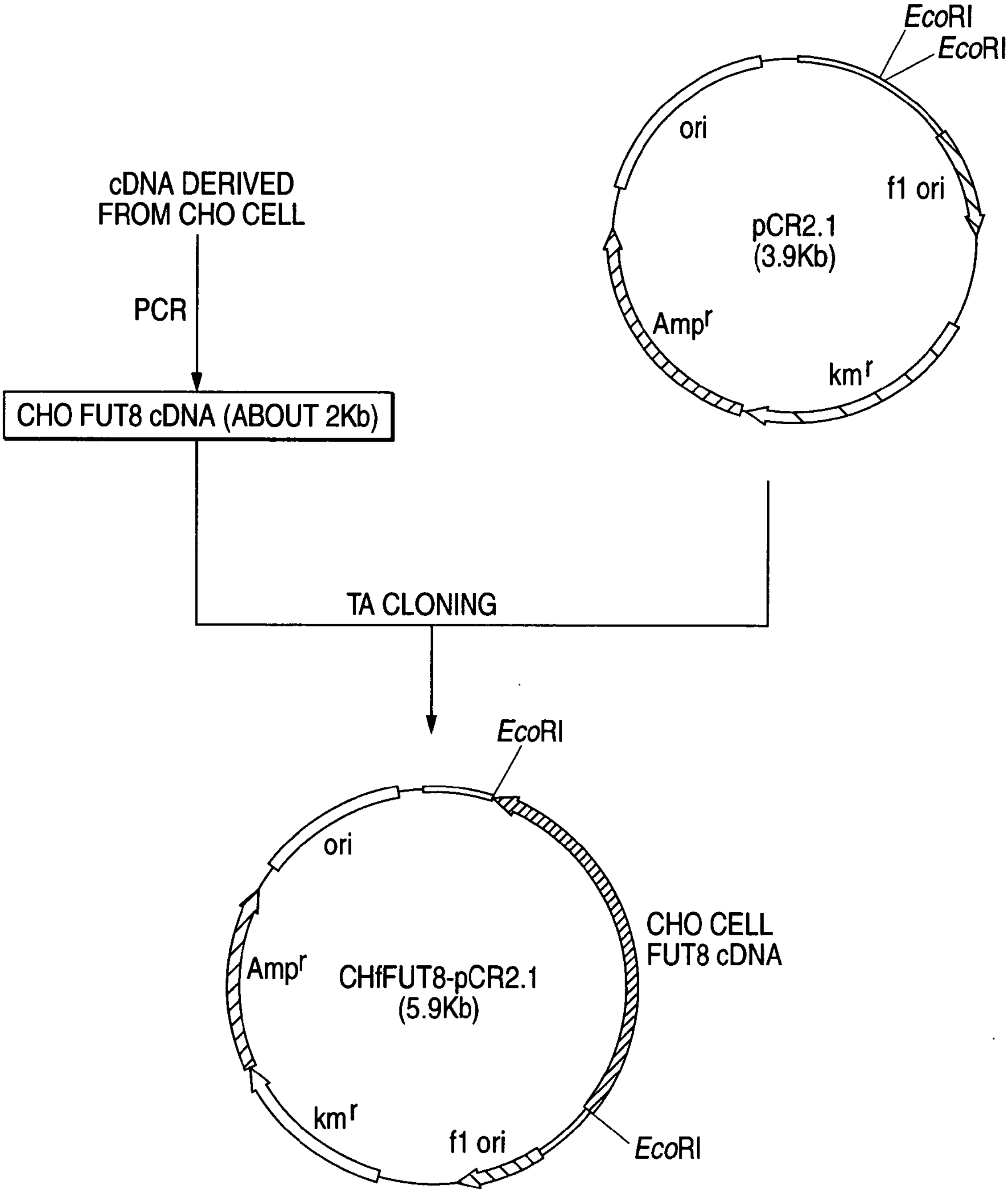
cDNA DERIVED
FROM CHO CELL
PCR
CHO FUT8 cDNA (ABOUT 2Kb)
*Eco*RI
*Eco*RI
ori
f1 ori
pCR2.1
(3.9Kb)
Amp^r
km^r
TA CLONING
*Eco*RI
ori
Amp^r
CHfFUT8-pCR2.1
(5.9Kb)
CHO CELL
FUT8 cDNA
km^r
f1 ori
*Eco*RI

FIG. 33

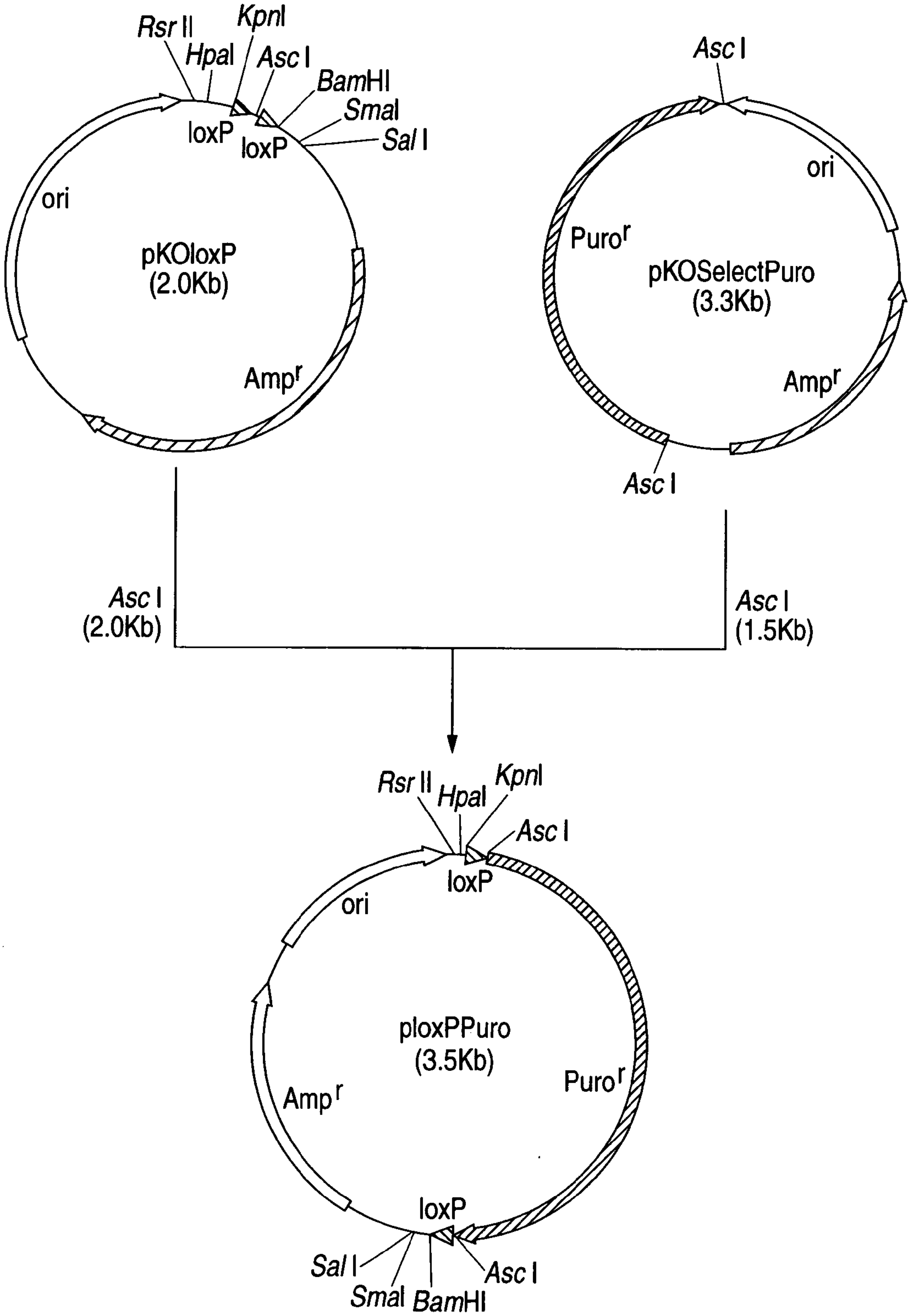
Rsr II
Hpal
KpnI
Asc I
BamHI
SmaI
Sal I
loxP
loxP
ori
pKOloxP
(2.0Kb)
Amp^r
Asc I
ori
Puro^r
pKOSelectPuro
(3.3Kb)
Amp^r
Asc I
Asc I
(2.0Kb)
Asc I
(1.5Kb)
Rsr II
Hpal
KpnI
Asc I
loxP
ori
ploxPPuro
(3.5Kb)
Amp^r
Puro^r
loxP
Sal I
SmaI
BamHI
Asc I

FIG. 34

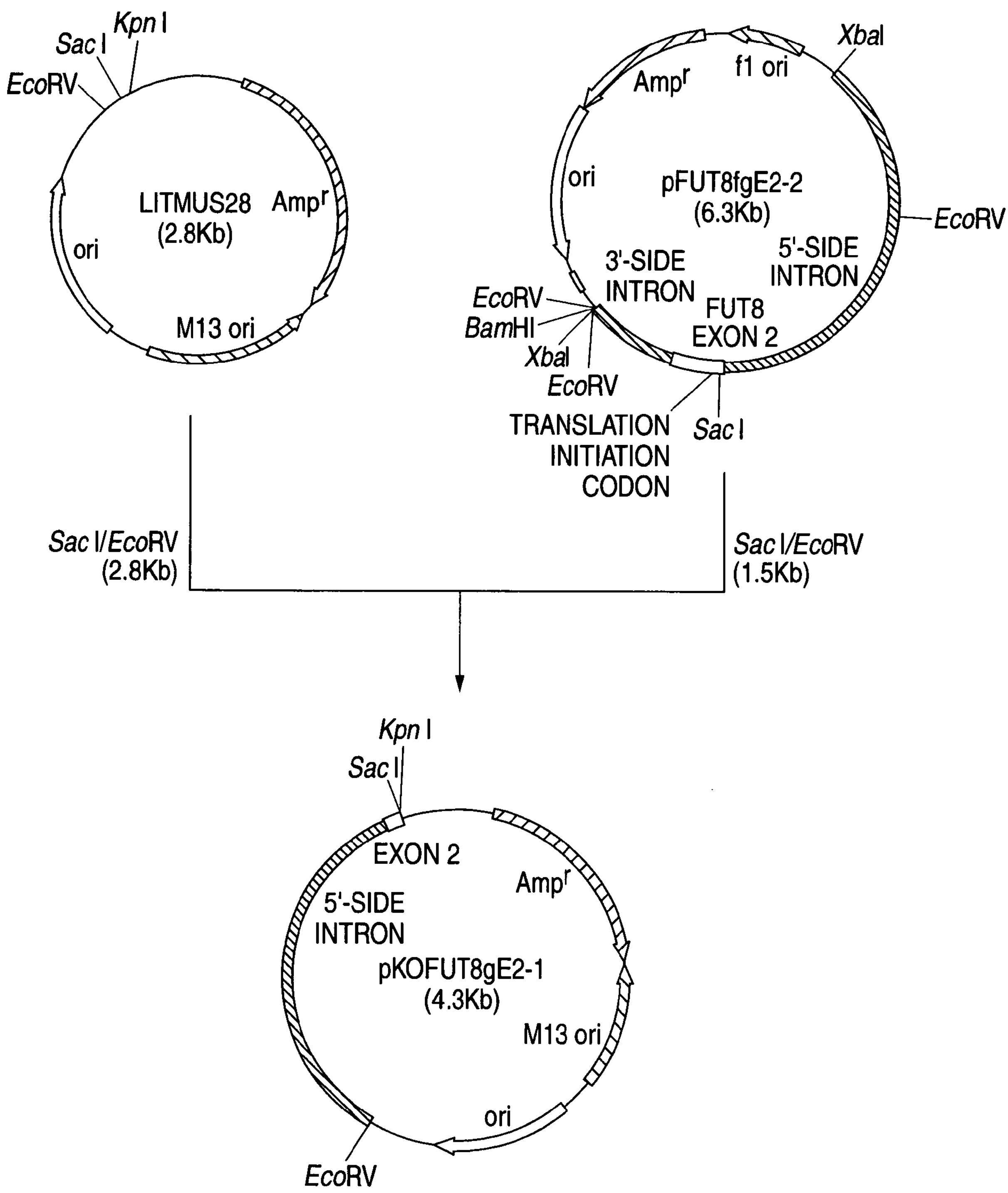
Kpn I
Sac I
EcoRV
LITMUS28
(2.8Kb)
Amp^r
ori
M13 ori
Amp^r
f1 ori
XbaI
ori
pFUT8fgE2-2
(6.3Kb)
EcoRV
3'-SIDE
INTRON
5'-SIDE
INTRON
EcoRV
BamHI
XbaI
EcoRV
FUT8
EXON 2
TRANSLATION
INITIATION
CODON
Sac I
Sac I/EcoRV
(2.8Kb)
Sac I/EcoRV
(1.5Kb)
Kpn I
Sac I
EXON 2
Amp^r
5'-SIDE
INTRON
pKOFUT8gE2-1
(4.3Kb)
M13 ori
ori
EcoRV

FIG. 35

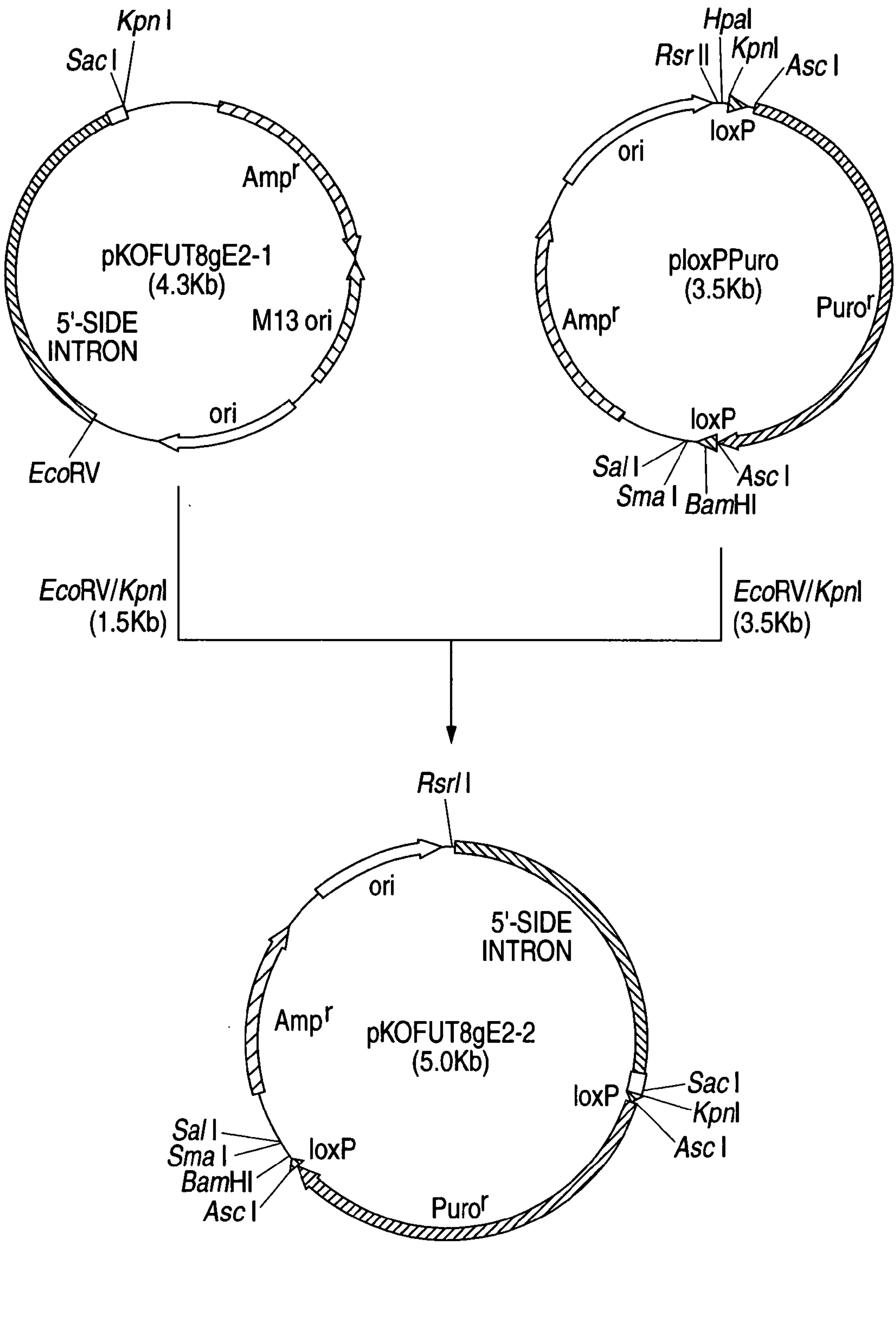

Kpn I
Sac I
Amp^r
pKOFUT8gE2-1
(4.3Kb)
5'-SIDE
INTRON
M13 ori
ori
EcoRV
Hpa I
Rsr II
Kpn I
Asc I
loxP
ori
ploxPPuro
(3.5Kb)
Amp^r
Puro^r
loxP
Sal I
Sma I
BamHI
Asc I
EcoRV/KpnI
(1.5Kb)
EcoRV/KpnI
(3.5Kb)
RsrII
ori
5'-SIDE
INTRON
Amp^r
pKOFUT8gE2-2
(5.0Kb)
loxP
Sac I
KpnI
Asc I
Sal I
Sma I
BamHI
Asc I
loxP
Puro^r

FIG. 36

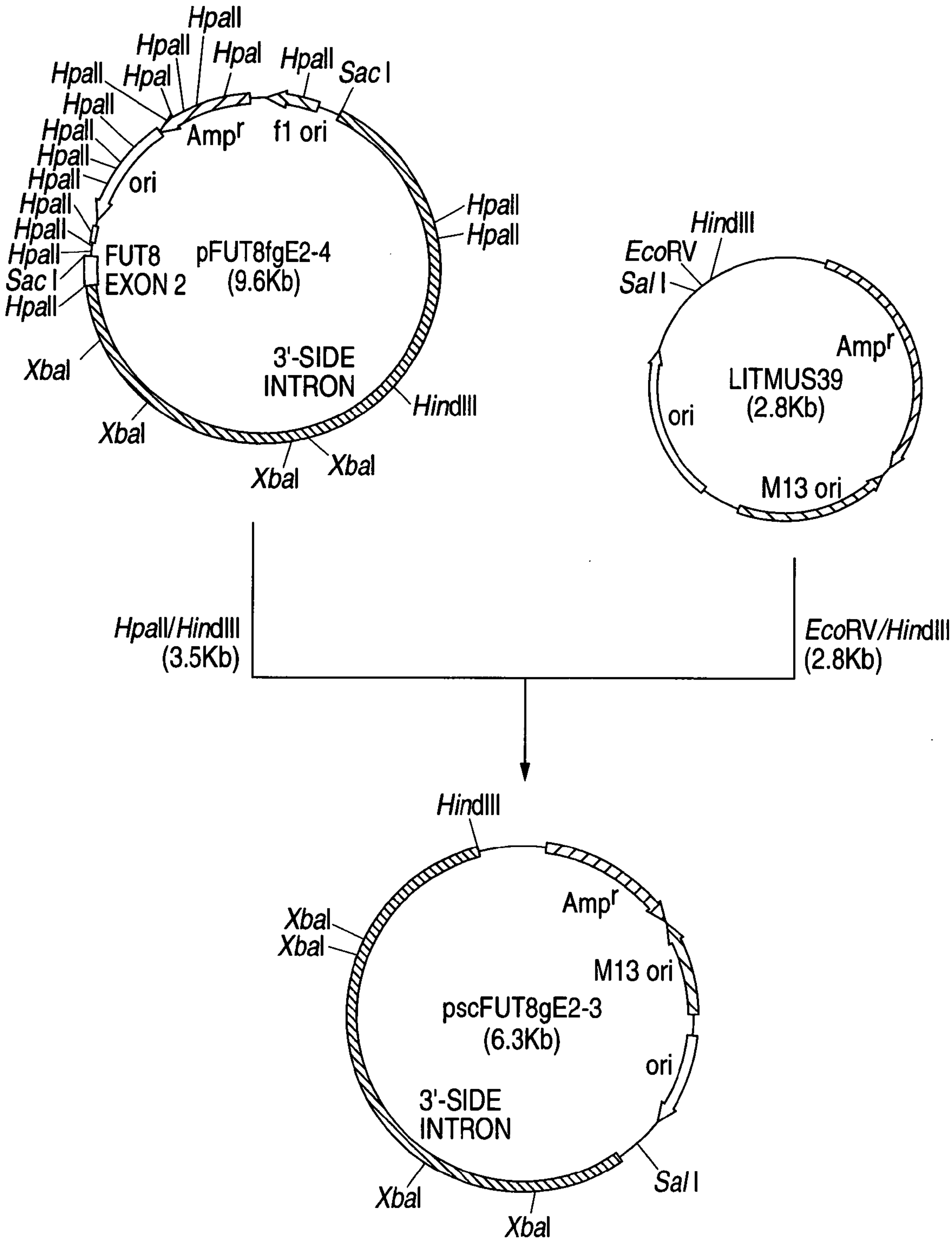
HpaII
HpaII
HpaI
HpaII
SacI
HpaII
HpaI
HpaII
HpaII
HpaII
HpaII
HpaII
HpaII
HpaII
SacI
HpaII
Amp^r
f1 ori
ori
FUT8
EXON 2
pFUT8fgE2-4
(9.6Kb)
3'-SIDE
INTRON
HpaII
HpaII
HindIII
XbaI
XbaI
XbaI
XbaI
HindIII
EcoRV
SalI
Amp^r
LITMUS39
(2.8Kb)
ori
M13 ori
HpaII/HindIII
(3.5Kb)
EcoRV/HindIII
(2.8Kb)
HindIII
XbaI
XbaI
Amp^r
M13 ori
pscFUT8gE2-3
(6.3Kb)
ori
3'-SIDE
INTRON
XbaI
XbaI
SalI

FIG. 37

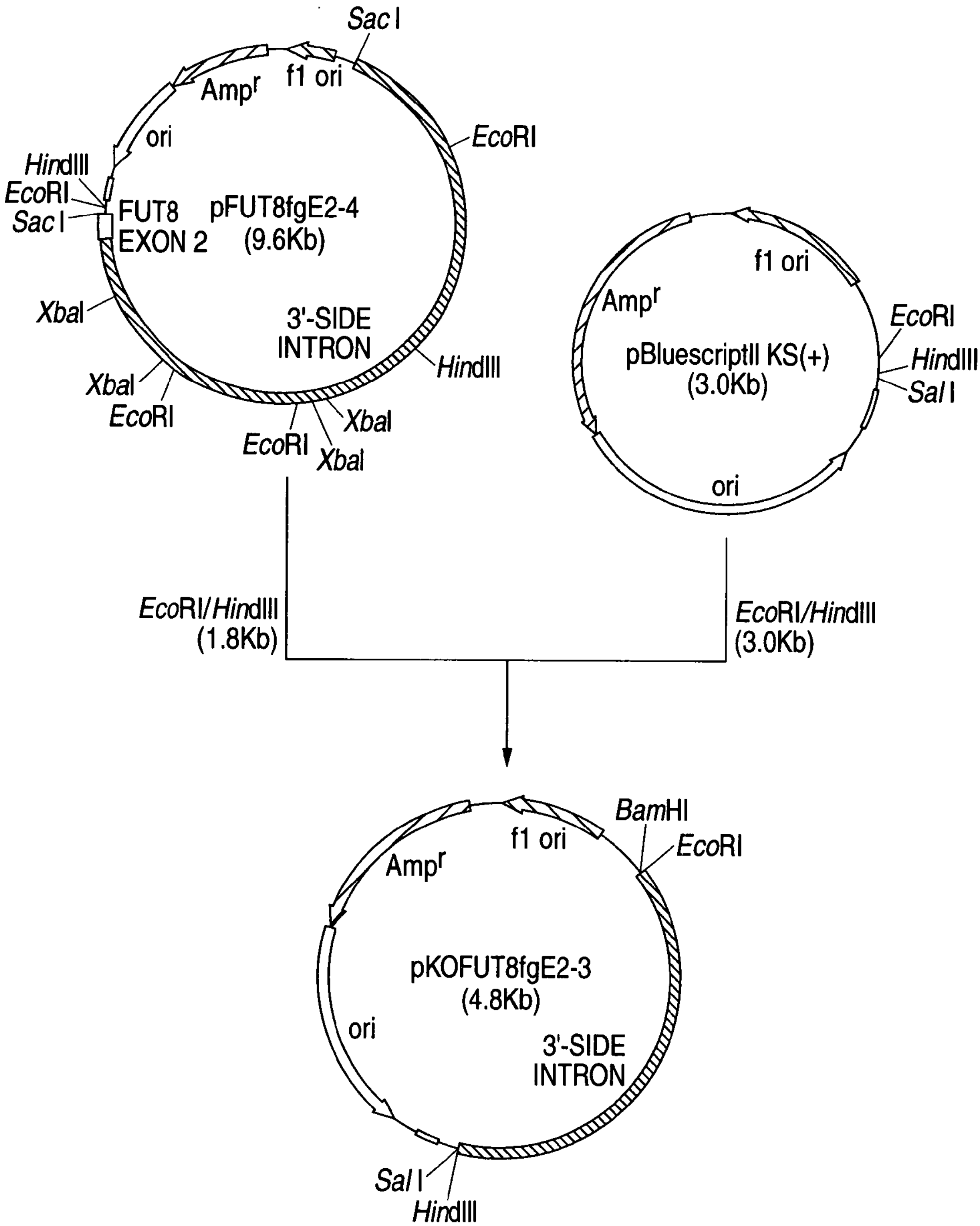

SacI
f1 ori
Amp^r
EcoRI
ori
HindIII
EcoRI
SacI
FUT8
EXON 2
pFUT8fgE2-4
(9.6Kb)
XbaI
3'-SIDE
INTRON
HindIII
XbaI
EcoRI
XbaI
EcoRI
XbaI
f1 ori
Amp^r
EcoRI
HindIII
SalI
pBluescriptII KS(+)
(3.0Kb)
ori
EcoRI/HindIII
(1.8Kb)
EcoRI/HindIII
(3.0Kb)
BamHI
EcoRI
f1 ori
Amp^r
pKOFUT8fgE2-3
(4.8Kb)
ori
3'-SIDE
INTRON
SalI
HindIII

*FIG. 38*

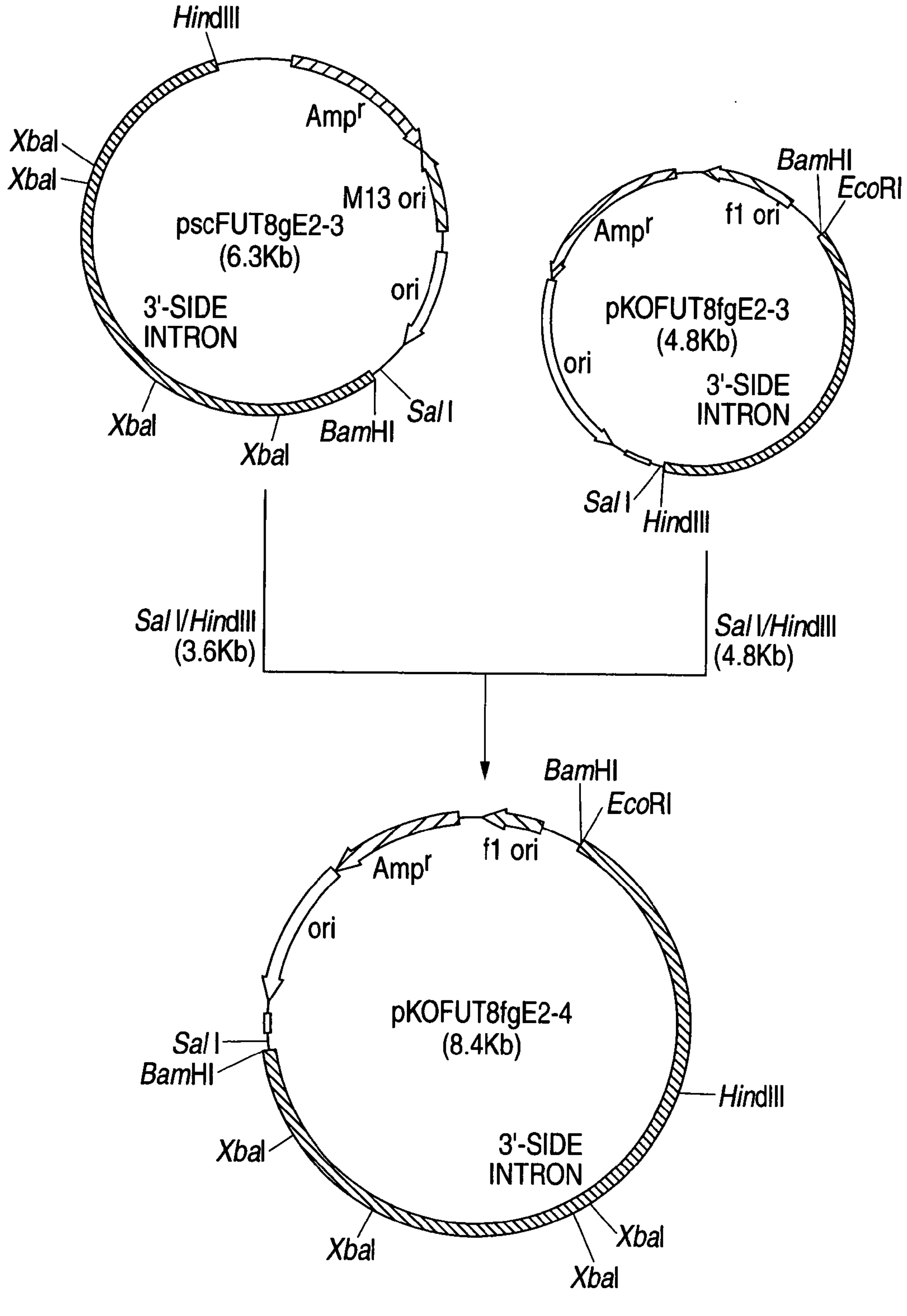
HindIII
Amp^r
XbaI
XbaI
M13 ori
pscFUT8gE2-3
(6.3Kb)
ori
3'-SIDE
INTRON
XbaI
XbaI
BamHI
SalI
BamHI
EcoRI
f1 ori
Amp^r
pKOFUT8fgE2-3
(4.8Kb)
ori
3'-SIDE
INTRON
SalI
HindIII
SalI/HindIII
(3.6Kb)
SalI/HindIII
(4.8Kb)
BamHI
EcoRI
f1 ori
Amp^r
ori
pKOFUT8fgE2-4
(8.4Kb)
SalI
BamHI
HindIII
XbaI
3'-SIDE
INTRON
XbaI
XbaI
XbaI

FIG. 39

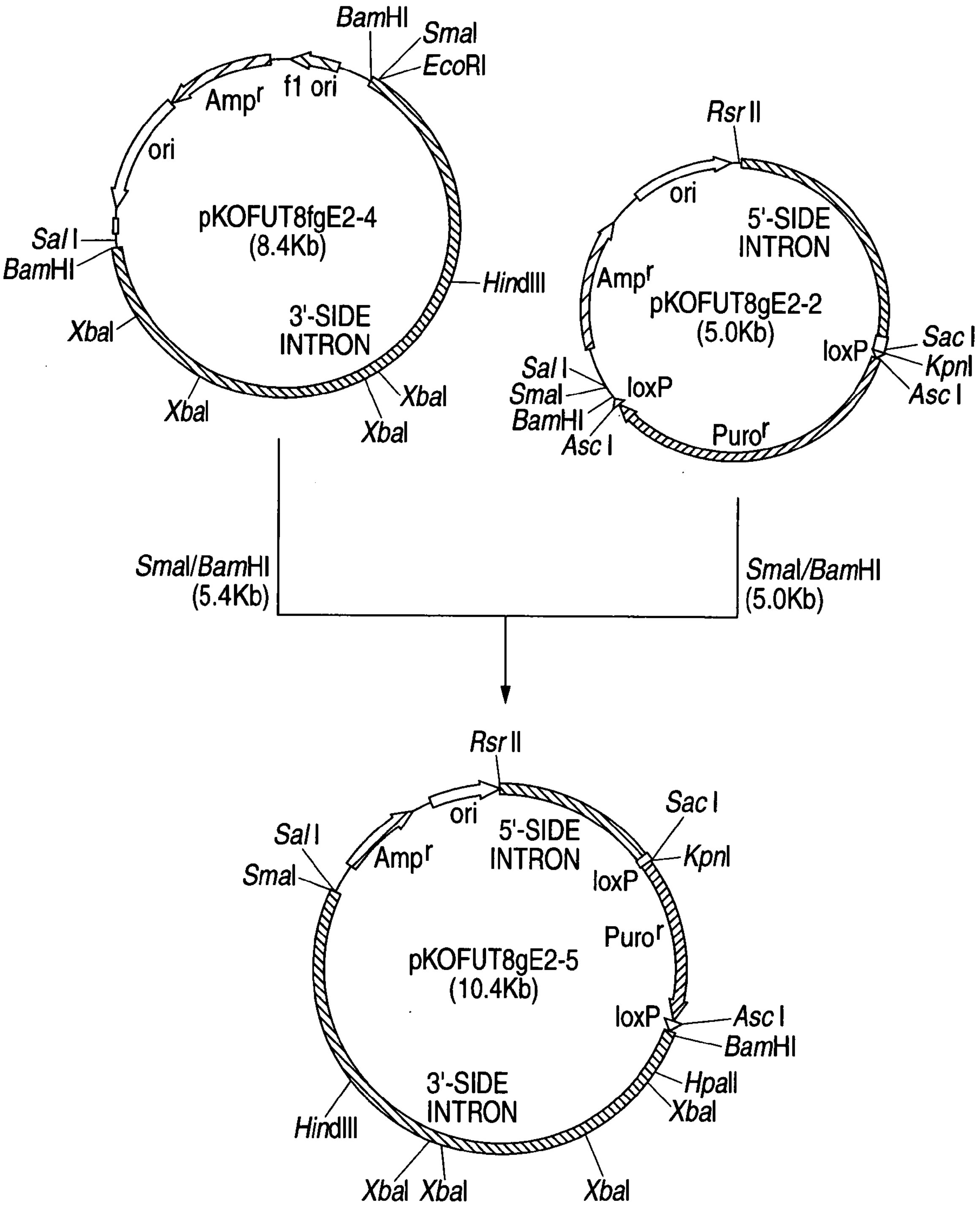
BamHI
SmaI
EcoRI
Amp^r
f1 ori
ori
pKOFUT8fgE2-4
(8.4Kb)
SalI
BamHI
HindIII
3'-SIDE INTRON
XbaI
XbaI
XbaI
XbaI
RsrII
ori
5'-SIDE INTRON
Amp^r
pKOFUT8gE2-2
(5.0Kb)
loxP
SacI
KpnI
AscI
SalI
SmaI
BamHI
AscI
loxP
Puro^r
SmaI/BamHI
(5.4Kb)
SmaI/BamHI
(5.0Kb)
RsrII
ori
5'-SIDE INTRON
SacI
KpnI
SalI
SmaI
Amp^r
loxP
Puro^r
pKOFUT8gE2-5
(10.4Kb)
loxP
AscI
BamHI
HpaII
XbaI
3'-SIDE INTRON
HindIII
XbaI
XbaI
XbaI

FIG. 40

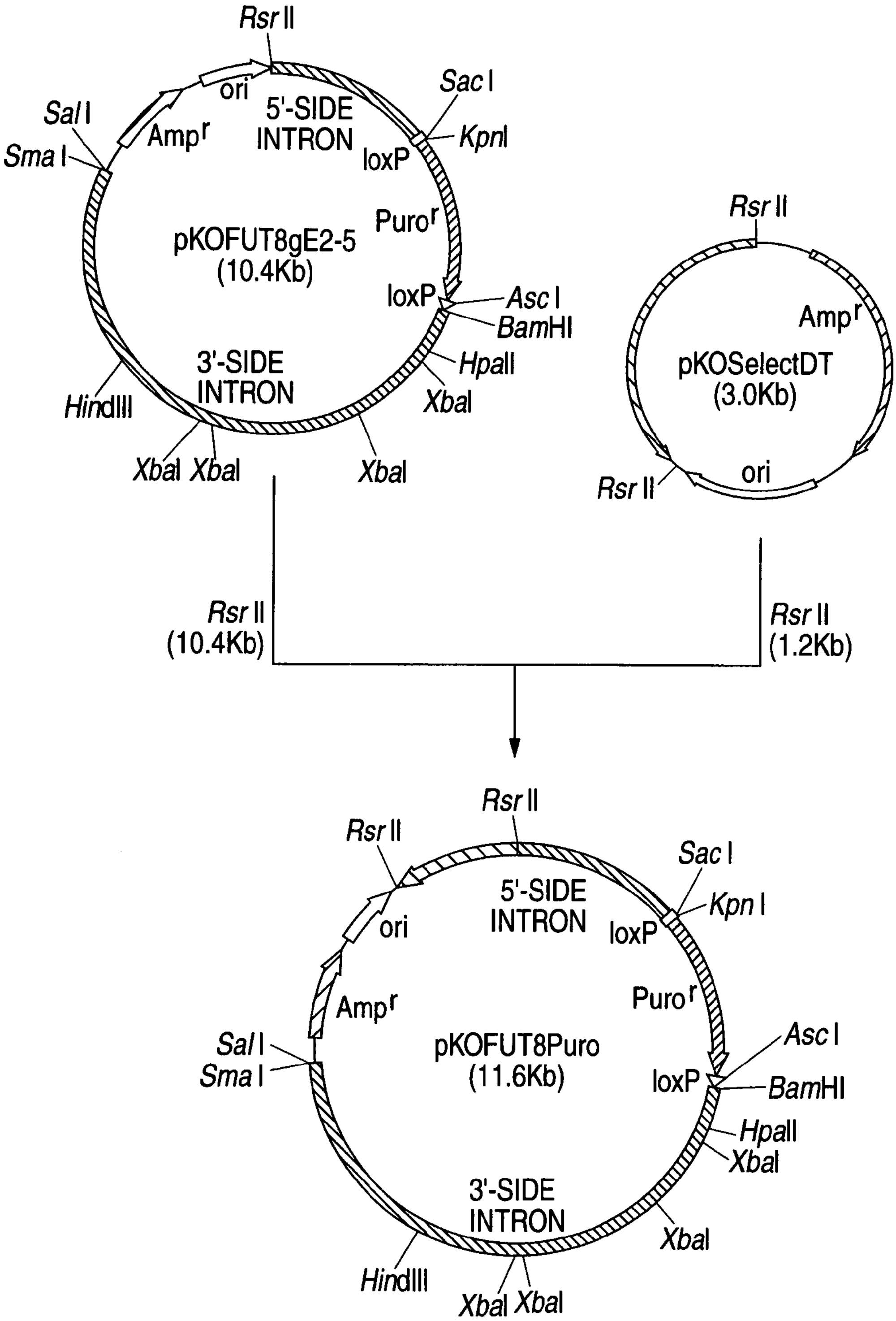
Rsr II
Sac I
Kpn I
Sal I
Sma I
ori
5'-SIDE INTRON
Amp^r
loxP
Puro^r
pKOFUT8gE2-5
(10.4Kb)
loxP
Asc I
BamHI
Hpa II
Xba I
3'-SIDE INTRON
Hind III
Xba I
Xba I
Xba I
Rsr II
Amp^r
pKOSelectDT
(3.0Kb)
ori
Rsr II
Rsr II
(10.4Kb)
Rsr II
(1.2Kb)
Rsr II
Rsr II
Sac I
Kpn I
5'-SIDE INTRON
ori
loxP
Amp^r
Puro^r
Sal I
Sma I
pKOFUT8Puro
(11.6Kb)
loxP
Asc I
BamHI
Hpa II
Xba I
3'-SIDE INTRON
Xba I
Hind III
Xba I
Xba I

FIG. 41

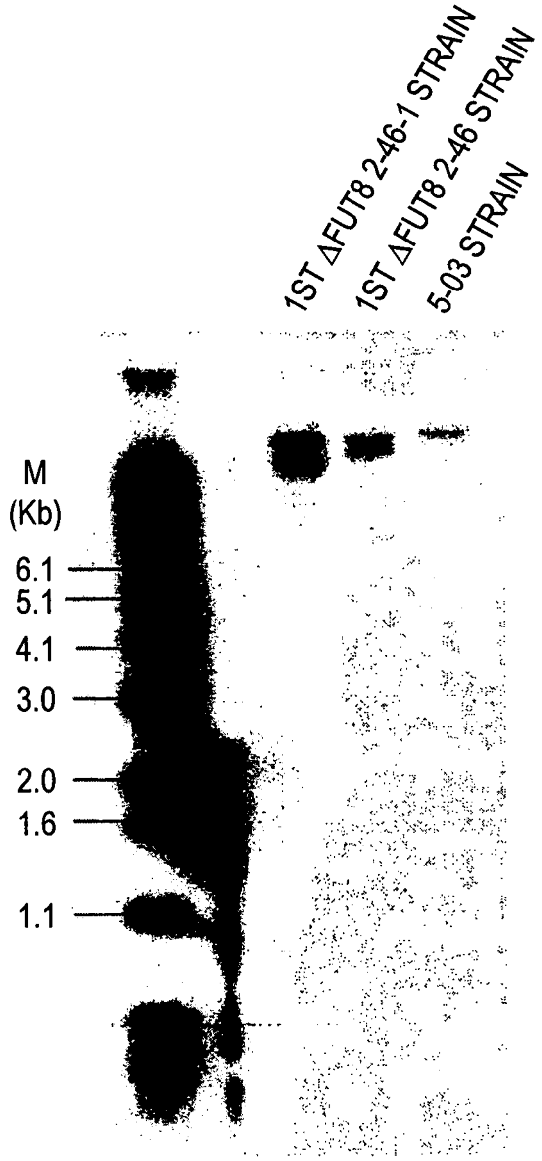
1ST ΔFUT8 2-46-1 STRAIN
1ST ΔFUT8 2-46 STRAIN
5-03 STRAIN
M
(Kb)
6.1
5.1
4.1
3.0
2.0
1.6
1.1

FIG. 42

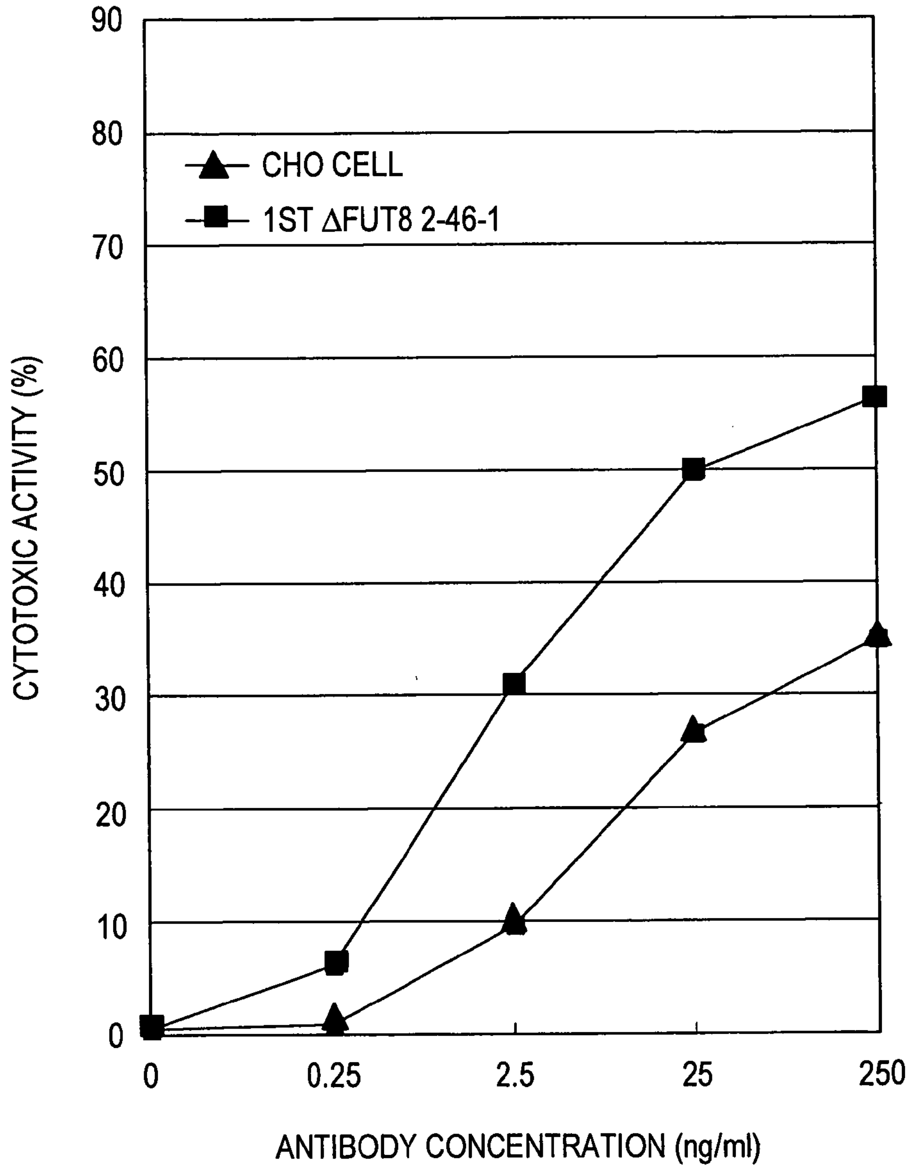
90
80
70
60
50
40
30
20
10
0
CYTOTOXIC ACTIVITY (%)
CHO CELL
1ST ΔFUT8 2-46-1
0
0.25
2.5
25
250
ANTIBODY CONCENTRATION (ng/ml)

FIG. 43

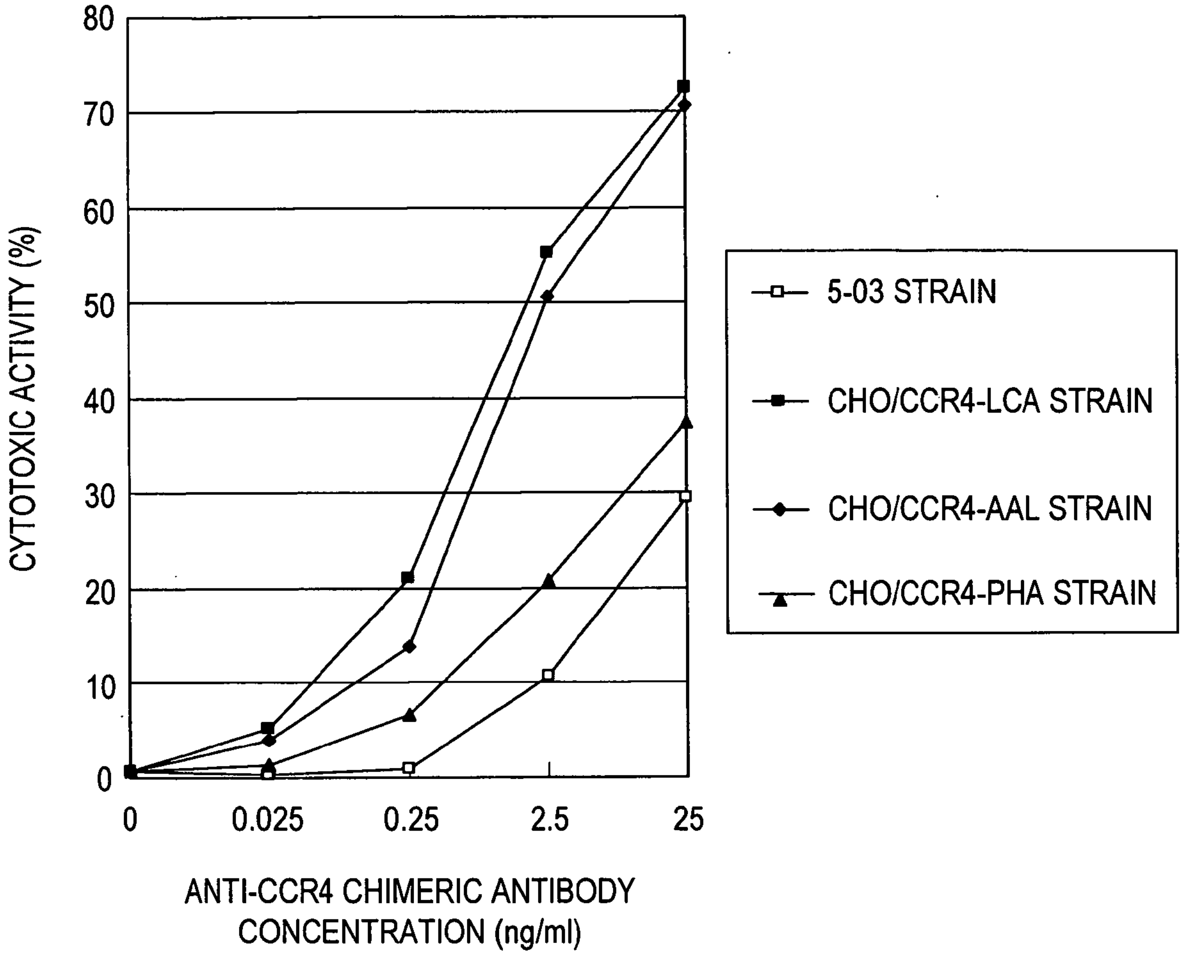
80
70
60
50
40
30
20
10
0
CYTOTOXIC ACTIVITY (%)
0
0.025
0.25
2.5
25
ANTI-CCR4 CHIMERIC ANTIBODY CONCENTRATION (ng/ml)
5-03 STRAIN
CHO/CCR4-LCA STRAIN
CHO/CCR4-AAL STRAIN
CHO/CCR4-PHA STRAIN

FIG. 44

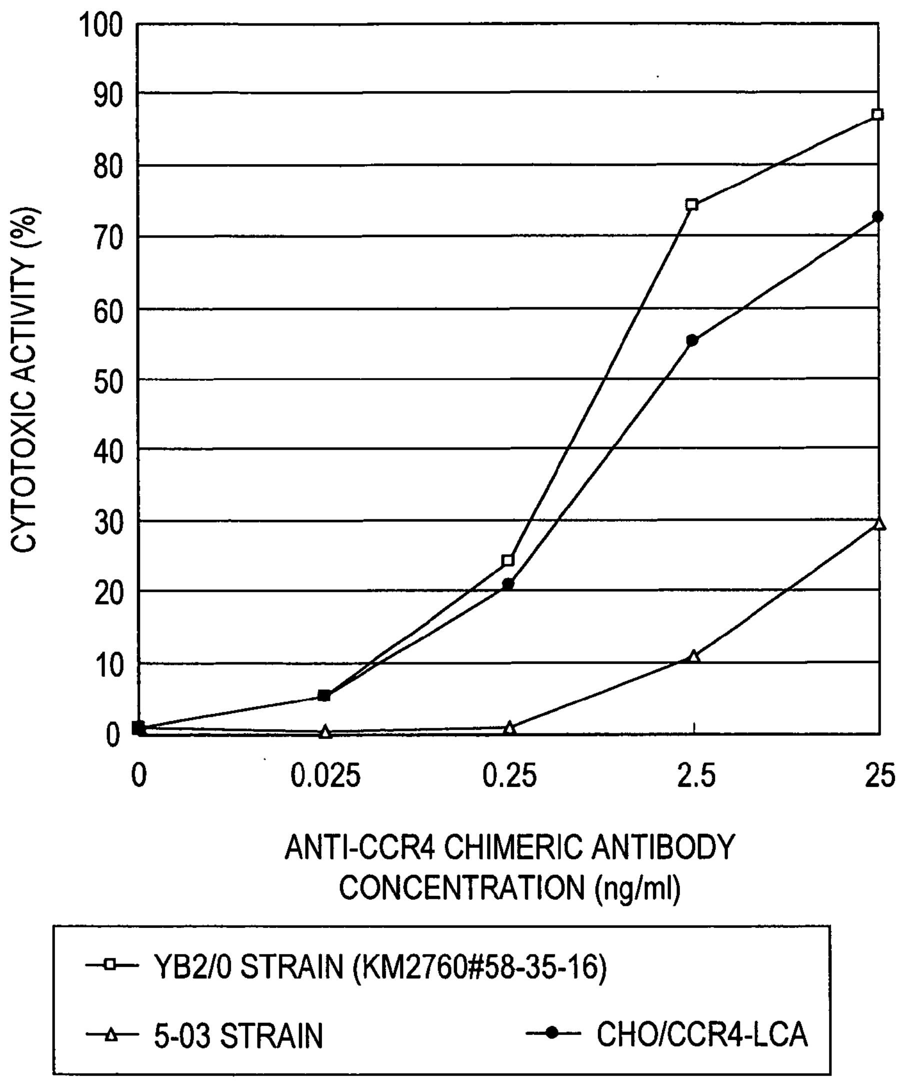
100
90
80
70
60
50
40
30
20
10
0
CYTOTOXIC ACTIVITY (%)
0
0.025
0.25
2.5
25
ANTI-CCR4 CHIMERIC ANTIBODY CONCENTRATION (ng/ml)
YB2/0 STRAIN (KM2760#58-35-16)
5-03 STRAIN
CHO/CCR4-LCA

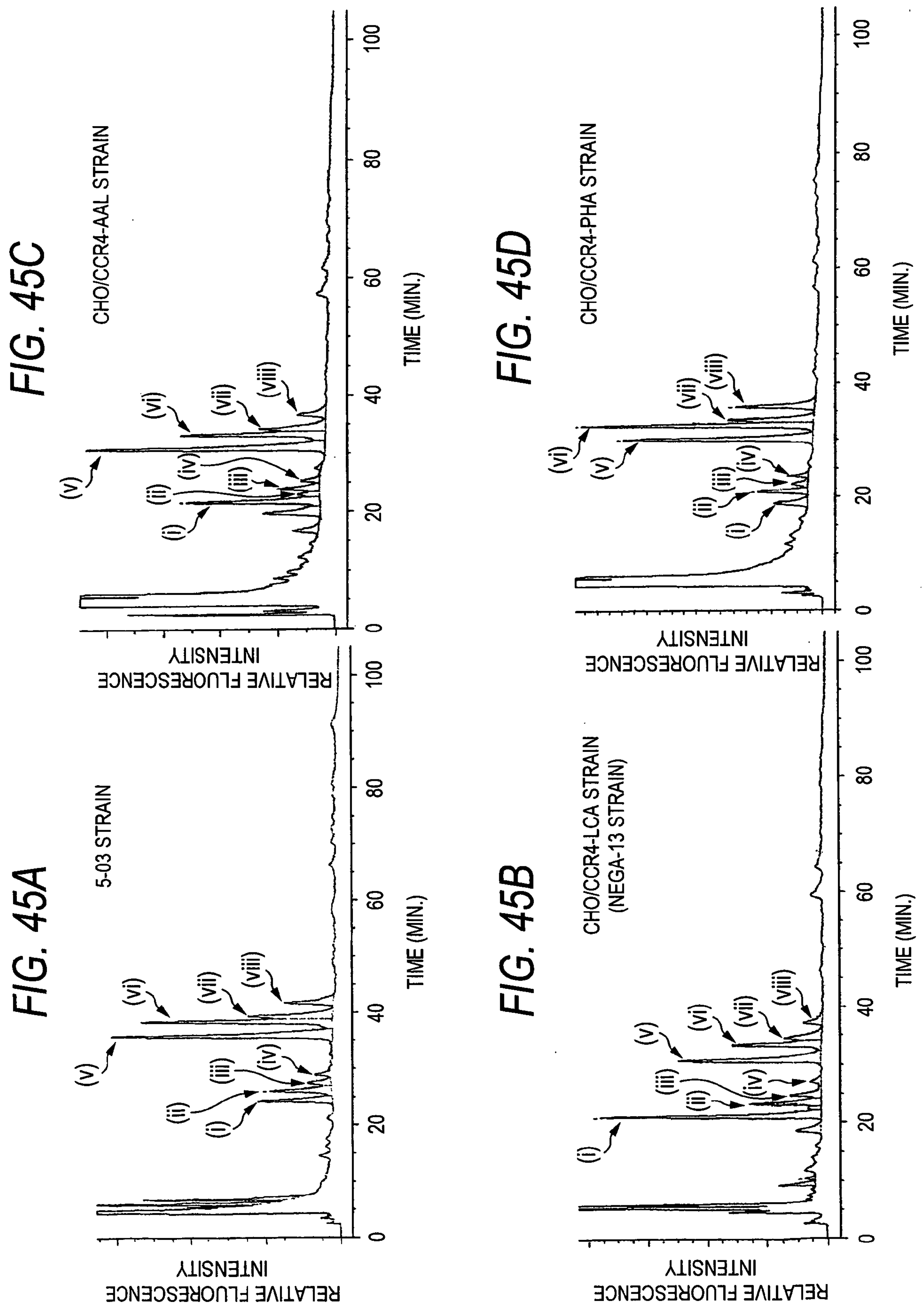


FIG. 45A

FIG. 45B

FIG. 45C

FIG. 45D

*FIG. 46*

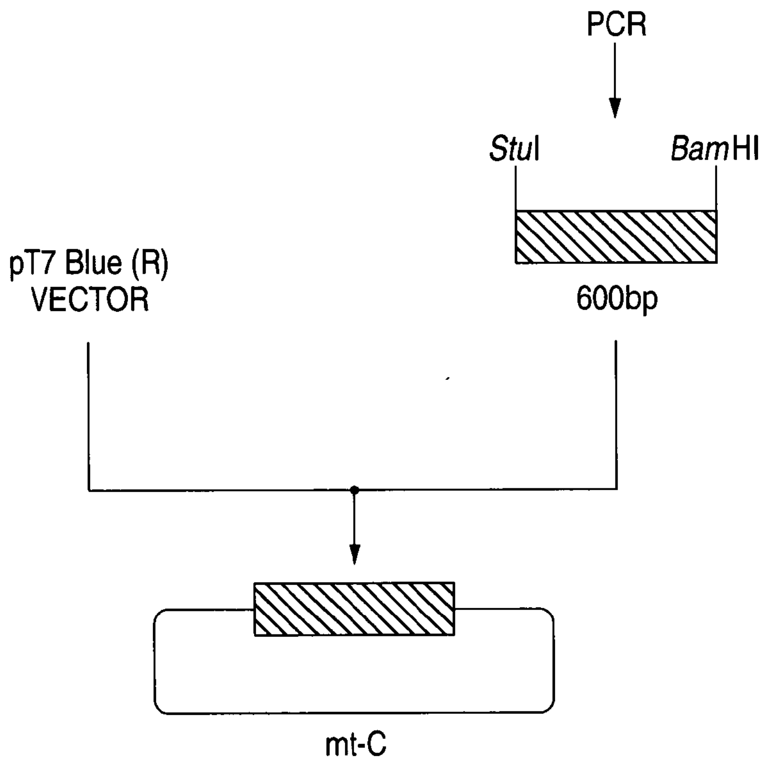

FIG. 47

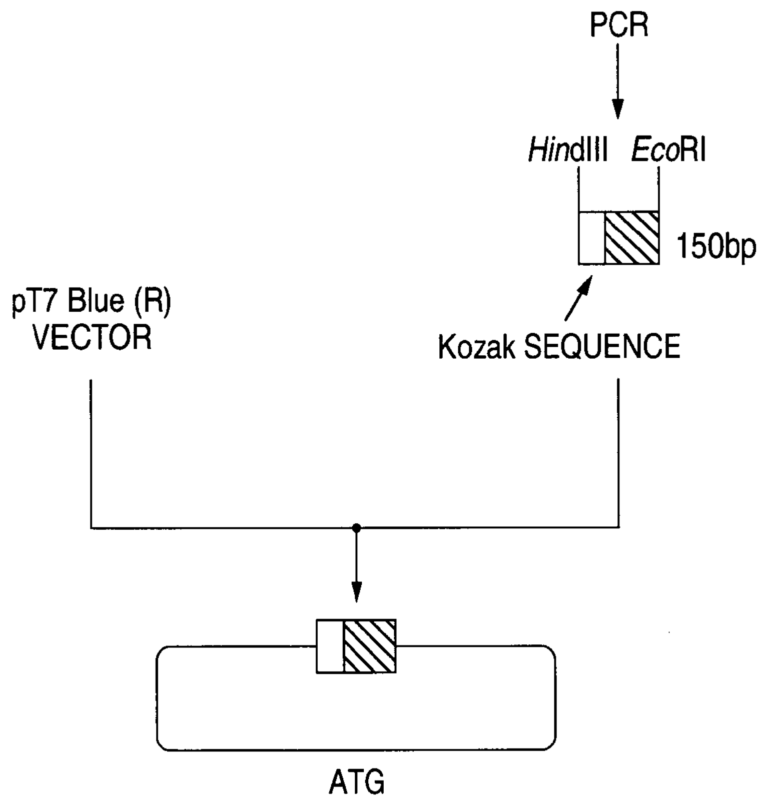
PCR
HindIII EcoRI
150bp
pT7 Blue (R) VECTOR
Kozak SEQUENCE
ATG

FIG. 48

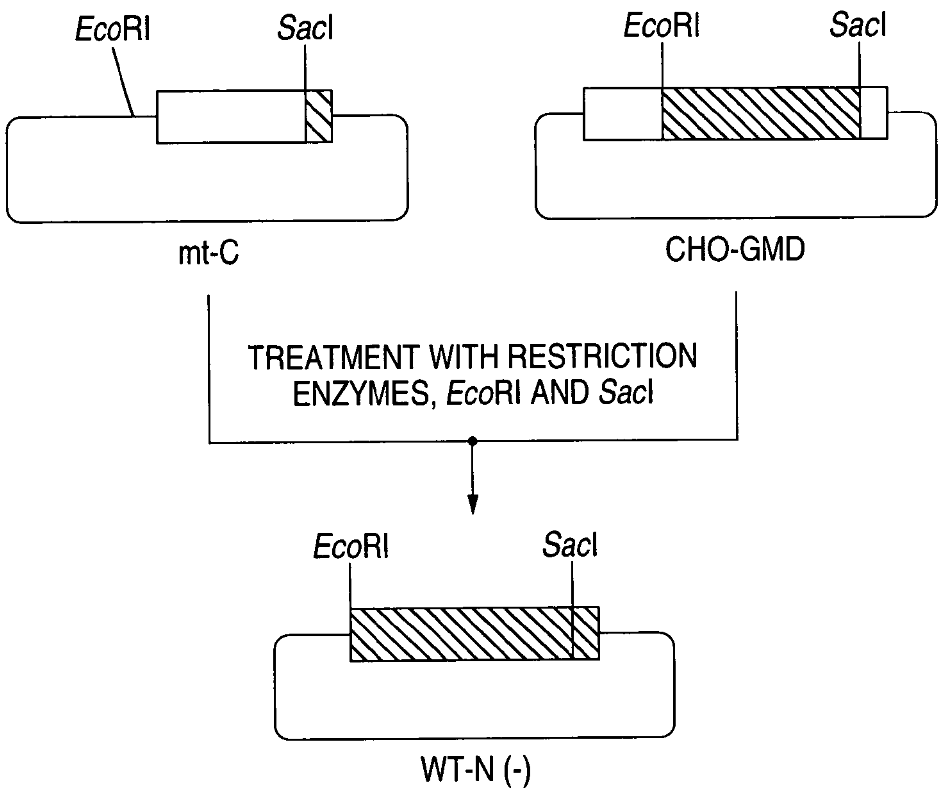
EcoRI
SacI
mt-C
EcoRI
SacI
CHO-GMD
TREATMENT WITH RESTRICTION ENZYMES, EcoRI AND SacI
EcoRI
SacI
WT-N (-)

FIG. 49

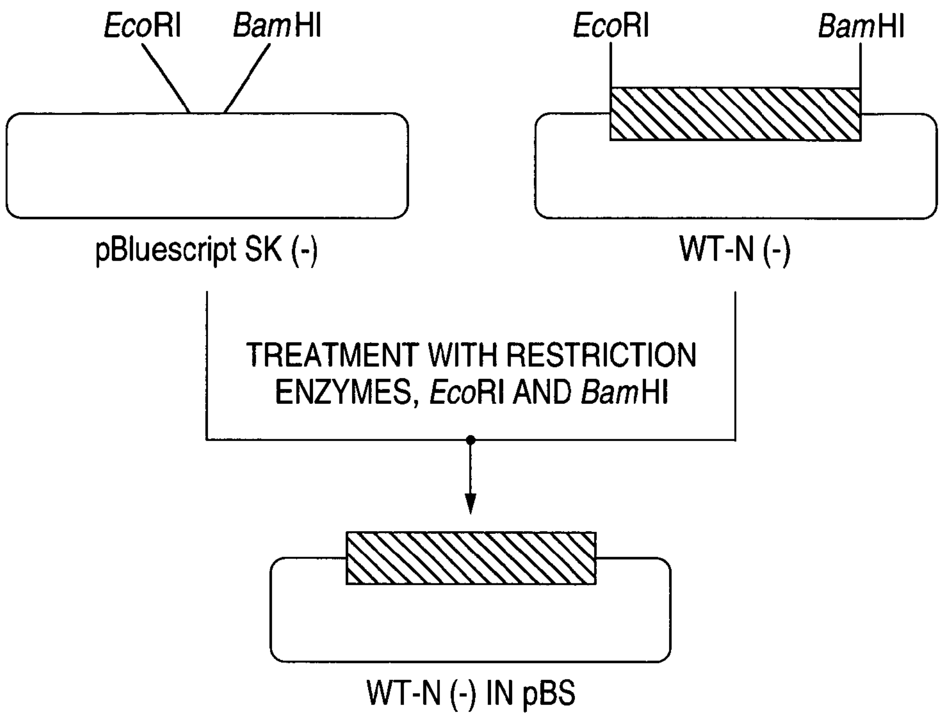
EcoRI
BamHI
pBluescript SK (-)
EcoRI
BamHI
WT-N (-)
TREATMENT WITH RESTRICTION ENZYMES, EcoRI AND BamHI
WT-N (-) IN pBS

FIG. 50

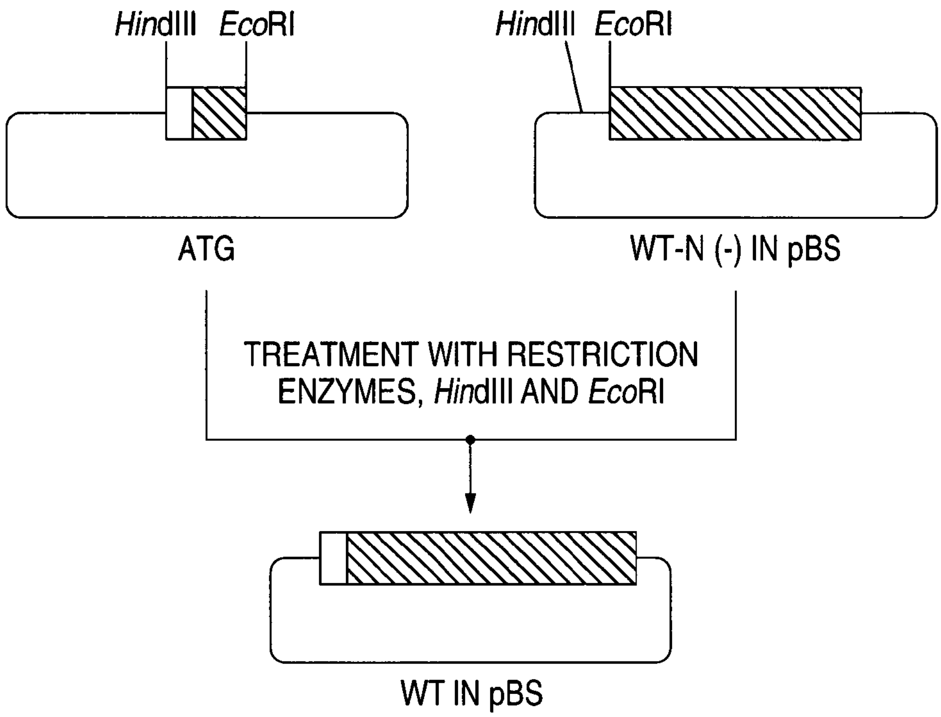
*Hind*III *Eco*RI
*Hind*III *Eco*RI
ATG
WT-N (-) IN pBS
TREATMENT WITH RESTRICTION ENZYMES, *Hind*III AND *Eco*RI
WT IN pBS

FIG. 51

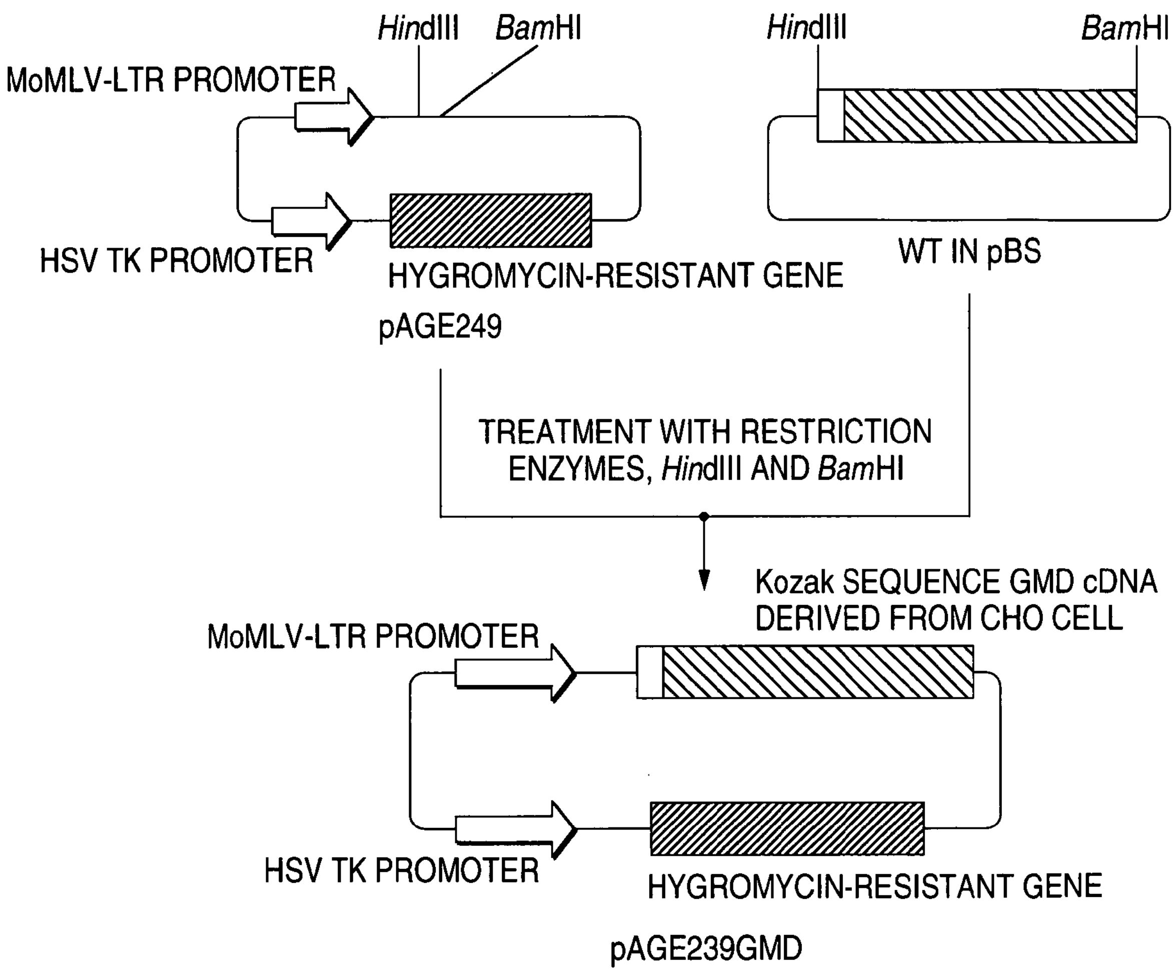
HindIII
BamHI
MoMLV-LTR PROMOTER
HSV TK PROMOTER
HYGROMYCIN-RESISTANT GENE
pAGE249
HindIII
BamHI
WT IN pBS
TREATMENT WITH RESTRICTION ENZYMES, HindIII AND BamHI
Kozak SEQUENCE GMD cDNA DERIVED FROM CHO CELL
MoMLV-LTR PROMOTER
HSV TK PROMOTER
HYGROMYCIN-RESISTANT GENE
pAGE239GMD

FIG. 52

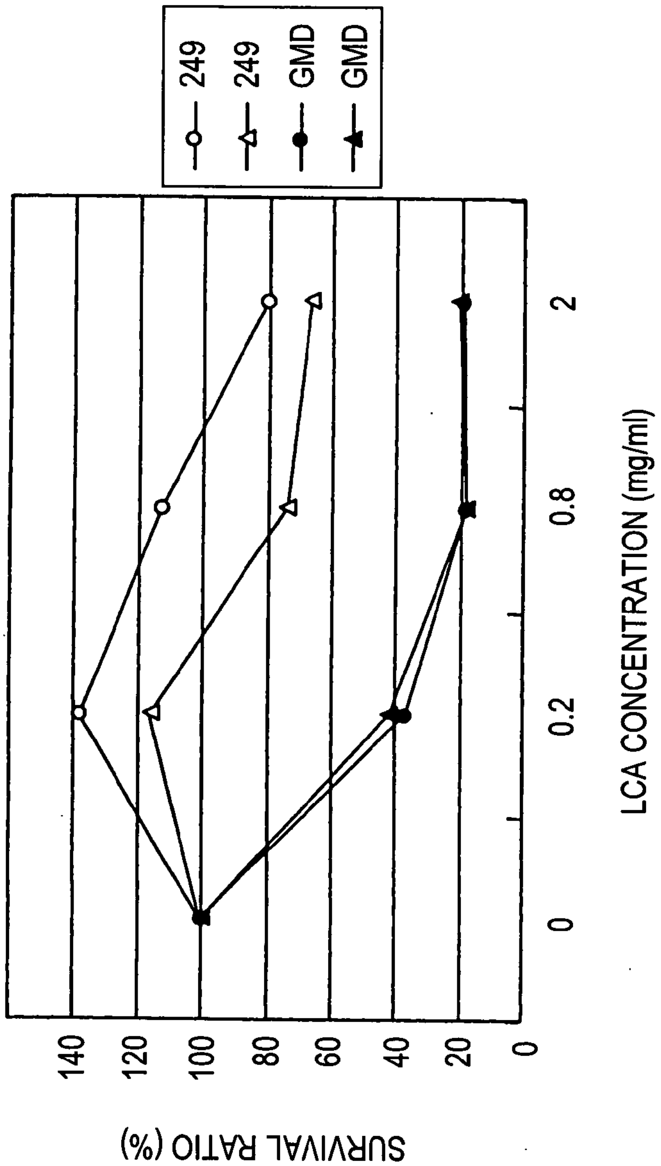

249
249
GMD
GMD
SURVIVAL RATIO (%)
140
120
100
80
60
40
20
0
0
0.2
0.8
2
LCA CONCENTRATION (mg/ml)

FIG. 53

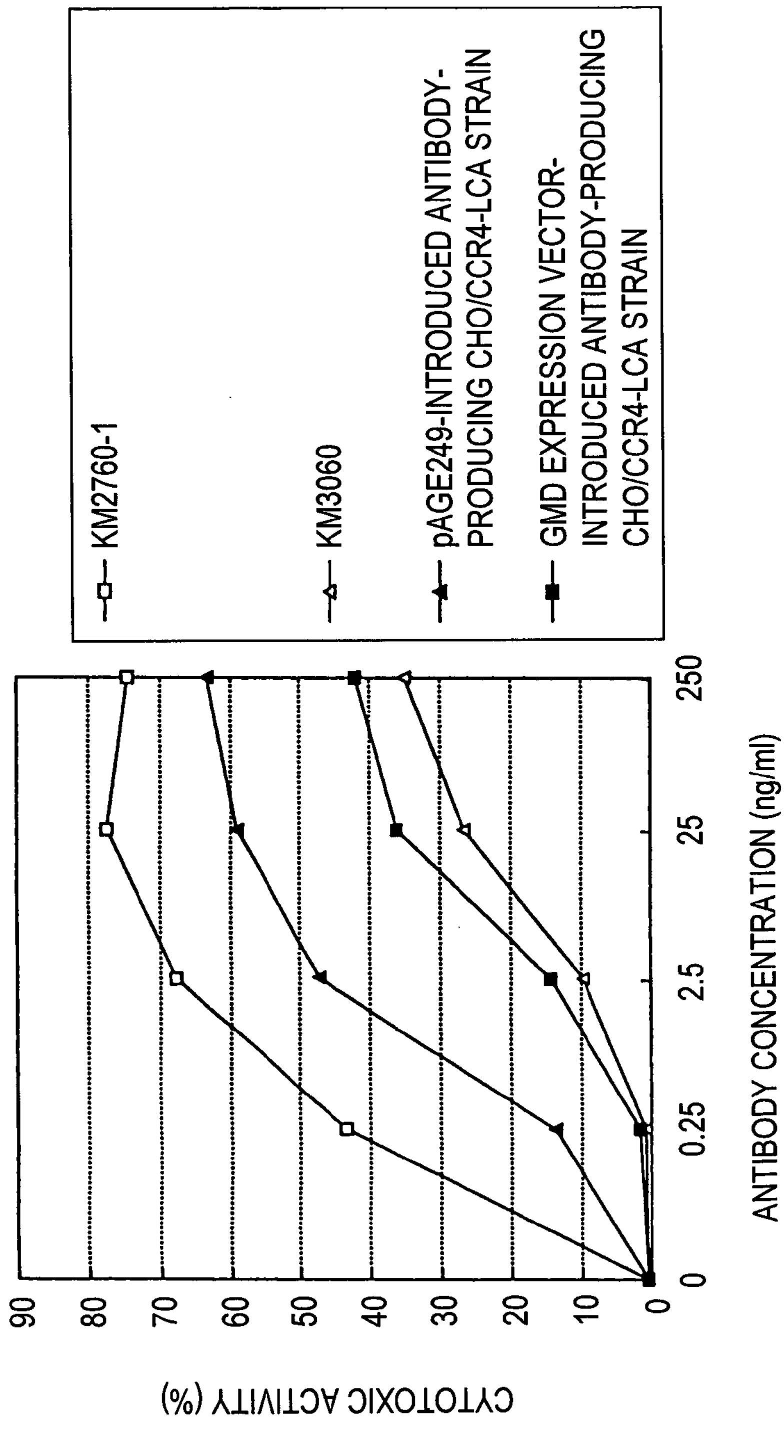
KM2760-1
KM3060
pAGE249-INTRODUCED ANTIBODY-PRODUCING CHO/CCR4-LCA STRAIN
GMD EXPRESSION VECTOR-INTRODUCED ANTIBODY-PRODUCING CHO/CCR4-LCA STRAIN
CYTOTOXIC ACTIVITY (%)
0
10
20
30
40
50
60
70
80
90
0
0.25
2.5
25
250
ANTIBODY CONCENTRATION (ng/ml)

FIG. 54

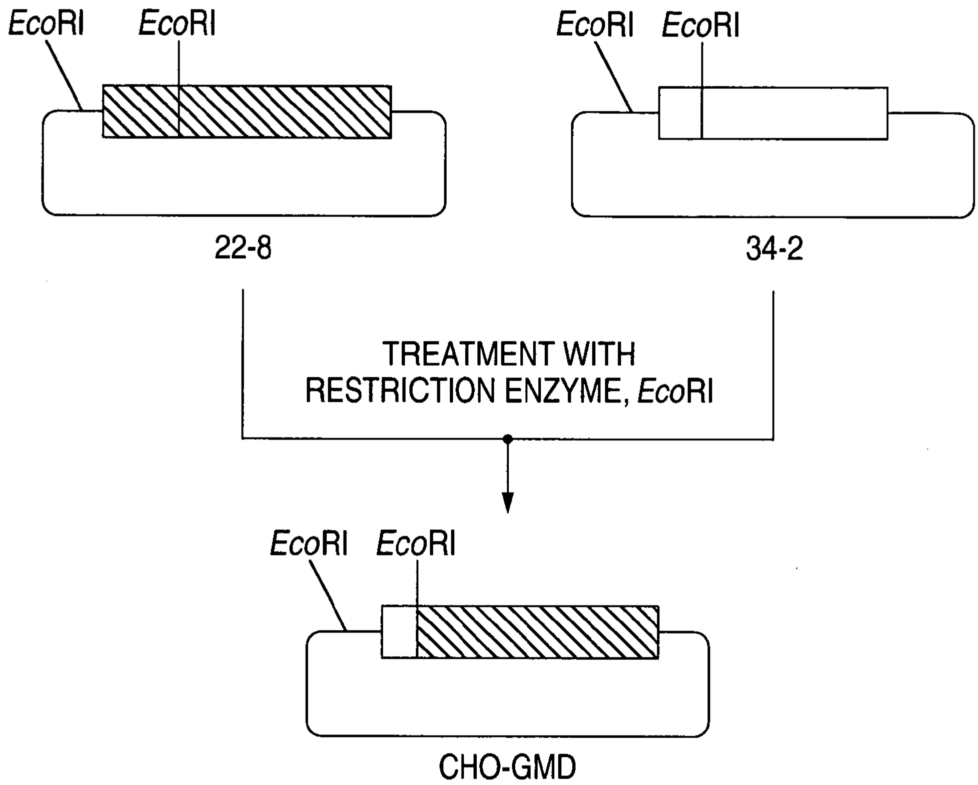
*Eco*RI
*Eco*RI
22-8
*Eco*RI
*Eco*RI
34-2
TREATMENT WITH
RESTRICTION ENZYME, *Eco*RI
*Eco*RI
*Eco*RI
CHO-GMD

FIG. 55

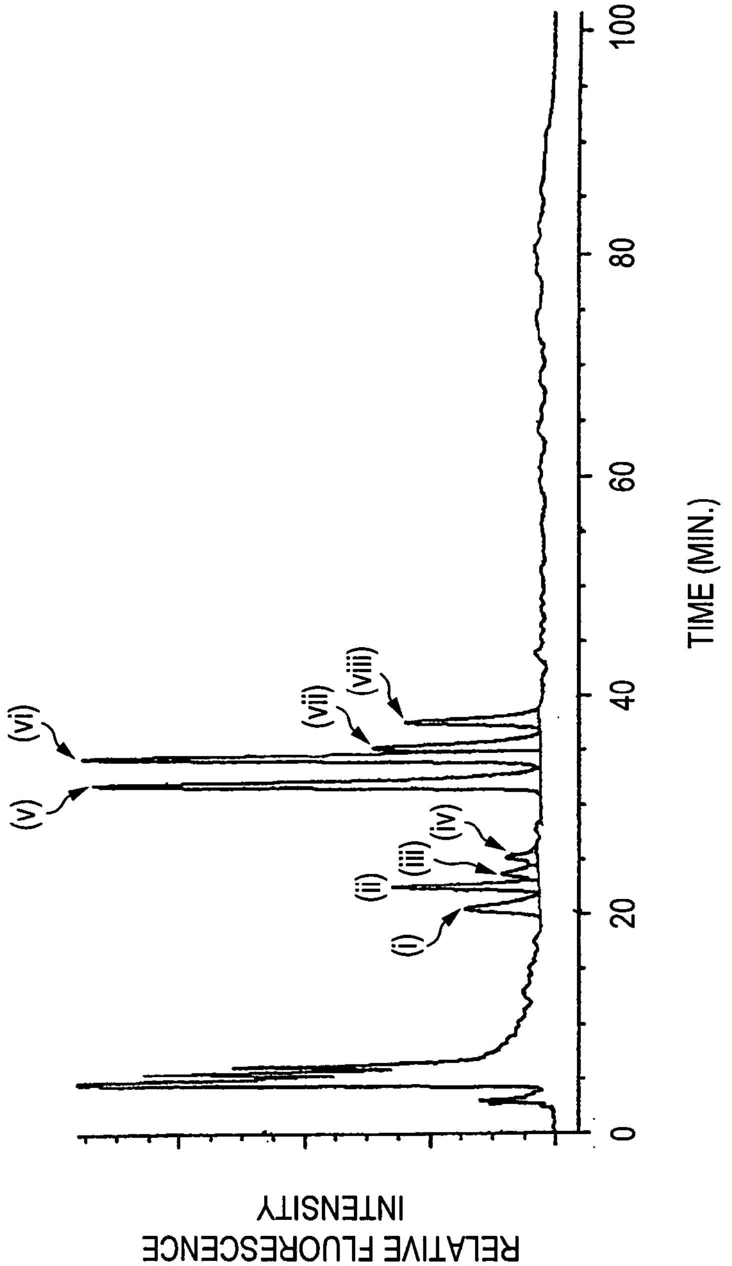
RELATIVE FLUORESCENCE INTENSITY
TIME (MIN.)
0
20
40
60
80
100
(i)
(ii)
(iii)
(iv)
(v)
(vi)
(vii)
(viii)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9954342 A
- JP 3022979 A
- JP 2227075 A
- JP 63000299 A
- JP 2606856 B
- JP 2517813 B
- JP 59140885 A
- JP 60070080 A
- WO 9400977 A
- JP 60251887 A
- JP 5336963 A
- JP 6823021 A
- JP 63309192 A
- JP 2257891 A
- JP 5304989 A
- EP 533199 A
- WO 9710354 A
- WO 0164754 A
- JP 11314512 A
- JP 2000308526 A


### Non-patent literature cited in the description


- Monoclonal Antibodies: Principles and Applications. Wiley-Liss, Inc, 1995
- *Chemical Immunology,* 1997, vol. 65, 88
- *Eur. J. Immunol.,* 1993, vol. 23, 1098
- *Immunology,* 1995, vol. 86, 319
- *Molecular Immunol.,* 1995, vol. 32, 1311
- *Glycobiology,* 1995, vol. 5, 813
- Biochemical Experimentation Method 23 - Method for Studying Glycoprotein Sugar Chain. Gakujutsu Shuppan Center, 1989
- *Biochemistry,* 1997, vol. 36, 130
- Glycobiology Series 2 - Destiny of Sugar Chain in Cell. Kodan-sha Scientific, 1993
- *Molecular Immunol.,* 1989, vol. 26, 1113
- *Somatic Cell Mol. Genet.,* 1986, vol. 12, 51
- *J. Biol. Chem.,* 1989, vol. 261, 13848
- *Science,* 1991, vol. 252, 1668
- *J. Biol. Chem.,* 1984, vol. 261, 13370
- *J. Biol Chem.,* 1984, vol. 261, 13370
- Biochemical Experimentation Methods 23 - Method for Studying Glycoprotein Sugar Chain. Japan Scientific Societies Press, 1989
- *J. Liq. Chromatogr.,* 1983, vol. 6, 1557
- *Journal of Experimental Medicine,* 1958, vol. 108, 945
- *Proc. Natl. Acad Sci. USA,* 1968, vol. 60, 1275
- *Genetics,* 1968, vol. 55, 513
- *Chromosoma,* 1973, vol. 41, 129
- *Methods in Cell Science,* 1996, vol. 18, 115
- *Radiation Research,* 1997, vol. 148, 260
- *Proc. Natl. Acad Sci. USA,* 1980, vol. 77, 4216
- *Proc. Natl. Acad Sci.,* 1968, vol. 60, 1275
- *Cell,* 1975, vol. 6, 121
- *Molecular Cell Genetics,* 883-900
- A Laboratory Manual. Molecular Cloning. Cold Spring Harbor Laboratory Press, 1989
- Molecular Cloning, Second Edition. Current Protocols in Molecular Biology. John Wiley & Sons, 1987
- Current Protocols in Molecular Biology. DNA Cloning 1: Core Techniques, A Practical Approach. Oxford University, 1995
- Current Protocols in Molecular Biology; Nucleic Acids Research. Molecular Cloning. 1982, vol. 10, 6487
- *Proc. Natl. Acad. Sci. USA,* 1982, vol. 79, 6409
- *Gene,* 1985, vol. 34, 315
- *Nucleic Acids Research,* 1985, vol. 13, 4431
- *Proc. Natl. Acad. Sci. USA,* 1985, vol. 82, 488
- *J. Mol. Biol.,* 1990, vol. 215, 403
- *Methods in Enzymology,* 1990, vol. 183, 63
- Monoclonal Antibodies: Principles and Applications. Wiley-Liss, Inc, 1955
- *Cell Technology,* 1993, vol. 12, 239
- *BIO/TECHNOLOGY,* 1999, vol. 17, 1097
- 5. *Hum. Mol. Genet.,* 1995, 1083
- *Cell Technology,* 1994, vol. 13, 255
- *Proc. Natl. Acad Sci. USA,* 1999, vol. 96, 1886
- New Biochemical Experimentation Series 3 - Saccharides I, Glycoprotein. Tokyo Kagaku Dojin, 1988
- Cell Engineering, Supplement, Experimental Protocol Series, Glycobiology Experimental Protocol, Glycoprotein, Glycolipid and Proteoglycan. Shujun-sha, 1996

- Current Protocols in Molecular Biology. Molecular Cloning
- *Methods in Enrymology,* 1987, vol. 154, 3
- *Analytical Biochemistry,* 1987, vol. 162, 156
- *Experimental Medicine,* 1991, vol. 9, 1937
- A Laboratory Manual. 1989
- *Strategies,* 1992, vol. 5, 58
- *Nucleic Acids Research,* 1989, vol. 17, 9494
- *DNA Cloning, A Practical Approach,* 1985, vol. 1, 49
- *Mol. Cell. Biol.,* 1983, vol. 3, 280
- *Gene,* 1985, vol. 33, 103
- *Strategies,* 1992, vol. 5, 81
- *Genetics,* 1954, vol. 39, 440
- *Science,* 1983, vol. 222, 778
- *J. Mol. Biol.,* 1983, vol. 166, 1
- *J. Mol. Biol.,* 1966, vol. 16, 118
- *Gene,* 1985, vol. 38, 275
- **SUGANO et al.** *Gene,* 1994, vol. 138, 171
- *Gene,* 1997, vol. 200, 149
- *Protein, Nucleic Acid and Protein,* 1996, vol. 41, 603
- *Experimental Medicine,* 1993, vol. 11, 2491
- cDNA Cloning. Yodo-sha, 1996
- Methods for Preparing Gene Libraries. Yodo-sha, 1994
- PCR Protocols. Academic Press, 1990
- **SANGER et al.** *Proc. Natl. Acad. Sci. USA,* 1977, vol. 74, 5463
- Molecular Cloning
- *Cell Technology,* 1997, vol. 16, 1463
- Manipulating the Mouse Embryo. A Laboratory Manual. Cold Spring Harbor Laboratory Press, 1994
- Gene Targeting, A Practical Approach. IRL Press at Oxford University Press, 1993
- Biomanual Series 8, Gene Targeting, Preparation of Mutant Mice using ES Cells. Yodo-sha, 1995
- Biomanual Series 8, Gene Targeting, Preparation of-Mutant Mice using ES Cells. Yodo-sha, 1995
- Molecular Cloning,
- *Proc. Natl. Acad. Sci. USA,* 1977, vol. 74, 5463
- *Science,* 1996, vol. 273, 1386
- *Nature Medicine,* 1998, vol. 4, 285
- *Hepatology,* 1997, vol. 25, 1462
- *Gene Therapy,* 1999, vol. 5, 1960
- *J Mol. Med.,* 1997, vol. 75, 829
- *Proc. Natl. Acad. Sci. USA,* 1999, vol. 96, 8774
- *Proc. Natl. Acad. Sci. USA,* 1999, vol. 96, 8768
- *Nuc. Acids. Res.,* 1999, vol. 27, 1323
- *Invest. Dematol.,* 1998, vol. 111, 1172
- *Nature Biotech.,* 1998, vol. 16, 1343
- *Nature Biotech.,* 2000, vol. 18, 43
- *Nature Biotech.,* 2000, vol. 18, 555
- *Nature,* 1998, vol. 391, 806
- *Proc. Natl. Acad. Sci. USA,* 1998, vol. 95, 15502
- *Nature,* 1998, vol. 395, 854
- *Proc. Natl. Acad. Sci. USA,* 1999, vol. 96, 5049
- *Cell,* 1998, vol. 95, 1017
- *Proc. Natl. Acad. Sci. USA,* 1999, vol. 96, 1451
- *Proc. Natl. Acad. Sci. USA,* 1998, vol. 95, 13959
- *Nature Cell Biol.,* 2000, vol. 2, 70
- *Nature Genet.,* 2000, vol. 25, 35
- *Structure,* 2000, vol. 8, 2
- Tissue Culture Techniques. Asakura Shoten, 1996
- *Nature Genet.,* 2000, vol. 24, 314
- *Bioscience and Industry,* 1992, vol. 50, 322
- *Chemistry,* 1991, vol. 46, 681
- *Biotechnology,* 1992, vol. 9, 358
- *Trends in Biotechnology,* 1992, vol. 10, 87
- *Trends in Biotechnology,* 1992, vol. 10, 152
- *Cell Engineering,* 1997, vol. 16, 1463
- *Trends in Biotechnology,* 1992, vol. 10, 132
- *Nature,* 1987, vol. 326 (6110), 295
- *Cell,* 1987, vol. 51 (3), 503
- Manipulating Mouse Embryo
- *Tissue Culture,* 1994, vol. 20
- *Tissue Culture,* 1995, vol. 21
- *Trends in Biotechnology,* 1997, vol. 15, 45
- Antibodies. A Laboratory Manual. Cold Spring Harbor Laboratory, 1988
- Monoclonal Antibodies. Principles and Practice. Acad. Press, 1993
- Antibody Engineering. A Practical Approach. IRL Press at Oxford University Press
- *Methods in Enzymology,* 1990, vol. 194, 182
- *Proc. Natl. Acad. Sci. USA,* 1978, vol. 84, 1929
- *J. Bacteriol.,* 1983, vol. 153, 163
- *Proc. Natl. Acad. Sci. USA,* 1978, vol. 75, 1929
- *Cytotechnology,* 1990, vol. 3, 133
- *Nature,* 1987, vol. 329, 840
- *J. Biochemistry,* 1987, vol. 101, 1307
- *Proc. Natl. Acad. Sci. USA,* 1987, vol. 84, 7413
- Manipulating the Mouse Embryo. A Laboratory Manual
- **YODO-SHA.** Biomanual Series 4-Gene Transfer and Expression Analysis. 1994
- Current Protocols in Molecular Biology, Baculovirus Expression Vectors. A Laboratory Manual. W.H. Freeman and Company, 1992
- *Bio/Technology,* 1988, vol. 6, 47
- *The Journal of the American Medical Association,* 1967, vol. 199, 519
- *Science,* 1952, vol. 122, 501

- *Virology,* 1959, vol. 8, 396
- *Proceeding of the Society for the Biological Medicine,* 1950, vol. 73, 1
- Developmental Engineering Experimentation Manual-Preparation of Transgenic Mice. Kodan-sha, 1987
- *Nature,* 1962, vol. 195, 788
- **PAULSON et al.** *J. Biol. Chem.,* 1989, vol. 264, 17619
- **LOWE et al.** *Proc. Natl. Acad. Sci. USA,* 1989, vol. 86, 8227
- *Genes Develop.,* 1990, vol. 4, 1288
- *American Journal of Clinical Nutrition,* 1996, vol. 63, 627S
- *Bio/Technology,* 1991, vol. 9, 830
- *J. Biochem.,* 1987, vol. 101, 1307
- *Gene,* 1984, vol. 27, 223
- *Proc. Natl. Acad. Sci. USA,* 1981, vol. 78, 1527
- *Cytotechnology,* 1990, vol. 4, 173
- *Biochem. Biophys. Res. Commun.,* 1987, vol. 149, 960
- *Cell,* 1985, vol. 41, 479
- *Cell,* 1983, vol. 33, 717
- *J. Immunol. Methods,* 1994, vol. 167, 271
- *Methods in Enzymology,* 1987, vol. 154, 3
- Current Protocols in Molecular Biology. Molecular Cloning. vol. 1-34
- *DNA Cloning, A Practical Approach,* 1985, vol. I, 49
- Sequences of Proteins of Immunological Interest. US Dep. Health and Human Services, 1991
- Antibodies: A Laboratory Manual. Cold Spring Harbor Laboratory, 1998
- Monoclonal Antibodies: Principles and Practice. Academic Press Limited, 1996
- Antibodies, A Laboratory Manual. Cold Spring Harbor Laboratory, 1988
- *Nature,* 1970, vol. 227, 680
- *Cancer Immunol. Immunother.,* 1993, vol. 36, 373
- *J. Liq. Chromatogr.,* 1983, vol. 6, 1577
- *Agric. Biol. Chem.,* 1991, vol. 55 (1), 283-284
- *Anal. Biochem.,* 1988, vol. 171, 73
- Biochemical Experimentation Methods 23 - Methods for Studying Glycoprotein Sugar Chains. Japan Scientific Societies Press, 1989
- *J. Biochem.,* 1984, vol. 95, 197
- Immunoassay. 1987
- Enzyme Antibody Method. Gakusai Kikaku, 1985
- *Science,* 1998, vol. 280, 1197
- **OHTA et al.** *Anticancer Res.,* 1993, vol. 13, 331-336
- **OHTA et al.** *Cancer Immunol. Immunother.,* 1993, vol. 36, 260-266
- **NAKAMURA et al.** *Cancer Res.,* 1994, vol. 54, 1511-1516
- **CARTER et al.** *Proc. Natl. Acad. Sci. USA,* 1992, vol. 89, 4285-4289
- **JUNGBLUTH et al.** *British J. Cancer,* 2000, vol. 83, 493-497
- **ONO et al.** *Molecular Immunol.,* 1999, vol. 36, 387-395
- **OGATA et al.** *Cancer,* 2000, vol. 88, 2909-2911
- **MATSUZAKI et al.** *Proc. Natl. Acad. Sci. USA,* 1989, vol. 86, 9911-9915
- **KUO et al.** *J. Biol. Chem.,* 1990, vol. 265, 16455-16463
- **YAO et al.** *J. Neurosci. Res.,* 1995, vol. 40, 647-659
- **MURPHY et al.** *J. Urology,* 1998, vol. 160, 2396-2401
- **PRESTA et al.** *Cancer Res.,* 1997, vol. 57, 4593-4599
- **KANNO et al.** *Oncogene,* 2000, vol. 19, 2138-2146
- **ABRAMS et al.** *Immunol. Rev.,* 1992, vol. 127, 5-24
- **SATO et al.** *Molecular Immunol.,* 1994, vol. 31, 371-381
- **BIORD et al.** *Cytokine,* 1991, vol. 3, 562-567
- **TEMPEST et al.** *Hybridoma,* 1994, vol. 13, 183-190
- **AMRANI et al.** *Molecular Pharmacol.,* 2000, vol. 58, 237-245
- **CAMPBELL et al.** *Nature,* 1999, vol. 400, 776-780
- **PERI et al.** *J. Immuno. Meth.,* 1994, vol. 174, 249-257
- **WU et al.** *J. Exp. Med.,* 1997, vol. 186, 1373-1381
- **CO et al.** *J. Immunol.,* 1994, vol. 152, 2968-2976
- **FERNS et al.** *Science,* 1991, vol. 253, 1129-1132
- **SHULMAN et al.** *J. Biol. Chem.,* 1997, vol. 272, 17400-17404
- **PETER et al.** *Circulation,* 2000, vol. 101, 1158-1164
- **TUGARINOV et al.** *Structure,* 2000, vol. 8, 385-395
- **SCHULZE-KOOPS et al.** *J. Rheumatology,* 1998, vol. 25, 2065-2076
- **KARNALI et al.** *J. Clin. Microbiol.,* 1999, vol. 37, 396-399
- *Cancer Immunology Immunotherapy,* 1993, vol. 36, 373
- *Cancer Research,* 1994, vol. 54, 1511
- **J.V. KILMARIN et al.** *J. Cell. Biol.,* 1982, vol. 93, 576-582

- **G. URLAUB ; L.A. CHASIN.** *Proc. Natl. Acad. Sci. USA,* 1980, vol. 77, 4216-4220
- **YARRANTON et al.** *BIO/TECHNOLOGY,* 1992, vol. 10, 169
- *Method of Enzymology,* 1982, vol. 83, 263
- Antibodies: A Laboratory Manual. Cold Spring Harbor Laboratory, 1988
- *J. Biochem.,* 1997, vol. 121, 626
- *J. Biol. Chem.,* 1995, vol. 271, 27810
- **COHEN et al.** *Proc. Natl. Acad. Sci. USA,* 1972, vol. 69, 2110
- *Nucleic Acids Research,* 1979, vol. 7, 1513
- *Nucleic Acids Research,* 1983, vol. 11, 1759
- *J. Biol. Chem.,* 1994, vol. 269, 14730
- *Anal. Biochem.,* 1985, vol. 150, 76
- Current Protocols in Molecular Biology, A Laboratory Manual. 1989
- *Analytical Biochemistry,* 1992, vol. 201, 331
- *Nucleic Acids Research,* 1976, vol. 3, 2303
- *Proc. Natl. Acad. Sci. USA,* 1979, vol. 76, 3683
- *Advances in Protein Chemistry,* 1962, vol. 12, 303